# EUROPEAN PATENT SPECIFICATION

(11) **EP 2 583 974 B1**
(45) Date of publication and mention of the grant of the patent: **26.04.2017**
(21) Application number: 11186173.8
(22) Date of filing: 21.10.2011
(51) Int. Cl.: C07K 14/15, C12N 7/02, C12N 15/867

(54) **Pseudotyping of foamy viruses**
Pseudotypisierung von schaumigen Viren
Pseudotypage de virus mousseux

(43) Date of publication of application: 24.04.2013
(73) Proprietor: Technische Universität Dresden, 01069 Dresden (DE)
(72) Inventor: Lindemann, Dirk, Prof. Dr., 01257 Dresden (DE); Ho, Yu-Ping, 11445 Taipei (TW); Schnabel, Viktor, 01277 Dresden (DE)
(74) Representative: Kailuweit & Uhlemann Patentanwälte Partnerschaft mbB

(56) References cited:
- SU RUIJUN ET AL: "Transduction of primate cells with feline foamy virus envelope pseudotyped prototype foamy virus vectors", 46TH ANNUAL MEETING OF THE AMERICAN-SOCIETY-OF-HEMATOLOGY; SAN DIEGO, CA, USA; DECEMBER 04 -07, 2004 BLOOD, vol. 104, no. 11, Part. 2, ABS.5276, November 2004 (2004-11), XP002667733, Retrieved from the Internet: URL:http://abstracts.hematologylibrary.org /cgi/content/abstract/104/11/5276?maxtosho w=&hits=10&RESULTFORMAT=&fulltext=Transduc tion+of+primate+cells+with+feline+foamy+vi rus+envelope+pseudotyped+prototype+foamy+v irus+vec&searchid=1&FIRSTINDEX=0&volume=10 4&issue=11&resourcetype=HWCIT [retrieved on 2012-01-20]
- ZHADINA MARIA ET AL: "Ubiquitin-dependent virus particle budding without viral protein ubiquitination.", PROCEEDINGS OF THE NATIONAL ACADEMY OF SCIENCES OF THE UNITED STATES OF AMERICA, vol. 104, no. 50, 11 December 2007 (2007-12-11), pages 20031-20036, XP002667734, ISSN: 1091-6490
- LINDEMANN DIRK ET AL: "Foamy virus biology and its application for vector development.", VIRUSES MAY 2011 LNKD- PUBMED:21994746, vol. 3, no. 5, May 2011 (2011-05), pages 561-585, XP002667735, ISSN: 1999-4915
- WU MIN ET AL: "Packaging cell lines for simian foamy virus type 1 vectors", JOURNAL OF VIROLOGY, vol. 73, no. 5, May 1999 (1999-05), pages 4498-4501, XP002667736, ISSN: 0022-538X
- CORSON TIMOTHY W ET AL: "Design and Applications of Bifunctional Small Molecules: Why Two Heads Are Better Than One.", ACS CHEMICAL BIOLOGY, vol. 3, no. 11, 21 November 2008 (2008-11-21), pages 677-692, XP002667737, ISSN: 1554-8937
- AMARA J F ET AL: "A versatile synthetic dimerizer for the regulation of protein-protein interactions", PROCEEDINGS OF THE NATIONAL ACADEMY OF SCIENCES OF USA, vol. 94, no. 20, 30 September 1997 (1997-09-30), pages 10618-10623, XP002178325, NATIONAL ACADEMY OF SCIENCE, WASHINGTON, DC; US ISSN: 0027-8424, DOI: 10.1073/PNAS.94.20.10618

### SEQUENCE LISTING

<110> Technische Universität Dresden
<120> Pseudotyping of foamy viruses
<130> 00017P0185EP
<160> 167
<170> PatentIn version 3.5
<210> 1
   <211> 107
   <212> PRT
   <213> Artificial Sequence
<220>
   <223> rapamycin-binding heterodimerizable protein domain
<400> 1
   Gly Val Gln Val Glu Thr Ile Ser Pro Gly Asp Gly Arg Thr Phe Pro
   1               5                   10                  15
   Lys Arg Gly Gln Thr Cys Val Val His Tyr Thr Gly Met Leu Glu Asp
               20                  25                  30
   Gly Lys Lys Phe Asp Ser Ser Arg Asp Arg Asn Lys Pro Phe Lys Phe
           35                  40                  45
   Met Leu Gly Lys Gln Glu Val Ile Arg Gly Trp Glu Glu Gly Val Ala
       50                  55                  60
   Gln Met Ser Val Gly Gln Arg Ala Lys Leu Thr Ile Ser Pro Asp Tyr
   65                  70                  75                  80
   Ala Tyr Gly Ala Thr Gly His Pro Gly Ile Ile Pro Pro His Ala Thr
                   85                  90                  95
   Leu Val Phe Asp Val Glu Leu Leu Lys Leu Glu
               100                 105
<210> 2
   <211> 321
   <212> DNA
   <213> Artificial Sequence
<220>
   <223> rapamycin-binding heterodimerizable protein domain
<400> 2
   ggagtgcagg tggaaaccat ctccccagga gacgggcgca ccttccccaa gcgcggccag      60
   acctgcgtgg tgcactacac cgggatgctt gaagatggaa agaaatttga ttcctcccgg     120
   gacagaaaca agccctttaa gtttatgcta ggcaagcagg aggtgatccg aggctgggaa     180
   gaaggggttg cccagatgag tgtgggtcag agagccaaac tgactatatc tccagattat     240
   gcctatggtg ccactgggca cccaggcatc atcccaccac atgccactct cgtcttcgat     300
   gtggagcttc taaaactgga a                                               321
<210> 3
   <211> 93
   <212> PRT
   <213> Artificial Sequence
<220>
   <223> rapamycin-binding heterodimerizable protein domain
<400> 3
   Ile Leu Trp His Glu Met Trp His Glu Gly Leu Glu Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Leu Gln Ala
   65                  70                  75                  80
   Trp Asp Leu Tyr Tyr His Val Phe Arg Arg Ile Ser Lys
                   85                  90
<210> 4
   <211> 279
   <212> DNA
   <213> Artificial Sequence
<220>
   <223> rapamycin-binding heterodimerizable protein domain
<400> 4
   atcctctggc atgagatgtg gcatgaaggc ctggaagagg catctcgttt gtactttggg      60
   gaaaggaacg tgaaaggcat gtttgaggtg ctggagccct tgcatgctat gatggaacgg     120
   ggcccccaga ctctgaagga aacatccttt aatcaggcct atggtcgaga tttaatggag     180
   gcccaagagt ggtgcaggaa gtacatgaaa tcagggaatg tcaaggacct cctccaagcc     240
   tgggacctct attatcatgt gttccgacga atctcaaag                            279
<210> 5
   <211> 648
   <212> PRT
   <213> Human foamy virus
<400> 5
   Met Ala Ser Gly Ser Asn Val Glu Glu Tyr Glu Leu Asp Val Glu Ala
   1               5                   10                  15
   Leu Val Val Ile Leu Arg Asp Arg Asn Ile Pro Arg Asn Pro Leu His
               20                  25                  30
   Gly Glu Val Ile Gly Leu Arg Leu Thr Glu Gly Trp Trp Gly Gln Ile
           35                  40                  45
   Glu Arg Phe Gln Met Val Arg Leu Ile Leu Gln Asn Asp Asp Asn Glu
       50                  55                  60
   Pro Leu Gln Arg Pro Arg Tyr Glu Val Ile Gln Arg Ala Val Asn Pro
   65                  70                  75                  80
   His Thr Met Phe Met Ile Ser Gly Pro Leu Ala Glu Leu Gln Leu Ala
                   85                  90                  95
   Phe Gln Asp Leu Asp Leu Pro Glu Gly Pro Leu Arg Phe Gly Pro Leu
               100                 105                 110
   Ala Asn Gly His Tyr Val Gln Gly Asp Pro Tyr Ser Ser Ser Tyr Arg
           115                 120                 125
   Pro Val Thr Met Ala Glu Thr Ala Gln Met Thr Arg Asp Glu Leu Glu
       130                 135                 140
   Asp Val Leu Asn Thr Gln Ser Glu Ile Glu Ile Gln Met Ile Asn Leu
   145                 150                 155                 160
   Leu Glu Leu Tyr Glu Val Glu Thr Arg Ala Leu Arg Arg Gln Leu Ala
                   165                 170                 175
   Glu Arg Ser Ser Thr Gly Gln Gly Gly Ile Ser Pro Gly Ala Pro Arg
               180                 185                 190
   Ser Arg Pro Pro Val Ser Ser Phe Ser Gly Leu Pro Ser Leu Pro Ser
           195                 200                 205
   Ile Pro Gly Ile His Pro Arg Ala Pro Ser Pro Pro Arg Ala Thr Ser
       210                 215                 220
   Thr Pro Gly Asn Ile Pro Trp Ser Leu Gly Asp Asp Asn Pro Pro Ser
   225                 230                 235                 240
   Ser Ser Phe Pro Gly Pro Ser Gln Pro Arg Val Ser Phe His Pro Gly
                   245                 250                 255
   Asn Pro Phe Val Glu Glu Glu Gly His Arg Pro Arg Ser Gln Ser Arg
               260                 265                 270
   Glu Arg Arg Arg Glu Ile Leu Pro Ala Pro Val Pro Ser Ala Pro Pro
           275                 280                 285
   Met Ile Gln Tyr Ile Pro Val Pro Pro Pro Pro Pro Ile Gly Thr Val
       290                 295                 300
   Ile Pro Ile Gln His Ile Arg Ser Val Thr Gly Glu Pro Pro Arg Asn
   305                 310                 315                 320
   Pro Arg Glu Ile Pro Ile Trp Leu Gly Arg Asn Ala Pro Ala Ile Asp
                   325                 330                 335
   Gly Val Phe Pro Val Thr Thr Pro Asp Leu Arg Cys Arg Ile Ile Asn
               340                 345                 350
   Ala Ile Leu Gly Gly Asn Ile Gly Leu Ser Leu Thr Pro Gly Asp Cys
           355                 360                 365
   Leu Thr Trp Asp Ser Ala Val Ala Thr Leu Phe Ile Arg Thr His Gly
       370                 375                 380
   Thr Phe Pro Met His Gln Leu Gly Asn Val Ile Lys Gly Ile Val Asp
   385                 390                 395                 400
   Gln Glu Gly Val Ala Thr Ala Tyr Thr Leu Gly Met Met Leu Ser Gly
                   405                 410                 415
   Gln Asn Tyr Gln Leu Val Ser Gly Ile Ile Arg Gly Tyr Leu Pro Gly
               420                 425                 430
   Gln Ala Val Val Thr Ala Leu Gln Gln Arg Leu Asp Gln Glu Ile Asp
           435                 440                 445
   Asp Gln Thr Arg Ala Glu Thr Phe Ile Gln His Leu Asn Ala Val Tyr
       450                 455                 460
   Glu Ile Leu Gly Leu Asn Ala Arg Gly Gln Ser Ile Arg Ala Ser Val
   465                 470                 475                 480
   Thr Pro Gln Pro Arg Pro Ser Arg Gly Arg Gly Arg Gly Gln Asn Thr
                   485                 490                 495
   Ser Arg Pro Ser Gln Gly Pro Ala Asn Ser Gly Arg Gly Arg Gln Arg
               500                 505                 510
   Pro Ala Ser Gly Gln Ser Asn Arg Gly Ser Ser Thr Gln Asn Gln Asn
           515                 520                 525
   Gln Asp Asn Leu Asn Gln Gly Gly Tyr Asn Leu Arg Pro Arg Thr Tyr
       530                 535                 540
   Gln Pro Gln Arg Tyr Gly Gly Gly Arg Gly Arg Arg Trp Asn Asp Asn
   545                 550                 555                 560
   Thr Asn Asn Gln Glu Ser Arg Pro Ser Asp Gln Gly Ser Gln Thr Pro
                   565                 570                 575
   Arg Pro Asn Gln Ala Gly Ser Gly Val Arg Gly Asn Gln Ser Gln Thr
               580                 585                 590
   Pro Arg Pro Ala Ala Gly Arg Gly Gly Arg Gly Asn His Asn Arg Asn
           595                 600                 605
   Gln Arg Ser Ser Gly Ala Gly Asp Ser Arg Ala Val Asn Thr Val Thr
       610                 615                 620
   Gln Ser Ala Thr Ser Ser Thr Ala Glu Ser Ser Ser Ala Val Thr Ala
   625                 630                 635                 640
   Ala Ser Gly Gly Asp Gln Arg Asp
                   645
<210> 6
   <211> 1947
   <212> DNA
   <213> Human foamy virus
<400> 6
   atggcttcag gaagtaatgt tgaagaatat gaacttgatg ttgaagctct ggttgtaatt        60
   ttaagagata gaaatatacc aagaaatcct ttacatggag aagttatagg tcttcgcctt       120
   actgaaggat ggtggggaca aattgagaga tttcagatgg tacgtctaat attacaaaat       180
   gatgataatg aacctttaca gagacctaga tatgaggtaa tacaacgagc tgtaaaccct       240
   catacaatgt ttatgatatc aggaccatta gctgaacttc aattagcctt tcaggattta       300
   gatttacctg aaggtccatt gaggtttggt ccattggcaa atggacatta tgttcaagga       360
   gatccttata gtagttctta cagaccagta acaatggccg aaacagccca aatgactaga       420
   gatgaactgg aagatgttct taatactcaa agtgaaatag aaattcaaat gataaattta       480
   ttggagttgt atgaagttga aactagagct cttagaagac aattagctga gagatctagt       540
   acagggcaag gaggaatatc cccaggagct cctcgttctc gaccaccagt aagcagcttc       600
   tcagggttac caagtttgcc ctctatacct gggattcatc ccagggcacc ttcacctcca       660
   agggcaactt ctactccggg aaatattcct tggagtttag gagatgataa cccaccttca     720
   tctagttttc ctggaccctc tcaacctcgt gtttctttcc atccgggaaa tccttttgtt     780
   gaagaagaag gtcatagacc tagatcccag tctagagaaa ggagaagaga aattcttcct     840
   gctcctgtac cgtcagcacc tcctatgatt cagtatatac cagtaccacc tccaccaccg     900
   attggcacgg ttatacctat tcagcatatc agatctgtaa ctggagagcc tcctagaaac     960
   ccaagagaaa taccaatttg gctaggacga aatgctcctg ctatagatgg agtgttccct    1020
   gttacaacac cggatctaag atgcagaata attaatgcta tactaggagg aaatattggg    1080
   ctatcattaa cccctggaga ctgtttaaca tgggactcag cagtagccac cttatttatt    1140
   agaacccatg gaacttttcc aatgcatcag cttggaaatg taataaaagg catagttgat    1200
   caagaaggag tggcaacagc atatactttg ggaatgatgc tttctggaca aaattatcaa    1260
   ttagtttctg gaataattag aggatatttg cctggacaag ctgtagtaac tgcattacaa    1320
   cagcgtttag accaagaaat agatgatcaa acaagagcag agacttttat tcaacatcta    1380
   aatgctgtat atgaaattct cggccttaat gccagaggac aaagtatacg tgcttcagtg    1440
   actcctcaac cccgaccatc cagaggtaga ggtcgaggtc aaaatacttc tagaccctct    1500
   caaggaccag ctaatagcgg gcggggacga cagcgccctg cttctggtca aagcaacaga    1560
   ggatctagta ctcagaatca aaatcaagat aatttaaatc aaggaggata taatcttcga    1620
   ccccgtactt accaacctca aaggtacgga ggaggacgtg gacgaagatg gaacgataat    1680
   actaacaatc aagagtccag accatcagat caaggttctc aaactcctag gccaaatcaa    1740
   gcaggctctg gggtgcgtgg caatcagtca caaactccca gaccagctgc tggtcgcgga    1800
   ggaagaggta accacaaccg aaaccaacga tcatccggtg ctggtgactc acgcgctgtc    1860
   aataccgtga cacagagtgc cacgtcctcc acagctgaat cctcttcagc tgttacagcc    1920
   gcttccggcg gagatcaaag ggactaa                                        1947
<210> 7
   <211> 648
   <212> PRT
   <213> Human foamy virus
<400> 7
   Met Ala Ser Gly Ser Asn Val Glu Glu Tyr Glu Leu Asp Val Glu Ala
   1               5                   10                  15
   Leu Val Val Ile Leu Arg Asp Arg Asn Ile Pro Arg Asn Pro Leu His
               20                  25                  30
   Gly Glu Val Ile Gly Leu Arg Leu Thr Glu Gly Trp Trp Gly Gln Ile
           35                  40                  45
   Glu Arg Phe Gln Met Val Arg Leu Ile Leu Gln Asp Asp Asp Asn Glu
       50                  55                  60
   Pro Leu Gln Arg Pro Arg Tyr Glu Val Ile Gln Arg Ala Val Asn Pro
   65                  70                  75                  80
   His Thr Met Phe Met Ile Ser Gly Pro Leu Ala Glu Leu Gln Leu Ala
                   85                  90                  95
   Phe Gln Asp Leu Asp Leu Pro Glu Gly Pro Leu Arg Phe Gly Pro Leu
               100                 105                 110
   Ala Asn Gly His Tyr Val Gln Gly Asp Pro Tyr Ser Ser Ser Tyr Arg
           115                 120                 125
   Pro Val Thr Met Ala Glu Thr Ala Gln Met Thr Arg Asp Glu Leu Glu
       130                 135                 140
   Asp Val Leu Asn Thr Gln Ser Glu Ile Glu Ile Gln Met Ile Asn Leu
   145                 150                 155                 160
   Leu Glu Leu Tyr Glu Val Glu Thr Arg Ala Leu Arg Arg Gln Leu Ala
                   165                 170                 175
   Glu Arg Ser Ser Thr Gly Gln Gly Gly Ile Ser Pro Gly Ala Pro Arg
               180                 185                 190
   Ser Arg Pro Pro Val Ser Ser Phe Ser Gly Leu Pro Ser Leu Pro Ser
           195                 200                 205
   Ile Pro Gly Ile His Pro Arg Ala Pro Ser Pro Pro Arg Ala Thr Ser
       210                 215                 220
   Thr Pro Gly Asn Ile Pro Trp Ser Leu Gly Asp Asp Asn Pro Pro Ser
   225                 230                 235                 240
   Ser Ser Phe Pro Gly Pro Ser Gln Pro Arg Val Ser Phe His Pro Gly
                   245                 250                 255
   Asn Pro Phe Val Glu Glu Glu Gly His Arg Pro Arg Ser Gln Ser Arg
               260                 265                 270
   Glu Arg Arg Arg Glu Ile Leu Pro Ala Pro Val Pro Ser Ala Pro Pro
           275                 280                 285
   Met Ile Gln Tyr Ile Pro Val Pro Pro Pro Pro Pro Ile Gly Thr Val
       290                 295                 300
   Ile Pro Ile Gln His Ile Arg Ser Val Thr Gly Glu Pro Pro Arg Asn
   305                 310                 315                 320
   Pro Arg Glu Ile Pro Ile Trp Leu Gly Arg Asn Ala Pro Ala Ile Asp
                   325                 330                 335
   Gly Val Phe Pro Val Thr Thr Pro Asp Leu Arg Cys Arg Ile Ile Asn
               340                 345                 350
   Ala Ile Leu Gly Gly Asn Ile Gly Leu Ser Leu Thr Pro Gly Asp Cys
           355                 360                 365
   Leu Thr Trp Asp Ser Ala Val Ala Thr Leu Phe Ile Arg Thr His Gly
       370                 375                 380
   Thr Phe Pro Met His Gln Leu Gly Asn Val Ile Lys Gly Ile Val Asp
   385                 390                 395                 400
   Gln Glu Gly Val Ala Thr Ala Tyr Thr Leu Gly Met Met Leu Ser Gly
                   405                 410                 415
   Gln Asn Tyr Gln Leu Val Ser Gly Ile Ile Arg Gly Tyr Leu Pro Gly
               420                 425                 430
   Gln Ala Val Val Thr Ala Leu Gln Gln Arg Leu Asp Gln Glu Ile Asp
           435                 440                 445
   Asp Gln Thr Arg Ala Glu Thr Phe Ile Gln His Leu Asn Ala Val Tyr
       450                 455                 460
   Glu Ile Leu Gly Leu Asn Ala Arg Gly Gln Ser Ile Arg Ala Ser Val
   465                 470                 475                 480
   Thr Pro Gln Pro Arg Pro Ser Arg Gly Arg Gly Arg Gly Gln Asn Thr
                   485                 490                 495
   Ser Arg Pro Ser Gln Gly Pro Ala Asn Ser Gly Arg Gly Arg Gln Arg
               500                 505                 510
   Pro Ala Ser Gly Gln Ser Asn Arg Gly Ser Ser Thr Gln Asn Gln Asn
           515                 520                 525
   Gln Asp Asn Leu Asn Gln Gly Gly Tyr Asn Leu Arg Pro Arg Thr Tyr
       530                 535                 540
   Gln Pro Gln Arg Tyr Gly Gly Gly Arg Gly Arg Arg Trp Asn Asp Asn
   545                 550                 555                 560
   Thr Asn Asn Gln Glu Ser Arg Pro Ser Asp Gln Gly Ser Gln Thr Pro
                   565                 570                 575
   Arg Pro Asn Gln Ala Gly Ser Gly Val Arg Gly Asn Gln Ser Gln Thr
               580                 585                 590
   Pro Arg Pro Ala Ala Gly Arg Gly Gly Arg Gly Asn His Asn Arg Asn
           595                 600                 605
   Gln Arg Ser Ser Gly Ala Gly Asp Ser Arg Ala Val Asn Thr Val Thr
       610                 615                 620
   Gln Ser Ala Thr Ser Ser Thr Asp Glu Ser Ser Ser Ala Val Thr Ala
   625                 630                 635                 640
   Ala Ser Gly Gly Asp Gln Arg Asp
                   645
<210> 8
   <211> 1947
   <212> DNA
   <213> Artificial Sequence
<220>
   <223> expression optimized polynoucleotide encoding for a human foamy virus derived capsid protein
<400> 8
   atggccagcg gcagcaacgt ggaggaatac gagctggacg tggaggccct ggtggtgatc      60
   ctgcgggacc ggaacatccc ccggaacccc ctgcacggcg aggtgatcgg cctgcggctg     120
   accgaggggt ggtggggcca gatcgagcgg ttccagatgg tgcggctgat cctgcaggac     180
   gacgacaacg agcccctgca gcggcccaga tacgaggtga tccagagggc cgtgaacccc     240
   cacaccatgt tcatgatcag cggccctctg gccgagctgc agctggcctt ccaggacctg     300
   gacctgcccg agggccccct gagattcggc cccctggcca acggccacta cgtgcagggc     360
   gacccctaca gcagcagcta ccggcccgtg accatggccg agaccgccca gatgacccgg     420
   gacgagctgg aagatgtgct gaacacccag agcgagatcg agatccagat gatcaacctg     480
   ctggaactgt acgaggtgga gaccagggcc ctgcggaggc agctggccga gcggagcagc     540
   acaggccagg gcggcatcag ccctggcgcc cctagaagca gaccccccgt gagcagcttc     600
   agcggcctgc ccagcctgcc tagcatcccc ggcatccacc ccagagcccc cagccccccc     660
   agagccacct ccacccccgg caacatccct tggagcctgg gcgacgacaa ccccccctcc     720
   agcagcttcc ctggccccag ccagcccaga gtgagcttcc accccggcaa tcccttcgtg     780
   gaggaagagg gccaccggcc cagaagccag agccgggagc ggcggagaga gatcctgcct     840
   gcccccgtgc ctagcgcccc tcccatgatc cagtacatcc ccgtgccccc tccccctccc     900
   atcggcaccg tgatccccat ccagcacatc agaagcgtga ccggcgagcc ccccagaaac     960
   cccagagaga tccccatctg gctgggccgg aacgccccag ccatcgacgg cgtgttcccc    1020
   gtgaccaccc ccgacctgcg gtgccggatc atcaacgcca tcctgggcgg caacatcggc 1080
   ctgagcctga cccctggcga ctgcctgacc tgggacagcg ccgtggccac cctgttcatc 1140
   cggacccacg gcaccttccc catgcaccag ctgggcaacg tgatcaaggg catcgtggac 1200
   caggaaggcg tcgccaccgc ctacaccctg ggcatgatgc tgtccggcca gaactaccag 1260
   ctggtgtccg gcatcatccg gggctacctg cccggacagg ccgtggtgac cgccctgcag 1320
   cagcggctgg atcaggaaat cgacgaccag accagagccg agaccttcat ccagcacctg 1380
   aacgccgtgt acgagatcct gggcctgaat gccaggggcc agagcatccg ggccagcgtg 1440
   accccccagc ccaggcccag ccggggcaga ggcagaggcc agaacacctc ccggcccagc 1500
   cagggacccg ccaacagcgg cagaggccgg cagagacccg ccagcggcca gtccaaccgg 1560
   ggcagcagca cccagaacca gaaccaggac aacctgaacc agggcggcta caacctgcgg 1620
   cccaggacct accagcccca gagatatggc ggcggacggg gcaggcggtg gaacgacaac 1680
   accaacaacc aggaaagccg gcccagcgac cagggcagcc agacccccag gcccaaccag 1740
   gccggctccg gcgtgagggg caaccagagc cagacacccc ggcctgccgc cggaagaggc 1800
   ggcaggggca atcacaaccg gaaccagaga agcagcggag ccggcgacag cagagccgtg 1860
   aacaccgtga cccagagcgc cacctccagc accgatgaaa gcagcagcgc cgtgaccgcc 1920
   gcctctggcg gcgaccagcg ggactga 1947
<210> 9
   <211> 775
   <212> PRT
   <213> Artificial Sequence
<220>
   <223> fusion protein of human foamy virus derived capsid protein and a rapamycin-binding heterodimerizable protein domain
<400> 9
   Met Val Ser Arg Gly Val Gln Val Glu Thr Ile Ser Pro Gly Asp Gly
   1               5                   10                  15
   Arg Thr Phe Pro Lys Arg Gly Gln Thr Cys Val Val His Tyr Thr Gly
               20                  25                  30
   Met Leu Glu Asp Gly Lys Lys Phe Asp Ser Ser Arg Asp Arg Asn Lys
           35                  40                  45
   Pro Phe Lys Phe Met Leu Gly Lys Gln Glu Val Ile Arg Gly Trp Glu
       50                  55                  60
   Glu Gly Val Ala Gln Met Ser Val Gly Gln Arg Ala Lys Leu Thr Ile
   65                  70                  75                  80
   Ser Pro Asp Tyr Ala Tyr Gly Ala Thr Gly His Pro Gly Ile Ile Pro
   85 90 95
   Pro His Ala Thr Leu Val Phe Asp Val Glu Leu Leu Lys Leu Glu Thr
   100 105 110
   Ser Gly Ser Gly Gly Gly Gly Ser Gly Gly Gly Gly Ser Gly Gly Gly
   115 120 125
   Ala Ser Gly Ser Asn Val Glu Glu Tyr Glu Leu Asp Val Glu Ala Leu
   130 135 140
   Val Val Ile Leu Arg Asp Arg Asn Ile Pro Arg Asn Pro Leu His Gly
   145 150 155 160
   Glu Val Ile Gly Leu Arg Leu Thr Glu Gly Trp Trp Gly Gln Ile Glu
   165 170 175
   Arg Phe Gln Met Val Arg Leu Ile Leu Gln Asp Asp Asp Asn Glu Pro
   180 185 190
   Leu Gln Arg Pro Arg Tyr Glu Val Ile Gln Arg Ala Val Asn Pro His
   195 200 205
   Thr Met Phe Met Ile Ser Gly Pro Leu Ala Glu Leu Gln Leu Ala Phe
   210 215 220
   Gln Asp Leu Asp Leu Pro Glu Gly Pro Leu Arg Phe Gly Pro Leu Ala
   225 230 235 240
   Asn Gly His Tyr Val Gln Gly Asp Pro Tyr Ser Ser Ser Tyr Arg Pro
   245 250 255
   Val Thr Met Ala Glu Thr Ala Gln Met Thr Arg Asp Glu Leu Glu Asp
   260 265 270
   Val Leu Asn Thr Gln Ser Glu Ile Glu Ile Gln Met Ile Asn Leu Leu
   275 280 285
   Glu Leu Tyr Glu Val Glu Thr Arg Ala Leu Arg Arg Gln Leu Ala Glu
   290 295 300
   Arg Ser Ser Thr Gly Gln Gly Gly Ile Ser Pro Gly Ala Pro Arg Ser
   305 310 315 320
   Arg Pro Pro Val Ser Ser Phe Ser Gly Leu Pro Ser Leu Pro Ser Ile
   325 330 335
   Pro Gly Ile His Pro Arg Ala Pro Ser Pro Pro Arg Ala Thr Ser Thr
   340 345 350
   Pro Gly Asn Ile Pro Trp Ser Leu Gly Asp Asp Asn Pro Pro Ser Ser
           355                 360                 365
   Ser Phe Pro Gly Pro Ser Gln Pro Arg Val Ser Phe His Pro Gly Asn
       370                 375                 380
   Pro Phe Val Glu Glu Glu Gly His Arg Pro Arg Ser Gln Ser Arg Glu
   385                 390                 395                 400
   Arg Arg Arg Glu Ile Leu Pro Ala Pro Val Pro Ser Ala Pro Pro Met
                   405                 410                 415
   Ile Gln Tyr Ile Pro Val Pro Pro Pro Pro Pro Ile Gly Thr Val Ile
               420                 425                 430
   Pro Ile Gln His Ile Arg Ser Val Thr Gly Glu Pro Pro Arg Asn Pro
           435                 440                 445
   Arg Glu Ile Pro Ile Trp Leu Gly Arg Asn Ala Pro Ala Ile Asp Gly
       450                 455                 460
   Val Phe Pro Val Thr Thr Pro Asp Leu Arg Cys Arg Ile Ile Asn Ala
   465                 470                 475                 480
   Ile Leu Gly Gly Asn Ile Gly Leu Ser Leu Thr Pro Gly Asp Cys Leu
                   485                 490                 495
   Thr Trp Asp Ser Ala Val Ala Thr Leu Phe Ile Arg Thr His Gly Thr
               500                 505                 510
   Phe Pro Met His Gln Leu Gly Asn Val Ile Lys Gly Ile Val Asp Gln
           515                 520                 525
   Glu Gly Val Ala Thr Ala Tyr Thr Leu Gly Met Met Leu Ser Gly Gln
       530                 535                 540
   Asn Tyr Gln Leu Val Ser Gly Ile Ile Arg Gly Tyr Leu Pro Gly Gln
   545                 550                 555                 560
   Ala Val Val Thr Ala Leu Gln Gln Arg Leu Asp Gln Glu Ile Asp Asp
                   565                 570                 575
   Gln Thr Arg Ala Glu Thr Phe Ile Gln His Leu Asn Ala Val Tyr Glu
               580                 585                 590
   Ile Leu Gly Leu Asn Ala Arg Gly Gln Ser Ile Arg Ala Ser Val Thr
           595                 600                 605
   Pro Gln Pro Arg Pro Ser Arg Gly Arg Gly Arg Gly Gln Asn Thr Ser
       610                 615                 620
   Arg Pro Ser Gln Gly Pro Ala Asn Ser Gly Arg Gly Arg Gln Arg Pro
   625                 630                 635                 640
   Ala Ser Gly Gln Ser Asn Arg Gly Ser Ser Thr Gln Asn Gln Asn Gln
                   645                 650                 655
   Asp Asn Leu Asn Gln Gly Gly Tyr Asn Leu Arg Pro Arg Thr Tyr Gln
               660                 665                 670
   Pro Gln Arg Tyr Gly Gly Gly Arg Gly Arg Arg Trp Asn Asp Asn Thr
           675                 680                 685
   Asn Asn Gln Glu Ser Arg Pro Ser Asp Gln Gly Ser Gln Thr Pro Arg
       690                 695                 700
   Pro Asn Gln Ala Gly Ser Gly Val Arg Gly Asn Gln Ser Gln Thr Pro
   705                 710                 715                 720
   Arg Pro Ala Ala Gly Arg Gly Gly Arg Gly Asn His Asn Arg Asn Gln
                   725                 730                 735
   Arg Ser Ser Gly Ala Gly Asp Ser Arg Ala Val Asn Thr Val Thr Gln
               740                 745                 750
   Ser Ala Thr Ser Ser Thr Asp Glu Ser Ser Ser Ala Val Thr Ala Ala
           755                 760                 765
   Ser Gly Gly Asp Gln Arg Asp
       770                 775
<210> 10
   <211> 2328
   <212> DNA
   <213> Artificial Sequence
<220>
   <223> polynucleotide encoding for a fusion protein of human foamy virus derived capsid protein and a rapamycin-binding heterodimerizable protein domain
<400> 10
   atggtgtcta gaggagtgca ggtggaaacc atctccccag gagacgggcg caccttcccc        60
   aagcgcggcc agacctgcgt ggtgcactac accgggatgc ttgaagatgg aaagaaattt       120
   gattcctccc gggacagaaa caagcccttt aagtttatgc taggcaagca ggaggtgatc       180
   cgaggctggg aagaaggggt tgcccagatg agtgtgggtc agagagccaa actgactata       240
   tctccagatt atgcctatgg tgccactggg cacccaggca tcatcccacc acatgccact       300
   ctcgtcttcg atgtggagct tctaaaactg gaaactagtg gatccggagg cggcggcagc 360
   ggcgggggcg ggagcggcgg cggggctagc ggcagcaacg tggaggaata cgagctggac 420
   gtggaggccc tggtggtgat cctgcgggac cggaacatcc cccggaaccc cctgcacggc 480
   gaggtgatcg gcctgcggct gaccgagggg tggtggggcc agatcgagcg gttccagatg 540
   gtgcggctga tcctgcagga cgacgacaac gagcccctgc agcggcccag atacgaggtg 600
   atccagaggg ccgtgaaccc ccacaccatg ttcatgatca gcggccctct ggccgagctg 660
   cagctggcct tccaggacct ggacctgccc gagggccccc tgagattcgg ccccctggcc 720
   aacggccact acgtgcaggg cgacccctac agcagcagct accggcccgt gaccatggcc 780
   gagaccgccc agatgacccg ggacgagctg gaagatgtgc tgaacaccca gagcgagatc 840
   gagatccaga tgatcaacct gctggaactg tacgaggtgg agaccagggc cctgcggagg 900
   cagctggccg agcggagcag cacaggccag ggcggcatca gccctggcgc ccctagaagc 960
   agaccccccg tgagcagctt cagcggcctg cccagcctgc ctagcatccc cggcatccac 1020
   cccagagccc ccagcccccc cagagccacc tccacccccg gcaacatccc ttggagcctg 1080
   ggcgacgaca accccccctc cagcagcttc cctggcccca gccagcccag agtgagcttc 1140
   caccccggca atcccttcgt ggaggaagag ggccaccggc ccagaagcca gagccgggag 1200
   cggcggagag agatcctgcc tgcccccgtg cctagcgccc ctcccatgat ccagtacatc 1260
   cccgtgcccc ctccccctcc catcggcacc gtgatcccca tccagcacat cagaagcgtg 1320
   accggcgagc cccccagaaa ccccagagag atccccatct ggctgggccg gaacgcccca 1380
   gccatcgacg gcgtgttccc cgtgaccacc cccgacctgc ggtgccggat catcaacgcc 1440
   atcctgggcg gcaacatcgg cctgagcctg acccctggcg actgcctgac ctgggacagc 1500
   gccgtggcca ccctgttcat ccggacccac ggcaccttcc ccatgcacca gctgggcaac 1560
   gtgatcaagg gcatcgtgga ccaggaaggc gtcgccaccg cctacaccct gggcatgatg 1620
   ctgtccggcc agaactacca gctggtgtcc ggcatcatcc ggggctacct gcccggacag 1680
   gccgtggtga ccgccctgca gcagcggctg gatcaggaaa tcgacgacca gaccagagcc 1740
   gagaccttca tccagcacct gaacgccgtg tacgagatcc tgggcctgaa tgccaggggc 1800
   cagagcatcc gggccagcgt gaccccccag cccaggccca gccggggcag aggcagaggc 1860
   cagaacacct cccggcccag ccagggaccc gccaacagcg gcagaggccg gcagagaccc 1920
   gccagcggcc agtccaaccg gggcagcagc acccagaacc agaaccagga caacctgaac 1980
   cagggcggct acaacctgcg gcccaggacc taccagcccc agagatatgg cggcggacgg 2040
   ggcaggcggt ggaacgacaa caccaacaac caggaaagcc ggcccagcga ccagggcagc 2100
   cagaccccca ggcccaacca ggccggctcc ggcgtgaggg gcaaccagag ccagacaccc 2160
   cggcctgccg ccggaagagg cggcaggggc aatcacaacc ggaaccagag aagcagcgga 2220
   gccggcgaca gcagagccgt gaacaccgtg acccagagcg ccacctccag caccgatgaa 2280
   agcagcagcg ccgtgaccgc cgcctctggc ggcgaccagc gggactga 2328
<210> 11
   <211> 761
   <212> PRT
   <213> Artificial Sequence
<220>
   <223> fusion protein of human foamy virus derived capsid protein and a rapamycin-binding heterodimerizable protein domain
<400> 11
   Met Val Ser Arg Ile Leu Trp His Glu Met Trp His Glu Gly Leu Glu
   1               5                   10                  15
   Glu Ala Ser Arg Leu Tyr Phe Gly Glu Arg Asn Val Lys Gly Met Phe
               20                  25                  30
   Glu Val Leu Glu Pro Leu His Ala Met Met Glu Arg Gly Pro Gln Thr
           35                  40                  45
   Leu Lys Glu Thr Ser Phe Asn Gln Ala Tyr Gly Arg Asp Leu Met Glu
       50                  55                  60
   Ala Gln Glu Trp Cys Arg Lys Tyr Met Lys Ser Gly Asn Val Lys Asp
   65                  70                  75                  80
   Leu Leu Gln Ala Trp Asp Leu Tyr Tyr His Val Phe Arg Arg Ile Ser
                   85                  90                  95
   Lys Thr Ser Gly Ser Gly Gly Gly Gly Ser Gly Gly Gly Gly Ser Gly
               100                 105                 110
   Gly Gly Ala Ser Gly Ser Asn Val Glu Glu Tyr Glu Leu Asp Val Glu
           115                 120                 125
   Ala Leu Val Val Ile Leu Arg Asp Arg Asn Ile Pro Arg Asn Pro Leu
       130                 135                 140
   His Gly Glu Val Ile Gly Leu Arg Leu Thr Glu Gly Trp Trp Gly Gln
   145                 150                 155                 160
   Ile Glu Arg Phe Gln Met Val Arg Leu Ile Leu Gln Asp Asp Asp Asn
                   165                 170                 175
   Glu Pro Leu Gln Arg Pro Arg Tyr Glu Val Ile Gln Arg Ala Val Asn
               180                 185                 190
   Pro His Thr Met Phe Met Ile Ser Gly Pro Leu Ala Glu Leu Gln Leu
           195                 200                 205
   Ala Phe Gln Asp Leu Asp Leu Pro Glu Gly Pro Leu Arg Phe Gly Pro
       210                 215                 220
   Leu Ala Asn Gly His Tyr Val Gln Gly Asp Pro Tyr Ser Ser Ser Tyr
   225                 230                 235                 240
   Arg Pro Val Thr Met Ala Glu Thr Ala Gln Met Thr Arg Asp Glu Leu
                   245                 250                 255
   Glu Asp Val Leu Asn Thr Gln Ser Glu Ile Glu Ile Gln Met Ile Asn
               260                 265                 270
   Leu Leu Glu Leu Tyr Glu Val Glu Thr Arg Ala Leu Arg Arg Gln Leu
           275                 280                 285
   Ala Glu Arg Ser Ser Thr Gly Gln Gly Gly Ile Ser Pro Gly Ala Pro
       290                 295                 300
   Arg Ser Arg Pro Pro Val Ser Ser Phe Ser Gly Leu Pro Ser Leu Pro
   305                 310                 315                 320
   Ser Ile Pro Gly Ile His Pro Arg Ala Pro Ser Pro Pro Arg Ala Thr
                   325                 330                 335
   Ser Thr Pro Gly Asn Ile Pro Trp Ser Leu Gly Asp Asp Asn Pro Pro
               340                 345                 350
   Ser Ser Ser Phe Pro Gly Pro Ser Gln Pro Arg Val Ser Phe His Pro
           355                 360                 365
   Gly Asn Pro Phe Val Glu Glu Glu Gly His Arg Pro Arg Ser Gln Ser
       370                 375                 380
   Arg Glu Arg Arg Arg Glu Ile Leu Pro Ala Pro Val Pro Ser Ala Pro
   385                 390                 395                 400
   Pro Met Ile Gln Tyr Ile Pro Val Pro Pro Pro Pro Pro Ile Gly Thr
                   405                 410                 415
   Val Ile Pro Ile Gln His Ile Arg Ser Val Thr Gly Glu Pro Pro Arg
               420                 425                 430
   Asn Pro Arg Glu Ile Pro Ile Trp Leu Gly Arg Asn Ala Pro Ala Ile
           435                 440                 445
   Asp Gly Val Phe Pro Val Thr Thr Pro Asp Leu Arg Cys Arg Ile Ile
       450                 455                 460
   Asn Ala Ile Leu Gly Gly Asn Ile Gly Leu Ser Leu Thr Pro Gly Asp
   465 470 475 480
   Cys Leu Thr Trp Asp Ser Ala Val Ala Thr Leu Phe Ile Arg Thr His
   485 490 495
   Gly Thr Phe Pro Met His Gln Leu Gly Asn Val Ile Lys Gly Ile Val
   500 505 510
   Asp Gln Glu Gly Val Ala Thr Ala Tyr Thr Leu Gly Met Met Leu Ser
   515 520 525
   Gly Gln Asn Tyr Gln Leu Val Ser Gly Ile Ile Arg Gly Tyr Leu Pro
   530 535 540
   Gly Gln Ala Val Val Thr Ala Leu Gln Gln Arg Leu Asp Gln Glu Ile
   545 550 555 560
   Asp Asp Gln Thr Arg Ala Glu Thr Phe Ile Gln His Leu Asn Ala Val
   565 570 575
   Tyr Glu Ile Leu Gly Leu Asn Ala Arg Gly Gln Ser Ile Arg Ala Ser
   580 585 590
   Val Thr Pro Gln Pro Arg Pro Ser Arg Gly Arg Gly Arg Gly Gln Asn
   595 600 605
   Thr Ser Arg Pro Ser Gln Gly Pro Ala Asn Ser Gly Arg Gly Arg Gln
   610 615 620
   Arg Pro Ala Ser Gly Gln Ser Asn Arg Gly Ser Ser Thr Gln Asn Gln
   625 630 635 640
   Asn Gln Asp Asn Leu Asn Gln Gly Gly Tyr Asn Leu Arg Pro Arg Thr
   645 650 655
   Tyr Gln Pro Gln Arg Tyr Gly Gly Gly Arg Gly Arg Arg Trp Asn Asp
   660 665 670
   Asn Thr Asn Asn Gln Glu Ser Arg Pro Ser Asp Gln Gly Ser Gln Thr
   675 680 685
   Pro Arg Pro Asn Gln Ala Gly Ser Gly Val Arg Gly Asn Gln Ser Gln
   690 695 700
   Thr Pro Arg Pro Ala Ala Gly Arg Gly Gly Arg Gly Asn His Asn Arg
   705 710 715 720
   Asn Gln Arg Ser Ser Gly Ala Gly Asp Ser Arg Ala Val Asn Thr Val
                   725                 730                 735
   Thr Gln Ser Ala Thr Ser Ser Thr Asp Glu Ser Ser Ser Ala Val Thr
               740                 745                 750
   Ala Ala Ser Gly Gly Asp Gln Arg Asp
           755                 760
<210> 12
   <211> 2286
   <212> DNA
   <213> Artificial Sequence
<220>
   <223> polynucleotide encoding for a fusion protein of human foamy virus derived capsid protein and a rapamycin-binding heterodimerizable protein domain
<400> 12
   atggtgtcta gaatcctctg gcatgagatg tggcatgaag gcctggaaga ggcatctcgt      60
   ttgtactttg gggaaaggaa cgtgaaaggc atgtttgagg tgctggagcc cttgcatgct     120
   atgatggaac ggggccccca gactctgaag gaaacatcct ttaatcaggc ctatggtcga     180
   gatttaatgg aggcccaaga gtggtgcagg aagtacatga aatcagggaa tgtcaaggac     240
   ctcctccaag cctgggacct ctattatcat gtgttccgac gaatctcaaa gactagtgga     300
   tccggaggcg gcggcagcgg cgggggcggg agcggcggcg gggctagcgg cagcaacgtg     360
   gaggaatacg agctggacgt ggaggccctg gtggtgatcc tgcgggaccg gaacatcccc     420
   cggaaccccc tgcacggcga ggtgatcggc ctgcggctga ccgaggggtg gtggggccag     480
   atcgagcggt tccagatggt gcggctgatc ctgcaggacg acgacaacga gcccctgcag     540
   cggcccagat acgaggtgat ccagagggcc gtgaaccccc acaccatgtt catgatcagc     600
   ggccctctgg ccgagctgca gctggccttc caggacctgg acctgcccga gggccccctg     660
   agattcggcc ccctggccaa cggccactac gtgcagggcg acccctacag cagcagctac     720
   cggcccgtga ccatggccga gaccgcccag atgacccggg acgagctgga agatgtgctg     780
   aacacccaga gcgagatcga gatccagatg atcaacctgc tggaactgta cgaggtggag     840
   accagggccc tgcggaggca gctggccgag cggagcagca caggccaggg cggcatcagc     900
   cctggcgccc ctagaagcag accccccgtg agcagcttca gcggcctgcc cagcctgcct     960
   agcatccccg gcatccaccc cagagccccc agccccccca gagccacctc cacccccggc    1020
   aacatccctt ggagcctggg cgacgacaac cccccctcca gcagcttccc tggccccagc    1080
   cagcccagag tgagcttcca ccccggcaat cccttcgtgg aggaagaggg ccaccggccc    1140
   agaagccaga gccgggagcg gcggagagag atcctgcctg cccccgtgcc tagcgcccct    1200
   cccatgatcc agtacatccc cgtgccccct ccccctccca tcggcaccgt gatccccatc    1260
   cagcacatca gaagcgtgac cggcgagccc cccagaaacc ccagagagat ccccatctgg     1320
   ctgggccgga acgccccagc catcgacggc gtgttccccg tgaccacccc cgacctgcgg     1380
   tgccggatca tcaacgccat cctgggcggc aacatcggcc tgagcctgac ccctggcgac     1440
   tgcctgacct gggacagcgc cgtggccacc ctgttcatcc ggacccacgg caccttcccc     1500
   atgcaccagc tgggcaacgt gatcaagggc atcgtggacc aggaaggcgt cgccaccgcc     1560
   tacaccctgg gcatgatgct gtccggccag aactaccagc tggtgtccgg catcatccgg     1620
   ggctacctgc ccggacaggc cgtggtgacc gccctgcagc agcggctgga tcaggaaatc     1680
   gacgaccaga ccagagccga gaccttcatc cagcacctga acgccgtgta cgagatcctg     1740
   ggcctgaatg ccaggggcca gagcatccgg gccagcgtga ccccccagcc caggcccagc     1800
   cggggcagag gcagaggcca gaacacctcc cggcccagcc agggacccgc caacagcggc     1860
   agaggccggc agagacccgc cagcggccag tccaaccggg gcagcagcac ccagaaccag     1920
   aaccaggaca acctgaacca gggcggctac aacctgcggc ccaggaccta ccagccccag     1980
   agatatggcg gcggacgggg caggcggtgg aacgacaaca ccaacaacca ggaaagccgg     2040
   cccagcgacc agggcagcca gacccccagg cccaaccagg ccggctccgg cgtgaggggc     2100
   aaccagagcc agacaccccg gcctgccgcc ggaagaggcg gcaggggcaa tcacaaccgg     2160
   aaccagagaa gcagcggagc cggcgacagc agagccgtga acaccgtgac ccagagcgcc     2220
   acctccagca ccgatgaaag cagcagcgcc gtgaccgccg cctctggcgg cgaccagcgg     2280
   gactga                                                                2286
<210> 13
   <211> 775
   <212> PRT
   <213> Artificial Sequence
<220>
   <223> fusion protein of human foamy virus derived capsid protein and a rapamycin-binding heterodimerizable protein domain
<400> 13
   Met Ala Ser Gly Ser Asn Val Glu Glu Tyr Glu Leu Asp Val Glu Ala
   1               5                   10                  15
   Leu Val Val Ile Leu Arg Asp Arg Asn Ile Pro Arg Asn Pro Leu His
               20                  25                  30
   Gly Glu Val Ile Gly Leu Arg Leu Thr Glu Gly Trp Trp Gly Gln Ile
           35                  40                  45
   Glu Arg Phe Gln Met Val Arg Leu Ile Leu Gln Asp Asp Asp Asn Glu
       50                  55                  60
   Pro Leu Gln Arg Pro Arg Tyr Glu Val Ile Gln Arg Ala Val Asn Pro
   65                  70                  75                  80
   His Thr Met Phe Met Ile Ser Gly Pro Leu Ala Glu Leu Gln Leu Ala
                   85                  90                  95
   Phe Gln Asp Leu Asp Leu Pro Glu Gly Pro Leu Arg Phe Gly Pro Leu
               100                 105                 110
   Ala Asn Gly His Tyr Val Gln Gly Asp Pro Tyr Ser Ser Ser Tyr Arg
           115                 120                 125
   Pro Val Thr Met Ala Glu Thr Ala Gln Met Thr Arg Asp Glu Leu Glu
       130                 135                 140
   Asp Val Leu Asn Thr Gln Ser Glu Ile Glu Ile Gln Met Ile Asn Leu
   145                 150                 155                 160
   Leu Glu Leu Tyr Glu Val Glu Thr Arg Ala Leu Arg Arg Gln Leu Ala
                   165                 170                 175
   Glu Arg Ser Ser Thr Gly Gln Gly Gly Ile Ser Pro Gly Ala Pro Arg
               180                 185                 190
   Ser Arg Pro Pro Val Ser Ser Phe Ser Gly Leu Pro Ser Leu Pro Ser
           195                 200                 205
   Ile Pro Gly Ile His Pro Arg Ala Pro Ser Pro Pro Arg Ala Thr Ser
       210                 215                 220
   Thr Pro Gly Asn Ile Pro Trp Ser Leu Gly Asp Asp Asn Pro Pro Ser
   225                 230                 235                 240
   Ser Ser Phe Pro Gly Pro Ser Gln Pro Arg Val Ser Phe His Pro Gly
                   245                 250                 255
   Asn Pro Phe Val Glu Glu Glu Gly His Arg Pro Arg Ser Gln Ser Arg
               260                 265                 270
   Glu Arg Arg Arg Glu Ile Leu Pro Ala Pro Val Pro Ser Ala Pro Pro
           275                 280                 285
   Met Ile Gln Tyr Ile Pro Val Pro Pro Pro Pro Pro Ile Gly Thr Val
       290                 295                 300
   Ile Pro Ile Gln His Ile Arg Ser Val Thr Gly Glu Pro Pro Arg Asn
   305                 310                 315                 320
   Pro Arg Glu Ile Pro Ile Trp Leu Gly Arg Asn Ala Pro Ala Ile Asp
   325 330 335
   Gly Val Phe Pro Val Thr Thr Pro Asp Leu Arg Cys Arg Ile Ile Asn
   340 345 350
   Ala Ile Leu Gly Gly Asn Ile Gly Leu Ser Leu Thr Pro Gly Asp Cys
   355 360 365
   Leu Thr Trp Asp Ser Ala Val Ala Thr Leu Phe Ile Arg Thr His Gly
   370 375 380
   Thr Phe Pro Met His Gln Leu Gly Asn Val Ile Lys Gly Ile Val Asp
   385 390 395 400
   Gln Glu Gly Val Ala Thr Ala Tyr Thr Leu Gly Met Met Leu Ser Gly
   405 410 415
   Gln Asn Tyr Gln Leu Val Ser Gly Ile Ile Arg Gly Tyr Leu Pro Gly
   420 425 430
   Gln Ala Val Val Thr Ala Leu Gln Gln Arg Leu Asp Gln Glu Ile Asp
   435 440 445
   Asp Gln Thr Arg Ala Glu Thr Phe Ile Gln His Leu Asn Ala Val Tyr
   450 455 460
   Glu Ile Leu Gly Leu Asn Ala Arg Gly Gln Ser Ile Arg Ala Ser Val
   465 470 475 480
   Thr Pro Gln Pro Arg Pro Ser Arg Gly Arg Gly Arg Gly Gln Asn Thr
   485 490 495
   Ser Arg Pro Ser Gln Gly Pro Ala Asn Ser Gly Arg Gly Arg Gln Arg
   500 505 510
   Pro Ala Ser Gly Gln Ser Asn Arg Gly Ser Ser Thr Gln Asn Gln Asn
   515 520 525
   Gln Asp Asn Leu Asn Gln Gly Gly Tyr Asn Leu Arg Pro Arg Thr Tyr
   530 535 540
   Gln Pro Gln Arg Tyr Gly Gly Gly Arg Gly Arg Arg Trp Asn Asp Asn
   545 550 555 560
   Thr Asn Asn Gln Glu Ser Arg Pro Ser Asp Gln Gly Ser Gln Thr Pro
   565 570 575
   Arg Pro Asn Gln Ala Gly Ser Gly Val Arg Gly Asn Gln Ser Gln Thr
   580 585 590
   Pro Arg Pro Ala Ala Gly Arg Gly Gly Arg Gly Asn His Asn Arg Asn
   595 600 605
   Gln Arg Ser Ser Gly Ala Gly Asp Ser Arg Ala Val Asn Thr Val Thr
   610 615 620
   Gln Ser Ala Thr Ser Ser Thr Asp Glu Ser Ser Ser Ala Val Thr Ala
   625 630 635 640
   Ala Ser Gly Gly Asp Gln Arg Asp Ser Gly Gly Gly Gly Ser Gly Gly
   645 650 655
   Gly Gly Ser Gly Gly Gly Ala Ser Ser Arg Gly Val Gln Val Glu Thr
   660 665 670
   Ile Ser Pro Gly Asp Gly Arg Thr Phe Pro Lys Arg Gly Gln Thr Cys
   675 680 685
   Val Val His Tyr Thr Gly Met Leu Glu Asp Gly Lys Lys Phe Asp Ser
   690 695 700
   Ser Arg Asp Arg Asn Lys Pro Phe Lys Phe Met Leu Gly Lys Gln Glu
   705 710 715 720
   Val Ile Arg Gly Trp Glu Glu Gly Val Ala Gln Met Ser Val Gly Gln
   725 730 735
   Arg Ala Lys Leu Thr Ile Ser Pro Asp Tyr Ala Tyr Gly Ala Thr Gly
   740 745 750
   His Pro Gly Ile Ile Pro Pro His Ala Thr Leu Val Phe Asp Val Glu
   755 760 765
   Leu Leu Lys Leu Glu Thr Ser
   770 775
<210> 14
   <211> 2328
   <212> DNA
   <213> Artificial Sequence
<220>
   <223> polynucleotide encoding for a fusion protein of human foamy virus derived capsid protein and a rapamycin-binding heterodimerizable protein domain
<400> 14
   atggccagcg gcagcaacgt ggaggaatac gagctggacg tggaggccct ggtggtgatc        60
   ctgcgggacc ggaacatccc ccggaacccc ctgcacggcg aggtgatcgg cctgcggctg       120
   accgaggggt ggtggggcca gatcgagcgg ttccagatgg tgcggctgat cctgcaggac 180
   gacgacaacg agcccctgca gcggcccaga tacgaggtga tccagagggc cgtgaacccc 240
   cacaccatgt tcatgatcag cggccctctg gccgagctgc agctggcctt ccaggacctg 300
   gacctgcccg agggccccct gagattcggc cccctggcca acggccacta cgtgcagggc 360
   gacccctaca gcagcagcta ccggcccgtg accatggccg agaccgccca gatgacccgg 420
   gacgagctgg aagatgtgct gaacacccag agcgagatcg agatccagat gatcaacctg 480
   ctggaactgt acgaggtgga gaccagggcc ctgcggaggc agctggccga gcggagcagc 540
   acaggccagg gcggcatcag ccctggcgcc cctagaagca gaccccccgt gagcagcttc 600
   agcggcctgc ccagcctgcc tagcatcccc ggcatccacc ccagagcccc cagccccccc 660
   agagccacct ccacccccgg caacatccct tggagcctgg gcgacgacaa ccccccctcc 720
   agcagcttcc ctggccccag ccagcccaga gtgagcttcc accccggcaa tcccttcgtg 780
   gaggaagagg gccaccggcc cagaagccag agccgggagc ggcggagaga gatcctgcct 840
   gcccccgtgc ctagcgcccc tcccatgatc cagtacatcc ccgtgccccc tccccctccc 900
   atcggcaccg tgatccccat ccagcacatc agaagcgtga ccggcgagcc ccccagaaac 960
   cccagagaga tccccatctg gctgggccgg aacgccccag ccatcgacgg cgtgttcccc 1020
   gtgaccaccc ccgacctgcg gtgccggatc atcaacgcca tcctgggcgg caacatcggc 1080
   ctgagcctga cccctggcga ctgcctgacc tgggacagcg ccgtggccac cctgttcatc 1140
   cggacccacg gcaccttccc catgcaccag ctgggcaacg tgatcaaggg catcgtggac 1200
   caggaaggcg tcgccaccgc ctacaccctg ggcatgatgc tgtccggcca gaactaccag 1260
   ctggtgtccg gcatcatccg gggctacctg cccggacagg ccgtggtgac cgccctgcag 1320
   cagcggctgg atcaggaaat cgacgaccag accagagccg agaccttcat ccagcacctg 1380
   aacgccgtgt acgagatcct gggcctgaat gccaggggcc agagcatccg ggccagcgtg 1440
   accccccagc ccaggcccag ccggggcaga ggcagaggcc agaacacctc ccggcccagc 1500
   cagggacccg ccaacagcgg cagaggccgg cagagacccg ccagcggcca gtccaaccgg 1560
   ggcagcagca cccagaacca gaaccaggac aacctgaacc agggcggcta caacctgcgg 1620
   cccaggacct accagcccca gagatatggc ggcggacggg gcaggcggtg gaacgacaac 1680
   accaacaacc aggaaagccg gcccagcgac cagggcagcc agacccccag gcccaaccag 1740
   gccggctccg gcgtgagggg caaccagagc cagacacccc ggcctgccgc cggaagaggc 1800
   ggcaggggca atcacaaccg gaaccagaga agcagcggag ccggcgacag cagagccgtg 1860
   aacaccgtga cccagagcgc cacctccagc accgatgaaa gcagcagcgc cgtgaccgcc 1920
   gcctctggcg gcgaccagcg ggactccgga ggcggcggca gcggcggggg cgggagcggc 1980
   ggcggggcta gctctagagg agtgcaggtg gaaaccatct ccccaggaga cgggcgcacc 2040
   ttccccaagc gcggccagac ctgcgtggtg cactacaccg ggatgcttga agatggaaag 2100
   aaatttgatt cctcccggga cagaaacaag ccctttaagt ttatgctagg caagcaggag 2160
   gtgatccgag gctgggaaga aggggttgcc cagatgagtg tgggtcagag agccaaactg 2220
   actatatctc cagattatgc ctatggtgcc actgggcacc caggcatcat cccaccacat 2280
   gccactctcg tcttcgatgt ggagcttcta aaactggaaa ctagttag 2328
<210> 15
   <211> 775
   <212> PRT
   <213> Artificial Sequence
<220>
   <223> fusion protein of human foamy virus derived capsid protein and a rapamycin-binding heterodimerizable protein domain
<400> 15
   Met Ala Ser Gly Ser Asn Val Glu Glu Tyr Glu Leu Asp Val Glu Ala
   1               5                   10                  15
   Leu Val Val Ile Leu Arg Asp Arg Asn Ile Pro Arg Asn Pro Leu His
               20                  25                  30
   Gly Glu Val Ile Gly Leu Arg Leu Thr Glu Gly Trp Trp Gly Gln Ile
           35                  40                  45
   Glu Arg Phe Gln Met Val Arg Leu Ile Leu Gln Asp Asp Asp Asn Glu
       50                  55                  60
   Pro Leu Gln Arg Pro Arg Tyr Glu Val Ile Gln Arg Ala Val Asn Pro
   65                  70                  75                  80
   His Thr Met Phe Met Ile Ser Gly Pro Leu Ala Glu Leu Gln Leu Ala
                   85                  90                  95
   Phe Gln Asp Leu Asp Leu Pro Glu Gly Pro Leu Arg Phe Gly Pro Leu
               100                 105                 110
   Ala Asn Gly His Tyr Val Gln Gly Asp Pro Tyr Ser Ser Ser Tyr Arg
           115                 120                 125
   Pro Val Thr Met Ala Glu Thr Ala Gln Met Thr Arg Asp Glu Leu Glu
       130                 135                 140
   Asp Val Leu Asn Thr Gln Ser Glu Ile Glu Ile Gln Met Ile Asn Leu
   145                 150                 155                 160
   Leu Glu Leu Tyr Glu Val Glu Thr Arg Ala Leu Arg Arg Gln Leu Ala
                   165                 170                 175
   Glu Arg Ser Ser Thr Gly Gln Gly Gly Ile Ser Pro Gly Ala Pro Arg
               180                 185                 190
   Ser Arg Pro Pro Val Ser Ser Phe Ser Gly Leu Pro Ser Leu Pro Ser
           195                 200                 205
   Ile Pro Gly Ile His Pro Arg Ala Pro Ser Pro Pro Arg Ala Thr Ser
       210                 215                 220
   Thr Pro Gly Asn Ile Pro Trp Ser Leu Gly Asp Asp Asn Pro Pro Ser
   225                 230                 235                 240
   Ser Ser Phe Pro Gly Pro Ser Gln Pro Arg Val Ser Phe His Pro Gly
                   245                 250                 255
   Asn Pro Phe Val Glu Glu Glu Gly His Arg Pro Arg Ser Gln Ser Arg
               260                 265                 270
   Glu Arg Arg Arg Glu Ile Leu Pro Ala Pro Val Pro Ser Ala Pro Pro
           275                 280                 285
   Met Ile Gln Tyr Ile Pro Val Pro Pro Pro Pro Pro Ile Gly Thr Val
       290                 295                 300
   Ile Pro Ile Gln His Ile Arg Ser Val Thr Gly Glu Pro Pro Arg Asn
   305                 310                 315                 320
   Pro Arg Glu Ile Pro Ile Trp Leu Gly Arg Asn Ala Pro Ala Ile Asp
                   325                 330                 335
   Gly Val Phe Pro Val Thr Thr Pro Asp Leu Arg Cys Arg Ile Ile Asn
               340                 345                 350
   Ala Ile Leu Gly Gly Asn Ile Gly Leu Ser Leu Thr Pro Gly Asp Cys
           355                 360                 365
   Leu Thr Trp Asp Ser Ala Val Ala Thr Leu Phe Ile Arg Thr His Gly
       370                 375                 380
   Thr Phe Pro Met His Gln Leu Gly Asn Val Ile Lys Gly Ile Val Asp
   385                 390                 395                 400
   Gln Glu Gly Val Ala Thr Ala Tyr Thr Leu Gly Met Met Leu Ser Gly
                   405                 410                 415
   Gln Asn Tyr Gln Leu Val Ser Gly Ile Ile Arg Gly Tyr Leu Pro Gly
               420                 425                 430
   Gln Ala Val Val Thr Ala Leu Gln Gln Arg Leu Asp Gln Glu Ile Asp
   435 440 445
   Asp Gln Thr Arg Ala Glu Thr Phe Ile Gln His Leu Asn Ala Val Tyr
   450 455 460
   Glu Ile Leu Gly Leu Asn Ala Arg Gly Gln Ser Ile Arg Ala Ser Val
   465 470 475 480
   Thr Pro Gln Pro Arg Pro Ser Arg Gly Arg Gly Arg Gly Gln Asn Thr
   485 490 495
   Ser Arg Pro Ser Gln Gly Pro Ala Asn Ser Gly Arg Gly Arg Gln Arg
   500 505 510
   Pro Ala Ser Gly Gln Ser Asn Arg Gly Ser Ser Thr Gln Asn Gln Asn
   515 520 525
   Gln Asp Asn Leu Asn Gln Gly Gly Tyr Asn Leu Arg Pro Arg Thr Tyr
   530 535 540
   Gln Pro Gln Arg Tyr Gly Gly Gly Arg Gly Arg Arg Trp Asn Asp Asn
   545 550 555 560
   Thr Asn Asn Gln Glu Ser Arg Pro Ser Asp Gln Gly Ser Gln Thr Pro
   565 570 575
   Arg Pro Asn Gln Ala Gly Ser Gly Val Arg Gly Asn Gln Ser Gln Thr
   580 585 590
   Pro Arg Pro Ala Ala Gly Arg Gly Gly Arg Gly Asn His Asn Arg Asn
   595 600 605
   Gln Arg Ser Ser Gly Ala Gly Asp Ser Arg Ala Val Asn Thr Val Thr
   610 615 620
   Gln Ser Ala Thr Ser Ser Thr Asp Glu Ser Ser Ser Ala Val Thr Ala
   625 630 635 640
   Ala Ser Gly Gly Asp Gln Arg Asp Ser Gly Gly Gly Gly Ser Gly Gly
   645 650 655
   Gly Gly Ser Gly Gly Gly Ala Ser Ser Arg Gly Val Gln Val Glu Thr
   660 665 670
   Ile Ser Pro Gly Asp Gly Arg Thr Phe Pro Lys Arg Gly Gln Thr Cys
   675 680 685
   Val Val His Tyr Thr Gly Met Leu Glu Asp Gly Lys Lys Phe Asp Ser
   690 695 700
   Ser Arg Asp Arg Asn Lys Pro Phe Lys Phe Met Leu Gly Lys Gln Glu
   705                 710                 715                 720
   Val Ile Arg Gly Trp Glu Glu Gly Val Ala Gln Met Ser Val Gly Gln
                   725                 730                 735
   Arg Ala Lys Leu Thr Ile Ser Pro Asp Tyr Ala Tyr Gly Ala Thr Gly
               740                 745                 750
   His Pro Gly Ile Ile Pro Pro His Ala Thr Leu Val Phe Asp Val Glu
           755                 760                 765
   Leu Leu Lys Leu Glu Thr Ser
       770                 775
<210> 16
   <211> 2328
   <212> DNA
   <213> Artificial Sequence
<220>
   <223> polynucleotide encoding for a fusion protein of human foamy virus derived capsid protein and a rapamycin-binding heterodimerizable protein domain
<400> 16
   atggccagcg gcagcaacgt ggaggaatac gagctggacg tggaggccct ggtggtgatc      60
   ctgcgggacc ggaacatccc ccggaacccc ctgcacggcg aggtgatcgg cctgcggctg     120
   accgaggggt ggtggggcca gatcgagcgg ttccagatgg tgcggctgat cctgcaggac     180
   gacgacaacg agcccctgca gcggcccaga tacgaggtga tccagagggc cgtgaacccc     240
   cacaccatgt tcatgatcag cggccctctg gccgagctgc agctggcctt ccaggacctg     300
   gacctgcccg agggccccct gagattcggc cccctggcca acggccacta cgtgcagggc     360
   gacccctaca gcagcagcta ccggcccgtg accatggccg agaccgccca gatgacccgg     420
   gacgagctgg aagatgtgct gaacacccag agcgagatcg agatccagat gatcaacctg     480
   ctggaactgt acgaggtgga gaccagggcc ctgcggaggc agctggccga gcggagcagc     540
   acaggccagg gcggcatcag ccctggcgcc cctagaagca gaccccccgt gagcagcttc      600
   agcggcctgc ccagcctgcc tagcatcccc ggcatccacc ccagagcccc cagccccccc     660
   agagccacct ccacccccgg caacatccct tggagcctgg gcgacgacaa ccccccctcc    720
   agcagcttcc ctggccccag ccagcccaga gtgagcttcc accccggcaa tcccttcgtg     780
   gaggaagagg gccaccggcc cagaagccag agccgggagc ggcggagaga gatcctgcct     840
   gcccccgtgc ctagcgcccc tcccatgatc cagtacatcc ccgtgccccc tccccctccc    900
   atcggcaccg tgatccccat ccagcacatc agaagcgtga ccggcgagcc ccccagaaac     960
   cccagagaga tccccatctg gctgggccgg aacgccccag ccatcgacgg cgtgttcccc 1020
   gtgaccaccc ccgacctgcg gtgccggatc atcaacgcca tcctgggcgg caacatcggc 1080
   ctgagcctga cccctggcga ctgcctgacc tgggacagcg ccgtggccac cctgttcatc 1140
   cggacccacg gcaccttccc catgcaccag ctgggcaacg tgatcaaggg catcgtggac 1200
   caggaaggcg tcgccaccgc ctacaccctg ggcatgatgc tgtccggcca gaactaccag 1260
   ctggtgtccg gcatcatccg gggctacctg cccggacagg ccgtggtgac cgccctgcag 1320
   cagcggctgg atcaggaaat cgacgaccag accagagccg agaccttcat ccagcacctg 1380
   aacgccgtgt acgagatcct gggcctgaat gccaggggcc agagcatccg ggccagcgtg 1440
   accccccagc ccaggcccag ccggggcaga ggcagaggcc agaacacctc ccggcccagc 1500
   cagggacccg ccaacagcgg cagaggccgg cagagacccg ccagcggcca gtccaaccgg 1560
   ggcagcagca cccagaacca gaaccaggac aacctgaacc agggcggcta caacctgcgg 1620
   cccaggacct accagcccca gagatatggc ggcggacggg gcaggcggtg gaacgacaac 1680
   accaacaacc aggaaagccg gcccagcgac cagggcagcc agacccccag gcccaaccag 1740
   gccggctccg gcgtgagggg caaccagagc cagacacccc ggcctgccgc cggaagaggc 1800
   ggcaggggca atcacaaccg gaaccagaga agcagcggag ccggcgacag cagagccgtg 1860
   aacaccgtga cccagagcgc cacctccagc accgatgaaa gcagcagcgc cgtgaccgcc 1920
   gcctctggcg gcgaccagcg ggactccgga ggcggcggca gcggcggggg cgggagcggc 1980
   ggcggggcta gctctagagg agtgcaggtg gaaaccatct ccccaggaga cgggcgcacc 2040
   ttccccaagc gcggccagac ctgcgtggtg cactacaccg ggatgcttga agatggaaag 2100
   aaatttgatt cctcccggga cagaaacaag ccctttaagt ttatgctagg caagcaggag 2160
   gtgatccgag gctgggaaga aggggttgcc cagatgagtg tgggtcagag agccaaactg 2220
   actatatctc cagattatgc ctatggtgcc actgggcacc caggcatcat cccaccacat 2280
   gccactctcg tcttcgatgt ggagcttcta aaactggaaa ctagttag 2328
<210> 17
   <211> 647
   <212> PRT
   <213> Simian foamy virus
<400> 17
   Met Ala Ala Ile Glu Gly Asp Leu Asp Val Gln Ala Leu Ala Asn Leu
   1               5                   10                  15
   Phe Asn Asp Leu Gly Ile Asn Arg Asn Pro Arg His Arg Glu Val Ile
               20                  25                  30
   Ala Leu Arg Met Thr Gly Gly Trp Trp Gly Pro Ala Thr Arg Tyr Asn
           35                  40                  45
   Leu Val Ser Leu Leu Leu Gln Asp Asp Gln Gly Gln Pro Leu Pro Gln
       50                  55                  60
   Pro Arg Trp Arg Ala Glu Gly Arg Ala Ala Asn Pro Ala Val Met Phe
   65                  70                  75                  80
   Thr Leu Glu Ala Pro Trp Gln Asp Leu Arg Leu Ala Phe Asp Asn Ile
                   85                  90                  95
   Asp Val Gly Glu Gly Thr Leu Arg Phe Gly Pro Leu Ala Asn Gly Asn
               100                 105                 110
   Tyr Ile Pro Gly Asp Glu Phe Ser Leu Glu Phe Leu Pro Pro Ala Met
           115                 120                 125
   Gln Glu Ile Thr Gln Met Gln Arg Asp Glu Leu Glu Glu Val Leu Asp
       130                 135                 140
   Val Val Gly Gln Ile Thr Met Gln Met Asn Asp Leu Ile Gly Met Gln
   145                 150                 155                 160
   Asp Ala Gln Ile Arg Gly Leu Glu Gly Gln Leu Arg Gly Leu Arg Gly
                   165                 170                 175
   Asn Leu Pro Val Ala Gly Thr Pro Pro Pro Pro Pro Pro Ser Leu Asp
               180                 185                 190
   Leu Gln Pro Ala Ala Ala Ser Ser Pro Tyr Val Ala Pro Ala Pro Ser
           195                 200                 205
   Ala Pro Ala Ala Ser Ala Ala Ala Ala Asp Leu Gly Trp Phe Ala Gly
       210                 215                 220
   Gly Pro Gly Pro Gly Ser Leu Asp Pro Arg Leu Ala Arg Val Ala Tyr
   225                 230                 235                 240
   Asn Pro Phe Leu Pro Gly Pro Ser Asp Gly Ser Gly Ala Ala Pro Ala
                   245                 250                 255
   Gln Pro Ser Ala Pro Pro Val Ala Ser Pro Leu Pro Ser Leu Leu Pro
               260                 265                 270
   Ala Gln Pro Met Gln Pro Val Ile Gln Tyr Val His Pro Pro Pro Ile
           275                 280                 285
   Asn Pro Ala Gln Gln Val Ile Pro Ile Gln His Ile Arg Ala Val Thr
       290                 295                 300
   Gly Asn Ala Pro Ser Asn Pro Arg Glu Ile Pro Met Trp Ile Gly Arg
   305 310 315 320
   Asn Ala Ser Ala Ile Glu Gly Val Phe Pro Ile Pro Thr Ser Asp Ile
   325 330 335
   Arg Ser Arg Val Ile Asn Ala Leu Leu Gly Arg Gln Leu Gly Leu Asn
   340 345 350
   Leu Asp Pro Gln His Cys Ile Thr Trp Ala Ser Ala Ile Ala Thr Leu
   355 360 365
   Tyr Val Arg Thr His Gly Ser Tyr Pro Leu His Gln Leu Ala Glu Val
   370 375 380
   Leu Arg Arg Val Ser Asn Ser Glu Gly Ala Ala Ala Ala Trp Gln Leu
   385 390 395 400
   Gly Met Met Leu Thr Asn Gln Asp Tyr Asn Leu Val Trp Gly Met Val
   405 410 415
   Arg Pro Leu Leu Pro Gly Gln Ala Val Val Thr Ala Met Gln His Arg
   420 425 430
   Leu Asp Gln Glu Val Ser Asp Ala Ala Arg Ile Val Ser Phe Val Asn
   435 440 445
   His Leu Asn Ala Val Tyr Glu Leu Leu Gly Leu Asn Ala Arg Gly Gln
   450 455 460
   Asn Leu Arg Val Ser Thr Gly Gly Gln Thr Thr Ala Arg Thr Ser Ala
   465 470 475 480
   Gly Arg Gly Ala Arg Gly Arg Arg Ser Gln Gln Gly Thr Pro Gly Arg
   485 490 495
   Gln Ser Ser Gly Gln Ala Pro Pro Gln Gly Arg Arg Ser Ser Gln Gly
   500 505 510
   Gln Gln Pro Arg Gln Ser Glu Ser Gly Asp Gln Asn Asn Gln Arg Gln
   515 520 525
   Leu Gln Gly Gly Asn Asn Arg Gly Gly Tyr Asn Leu Arg Pro Arg Thr
   530 535 540
   Tyr Gln Pro Gln Arg Tyr Gly Gly Gly Arg Gly Arg Arg Trp Asn Asp
   545 550 555 560
   Gln Thr Ala Arg Ala Asp Asn Gln Gln Arg Ser Gln Ser Gln Gln Pro
   565 570 575
   Gln Ser Glu Ala Arg Gly Glu Gln Ser Arg Thr Ser Gly Ala Gly Arg
   580 585 590
   Glu Gln Gly Gly Arg Gly Asn Gln Asn Arg Asn Gln Arg Ser Ala Gly
   595 600 605
   Glu Asn Thr Asp Arg Ser Val Asn Thr Val Thr Ala Thr Ser Ala Ser
   610 615 620
   Ile Ser Ala Ser Gly Gln Asn Gly Ser Ser Thr Thr Pro Pro Ala Ser
   625 630 635 640
   Gly Ser Gly Asn Gln Gly Asn
   645
<210> 18
   <211> 1944
   <212> DNA
   <213> Simian foamy virus
<400> 18
   atggcagcaa tagaaggtga tttagatgtc caagctttag ccaatctatt taatgatctg        60
   ggaattaaca ggaatcccag acatagagaa gtaattgccc tccgaatgac tggaggatgg       120
   tggggaccag ctactagata taatctagta tccttgttgt tacaagatga ccagggacaa       180
   cctcttccac aacctaggtg gagagctgaa ggtcgggctg ctaatccagc agtaatgttt       240
   actctagaag ctccctggca agatcttagg ttagcctttg ataatataga tgtaggagaa       300
   ggaactttac gatttgggcc actagccaat ggtaattata taccgggaga tgaattttcc       360
   ctagaatttc ttccacctgc aatgcaagaa atcactcaga tgcaacgaga tgaattagaa       420
   gaagtattgg atgtagtagg tcaaataaca atgcaaatga atgatttaat aggaatgcaa       480
   gatgcacaaa ttagaggtct tgaaggacaa ttgagagggc tacgaggcaa tttaccagtt       540
   gctggtactc cacctccacc accacctagt ttagacttac agcctgctgc tgcctcttca       600
   ccttatgtag caccagcacc ctcagcccca gctgcttctg cggctgctgc ggacctagga       660
   tggtttgctg gaggccctgg gccaggatcc ttagacccaa gactggccag ggtagcttat       720
   aatccatttt tgcctgggcc aagtgatggt tctggggcag ccccggctca gcctagtgct       780
   cctccagtgg cttcaccctt accatcacta ttaccagccc agccaatgca gccagtaatt       840
   caatatgtgc atccgcctcc tataaatcca gcccaacagg ttataccaat acaacatata       900
   cgagcagtaa ctggaaatgc tccctcaaat ccacgagaaa ttcctatgtg gattggaaga       960
   aatgcttcag caatagaagg agtattcccc atacctacat ctgatataag aagtagggta      1020
   attaatgcct tacttggaag acaacttgga ctaaatttag atccccagca ttgtataacc      1080
   tgggcctcag caatagcaac tctctatgta agaacccatg gttcttaccc tctccatcaa 1140
   cttgctgaag tcttgagaag agtctctaat tcagaaggag ctgctgctgc ctggcaactt 1200
   ggaatgatgt taacaaatca agattataat cttgtatggg gaatggttag acccttacta 1260
   ccagggcaag ctgtagttac tgcaatgcag caccgactgg accaagaagt cagcgatgct 1320
   gccagaatag tttcttttgt caatcatctg aatgctgtat atgaactctt aggcttaaat 1380
   gctcgaggac aaaaccttag agtatccact gggggacaaa ctacggctag gacctctgct 1440
   ggaagaggtg ctcggggccg tagaagtcaa caaggaaccc caggaagaca atcctctggg 1500
   caagcaccac cacaaggtag aagaagttcc caaggacaac agcctagaca gtctgagtct 1560
   ggtgatcaga ataatcaaag acagttacaa ggagggaata atcgaggagg atacaatttg 1620
   agaccacgca cctaccaacc gcagagatac gggggcggac gaggccgccg atggaatgat 1680
   cagaccgcaa gagcagataa tcaacaacgt tcccagtctc aacaaccgca gtcagaggct 1740
   cgtggcgagc agtcacgaac atctggtgcg gggcgtgaac aaggaggaag agggaaccaa 1800
   aaccgaaacc aacgatccgc tggtgaaaac acagacagat ctgtgaatac agtgacagct 1860
   acatccgcct cgatctctgc ttcaggtcaa aatggatcct ctacaactcc tccagcctct 1920
   ggaagcggaa atcaagggaa ctaa 1944
<210> 19
   <211> 643
   <212> PRT
   <213> Simian foamy virus
<400> 19
<210> 20
   <211> 1932
   <212> DNA
   <213> Simian foamy virus
<400> 20
   atgggtgatc acaacttgaa tgttcaagaa ctcttaaacc ttttccagaa tctgggtatt        60
   cccagacaac caaaccatag agaagtcata ggacttcgaa tgttaggagg ctggtggggt       120
   ccaggcaccc gttatattct ggtttctatc tttttacaag atgattctgg acaacctttg       180
   caacaacctc gatggagacc cgagggtaga cctgttaatc ctttagtaca caataccata       240
   gaagcccctt ggggggaatt aagacaagca tttgaagacc tagatgtggc ggaaggaact       300
   ttaaggtttg gtcctctagc taatgggaat tggattcctg gagatgaata ttccatggaa       360
   ttccaacccc cattagccca ggaaattgct caaatgcaga gggatgaatt agaagaaatt       420
   ttagatataa caggacaaat ttgtgcacaa gtcatagatt tagtagatat gcaagatgca       480
   caaatcaggg gtttggaaag acgcatacaa gatagactag gtttgaggga taacttacca       540
   gttgccggaa tacaagcacc accatctagt ccaattggtc aacctattgc atcatcttca       600
   cttcagccca ttccaggatc tagctcatca ccagcagatc tagatgggat ctggacacca       660
   aggcaaatag accctagatt gtctagggta gcctataacc cctttttgcc aggatctagt       720
   gatgggtcag ggggatcaat cccggttcag ccgagtgctc ctccagcggt tcttccatcc        780
   ttaccctcgc tacctgctcc agtgtcacag ccaataattc agtatgttgc tcaacctccc       840
   gttcctgctc ctcaagctat tcctattcaa catattcgag cagtgacagg aaacacacca       900
   actaatccaa gagacatacc tatgtggctt ggaagacact cggcagccat agaaggagtt       960
   tttcctatga ctacacctga tcttcgttgc cgagtggtca acgccttaat aggaggcagt      1020
   cttggacttt ctttagaacc tattcattgt gttaattggg cagctgttgt agctgcctta      1080
   tatgtaagaa cacatggatc atatccaata catgaattgg ctaatgttct acgggcagtt      1140
   gttacccaag aaggagtagc cacaggattt cagctcggga ttatgttatc taatcaagat      1200
   tataatctcg tatggggaat tttacgtccc cttttgcctg gacaagctgt agttacagct      1260
   atgcagcaaa gactggatca agaagttaat gatgctgcta gaattacctc cttcaatgga      1320
   catttgaatg atatatatca actcctaggg ttgaatgccc gaggccaaag cattgctagg      1380
   gcccaaagtg cttcgacctc tgggaattct gcctctgcag gaagaggaag acgggggcag      1440
   agaactcaac agcaagctgg ccgtcaacaa caacagcaaa caagaagaac taaccaggga      1500
   aatcagggac aaagagataa taatcaaaga cagtcttctg gaggtaatca aggacagaga      1560
   ggacagggag gatatgactt aagacctaga acgtatcagc cacaaagata cggaggagga     1620
   cgaggaagaa gatggaacga taaccagcaa cagcaacaag cacagccagg cagatcatct     1680
   gatcaacctc gttcccagag tcagcaaccg caaccagagg ctcgtggcga tcagtcacga     1740
   acatctggtg ctgggcgcgg ccaacaagga agagggaatc aaaaccgaaa tcaacgccgg     1800
   gctgatgcaa acaatactcg gaatgtggat actgtgacag caaccacaac ttcctcctcc     1860
   acggctagtt caggtcaaaa tggatcctct acaactcctc cagcctctgg aagcagaaat     1920
   caaggggact aa                                                          1932
<210> 21
   <211> 653
   <212> PRT
   <213> Simian foamy virus
<400> 21
   Met Ala Ser Gly Ser Asn Val Glu Glu Tyr Glu Leu Asp Val Glu Ala
   1               5                   10                  15
   Leu Val Val Ile Leu Arg Asp Arg Asn Ile Gly Arg Asn Pro Leu His
               20                  25                  30
   Gly Glu Ile Ile Gly Leu Arg Leu Thr Glu Gly Trp Trp Gly Gln Leu
           35                  40                  45
   Glu Arg Phe Gln Met Val Arg Leu Ile Leu Gln Asp Glu Asp Asn Glu
       50                  55                  60
   Pro Leu Gln Arg Pro Arg His Glu Ile Ile Pro Arg Ala Val Asn Pro
   65                  70                  75                  80
   His Thr Met Phe Val Leu Ser Gly Pro Leu Ala Glu Leu Gln Leu Ala
                   85                  90                  95
   Phe Gln Asp Leu Asp Leu Pro Glu Gly Pro Leu Arg Phe Gly Pro Leu
               100                 105                 110
   Ala Asn Gly His Tyr Val Glu Gly Asp Pro Tyr Ser Arg Ser Tyr Arg
           115                 120                 125
   Pro Val Thr Met Ala Glu Thr Ala Gln Met Thr Arg Asp Glu Leu Glu
       130                 135                 140
   Asp Thr Leu Asn Thr Gln Ser Glu Ile Glu Ile Gln Met Ile Asn Leu
   145                 150                 155                 160
   Leu Glu Leu Tyr Glu Val Glu Thr Arg Ala Leu Arg Arg Gln Leu Ala
                   165                 170                 175
   Glu Arg Ser Ser Ile Gly Gln Gly Gly Ile Ser Pro Gly Ala Ser His
               180                 185                 190
   Ser Arg Pro Pro Val Ser Ser Phe Ser Gly Leu Pro Ser Leu Pro Ala
           195                 200                 205
   Ile Pro Gly Ile His Thr Arg Ala Pro Ser Pro Pro Arg Ala Thr Ser
       210                 215                 220
   Thr Pro Gly Asn Ile Pro Arg Ser Leu Gly Asp Asp Asn Met Pro Ser
   225                 230                 235                 240
   Ser Ser Phe Ala Gly Pro Ser Gln Pro Arg Val Ser Phe His Pro Gly
                   245                 250                 255
   Asn Pro Phe Ala Glu Ala Glu Gly His Arg Pro Arg Ser Gln Ser Arg
               260                 265                 270
   Glu Arg Arg Arg Asp Ile Pro Ser Ala Pro Val Ile Ser Ala Pro Val
           275                 280                 285
   Pro Ser Ala Pro Pro Met Ile Gln Tyr Ile Pro Val Pro Pro Pro Pro
       290                 295                 300
   Pro Val Gly Ala Val Ile Pro Ile Gln His Ile Arg Ser Val Thr Gly
   305                 310                 315                 320
   Glu Pro Pro Arg Asn Pro Arg Glu Ile Pro Ile Trp Leu Gly Arg Asn
                   325                 330                 335
   Ala Pro Ala Ile Asp Gly Val Phe Pro Thr Thr Thr Pro Asp Leu Arg
               340                 345                 350
   Cys Arg Ile Ile Asn Ala Leu Leu Gly Gly Asn Leu Gly Leu Ser Leu
           355                 360                 365
   Thr Pro Gly Asp Cys Ile Thr Trp Asp Ser Ala Val Ala Thr Leu Phe
       370                 375                 380
   Ile Arg Thr Tyr Gly Gln Tyr Pro Leu His Gln Leu Gly Asn Val Leu
   385                 390                 395                 400
   Lys Gly Ile Ala Asp Gln Glu Gly Val Ala Thr Ala Tyr Thr Leu Gly
                   405                 410                 415
   Met Met Leu Ser Gly Gln Asn Tyr Gln Leu Val Ser Gly Ile Ile Arg
               420                 425                 430
   Gly Tyr Leu Pro Gly Gln Ala Val Val Thr Ala Met Gln Gln Arg Leu
           435                 440                 445
   Asp Gln Glu Ile Asp Asp Gln Thr Arg Ala Glu Thr Phe Ile Gln His
       450                 455                 460
   Leu Asn Ala Val Tyr Glu Ile Leu Gly Leu Asn Ala Arg Gly Gln Ser
   465                 470                 475                 480
   Ile Arg Ala Ser Val Thr Pro Gln Pro Arg Pro Ser Arg Gly Arg Gly
                   485                 490                 495
   Arg Gly Gln Ser Ala Pro Glu Pro Ser Gln Gly Pro Val Asn Ser Gly
               500                 505                 510
   Arg Gly Arg Gln Cys Pro Ala Pro Gly Gln Asn Asp Arg Gly Ser Asn
           515                 520                 525
   Ile Gln Asn Gln Gly Gln Glu Asn Ser Ser Gln Gly Gly Tyr Asn Leu
       530                 535                 540
   Arg Ser Arg Thr Tyr Gln Pro Gln Arg Tyr Gly Gly Gly Arg Gly Arg
   545                 550                 555                 560
   Arg Trp Asn Glu Asn Thr Asn Asn Ser Glu Thr Arg Pro Thr Glu Gln
                   565                 570                 575
   Ser Pro Gln Thr Pro Arg Pro Ile Gln Ala Gly Ser Gly Val Arg Gly
               580                 585                 590
   Asn Gln Ser Gln Thr Tyr Lys Pro Ala Ala Gly Arg Gly Gly Arg Gly
           595                 600                 605
   Asn Gln Asn Arg Asn Gln Arg Ser Ser Gly Ala Gly Asp Ser Arg Ala
       610                 615                 620
   Val Asn Thr Val Thr Gln Ser Ala Thr Ser Ser Thr Asp Glu Ser Ser
   625                 630                 635                 640
   Ser Thr Thr Thr Ala Ala Pro Ser Gly Gly Gln Gly Asn
                   645                 650
<210> 22
   <211> 1962
   <212> DNA
   <213> Simian foamy virus
<400> 22
   atggcttcag gaagtaatgt tgaagaatat gaacttgatg ttgaagctct ggttgtaatt        60
   ttaagagata gaaatatagg gagaaatcct ttacatggag aaattatagg tcttcgccta       120
   actgaaggat ggtggggaca attagagagg tttcaaatgg tgcggctaat attacaagat      180
   gaggataatg agcctttaca aagaccaaga catgagataa taccgcgagc agtaaatcct      240
   catactatgt tcgtgttatc aggaccattg gctgaacttc aattagcttt tcaggactta      300
   gatttgcctg aaggaccttt aaggtttgga ccactagcaa acggccatta tgttgaaggg      360
   gatccatata gtagatcata tagaccagta actatggccg aaacagctca gatgactaga      420
   gatgaattag aagatactct taatactcaa agtgaaatag aaattcaaat gataaattta      480
   ttggaattgt atgaagtcga aacaagggct ctgcgaagac aactagcaga aagatctagt      540
   atagggcaag gaggaatatc cccaggagct tctcattcac gaccaccagt aagtagcttt      600
   tcagggttac ctagcttacc tgcaatacct ggcatacata ccagagcacc ctcacctcca      660
   cgagcaactt ccactccagg aaatattcct cggagtttgg gggatgataa tatgccttca      720
   tctagttttg ctggaccctc tcaacctcga gtctctttcc atccgggaaa tcctttcgct      780
   gaagccgagg gccatagacc aagatctcag tccagagaaa gaagaagaga tataccctca      840
   gctcctgtaa tatctgctcc cgtcccatca gcacctccta tgattcagta tatacctgtg      900
   ccacctccac caccagtcgg cgcagttatt cctattcagc atataagatc tgttacagga      960
   gagcctccaa ggaatcctag ggaaatacct atttggctgg gacgaaatgc tccagccata     1020
   gatggagtat ttcctactac tacaccagat ttaaggtgta gaattattaa tgctttgttg     1080
   ggaggaaatt taggactatc tctaactccc ggagattgta ttacatggga ctcagcagtc     1140
   gctactctgt tcattagaac atatggacaa tatccattac atcagcttgg aaatgtgtta     1200
   aaaggaatag ctgatcaaga aggagtagca acagcatata ctctagggat gatgctttct     1260
   ggacaaaact atcaactagt ttcaggaatt atcagagggt atttgcctgg tcaagcagtg     1320
   gtgactgcca tgcaacagcg tctagatcaa gaaatagatg accaaacaag agcagagact     1380
   tttattcaac atttaaatgc tgtgtatgaa attctaggcc tcaatgccag aggacaaagc     1440
   atacgtgcct cagtgactcc tcaaccccga ccatcaagag gtagaggtag aggtcaaagt     1500
   gctcctgagc cctctcaagg accagtaaat agcgggcgcg gacgtcagtg ccctgcccct     1560
   ggtcaaaacg atagaggatc taacattcaa aatcaaggcc aagagaattc aagtcaagga     1620
   ggatataatc ttcgatcccg tacttaccaa cctcaaaggt acggaggagg acgaggacga     1680
   agatggaatg aaaatactaa caattcagaa accagaccga ctgaacaaag tcctcaaacc     1740
   ccaagaccaa ttcaggcagg ttctggagtg cgtggcaatc agtcacaaac ttacaagcca     1800
   gctgctggtc gcggaggaag aggcaatcaa aaccgaaacc aacgatcatc cggtgctggt     1860
   gactcacgcg cagttaatac tgtaacacaa agtgccacgt cctccacaga tgaatcctct     1920
   tcaactacta cagccgctcc cagcggaggt caagggaact aa                        1962
<210> 23
   <211> 644
   <212> PRT
   <213> Simian foamy virus
<400> 23
   Met Ala Ser Asn Ser Asn Val Glu Glu Tyr Leu Leu Asp Val Glu Ala
   1               5                   10                  15
   Leu Val Asp Ile Leu Arg Glu Arg His Ile Pro Arg Asn Pro Met His
               20                  25                  30
   Gln Glu Ile Ile Gly Leu Arg Met Thr Glu Gly Trp Trp Gly Gln Met
           35                  40                  45
   Glu Arg Phe Gln Met Thr Arg Leu Ile Leu Gln Asp Asp Asp Gly Glu
       50                  55                  60
   Pro Leu Gln Gln Pro Arg Tyr Glu Val Val Pro Arg Ala Val Asn Pro
   65                  70                  75                  80
   Arg Thr Gln Phe Val Ile Ser Gly Thr Leu Ala Glu Leu Arg Leu Ala
                   85                  90                  95
   Phe Leu Glu Leu Asp Leu Pro Glu Gly Ala Leu Arg Tyr Gly Pro Leu
               100                 105                 110
   Ala Asn Gly His Tyr Ile Gln Gly Asp Pro Tyr Ser Leu Ser Tyr Arg
           115                 120                 125
   Pro Val Thr Met Ala Glu Thr Ala His Met Thr Arg Asp Glu Leu Glu
       130                 135                 140
   Asp Val Leu Asn Val Gln Ser Glu Ile Gly Ile Gln Leu Ile Asn Ile
   145                 150                 155                 160
   Leu Glu Leu Gln Glu Val Glu Val Arg Ala Leu Arg Arg Gln Leu Ala
                   165                 170                 175
   Glu Asn Ser Ser Val Arg Arg Gly Gly Met Ser Pro Gly Ala Pro Ile
               180                 185                 190
   Pro Arg Pro Pro Val Ser Ser Tyr Ala Gly Leu Pro Ser Leu Pro Pro
           195                 200                 205
   Ala Pro Gly Ile Phe Pro Ser Ala Pro Ser Pro Pro Gln Ala Ser Ser
       210                 215                 220
   Thr Pro Gly Gln Arg Pro Trp Ser Leu Gly Asn Asp Leu Asp Gln Ser
   225                 230                 235                 240
   Val Ile Pro Gly Pro Ser Gln Pro Arg Val Ser Phe His Pro Ser Asn
   245 250 255
   Pro Phe Gln Ile Asp Ser Val Pro Arg Pro Arg Ser Ala Ser Arg Glu
   260 265 270
   Arg Pro Ala Val Glu Thr Gln Pro Ser Ala Pro Pro Leu Ile Gln Tyr
   275 280 285
   Val Pro Val Pro Pro Pro Val Phe Pro Pro Ser Val Asn Thr Val Ile
   290 295 300
   Pro Ile Gln His Ile Arg Ala Val Thr Gly Glu Pro Pro Arg Asn Pro
   305 310 315 320
   Arg Glu Ile Pro Ile Trp Leu Gly Arg Asn Ala Pro Ala Ile Glu Gly
   325 330 335
   Val Phe Pro Val Asn Ser Pro Glu Val Arg Cys Arg Val Ile Asn Ala
   340 345 350
   Ile Ile Gly Gly Asn Leu Gly Leu Ala Leu Thr Pro Thr Glu Cys Ala
   355 360 365
   Thr Trp Asp Ser Ala Val Ala Thr Leu Phe Ile Arg Thr His Gly Thr
   370 375 380
   Tyr Pro Met His Gln Leu Gly Asn Val Ile Lys Gly Ile Val Asp Gln
   385 390 395 400
   Glu Gly Val Ala Thr Gly Tyr Thr Leu Gly Met Met Leu Ser Gly Gln
   405 410 415
   Asn Phe Pro Leu Val Tyr Gly Ile Ile Arg Gly Phe Leu Pro Gly Gln
   420 425 430
   Ala Val Val Thr Ala Ile Gln Gln Arg Leu Asp Gln Glu Val Asp Asp
   435 440 445
   Gln Thr Arg Ser Asp Thr Phe Ile Gln His Pro Asn Ala Val Tyr Glu
   450 455 460
   Ile Leu Gly Leu Asn Ala Arg Gly Gln Ser Ile Arg Thr Gly Ala Asn
   465 470 475 480
   Ser Thr Pro Arg Pro Thr Arg Gly Arg Gly Arg Gly Ile Ser Arg Gln
   485 490 495
   Ser Gln Ser Ala Pro Gly Ala Gly Arg Gly Arg Gln Arg Ser Asp Gly
               500                 505                 510
   Asn Leu Gln Asp Asn Asn Pro Ser Ser Gln Asn Gln Gly Gln Ser Asn
           515                 520                 525
   Ser Asn Gln Arg Gly Tyr Asn Leu Arg Pro Arg Thr Tyr Gln Pro Ala
       530                 535                 540
   Arg Tyr Gly Gly Gly Arg Gly Arg Arg Trp Ser Asp Asn Thr Gln Thr
   545                 550                 555                 560
   Asn Arg Gln Thr Glu Ser Ser Thr Gln Asn Pro Arg Gln Ala Gln Thr
                   565                 570                 575
   Gly Gln Gly Val Arg Gly Asp Gln Ser Arg Ala Pro Arg Ser Gly Ala
               580                 585                 590
   Ser Arg Gly Gly Arg Gly Asn Gln Asn Arg Asn Gln Arg Pro Ser Gly
           595                 600                 605
   Ser Gly Asp Ser His Thr Val Asn Thr Val Thr Gln Ala Val Thr Ser
       610                 615                 620
   Ser Thr Thr Asp Glu Ser Ser Thr Val Ala Ser Ala Pro Thr Ser Gly
   625                 630                 635                 640
   Ser Gln Gly Glu
<210> 24
   <211> 1935
   <212> DNA
   <213> Simian foamy virus
<400> 24
   atggcctcta atagtaatgt tgaagaatat ctacttgatg ttgaagctct ggttgatata      60
   ttaagagaaa gacatatccc tcgaaatcct atgcatcaag aaatcatagg ccttcgtatg     120
   accgaaggat ggtggggaca aatggaaagg tttcaaatga cccggttaat attacaggat     180
   gacgacgggg aacctttaca gcaaccacgt tatgaagtag ttcccagagc tgtgaatcct     240
   agaacacagt ttgtaatatc tgggactttg gccgagttga gattggcctt tctggagtta     300
   gatttgcctg aaggagctct taggtacggg ccccttgcta atggacatta catacaagga     360
   gatccatata gtctttcata tagacctgtt actatggctg agactgctca tatgactaga     420
   gatgagctag aagatgtact aaacgtacag agtgagatag gaatacaatt aattaatatt     480
   ttagagttac aagaagtaga agttagagcc ttgagaaggc aattggcaga aaactcttct     540
   gtaagacgag ggggcatgtc cccaggagca cctataccac gaccacctgt tagcagttat     600
   gcagggttac caagtttgcc accagcgcca ggaatttttc ctagtgctcc ttctcctcca 660
   caagcatctt ccacaccagg acagagaccc tggtcattgg ggaatgattt agatcaatca 720
   gtgatccctg gaccgtctca acctagagtc tcttttcatc ccagtaatcc ctttcaaata 780
   gactctgtac caagacctcg gtcagcgtct agagaacgac cagctgtgga aacccaacca 840
   agtgctcctc cgctgattca atatgtgcca gtgccgcccc ctgtatttcc tccttctgta 900
   aacacagtta taccgattca gcacatacga gctgttactg gagagccacc acggaatccg 960
   cgagaaatcc caatatggct aggacgaaat gccccagcca ttgagggagt ttttcctgtg 1020
   aattcccctg aagttcgatg tcgtgtgatt aatgctatta ttgggggcaa tcttggcttg 1080
   gcactgacac ccactgaatg tgctacttgg gattctgccg ttgctactct atttataagg 1140
   acacatggaa cttatcctat gcatcaatta ggaaatgtaa ttaaaggaat agtggatcaa 1200
   gaaggagtag caacaggata tactttagga atgatgttat cggggcaaaa ttttccccta 1260
   gtatatggta ttattagggg atttcttcca ggacaggcag tagtgacagc tatacaacaa 1320
   cgtcttgatc aagaagtaga tgaccaaaca agatctgaca cttttataca acatccgaat 1380
   gctgtctatg aaattctagg ccttaatgca cgtggtcaga gcattaggac tggcgctaat 1440
   tctacgcctc gtcccacaag aggaagagga cgtgggataa gcagacaatc ccaaagtgct 1500
   cctggtgcag gccgaggacg gcaaaggtca gacggaaatc tacaagataa taatccctcg 1560
   agtcaaaatc aagggcaatc taatagcaat caaagaggat ataatctacg tccccgtact 1620
   taccagccgg caagatacgg aggaggacgt ggccgaaggt ggagtgataa tactcaaact 1680
   aatagacaga ctgaatctag tactcagaat cctaggcaag cccaaacggg tcaaggagta 1740
   cgtggcgatc agtcacgagc tccaagatct ggggcaagcc gaggaggaag aggaaatcaa 1800
   aatcgaaacc aaagaccgtc aggctctggt gattcacaca ctgtcaacac agtaacccaa 1860
   gctgtcacgt cctccacgac tgatgaatcc tctacagttg cttcagcccc tacaagcgga 1920
   agtcaagggg aataa 1935
<210> 25
   <211> 624
   <212> PRT
   <213> Simian foamy virus
<400> 25
   Met Ala Ala Gln Asn Phe Glu Leu Asp Val Gln Glu Leu Leu Asn Leu
   1               5                   10                  15
   Phe Gln Asp Asn Gly Val Thr Arg Asn Pro Arg His Leu Glu Thr Ile
               20                  25                  30
   Gly Leu Arg Met Leu Gly Gly Trp Trp Gly Glu Gln Glu Arg Tyr Gln
           35                  40                  45
   Ser Ala Arg Ile Ile Leu Gln Asp Asp Asp Gly Glu Pro Leu Gln Val
       50                  55                  60
   Pro Arg Trp Glu Glu Val Leu Arg Pro Val Asn Pro Leu Ala His Phe
   65                  70                  75                  80
   Val Ile Ser Ala Pro Trp Asp Gln Leu Arg Arg Ala Phe His Asp Leu
                   85                  90                  95
   Asp Val Gly Asn Gly Ala Leu Arg Phe Gly Pro Leu Ala Asn Gly Asn
               100                 105                 110
   Tyr Ile Pro Glu Asp Pro Tyr Ser Thr Ser Tyr Arg Pro Val Asn Pro
           115                 120                 125
   Gln Glu Met Ala Gln Met Gln Arg Asp Glu Leu Glu Glu Val Leu Glu
       130                 135                 140
   Val Gln Gly Glu Ile Glu Leu Gln Met Ile Asp Leu Ile Glu Met Gln
   145                 150                 155                 160
   Thr Ile Glu Ile Arg Gly Leu Arg Gln Leu Val Asn Glu Leu Gln Arg
                   165                 170                 175
   Glu Arg Asp Ser Gly Arg Gly Ala Ser Ile Pro Gly Ala Ser Ser Ser
               180                 185                 190
   Pro Pro Pro Gln Ser Tyr Val Gly Leu Pro Gly Met Pro Ser Val Ser
           195                 200                 205
   Gly Leu Ser Ser Leu Gln Pro Glu Ala Ser Ser Thr Pro Gly Gly Arg
       210                 215                 220
   Ala Pro Arg Val Ser Phe His Pro Ser Asn Pro Phe Val Arg Pro Pro
   225                 230                 235                 240
   Ser Pro Glu Arg Pro Arg Ala Arg Ser Ser Glu Arg Ile Pro Leu Pro
                   245                 250                 255
   Leu Gln Pro Pro Val Ile Gln Tyr Val Pro Val Pro Gln Pro Val Leu
               260                 265                 270
   Ala Pro Ala Pro Ala Pro Ser Pro Val Ile Pro Ile Gln His Ile Arg
           275                 280                 285
   Ala Val Thr Gly Glu Val Pro Asn Asn Pro Arg Asp Ile Pro Met Trp
       290                 295                 300
   Ile Gly Arg Asn Ala Pro Ala Ile Glu Gly Val Tyr Pro Val Thr Thr
   305                 310                 315                 320
   Pro Asp Leu Arg Ala Arg Ile Ile Asn Ala Leu Ile Gly Gly Lys Ser
                   325                 330                 335
   Gly Ile His Leu Thr Ala Pro Glu Ala Val Thr Trp Ala Ser Ala Val
               340                 345                 350
   Ala Ala Ile Phe Thr Arg Thr His Gly Ser Phe Pro Met His Asn Leu
           355                 360                 365
   Ser Ala Ile Leu Thr Gly Ile Ala Asn Gly Glu Gly Val Glu Ser Ala
       370                 375                 380
   Tyr Asn Leu Gly Met Met Leu Ser Asn Gly Asp Phe Asn Leu Val Tyr
   385                 390                 395                 400
   Gly Ile Val Arg Gly Leu Leu Pro Gly Gln Ala Ala Val Ala Tyr Met
                   405                 410                 415
   Gln Gln Arg Leu Asp Ala Glu Pro Ser Asp Ala Leu Arg Ala Gln Asn
               420                 425                 430
   Phe Ile Gln His Leu His Leu Val Tyr Glu Ile Leu Gly Leu Asn His
           435                 440                 445
   Arg Gly Gln Ser Ile Arg Thr Ser Leu Pro Thr Ser Thr Arg Pro Arg
       450                 455                 460
   Gly Gln Gly Arg Gly Arg Gly Gln Gly Gln Gly Ser Ile Pro Ser Thr
   465                 470                 475                 480
   Pro Arg Arg Pro Gln Ser Gly Arg Gly Leu Ser Thr Pro Asn Arg Gly
                   485                 490                 495
   Ser Asn Asn Ala Asn Asn Asn Thr Gln Ser Asn Val Gln Thr Glu Thr
               500                 505                 510
   Pro Arg Arg Ser Phe Gly Gly Tyr Asn Leu Arg Pro Asn Thr Phe Arg
           515                 520                 525
   Pro Gln Arg Tyr Gly Gly Gly Gln Gly Gln Arg Arg Asp Ser Gln Pro
       530                 535                 540
   Asp Arg Arg Ser Gln Gly Ser Ser Gln Asn Asn Arg Pro Ser Ala Pro
   545                 550                 555                 560
   Leu Glu Ser Arg Gly Glu Gln Ser Arg Gly Pro Gly Gly Gly Gly Arg
   565 570 575
   Ala Gly Gly Arg Arg Asn Gln Asn Arg Asn Ser Gly Gln Gly Asn Glu
   580 585 590
   Ser Ser Ser His Ala Val Asn Ala Val Thr Gln Ser Ala Val Thr Glu
   595 600 605
   Gln Gln Asn Glu Ser Pro Thr Pro Pro Pro Thr Ser Gly Gly Arg Ser
   610 615 620
<210> 26
   <211> 1875
   <212> DNA
   <213> Simian foamy virus
<400> 26
   atggcagcac aaaactttga actagatgtt caagaattat taaatttatt ccaagataat      60
   ggagttacta ggaatccccg tcacctagag accattggtc tacgaatgct aggtggatgg     120
   tggggagaac aagaaagata tcagtctgct agaataattt tacaagatga tgatggtgaa     180
   cctttgcaag ttccccgatg ggaagaagtt ttaaggcctg taaatccttt agcacatttt     240
   gttatatctg caccatggga tcagttacgc agagcattcc atgatttgga tgtgggtaat     300
   ggagccctaa gattcggacc attggctaat ggaaattata ttccagaaga tccctatagt     360
   acttcctata ggcctgtaaa cccacaggaa atggcacaaa tgcagagaga tgaattagag     420
   gaagtcttgg aggtacaagg agaaattgaa ttacaaatga tagatcttat agaaatgcaa     480
   accattgaga ttaggggatt gaggcaatta gttaatgaat tacagagaga aagggatagt     540
   ggacgaggag catctattcc aggagcatcc tcatcacctc caccacaatc atatgtagga     600
   ttgcctggaa tgccttctgt aagtggactt tctagtttgc aaccagaagc aagctctacc     660
   ccaggaggta gagctccaag ggtgtcattc catcctagta atcctttcgt acgacctcct     720
   tcacccgaga ggcctagagc aagatccagt gaaaggattc ctttgccact tcaaccgcca     780
   gtaatacaat atgtgcccgt gccacaacca gttttggccc cagcacctgc tccaagtcca     840
   gtaattccca tacagcatat tagagctgtt actggtgaag ttcctaacaa tcctcgagat     900
   attcccatgt ggataggacg aaatgcccca gcaatagaag gagtatatcc tgtcactaca     960
   cctgatttac gggcccgaat aataaatgcc ctcataggag ggaaaagtgg aatacattta    1020
   acagctccgg aagcagtaac ctgggcctct gcagttgcag ccatcttcac taggacacat    1080
   ggtagttttc ctatgcacaa tctctctgca atattaactg gaatagctaa tggagaaggc    1140
   gtggaatctg cttataattt aggaatgatg ctctctaatg gagattttaa tttggtttat    1200
   ggaatagtca gaggactgtt gcctggacag gcagcagttg cttatatgca gcaaaggctt    1260
   gatgctgaac ctagtgatgc tctcagagca caaaacttca tacagcattt acatttggta    1320
   tatgaaattc ttggacttaa tcacagggga caaagcataa ggacttcctt gcccacatca 1380
   accagaccta gaggacaggg acgtggccgt gggcaaggac aaggcagtat tccttctaca 1440
   cccagacgcc cacagtctgg aagaggacta tctactccta acagaggaag caataatgct 1500
   aataataaca cacagtctaa tgttcaaact gaaacccctc gaagaagttt cgggggatac 1560
   aatttgagac ccaatacatt tcgtccccag agatacggag gaggacaagg acaacgacgt 1620
   gactcccaac cagaccggcg gtcacagggc agttcccaga ataataggcc gtctgcgccc 1680
   cttgagtcac ggggcgagca gtcccgtggc ccaggcggag gcggacgtgc tggaggaaga 1740
   aggaaccaaa accgcaattc aggtcaagga aatgagtcat catcacacgc tgtaaatgca 1800
   gtcactcaat ctgctgtaac agaacaacaa aatgagtctc caacacctcc tccaacctct 1860
   ggaggcagaa gttaa 1875
<210> 27
   <211> 613
   <212> PRT
   <213> Simian foamy virus
<400> 27
   Met Ala Gln Gly Asp Pro Ile Ile Asn Gln Gly Asp Leu Asn Val Val
   1 5 10 15
   Glu Leu Thr His Leu Leu Arg Glu Asn Gly Leu Pro Gly Asn Pro Arg
   20 25 30
   Asn Gly Gln Thr Tyr Ala Ile Ala Met Thr Glu Gly Trp Trp Gly Asp
   35 40 45
   His Pro Arg Tyr Arg Ile Ile Arg Val Val Phe Gln Asp Thr Ser Gly
   50 55 60
   Asn Pro Leu Ser Gln Pro Gln Trp Met Asp Glu Asp Arg Gln Met Asn
   65 70 75 80
   Pro Leu Gln Asp Pro Val Val Gly Cys Thr Phe Asn Gln Ala Ser Arg
   85 90 95
   Val Phe Asp Gln Ile Asp Ile Gly Glu Gly Pro Ser Arg Phe Gly Pro
   100 105 110
   Leu Ala Asp Gly Met Phe Leu Ile Thr Asp Asn Ala Trp Met Asp Phe
   115 120 125
   Val Pro Leu Ser Ala Met Glu Ile Thr Asp Ile Asn Asn Glu Arg Thr
   130 135 140
   Leu Arg Asp His Leu Thr Phe Cys Cys Thr Leu Leu Glu Asn Met Met
   145                 150                 155                 160
   Ala Asp Ile Ile Val Thr Gln Arg Glu Asn Arg Asp Leu Asn Thr Thr
                   165                 170                 175
   Ile Asn Asn Leu Arg Ala Thr Ile Pro Val Ala Ser Gln Ala Leu Pro
               180                 185                 190
   His Ser Ser Leu Pro Ala Thr Pro Met Ala Ala Ser Thr Pro Gly Gly
           195                 200                 205
   Pro Ser Gly Leu Pro Pro Ser Thr Phe Asn Ile Gly Ala Val Gly Pro
       210                 215                 220
   Ser Tyr Gln Pro Pro Ala Gln Leu Pro Ser Ala Pro Pro Ile Ala Leu
   225                 230                 235                 240
   Pro Val Ile Pro Ser Ala Pro Leu Ala Pro Ala Val Pro Leu Pro Gln
                   245                 250                 255
   Ser Gln Pro Pro Gln Pro Met Tyr Asn Pro Tyr Met Met Ala Gly Asn
               260                 265                 270
   Leu Gln Gly Asn Val Leu Pro Ile Ser Gln Ile Lys Ala Val Ile Gly
           275                 280                 285
   Glu Thr Pro Thr Asp His Lys Ala Val Pro Leu Trp Val Ala Lys His
       290                 295                 300
   Ala Ala Ala Ile Glu Gly Val Phe Pro Thr Gly Ser Pro Glu Val Arg
   305                 310                 315                 320
   Cys Arg Val Leu Asn Ser Leu Leu Thr Gly His Gly Gly Met Met Leu
                   325                 330                 335
   His Pro Val Asp Cys Val Ser Trp Thr Asn Ala Ala Ser Val Leu Phe
               340                 345                 350
   Gln Arg Val His Gly Val Val Pro Leu His Gln Leu Pro Lys Thr Leu
           355                 360                 365
   Glu Glu Val Ala Lys Thr Glu Gly Leu Leu Val Ala Tyr Asn Ile Gly
       370                 375                 380
   Met Thr Phe Thr Ser Asn Asn Phe Asp Leu Ile Trp Gly Ile Ile Arg
   385                 390                 395                 400
   Pro Leu Val Pro Gly Gln Ala Ala Val Ala Met Leu Gln Gly Tyr Leu
                   405                 410                 415
   Asp Gln Tyr Pro Arg Pro Gln Asp Lys Ile Glu His Phe Pro Arg Phe
               420                 425                 430
   Leu Arg Arg Thr Phe Glu Val Leu Gly Leu Asn Phe Leu Gly Gln Ser
           435                 440                 445
   Ile Arg Ser Thr Gln Ala Pro Ser Arg Pro Asn Ser Ala Asn Arg Gly
       450                 455                 460
   Thr Asn Thr Ala Arg Gly Arg Gly Gly Asn Arg Gly Arg Gly Arg Gly
   465                 470                 475                 480
   Arg Gly Val Pro Pro Gly Pro Pro Pro Gly Arg Gly Thr Ala Thr Pro
                   485                 490                 495
   Gln Ala Ala Gly Thr Ser Arg Ser Ser Ser Gly Ser Phe Gly Glu Gln
               500                 505                 510
   Pro Arg Tyr Asn Leu Arg Pro Gln Val Asn Arg Pro Ser Arg Tyr Gly
           515                 520                 525
   Gly Gly Pro Asn Pro Ala Arg Asn Asn Pro Glu Pro Gln Gly Gly Asp
       530                 535                 540
   Thr Ser Ser Thr Pro Arg Gly Gln Gly Glu Arg Ser Gly Arg Gln Arg
   545                 550                 555                 560
   Asn Asn Asn Ala Arg Gln Asn Gln Gln Pro Pro Arg Arg Pro Asp Arg
                   565                 570                 575
   Thr Val Asn Thr Val Arg Thr Ala Gln Pro Asn Pro Thr Val Ala Ser
               580                 585                 590
   Gly Ser Gln Asp Asp Asn Ser Thr Pro Pro Ser Thr Ala Arg Gly Ser
           595                 600                 605
   Glu Glu Asn Arg Ala
       610
<210> 28
   <211> 1842
   <212> DNA
   <213> Simian foamy virus
<400> 28
   atggctcaag gggatcctat aataaatcaa ggagatttaa atgtagttga attaactcat      60
   ttattaagag aaaatggttt accaggaaat cctcgaaacg gccagactta tgccatcgct     120
   atgactgaag gctggtgggg agatcatcct aggtatcgaa taataagagt ggtttttcag 180
   gatactagtg gaaatcctct atcacaacca caatggatgg atgaagatag acagatgaat 240
   ccattacaag acccagtagt aggttgtact tttaatcagg cttcaagagt gtttgatcag 300
   atagacatag gagaagggcc atcacgtttt gggcctttgg cagatggaat gttccttatc 360
   accgataatg catggatgga tttcgttccg ctgtcagcta tggaaataac agatattaac 420
   aatgaaagaa ctctcagaga tcatcttaca ttttgttgta ccctcctaga gaatatgatg 480
   gcagacataa ttgttacaca aagagaaaat agggatttaa atactacaat aaataacttg 540
   agagcaacta taccggtagc tagtcaagca ctcccacact ctagtctacc cgctacacca 600
   atggctgcct ctacacctgg aggaccatct gggttgccac catcaacctt taacatagga 660
   gcagtaggtc cttcctatca gccaccagct caattaccat cagcgcctcc cattgcacta 720
   ccagtaatac catcagcacc attggctcca gctgttcctt tgcctcaatc tcagccacca 780
   cagccaatgt ataatcctta tatgatggca ggcaacctac aagggaatgt cttgccaata 840
   tcacaaataa aggctgtaat tggggaaacc cccacagatc ataaagcagt accgttatgg 900
   gtggcaaaac atgcagcagc aattgagggg gtatttccta ccgggagtcc tgaagttcga 960
   tgtagagtat tgaactcctt gttaacagga catggaggta tgatgctaca tccagtggat 1020
   tgtgtctcct ggaccaatgc tgcaagtgtc ttatttcaga gagtacacgg tgtagttcca 1080
   ctacaccagc tacctaagac tttagaagaa gtcgccaaaa ctgaaggctt acttgtagca 1140
   tataacatag gaatgacatt tacaagtaat aattttgatt taatttgggg tattattaga 1200
   cccctagtac ctgggcaagc agcagtagct atgcttcagg gatatttaga ccaatatcct 1260
   cgacctcaag acaaaattga acatttccct cggtttttga gacgtacctt tgaagtactt 1320
   ggcctaaact tcttaggaca aagcatacgt tctacccagg ctccttctcg tcctaattca 1380
   gcaaatcgag gaaccaatac tgcaagaggg cgaggaggaa accgaggacg agggagagga 1440
   cgaggagttc caccaggacc acctcctggt agaggaacag caacgccgca agcagcagga 1500
   acatcaagat catcgtcagg ctcttttgga gaacagccaa ggtacaatct aaggcctcag 1560
   gttaacaggc cctccagata cggaggagga cctaatcctg cccgaaataa tcctgaacct 1620
   caaggaggtg acactagctc aactccacga ggccagggag aacgatcggg acggcagaga 1680
   aacaacaacg cccggcaaaa tcaacaacct ccacgaagac ctgatcgaac agttaacacc 1740
   gtaagaacag ctcaaccaaa tcctacagtt gcttccggaa gtcaagatga caactccacc 1800
   cctccttcaa ctgcccgtgg aagtgaagaa aacagagctt aa 1842
<210> 29
   <211> 623
   <212> PRT
   <213> Simian foamy virus
<400> 29
   Met Ala Gln Gly Asn Gln Pro Glu His Ile Asp Ile Gly His Leu Thr
   1               5                   10                  15
   Ile Leu Leu Arg Glu Asn Gly Leu Pro Thr Asn Pro Pro His Gly Thr
               20                  25                  30
   Glu Tyr Ala Val Arg Met Thr Glu Gly Trp Trp Gly Asp Tyr Ala Arg
           35                  40                  45
   Phe Thr Val Ile Arg Val Ala Phe Gln Asp Asn Gln Gly Asn Pro Leu
       50                  55                  60
   Pro Arg Pro Glu Trp Glu Tyr Ile Asn Arg Asp Val Arg Pro Leu Asn
   65                  70                  75                  80
   Asp Ser Ile Ile Gly Cys Thr Phe Arg Gln Ala Gln Gln Ala Phe Asn
                   85                  90                  95
   Asn Ile Asp Leu Ala Arg Thr Pro Ser Arg Tyr Gly Pro Leu Ser Asn
               100                 105                 110
   Gly Leu Phe Leu Ile Thr Asp Pro Glu Trp Leu Thr Phe Asn Pro Leu
           115                 120                 125
   Ser Ala Val Glu Ile Ala Asp Leu Asp Asp Gln Glu Ile Arg Asp Phe
       130                 135                 140
   Leu Ala His Ala Thr Ile Val Leu Asp Asn Val Met Ala Asp Val Val
   145                 150                 155                 160
   Ile Gln Gln Arg Glu Ile Glu Asp Leu Thr Lys Thr Asn Leu Arg Leu
                   165                 170                 175
   Arg Asn Ala Leu Ala Ser Ser Gly Thr Tyr Gln Pro Ile Gly Met Pro
               180                 185                 190
   Ile Ala Gln Ser Ser Pro Ala Gly Pro Ser Ala Ser Val Ala Gly Pro
           195                 200                 205
   His Met Pro Ala Met Pro Ser Val Pro Tyr Ile Ser Ala Ile Pro Ser
       210                 215                 220
   Ala Pro Pro Ala Pro Ile Pro Val Ala Pro Ala Ala Ala Pro Pro Leu
   225                 230                 235                 240
   Gly Pro Leu Pro Ser Ala Pro Ala Pro Pro Val Leu Pro Val Ile Gln
                   245                 250                 255
   Pro Asn Thr Phe Gly Thr Asp Gly Asn Gly Tyr Thr Ala Val Gly Pro
               260                 265                 270
   Val Pro Val Leu Pro Ile Ala Gln Ile Lys Ala Val Ile Gly Glu Ala
           275                 280                 285
   Pro Thr Asp Ala Arg Gln Ile Pro Leu Trp Val Ala Lys His Ala Ala
       290                 295                 300
   Ala Ile Glu Gly Thr Phe Pro Thr Gly Ser Ala Asp Val Arg Cys Arg
   305                 310                 315                 320
   Val Leu Asn Ala Leu Leu Thr Ser His Gly Gly Met Thr Leu Ser Pro
                   325                 330                 335
   Asn Glu Cys Gly Thr Trp Ser Leu Ala Ala Ala Ala Leu Tyr Gln Arg
               340                 345                 350
   Ile Tyr Gly Val Ile Pro Leu His Asp Leu Pro His Thr Met Ala Glu
           355                 360                 365
   Val Ala Arg Arg Glu Gly Ile Leu Val Ala Phe Asn Met Gly Met Thr
       370                 375                 380
   Phe Thr Asn Asn Ser Phe Asp Val Val Trp Gly Ile Ile Arg Pro Leu
   385                 390                 395                 400
   Leu Pro Gly Gln Ala Ser Val Ala Met Leu Gln Gly Tyr Leu Asp Gln
                   405                 410                 415
   Tyr Gln Gly Gln Gln Gln Lys Ala Gln Ala Phe Pro Thr Leu Leu Arg
               420                 425                 430
   Arg Thr Phe Glu Thr Leu Gly Leu Asn Tyr Leu Gly Gln Ser Ile Arg
           435                 440                 445
   Ser Asn Gln Pro Ala Arg Thr Ser Gly Ser Val Ala Gly Ser Ile Thr
       450                 455                 460
   Gln Gly Arg Gly Arg Gly Gln Pro Arg Ser Arg Pro Pro Asn Arg Ala
   465                 470                 475                 480
   Ser Asn Arg Ser Asn Asn Pro Pro Arg Pro Pro Pro Pro Arg Ser Gln
                   485                 490                 495
   Glu Gln Gln Pro Ser Pro Ala Gly Arg Gly Ser Ser Ser Ser Ser Ala
               500                 505                 510
   Pro Pro Gln Arg Ser Gly Tyr Asn Leu Arg Gln Gln Ile Asn Arg Pro
               515                 520                 525
   Gln Arg Leu Gly Gly Gly Pro Asn Pro Tyr Arg Asn Leu Glu Asn Arg
       530                 535                 540
   Asn Ile Pro Arg Ser Glu Thr Val Pro Ser Ser Arg Arg Ala Glu Thr
   545                 550                 555                 560
   Ser Glu Thr Asn Arg Pro Asn Gln Ala Arg Gly Gly Gln Gln Gly Ser
                   565                 570                 575
   Ser Asn Arg Asn Gln Thr Arg Ser Ile Asn Thr Val Arg Ala Ser Asp
               580                 585                 590
   Ser Gln Val Ile Pro Ser Ala Pro Pro Ala Glu Thr Asn Pro Ala Ala
           595                 600                 605
   Thr Ala Val Thr Pro Asn Gly Pro Asn Gln His Ser Gly Arg Asn
       610                 615                 620
<210> 30
   <211> 1872
   <212> DNA
   <213> Simian foamy virus
<400> 30
   atggctcaag ggaatcaacc tgaacatatt gatattggtc atttaactat tctcttacga      60
   gaaaatggct tacccaccaa tcctcctcat gggacagagt atgctgtccg aatgactgaa     120
   ggatggtggg gggattatgc aagatttact gtgattagag tggcttttca agataatcaa     180
   ggaaatcctt tacctcgacc agaatgggaa tatattaata gagatgttag gccattaaat     240
   gattcaatta taggttgtac ttttagacaa gctcaacaag cttttaataa tatagattta     300
   gcaagaactc catccagata tgggccttta tctaatggtc tctttttaat aacagatccg     360
   gaatggttaa cattcaatcc tttatctgca gttgagatag cagatttaga tgatcaggaa     420
   ataagggact ttttggccca tgccactata gttttagaca atgtgatggc tgatgtagtt     480
   atacaacaaa gagaaataga agacttaacc aaaacaaatt taagacttag aaatgccttg     540
   gctagttcag gtacttatca gcctatagga atgcctatag ctcagtcttc tccagcaggc     600
   ccctctgcgt cagtagcagg accccatatg ccggctatgc catcagtacc ttatatttct     660
   gctatacctt cggctcctcc agcaccaatt ccagttgctc ctgctgcagc tcctcctttg     720
   ggacctttgc catcggctcc agctccacct gttctgcctg ttatacagcc aaatacattt     780
   ggaactgatg gcaatggata tactgcagta ggacctgtgc cagttttacc cattgctcaa     840
   ataaaagctg taatagggga agcccctact gatgctagac aaatcccctt gtgggtagct     900
   aaacatgctg cagctattga aggtactttc cctacaggct cagccgatgt gaggtgtaga     960
   gttttaaatg ccctattaac cagtcatggg ggtatgacct tgtctccaaa tgaatgcggc     1020
   acatggtccc tagctgcagc tgctctttat caaagaatat atggggtcat accattacat     1080
   gacttaccac atactatggc ggaagtggct agaagagaag gtattttagt cgctttcaat     1140
   atgggtatga cctttactaa taattccttt gatgtagtgt gggggatcat aagacctctt     1200
   ttgcctggac aagcctcagt ggctatgctt caaggttatt tagatcagta ccagggccaa     1260
   caacaaaaag cacaagcttt tccgacgttg ttacgacgaa cttttgaaac tttaggtcta     1320
   aattatttag gacaaagtat tagatctaat caaccagcaa gaacctctgg ttctgttgca     1380
   ggtagtataa cccaaggacg aggaagaggt caaccgagat ctagaccacc gaatagagcc     1440
   tctaataggt ctaataaccc gcctagacct cctccccctc gctctcaaga acagcaaccc     1500
   tctccagcag ggcgtggttc ctctagttca tcagcacctc ctcagaggtc aggttataat     1560
   ttaaggcagc aaattaatag acctcaaaga cttggaggcg gtccaaaccc gtatagaaac     1620
   ctagaaaata gaaatattcc aagaagtgaa acggtacctt cctctcggcg agctgagact     1680
   tcagaaacaa accggccaaa tcaagcaagg ggcggacagc aaggaagttc aaatcgaaac     1740
   caaacacgct ctattaacac tgtcagagca agtgactcac aagtcatacc ttccgctccg     1800
   ccggctgaaa caaacccagc tgctacagct gtgaccccaa atggcccaaa ccaacattcc     1860
   ggtagaaatt aa                                                          1872
<210> 31
   <211> 572
   <212> PRT
   <213> Simian foamy virus
<400> 31
   Met Ala Arg Gly Asn Leu Asp Ile Ala Arg Leu Thr Val Leu Leu Gln
   1               5                   10                  15
   Glu Asn Ser Leu Ser Gly Asn Pro Pro His Leu Thr His Tyr Val Leu
               20                  25                  30
   Arg Met Thr Glu Gly Trp Trp Gly Pro Phe Pro Arg Tyr Thr Arg Val
           35                  40                  45
   Arg Ile Ile Leu Gln Asp Glu Ser Gly Asn Pro Leu Ser Gln Pro Arg
       50                  55                  60
   Trp Glu Leu Val Asp Arg Met Phe Asn Pro Leu Arg Asp Pro Ile Leu
   65                  70                  75                  80
   Glu Thr Thr Leu Asp Glu Met Asp Arg Val Phe Asp Gly Ile Asn Leu
                   85                  90                  95
   Ser Pro Gly Thr Glu Arg Tyr Gly Pro Leu Cys Asp Gly Asn Phe Leu
               100                 105                 110
   Tyr Thr Asp Asp Ala Trp Asn Asp Phe Asn Pro Met Ser Ala Leu Glu
           115                 120                 125
   Val Ala Asp Val Glu Leu Thr Pro Ala Leu Tyr Arg Glu His Leu Asn
       130                 135                 140
   Phe Leu Ser Arg Val Ala Glu Gly Met Leu Gly Asp Ile Ile Met Leu
   145                 150                 155                 160
   Lys Gln Glu Asn Glu Asp Ala His Glu Thr Ile Thr Arg Leu Arg Glu
                   165                 170                 175
   Arg Ile Ile Asn Gln Pro Thr Ser Ala Pro Ile Ile Thr Ser Thr Pro
               180                 185                 190
   Gly Gly Val Ser Gly Leu Pro Pro Ser Val Tyr Gln Pro Thr Gly Leu
           195                 200                 205
   His Glu Thr Tyr Ile Gln Pro Ser Arg Pro Met Gln Pro Ser Ala Pro
       210                 215                 220
   Pro Leu Pro Leu Met Gly Asp Leu Gly Ser Gly Ile Leu Ser Val Leu
   225                 230                 235                 240
   Pro Ile Ser Gln Ile Arg Thr Val Ile Gly Asn Thr Pro Val Asp Pro
                   245                 250                 255
   Lys Lys Val Pro Leu Trp Ile Ala Lys Ser Ala Ser Ala Ile Glu Gly
               260                 265                 270
   Val Met Pro Thr Asn Thr Pro Asp Ile Arg Cys Arg Leu Val Asn Ala
           275                 280                 285
   Leu Leu Pro Gln His Gly Gly Leu Ile Leu Gln Pro His Glu Cys Asn
       290                 295                 300
   Ser Trp Thr Gln Ile Ala Ser Ala Leu Tyr Thr Arg Val Asn Gly Met
   305                 310                 315                 320
   Ile Pro Leu His Ala Leu Pro Gln Thr Leu Ser Gln Val Thr Lys Glu
                   325                 330                 335
   Glu Gly Ile Leu Val Ala Tyr Gln Ile Gly Met Thr Phe Thr Gly Gln
               340                 345                 350
   Asn Phe Pro Leu Thr Trp Gly Ile Leu Arg Pro Leu Leu Pro Gly Gln
           355                 360                 365
   Ala Val Val Ala Met Met Gln Gly Tyr Leu Asp Gln Tyr Pro Thr Asp
       370                 375                 380
   Asp Leu Lys Ala Val Asn Phe Ala Ser Ile Leu Arg Arg Val Phe Asp
   385                 390                 395                 400
   Ile Leu Gly Leu Asn Tyr Met Gly Gln Asn Ile Arg Asn Gln Ser Ser
                   405                 410                 415
   Pro Ser Leu Thr Val Ser Ser Ala Arg Gly Ala Thr Gly Arg Gly Arg
               420                 425                 430
   Gly Arg Ser Arg Asn Ile Gly Arg Gly Arg Gly Thr Thr Pro Asn Phe
           435                 440                 445
   Gln Gly Arg Gly Gln Pro Ala Thr Thr Pro Thr Val Thr Thr Ser Gly
       450                 455                 460
   Ser Thr Ser Ser Thr Thr Glu Ser Gln Gly Arg Asn Ser Asn Pro Asn
   465                 470                 475                 480
   Tyr Asn Phe Arg Pro Arg Val Asn Gln Pro Pro Arg Tyr Gly Gly Gly
                   485                 490                 495
   Pro Asn Pro Tyr Arg Asn Arg Glu Pro Leu Asn Asn Pro Pro Asn Arg
               500                 505                 510
   Glu Asn Thr Thr Gln Asn Asn Thr Arg Arg Pro Gly Ser Gln Gly Gln
           515                 520                 525
   Asn Arg Asn Arg Asn Gln Asn Arg Thr Val Asn Ala Val Gln Val Val
       530                 535                 540
   Pro Asn Gln Asp Asn Thr Asn Gln Pro Asn Ile Asn Pro Asn Gln Asn
   545                 550                 555                 560
   Val Ser Thr Pro Ala Ser Ala Ser Gly Gly Asn Ser
                   565                 570
<210> 32
   <211> 1719
   <212> DNA
   <213> Simian foamy virus
<400> 32
   atggctcgag gaaatctgga tattgctcga ttaactgttt tattacagga aaatagcctc 60
   tctggcaatc ctcctcattt aactcattat gtattaagaa tgactgaggg atggtggggg 120
   ccttttcccc gatataccag agtaagaata atattacaag atgaaagtgg aaaccccctc 180
   agtcaacctc gctgggaatt agtagataga atgtttaacc cattaagaga tcccatttta 240
   gaaacaactt tagatgagat ggatcgagtt tttgacggta taaacttatc cccagggact 300
   gaaagatatg ggcctttatg tgatggaaat tttttatata cagatgatgc ttggaatgat 360
   tttaatccca tgtctgctct agaagtagct gatgtagaac ttacacctgc tctatataga 420
   gaacatctaa actttttaag cagagtagct gaaggaatgc tgggtgatat aataatgcta 480
   aaacaagaaa atgaagatgc tcatgaaact attactagac taagagaaag aataataaac 540
   caacccacta gcgcccctat aattacttca acaccaggtg gcgttagtgg tttaccacca 600
   tctgtatatc aaccaacggg attacatgaa acatatatac aaccctctag acctatgcag 660
   ccttctgctc caccactacc tttaatggga gacttaggct caggaatatt gtctgtccta 720
   cctatttcac aaattagaac tgtcataggg aatacaccag tagaccctaa aaaggtaccg 780
   ttatggatag ccaagagtgc tagtgccata gaaggagtta tgcccactaa tactccagat 840
   ataagatgtc gactggtgaa tgctttacta cctcagcatg gaggtttgat tttacaacca 900
   catgagtgca actcctggac acaaatagcc tctgcccttt ataccagagt taatggaatg 960
   atacccttgc atgcattacc acagacttta tcacaagtaa ctaaagaaga aggaattcta 1020
   gtcgcctatc aaattggaat gacgtttacg ggacaaaact tccctttaac ttggggaatt 1080
   ttgcgtcccc tattaccagg acaggcagtg gtagctatga tgcaaggata tctggatcaa 1140
   tatcctacgg atgatttaaa ggcagtaaat tttgcttcca tcctaaggag agtatttgat 1200
   atattaggct taaattacat gggacaaaat attaggaatc aatcatctcc ctctcttacc 1260
   gttagttctg cccgaggagc cactggtaga gggcgaggaa gaagccgcaa tataggccga 1320
   ggcagaggca caacgccaaa tttccaaggg cgtggacagc cagcaacaac tccaacagta 1380
   accacatctg gttctacaag ttcyacaact gaaagtcagg gaagaaacag taatccaaat 1440
   tataatttca ggcctagagt aaatcaacct ccccgatatg gtggaggacc aaatccctat 1500
   aggaatcgtg agccattaaa caacccacct aatcgagaaa atactactca aaataacact 1560
   cgaagacccg gaagtcaagg gcaaaatcga aatcgaaacc aaaataggac ggttaatgcc 1620
   gtacaagttg ttcctaatca agacaataca aatcaaccta atataaatcc aaatcaaaat 1680
   gtatcaaccc cagcatcagc ttcaggtgga aattcatga 1719
<210> 33
   <211> 544
   <212> PRT
   <213> Bovine foamy virus
<400> 33
   Met Ala Leu Asn Asp Phe Asp Pro Ile Ala Leu Gln Gly Tyr Leu Pro
   1 5 10 15
   Ala Pro Arg Val Leu Gln His Asn Asp Ile Ile Ile Cys Arg Ala Thr
   20 25 30
   Ser Gly Pro Trp Gly Ile Gly Asp Arg Tyr Asn Leu Ile Arg Ile His
   35 40 45
   Leu Gln Asp Pro Ala Gly Gln Pro Leu Pro Ile Pro Gln Trp Glu Pro
   50 55 60
   Ile Pro Asn Arg Thr Ala Asn Pro Arg Thr Gln Pro Tyr Pro Val Val
   65 70 75 80
   Ser Ala Pro Met Ala Thr Leu Glu Asn Ile Leu Asn Asn Phe His Ile
   85 90 95
   Pro His Gly Val Ser Arg Tyr Gly Pro Leu Glu Gly Gly Asp Tyr Gln
   100 105 110
   Pro Gly Glu Gln Tyr Ser Gln Gly Phe Cys Pro Val Thr Gln Ala Glu
   115 120 125
   Ile Ala Leu Leu Asn Gly Gln His Leu Glu Glu Glu Ile Thr Ile Leu
   130 135 140
   Arg Glu Ile Thr His Arg Leu Met Gln Gly Val Arg Pro Pro Ala Val
   145 150 155 160
   Pro Gln Gly Pro Ala Pro Pro Pro Pro Pro Ala Gln Pro Pro Ala Pro
   165 170 175
   Leu Pro Ala Pro Pro Ile Gly Pro Pro Pro Pro Ala Ala Pro Ala Pro
   180 185 190
   Ala Pro Gly Pro Met Pro Val Pro Gln His Leu Pro Ile Thr His Ile
   195 200 205
   Arg Ala Val Ile Gly Glu Thr Pro Ala Asn Ile Arg Glu Val Pro Leu
   210 215 220
   Trp Leu Ala Arg Ala Val Pro Ala Leu Gln Gly Val Tyr Pro Val Gln
   225 230 235 240
   Asp Ala Val Met Arg Ser Arg Thr Val Asn Ala Leu Thr Val Arg His
   245 250 255
   Pro Gly Leu Ala Leu Glu Pro Leu Glu Cys Gly Ser Trp Gln Glu Cys
   260 265 270
   Leu Ala Ala Leu Trp Gln Arg Thr Phe Gly Ala Thr Ala Leu His Ala
           275                 280                 285
   Leu Gly Asp Thr Leu Gly Gln Ile Ala Asn Ser Asp Gly Ile Val Met
       290                 295                 300
   Ala Ile Glu Leu Gly Leu Leu Phe Ser Asp Asp Asn Trp Asp Leu Val
   305                 310                 315                 320
   Trp Gly Ile Cys Arg Arg Phe Leu Pro Gly Gln Ala Val Cys Val Ala
                   325                 330                 335
   Val Gln Ala Arg Leu Asp Pro Leu Pro Asp Asn Ala Thr Arg Ile Val
               340                 345                 350
   Met Ile Ser His Ile Ile Arg Asp Val Tyr Ala Ile Leu Gly Leu Asp
           355                 360                 365
   Pro Leu Gly Arg Pro Met Gln Gln Thr Leu Pro Arg Arg Asn Asn Gln
       370                 375                 380
   Pro Pro Arg Gln Gln Pro Gln Arg Arg Gln Gln Pro Arg Arg Thr Gly
   385                 390                 395                 400
   Asn Gln Glu Glu Arg Gly Gln Arg Asn Arg Gly Arg Gln Asn Ala Gln
                   405                 410                 415
   Thr Pro Arg Gln Glu Gly Asn Arg Leu Gln Asn Ser Gln Leu Pro Gly
               420                 425                 430
   Pro Arg Asp Cys Pro Asn Asn Ser Asn Gln Pro Arg Tyr Pro Leu Arg
           435                 440                 445
   Pro Asn Pro Gln Gln Pro Gln Arg Tyr Gly Gln Glu Gln Asn Arg Gly
       450                 455                 460
   Asn Asn Pro Asn Pro Tyr Arg Gln Pro Thr Pro Gly Asn Gly Asn Gln
   465                 470                 475                 480
   Asn Arg Asn Phe Ser Arg Gly Pro Ala Pro Val Asn Glu Gln Ser Arg
                   485                 490                 495
   Gly Arg Gly Arg Ser Ser Gln Gly Thr Asn Asn Thr Gly Ser Ser Ala
               500                 505                 510
   Val His Ser Val Arg Leu Thr Ser Ala Ala Pro Pro Ile Pro Pro Gln
           515                 520                 525
   Asp Ala Gly Thr Pro Pro Thr Ser Ser Gly Asn Gln Gly Gln Ser Ser
       530                 535                 540
<210> 34
   <211> 1635
   <212> DNA
   <213> Bovine foamy virus
<400> 34
   atggctctta atgacttcga ccctatagct ctccaggggt atttacccgc accacgagtt      60
   ttgcaacata atgatattat aatatgtaga gcaacctctg gaccatgggg gattggagac     120
   agatacaatc ttattaggat ccatttacaa gatcctgcag ggcaaccctt gcctattcct     180
   caatgggaac ccattcctaa tcggactgca aatcctcgga cccaacctta cccagtggtc     240
   tctgctccga tggcaactct agaaaacatc ttgaataact ttcatatacc tcatggggta     300
   tctagatatg gtcccctaga agggggtgat tatcagccag gagaacaata ttcccaggga    360
   ttttgtccag ttacacaggc tgagattgcc ctactaaatg gacaacatct ggaggaagag     420
   ataactattc taagagagat aactcataga ttgatgcaag gggtgagacc accagcggtc      480
   ccacaaggac ctgctcctcc accacctcca gcccagcctc cagctccgtt gcctgctccg     540
   cctattggac ctcctccacc tgctgctcct gcacctgctc cagggcctat gccagttcct     600
   caacaccttc caatcacaca tatacgtgct gtaatcggag aaacccccgc aaacatcaga     660
   gaagtccctc tatggttggc acgggcagta cctgccttac aaggtgttta cccggtccag     720
   gacgctgtta tgcgcagtcg aacagtgaat gctcttacag ttcggcatcc cgggctcgcc     780
   ttggaaccct tggaatgtgg gtcatggcaa gaatgtctgg cagcactatg gcaacgtact     840
   tttggagcca ctgctctcca tgcgcttgga gacacactag gacaaattgc caattcagat     900
   gggatagtga tggccataga actggggtta ttattctccg atgataattg ggatctagtc     960
   tggggtattt gtcgaagatt cctcccggga caagctgttt gtgtagccgt tcaggctagg    1020
   ctagacccac ttcccgataa tgcgacacga attgtgatga tttcacacat catacgtgat    1080
   gtctacgcca tcctagggct agatcccctg ggcagaccta tgcaacaaac tcttcccaga    1140
   cgcaacaacc aaccacctag acaacagccg caacggcgtc aacagccaag gcgtaccgga    1200
   aaccaagaag aaagaggtca gaggaatcgt ggcagacaga acgcccagac tccacgtcaa    1260
   gagggaaaca gattacaaaa ttcacaactt cctgggccac gggattgccc aaataattct    1320
   aatcaacctc gttatcccct caggcccaat cctcaacagc cacaacgata cggacaagaa    1380
   cagaaccgtg ggaacaatcc gaacccttat cgacagccaa cccctggaaa cgggaaccaa    1440
   aacaggaatt tcagccgggg tcccgccccc gtcaacgagc aaagtcgcgg acgtgggaga    1500
   agcagccaag gaacaaacaa cacaggaagt tctgcggtcc acagtgtgag attaacatct    1560
   gctgctccac ccataccccc acaagatgcc ggcactccgc ccacttcaag tggaaatcaa    1620
   gggcaatcat cttaa                                                     1635
<210> 35
   <211> 559
   <212> PRT
   <213> Equine foamy virus
<400> 35
   Met Ala Gln Asn Glu Thr Phe Asp Pro Val Ala Leu Gln Gly Tyr Tyr
   1               5                   10                  15
   Pro Ala Gly Gly Ile Leu Ala Asp Asn Asp Ile Ile Asn Ile Arg Phe
               20                  25                  30
   Thr Ser Gly Gln Trp Gly Ile Gly Asp Arg Trp Leu Gln Val Arg Leu
           35                  40                  45
   Arg Leu Val Asp Pro Asn Thr Gly Gln Pro Leu Ala Gln Pro Glu Tyr
       50                  55                  60
   Glu Asp Thr Gly Leu Pro Ala Glu Asn Arg Gly Ile Val Val Ala Val
   65                  70                  75                  80
   Ser His Asn Ala Ala Arg Asn Ile Phe Asn Asn Val Gln Pro Ala Gly
                   85                  90                  95
   Gly Pro Asn Arg His Gly Pro Leu His Asp Gly Gln Phe Gln Val Gly
               100                 105                 110
   Asp Asp Pro Ser Glu His Phe Val Pro Ile Glu Glu Asn Leu Ile Pro
           115                 120                 125
   Gln Glu Ile Val Asn Leu Gly Ala Ala Arg Arg Glu Val Arg Leu Leu
       130                 135                 140
   Arg Glu Met Cys Val Arg Leu Leu His Val Arg Arg Gln Met Met Gly
   145                 150                 155                 160
   Met Gly Met Pro Gly Ala Ile Gln Pro Gln Pro Pro Val Gly Pro Leu
                   165                 170                 175
   Pro Ala Pro Ala Gln Pro Pro Ile Pro Gly Pro Pro Val Pro Pro Pro
               180                 185                 190
   Val Pro Pro Pro Ala Pro Pro Ala Pro Val Asn Pro Pro Val Pro Pro
           195                 200                 205
   Val Gln Pro Ile His His Leu Pro Ile Thr His Ile Arg Ala Val Ile
       210                 215                 220
   Gly Glu Thr Pro Ala Gln Ile Arg Asp Val Pro Leu Trp Leu Ala Gln
   225                 230                 235                 240
   Ser Ile Pro Ala Leu Thr Gly Val Tyr Pro Ala Met Asp Ala Gly Thr
   245 250 255
   Leu Thr Arg Leu Val Asn Ala Ile Thr Ala Arg His Pro Gly Leu Ala
   260 265 270
   Leu Gly Met Asn Glu Ala Gly Ser Trp His Glu Ala Val His Leu Ile
   275 280 285
   Trp Gln Arg Thr Phe Gly Ala Thr Ala Leu His Ala Leu Ser Asp Val
   290 295 300
   Leu Lys Gly Ile Ala Gln Arg Asn Gly Val Val Met Ala Leu Glu Met
   305 310 315 320
   Gly Leu Met Phe Thr Asn Asp Asp Trp Asp Leu Thr Trp Ser Val Ile
   325 330 335
   Arg Arg Cys Leu Pro Gly Gln Ala Ser Val Val Thr Ile Gln Ala Arg
   340 345 350
   Leu Asp Ala Leu Pro Asn Asn Gln Ala Arg Ile Ile Gln Ala Gly Phe
   355 360 365
   Ile Ile Arg Glu Val Tyr Glu Val Leu Gly Leu Asp Pro Leu Gly Arg
   370 375 380
   Pro Leu Asn Phe Pro Gly Gly Leu Thr Gln Arg Asp Thr Ala Val Pro
   385 390 395 400
   Val Thr Arg Gly Arg Gly Arg Gly Arg Thr Gly Pro Arg Arg Gly Pro
   405 410 415
   Val Leu Pro Val Ser Ser Asn Gln Arg Arg Gln Glu Thr Ala Gly Gly
   420 425 430
   Asn Gln Pro Gln Thr Gln Pro Gln Gln Gln Asn Thr Phe Ser Asn Gln
   435 440 445
   Thr Asn Gln Arg Gly Asn Gln Arg Gln Trp Gln Asn Arg Gly Thr Asp
   450 455 460
   Ser Gln Arg Arg Tyr Phe Phe Arg Pro Arg Pro Ser Gln Pro Gln Arg
   465 470 475 480
   Tyr Gly Ser Asn Gln Gly Pro Asp Asn Pro Asn Pro Tyr Arg Gly Arg
   485 490 495
   Asp Ser Thr Asn Gln Ser Gly Gln Glu Arg Gln Leu Pro Gln Gln Gln
               500                 505                 510
   Gln Gly Ser Arg Arg Gly Pro Gly Arg Asn Thr Asn Ser Gly Asn Asn
           515                 520                 525
   Thr Val His Thr Val Arg Gln Val Glu Ser Ser Gln Leu Gln Gln Asn
       530                 535                 540
   Ala Ser Pro Thr Ala Ser Pro Ser Thr Asn Gln Gly Gln Gln Pro
   545                 550                 555
<210> 36
   <211> 1680
   <212> DNA
   <213> Equine foamy virus
<400> 36
   atggctcaaa acgagacttt tgatccagtg gctttacagg gatattaccc tgcaggaggt      60
   attttggccg ataatgatat tataaatata agatttacct cgggacaatg ggtataggga     120
   gatcgatggc tacaggtacg attacgtttg gttgatccaa atacaggaca accattggcc     180
   caacctgaat atgaagatac aggtttaccg gcggaaaata gaggtatagt agttgctgtc     240
   tctcataatg cagctagaaa tatttttaat aatgttcaac cagcaggggg acctaatagg     300
   catggcccac tacatgatgg tcaatttcaa gttggtgatg atccatcaga gcattttgtt     360
   cccatagagg aaaacttgat accacaagaa atagtgaatc taggagcagc aaggcgagaa     420
   gtgagactct tgagggaaat gtgtgtgcga ttattacatg ttagaaggca aatgatggga     480
   atgggaatgc caggagcaat acagccacag ccaccagtag ggcccctacc tgcaccagca     540
   caacctccta tacctggacc tcctgtgcca ccaccagtac ctcctccagc accaccggct     600
   ccagtgaatc ctccggttcc accagttcaa cctatacacc atttacctat tactcatatt     660
   agagcagtaa taggggaaac acccgctcaa ataagggacg tccccttatg gctggcacag     720
   tctataccag ccttaactgg tgtatatccc gctatggacg ctggaacttt aactcgcttg     780
   gttaatgcca tcactgctcg acatccaggt ttagcacttg gaatgaatga ggcaggaagt     840
   tggcatgaag cagttcatct catatggcag cgaacttttg gagcaactgc tttacatgcg     900
   ctaagcgatg tgttaaaagg catagcccaa aggaacggtg ttgtcatggc attagaaatg     960
   ggtcttatgt ttacaaatga tgactgggac ttgacttgga gtgtaatccg gcgatgtctt    1020
   cctggacagg ccagtgttgt aacaattcaa gctagacttg atgcgttgcc aaacaaccaa    1080
   gccaggatta tacaagctgg ttttataatc cgagaagtct atgaagtact ggggcttgat    1140
   cctcttggtc gacccctgaa cttcccaggg ggtttaacac aaagagacac cgctgttcct    1200
   gttacaagag gaagaggacg aggacgaaca ggcccccgac gaggaccagt tctgcccgtc    1260
   tcttcgaacc agcgacgaca ggaaacagct ggtggtaatc aaccgcaaac tcagccccaa    1320
   caacagaaca ctttttcgaa ccagacaaat cagcgtggaa atcaacgaca atggcaaaat    1380
   agaggaactg actcacagag aagatacttt tttagaccca gacccagcca accgcaaaga    1440
   tacggttcga atcaagggcc tgacaacccc aatccttatc gaggacgaga ctcaaccaat    1500
   cagagtggtc aagagagaca acttccccag caacaacagg gctcaagaag aggccctgga    1560
   agaaatacca atagtggaaa taacacagta catacagtga ggcaagttga atcttctcag    1620
   ctacaacaaa atgcaagccc tacagcctct ccaagtacaa atcaagggca acagccttaa    1680
<210> 37
   <211> 514
   <212> PRT
   <213> Feline foamy virus
<400> 37
   Met Ala Arg Glu Leu Asn Pro Leu Gln Leu Gln Gln Leu Tyr Ile Asn
   1               5                   10                  15
   Asn Gly Leu Gln Pro Asn Pro Gly His Gly Asp Ile Ile Ala Val Arg
               20                  25                  30
   Phe Thr Gly Gly Pro Trp Gly Pro Gly Asp Arg Trp Ala Arg Val Thr
           35                  40                  45
   Ile Arg Leu Gln Asp Asn Thr Gly Gln Pro Leu Gln Val Pro Gly Tyr
       50                  55                  60
   Asp Leu Glu Pro Gly Ile Ile Asn Leu Arg Glu Asp Ile Leu Ile Ala
   65                  70                  75                  80
   Gly Pro Tyr Asn Leu Ile Arg Thr Ala Phe Leu Asp Leu Glu Pro Ala
                   85                  90                  95
   Arg Gly Pro Glu Arg His Gly Pro Phe Gly Asp Gly Arg Leu Gln Pro
               100                 105                 110
   Gly Asp Gly Leu Ser Glu Gly Phe Gln Pro Ile Thr Asp Glu Glu Ile
           115                 120                 125
   Gln Ala Glu Val Gly Thr Ile Gly Ala Ala Arg Asn Glu Ile Arg Leu
       130                 135                 140
   Leu Arg Glu Ala Leu Gln Arg Leu Gln Ala Gly Gly Val Gly Arg Pro
   145                 150                 155                 160
   Ile Pro Gly Ala Val Leu Gln Pro Gln Pro Val Ile Gly Pro Val Ile
                   165                 170                 175
   Pro Ile Asn His Leu Arg Ser Val Ile Gly Asn Thr Pro Pro Asn Pro
               180                 185                 190
   Arg Asp Val Ala Leu Trp Leu Gly Arg Ser Thr Ala Ala Ile Glu Gly
           195                 200                 205
   Val Phe Pro Ile Val Asp Gln Val Thr Arg Met Arg Val Val Asn Ala
       210                 215                 220
   Leu Val Ala Ser His Pro Gly Leu Thr Leu Thr Glu Asn Glu Ala Gly
   225                 230                 235                 240
   Ser Trp Asn Ala Ala Ile Ser Ala Leu Trp Arg Lys Ala His Gly Ala
                   245                 250                 255
   Ala Ala Gln His Glu Leu Ala Gly Val Leu Ser Asp Ile Asn Lys Lys
               260                 265                 270
   Glu Gly Ile Gln Thr Ala Phe Asn Leu Gly Met Gln Phe Thr Asp Gly
           275                 280                 285
   Asn Trp Ser Leu Val Trp Gly Ile Ile Arg Thr Leu Leu Pro Gly Gln
       290                 295                 300
   Ala Leu Val Thr Asn Ala Gln Ser Gln Phe Asp Leu Met Gly Asp Asp
   305                 310                 315                 320
   Ile Gln Arg Ala Glu Asn Phe Pro Arg Val Ile Asn Asn Leu Tyr Thr
                   325                 330                 335
   Met Leu Gly Leu Asn Ile His Gly Gln Ser Ile Arg Pro Arg Val Gln
               340                 345                 350
   Thr Gln Pro Leu Gln Thr Arg Pro Arg Asn Pro Gly Arg Ser Gln Gln
           355                 360                 365
   Gly Gln Leu Asn Gln Pro Arg Pro Gln Asn Arg Ala Asn Gln Ser Tyr
       370                 375                 380
   Arg Pro Pro Arg Gln Gln Gln Gln His Ser Asp Val Pro Glu Gln Arg
   385                 390                 395                 400
   Asp Gln Arg Gly Pro Ser Gln Pro Pro Arg Gly Ser Gly Gly Gly Tyr
                   405                 410                 415
   Asn Phe Arg Arg Asn Pro Gln Gln Pro Gln Arg Tyr Gly Gln Gly Pro
               420                 425                 430
   Pro Gly Pro Asn Pro Tyr Arg Arg Phe Gly Asp Gly Gly Asn Pro Gln
   435 440 445
   Gln Gln Gly Pro Pro Pro Asn Arg Gly Pro Asp Gln Gly Pro Arg Pro
   450 455 460
   Gly Gly Asn Pro Arg Gly Gly Gly Arg Gly Gln Gly Pro Arg Asn Gly
   465 470 475 480
   Gly Gly Ser Ala Ala Ala Val His Thr Val Lys Ala Ser Glu Asn Glu
   485 490 495
   Thr Lys Asn Gly Ser Ala Glu Ala Val Asp Gly Gly Lys Lys Gly Gly
   500 505 510
   Lys Asp
<210> 38
   <211> 1545
   <212> DNA
   <213> Feline foamy virus
<400> 38
   atggctcgag aattaaatcc tctccaatta cagcaactgt atataaataa tggcttacaa      60
   cctaatccag gacatggaga tattattgct gtcagattta caggaggacc ttggggtcca     120
   ggtgatagat gggctagagt gacaatacga ttacaagata acacaggaca acctttacag     180
   gttcctggat atgatttgga acctgggata ataaatttga gagaggatat cttgatagca     240
   gggccatata atttaataag aactgccttt ttggacttag agcctgccag aggacctgaa     300
   aggcatggtc cctttggaga tggcagatta cagcctggag atggtttatc tgaaggattt     360
   caacctatca ctgatgaaga aatacaagca gaagtaggaa ctattggtgc tgctagaaat     420
   gagataagat tattacgaga agctttacag agattacaag ctggaggtgt gggtagacct     480
   ataccaggag cagttttaca accacaacca gtaatagggc cggtaatacc aattaatcat     540
   cttaggtcgg ttattggcaa tactccacca aatccacgag atgtcgccct atggcttgga     600
   agatctacag ccgctattga aggagtattt cccatagtgg accaagtcac tcgtatgagg     660
   gtagttaatg ccttagtagc atctcatccc ggcctaacgt tgactgagaa tgaggccggg     720
   agctggaatg ctgccatatc agctttatgg aggaaagctc acggtgctgc agctcagcat     780
   gaattggcag gagtattaag tgatattaat aaaaaggaag gcatacaaac tgcattcaac     840
   ctaggaatgc aatttacaga tggaaactgg tccttagtat ggggaataat caggactctt     900
   ttaccaggac aagccctagt aaccaatgct cagtcacaat ttgacctaat gggagatgat     960
   atacaacgag cagaaaattt ccccagggtc attaacaatc tatacactat gctgggtctc    1020
   aatatacatg ggcaaagtat tagacctcgg gtccaaacac agccactaca aactcgaccc    1080
   cggaacccgg gacgatctca acaaggtcaa ctaaatcagc cgagacccca aaatagagct    1140
   aaccaatctt atagaccccc tagacaacaa cagcaacact ctgatgttcc cgaacagaga    1200
   gatcagagag gaccgtcgca acctcctcgt ggaagtggag gaggatataa ttttagaaga    1260
   aatccgcagc agcctcagcg ctacggccaa ggaccaccag gaccaaaccc gtaccgacga    1320
   ttcggagacg gcggtaatcc tcaacagcag ggaccaccac caaaccgagg gcctgatcaa    1380
   ggacctcggc caggaggcaa tcccagagga ggaggaagag gtcaaggtcc aagaaatgga    1440
   ggaggaagcg ctgccgcagt acatacagta aaagcgtctg aaaacgaaac taaaaatgga    1500
   tctgctgaag ccgttgacgg tggaaagaaa gggggtaaag attaa                    1545
<210> 39
   <211> 511
   <212> PRT
   <213> Vesicular stomatitis virus
<400> 39
   Met Lys Cys Leu Leu Tyr Leu Ala Phe Leu Phe Ile Gly Val Asn Cys
   1               5                   10                  15
   Lys Phe Thr Ile Val Phe Pro His Asn Gln Lys Gly Asn Trp Lys Asn
               20                  25                  30
   Val Pro Ser Asn Tyr His Tyr Cys Pro Ser Ser Ser Asp Leu Asn Trp
           35                  40                  45
   His Asn Asp Leu Ile Gly Thr Ala Ile Gln Val Lys Met Pro Lys Ser
       50                  55                  60
   His Lys Ala Ile Gln Ala Asp Gly Trp Met Cys His Ala Ser Lys Trp
   65                  70                  75                  80
   Val Thr Thr Cys Asp Phe Arg Trp Tyr Gly Pro Lys Tyr Ile Thr Gln
                   85                  90                  95
   Ser Ile Arg Ser Phe Thr Pro Ser Val Glu Gln Cys Lys Glu Ser Ile
               100                 105                 110
   Glu Gln Thr Lys Gln Gly Thr Trp Leu Asn Pro Gly Phe Pro Pro Gln
           115                 120                 125
   Ser Cys Gly Tyr Ala Thr Val Thr Asp Ala Glu Ala Val Ile Val Gln
       130                 135                 140
   Val Thr Pro His His Val Leu Val Asp Glu Tyr Thr Gly Glu Trp Val
   145                 150                 155                 160
   Asp Ser Gln Phe Ile Asn Gly Lys Cys Ser Asn Tyr Ile Cys Pro Thr
                   165                 170                 175
   Val His Asn Ser Thr Thr Trp His Ser Asp Tyr Lys Val Lys Gly Leu
               180                 185                 190
   Cys Asp Ser Asn Leu Ile Ser Met Asp Ile Thr Phe Phe Ser Glu Asp
           195                 200                 205
   Gly Glu Leu Ser Ser Leu Gly Lys Glu Gly Thr Gly Phe Arg Ser Asn
       210                 215                 220
   Tyr Phe Ala Tyr Glu Thr Gly Gly Lys Ala Cys Lys Met Gln Tyr Cys
   225                 230                 235                 240
   Lys His Trp Gly Val Arg Leu Pro Ser Gly Val Trp Phe Glu Met Ala
                   245                 250                 255
   Asp Lys Asp Leu Phe Ala Ala Ala Arg Phe Pro Glu Cys Pro Glu Gly
               260                 265                 270
   Ser Ser Ile Ser Ala Pro Ser Gln Thr Ser Val Asp Val Ser Leu Ile
           275                 280                 285
   Gln Asp Val Glu Arg Ile Leu Asp Tyr Ser Leu Cys Gln Glu Thr Trp
       290                 295                 300
   Ser Lys Ile Arg Ala Gly Leu Pro Ile Ser Pro Val Asp Leu Ser Tyr
   305                 310                 315                 320
   Leu Ala Pro Lys Asn Pro Gly Thr Gly Pro Ala Phe Thr Ile Ile Asn
                   325                 330                 335
   Gly Thr Leu Lys Tyr Phe Glu Thr Arg Tyr Ile Arg Val Asp Ile Ala
               340                 345                 350
   Ala Pro Ile Leu Ser Arg Met Val Gly Met Ile Ser Gly Thr Thr Thr
           355                 360                 365
   Glu Arg Glu Leu Trp Asp Asp Trp Ala Pro Tyr Glu Asp Val Glu Ile
       370                 375                 380
   Gly Pro Asn Gly Val Leu Arg Thr Ser Ser Gly Tyr Lys Phe Pro Leu
   385                 390                 395                 400
   Tyr Met Ile Gly His Gly Met Leu Asp Ser Asp Leu His Leu Ser Ser
                   405                 410                 415
   Lys Ala Gln Val Phe Glu His Pro His Ile Gln Asp Ala Ala Ser Gln
   420 425 430
   Leu Pro Asp Asp Glu Ser Leu Phe Phe Gly Asp Thr Gly Leu Ser Lys
   435 440 445
   Asn Pro Ile Glu Leu Val Glu Gly Trp Phe Ser Ser Trp Lys Ser Ser
   450 455 460
   Ile Ala Ser Phe Phe Phe Ile Ile Gly Leu Ile Ile Gly Leu Phe Leu
   465 470 475 480
   Val Leu Arg Val Gly Ile His Leu Cys Ile Lys Leu Lys His Thr Lys
   485 490 495
   Lys Arg Gln Ile Tyr Thr Asp Ile Glu Met Asn Arg Leu Gly Lys
   500 505 510
<210> 40
   <211> 1536
   <212> DNA
   <213> Vesicular stomatitis virus
<400> 40
   atgaagtgcc ttttgtactt agccttttta ttcattgggg tgaattgcaa gttcaccata        60
   gtttttccac acaaccaaaa aggaaactgg aaaaatgttc cttctaatta ccattattgc       120
   ccgtcaagct cagatttaaa ttggcataat gacttaatag gcacagccat acaagtcaaa       180
   atgcccaaga gtcacaaggc tattcaagca gacggttgga tgtgtcatgc ttccaaatgg       240
   gtcactactt gtgatttccg ctggtatgga ccgaagtata taacacagtc catccgatcc       300
   ttcactccat ctgtagaaca atgcaaggaa agcattgaac aaacgaaaca aggaacttgg       360
   ctgaatccag gcttccctcc tcaaagttgt ggatatgcaa ctgtgacgga tgccgaagca       420
   gtgattgtcc aggtgactcc tcaccatgtg ctggttgatg aatacacagg agaatgggtt       480
   gattcacagt tcatcaacgg aaaatgcagc aattacatat gccccactgt ccataactct       540
   acaacctggc attctgacta taaggtcaaa gggctatgtg attctaacct catttccatg       600
   gacatcacct tcttctcaga ggacggagag ctatcatccc tgggaaagga gggcacaggg       660
   ttcagaagta actactttgc ttatgaaact ggaggcaagg cctgcaaaat gcaatactgc       720
   aagcattggg gagtcagact cccatcaggt gtctggttcg agatggctga taaggatctc       780
   tttgctgcag ccagattccc tgaatgccca gaagggtcaa gtatctctgc tccatctcag       840
   acctcagtgg atgtaagtct aattcaggac gttgagagga tcttggatta ttccctctgc       900
   caagaaacct ggagcaaaat cagagcgggt cttccaatct ctccagtgga tctcagctat       960
   cttgctccta aaaacccagg aaccggtcct gctttcacca taatcaatgg taccctaaaa      1020
   tactttgaga ccagatacat cagagtcgat attgctgctc caatcctctc aagaatggtc      1080
   ggaatgatca gtggaactac cacagaaagg gaactgtggg atgactgggc accatatgaa      1140
   gacgtggaaa ttggacccaa tggagttctg aggaccagtt caggatataa gtttccttta      1200
   tacatgattg gacatggtat gttggactcc gatcttcatc ttagctcaaa ggctcaggtg      1260
   ttcgaacatc ctcacattca agacgctgct tcgcaacttc ctgatgatga gagtttattt      1320
   tttggtgata ctgggctatc caaaaatcca atcgagcttg tagaaggttg gttcagtagt      1380
   tggaaaagct ctattgcctc ttttttcttt atcatagggt taatcattgg actattcttg       1440
   gttctccgag ttggtatcca tctttgcatt aaattaaagc acaccaagaa aagacagatt      1500
   tatacagaca tagagatgaa ccgacttgga aagtaa                                1536
<210> 41
   <211> 524
   <212> PRT
   <213> Rabies virus
<400> 41
   Met Val Pro Gln Ala Leu Leu Phe Val Pro Leu Leu Val Phe Pro Leu
   1               5                   10                  15
   Cys Phe Gly Lys Phe Pro Ile Tyr Thr Ile Pro Asp Lys Leu Gly Pro
               20                  25                  30
   Trp Ser Pro Ile Asp Ile His His Leu Ser Cys Pro Asn Asn Leu Val
           35                  40                  45
   Val Glu Asp Glu Gly Cys Thr Asn Leu Ser Gly Phe Ser Tyr Met Glu
       50                  55                  60
   Leu Lys Val Gly Tyr Ile Leu Ala Ile Lys Val Asn Gly Phe Thr Cys
   65                  70                  75                  80
   Thr Gly Val Val Thr Glu Ala Glu Thr Tyr Thr Asn Phe Val Gly Tyr
                   85                  90                  95
   Val Thr Thr Thr Phe Lys Arg Lys His Phe Arg Pro Thr Pro Asp Ala
               100                 105                 110
   Cys Arg Ala Ala Tyr Asn Trp Lys Met Ala Gly Asp Pro Arg Tyr Glu
           115                 120                 125
   Glu Ser Leu His Asn Pro Tyr Pro Asp Tyr Arg Trp Leu Arg Thr Val
       130                 135                 140
   Lys Thr Thr Lys Glu Ser Leu Val Ile Ile Ser Pro Ser Val Ala Asp
   145                 150                 155                 160
   Leu Asp Pro Tyr Asp Arg Ser Leu His Ser Arg Val Phe Pro Ser Gly
   165 170 175
   Lys Cys Ser Gly Val Ala Val Ser Ser Thr Tyr Cys Ser Thr Asn His
   180 185 190
   Asp Tyr Thr Ile Trp Met Pro Glu Asn Pro Arg Leu Gly Met Ser Cys
   195 200 205
   Asp Ile Phe Thr Asn Ser Arg Gly Lys Arg Ala Ser Lys Gly Ser Glu
   210 215 220
   Thr Cys Gly Phe Val Asp Glu Arg Gly Leu Tyr Lys Ser Leu Lys Gly
   225 230 235 240
   Ala Cys Lys Leu Lys Leu Cys Gly Val Leu Gly Leu Arg Leu Met Asp
   245 250 255
   Gly Thr Trp Val Ser Met Gln Thr Ser Asn Glu Thr Lys Trp Cys Pro
   260 265 270
   Pro Asp Lys Leu Val Asn Leu His Asp Phe Arg Ser Asp Glu Ile Glu
   275 280 285
   His Leu Val Val Glu Glu Leu Val Arg Lys Arg Glu Glu Cys Leu Asp
   290 295 300
   Ala Leu Glu Ser Ile Met Thr Thr Lys Ser Val Ser Phe Arg Arg Leu
   305 310 315 320
   Ser His Leu Arg Lys Leu Val Pro Gly Phe Gly Lys Ala Tyr Thr Ile
   325 330 335
   Phe Asn Lys Thr Leu Met Glu Ala Asp Ala His Tyr Lys Ser Val Arg
   340 345 350
   Thr Trp Asn Glu Ile Leu Pro Ser Lys Gly Cys Leu Arg Val Gly Gly
   355 360 365
   Arg Cys His Pro His Val Asn Gly Val Phe Phe Asn Gly Ile Ile Leu
   370 375 380
   Gly Pro Asp Gly Asn Val Leu Ile Pro Glu Met Gln Ser Ser Leu Leu
   385 390 395 400
   Gln Gln His Met Glu Leu Leu Glu Ser Ser Val Ile Pro Leu Val His
   405 410 415
   Pro Leu Ala Asp Pro Ser Thr Val Phe Lys Asp Gly Asp Glu Ala Glu
   420 425 430
   Asp Phe Val Glu Val His Leu Pro Asp Val His Asn Gln Val Ser Gly
           435                 440                 445
   Val Asp Leu Gly Leu Pro Asn Trp Gly Lys Tyr Val Leu Leu Ser Ala
       450                 455                 460
   Gly Ala Leu Thr Ala Leu Met Leu Ile Ile Phe Leu Met Thr Cys Cys
   465                 470                 475                 480
   Arg Arg Val Asn Arg Ser Glu Pro Thr Gln His Asn Leu Arg Gly Thr
                   485                 490                 495
   Gly Arg Glu Val Ser Val Thr Pro Gln Ser Gly Lys Ile Ile Ser Ser
               500                 505                 510
   Trp Glu Ser His Lys Ser Gly Gly Glu Thr Arg Leu
           515                 520
<210> 42
   <211> 1575
   <212> DNA
   <213> Rabies virus
<400> 42
   atggttcctc aggctctcct gtttgtaccc cttctggttt ttccattgtg ttttgggaaa      60
   ttccctattt acacgatacc agacaagctt ggtccctgga gtccgattga catacatcac     120
   ctcagctgcc caaacaattt ggtagtggag gacgaaggat gcaccaacct gtcagggttc     180
   tcctacatgg aacttaaagt tggatacatc ttagccataa aagtgaacgg gttcacttgc     240
   acaggcgttg tgacggaggc tgaaacctac actaacttcg ttggttatgt cacaaccacg     300
   ttcaaaagaa agcatttccg cccaacacca gatgcatgta gagccgcgta caactggaag     360
   atggccggtg accccagata tgaagagtct ctacacaatc cgtaccctga ctaccgctgg     420
   cttcgaactg taaaaaccac caaggagtct ctcgttatca tatctccaag tgtggcagat     480
   ttggacccat atgacagatc ccttcactcg agggtcttcc ctagcgggaa gtgctcagga     540
   gtagcggtgt cttctaccta ctgctccact aaccacgatt acaccatttg gatgcccgag     600
   aatccgagac tagggatgtc ttgtgacatt tttaccaata gtagagggaa gagagcatcc     660
   aaagggagtg agacttgcgg ctttgtagat gaaagaggcc tatataagtc tttaaaagga     720
   gcatgcaaac tcaagttatg tggagttcta ggacttagac ttatggatgg aacatgggtc     780
   tcgatgcaaa catcaaatga aaccaaatgg tgccctcccg ataagttggt gaacctgcac     840
   gactttcgct cagacgaaat tgagcacctt gttgtagagg agttggtcag gaagagagag     900
   gagtgtctgg atgcactaga gtccatcatg acaaccaagt cagtgagttt cagacgtctc     960
   agtcatttaa gaaaacttgt ccctgggttt ggaaaagcat ataccatatt caacaagacc    1020
   ttgatggaag ccgatgctca ctacaagtca gtcagaactt ggaatgagat cctcccttca    1080
   aaagggtgtt taagagttgg ggggaggtgt catcctcatg tgaacggggt gtttttcaat    1140
   ggtataatat taggacctga cggcaatgtc ttaatcccag agatgcaatc atccctcctc    1200
   cagcaacata tggagttgtt ggaatcctcg gttatccccc ttgtgcaccc cctggcagac    1260
   ccgtctaccg ttttcaagga cggtgacgag gctgaggatt ttgttgaagt tcaccttccc    1320
   gatgtgcaca atcaggtctc aggagttgac ttgggtctcc cgaactgggg gaagtatgta    1380
   ttactgagtg caggggccct gactgccttg atgttgataa ttttcctgat gacatgttgt    1440
   agaagagtca atcgatcaga acctacgcaa cacaatctca gagggacagg gagggaggtg    1500
   tcagtcactc cccaaagcgg gaagatcata tcttcatggg aatcacacaa gagtgggggt    1560
   gagaccagac tgtaa                                                     1575
<210> 43
   <211> 522
   <212> PRT
   <213> Mokola virus
<400> 43
   Met Asn Ile Pro Cys Phe Val Val Ile Leu Ser Leu Ala Thr Thr His
   1               5                   10                  15
   Ser Leu Gly Glu Phe Pro Leu Tyr Thr Ile Pro Glu Lys Ile Glu Lys
               20                  25                  30
   Trp Thr Pro Ile Asp Met Ile His Leu Ser Cys Pro Asn Asn Leu Leu
           35                  40                  45
   Ser Glu Glu Glu Gly Cys Asn Ala Glu Ser Ser Phe Thr Tyr Phe Glu
       50                  55                  60
   Leu Lys Ser Gly Tyr Leu Ala His Gln Lys Val Pro Gly Phe Thr Cys
   65                  70                  75                  80
   Thr Gly Val Val Asn Glu Ala Glu Thr Tyr Thr Asn Phe Val Gly Tyr
                   85                  90                  95
   Val Thr Thr Thr Phe Lys Arg Lys His Phe Arg Pro Thr Val Ala Ala
               100                 105                 110
   Cys Arg Asp Ala Tyr Asn Trp Lys Val Ser Gly Asp Pro Arg Tyr Glu
           115                 120                 125
   Glu Ser Leu His Thr Pro Tyr Pro Asp Ser Ser Trp Leu Arg Thr Val
       130                 135                 140
   Thr Thr Thr Lys Glu Ser Leu Leu Ile Ile Ser Pro Ser Ile Val Glu
   145 150 155 160
   Met Asp Ile Tyr Gly Arg Thr Leu His Ser Pro Met Phe Pro Ser Gly
   165 170 175
   Val Cys Ser Asn Val Tyr Pro Ser Val Pro Ser Cys Glu Thr Asn His
   180 185 190
   Asp Tyr Thr Leu Trp Leu Pro Glu Asp Pro Ser Leu Ser Leu Val Cys
   195 200 205
   Asp Ile Phe Thr Ser Ser Asn Gly Lys Lys Ala Met Asn Gly Ser Arg
   210 215 220
   Ile Cys Gly Phe Lys Asp Glu Arg Gly Phe Tyr Arg Ser Leu Lys Gly
   225 230 235 240
   Ala Cys Lys Leu Thr Leu Cys Gly Arg Pro Gly Ile Arg Leu Phe Asp
   245 250 255
   Gly Thr Trp Val Ser Phe Thr Lys Pro Asp Val His Val Trp Cys Thr
   260 265 270
   Pro Asn Gln Leu Ile Asn Ile His Asn Asp Arg Leu Asp Glu Ile Glu
   275 280 285
   His Leu Ile Val Glu Asp Ile Ile Lys Lys Arg Glu Glu Cys Leu Asp
   290 295 300
   Thr Leu Glu Thr Ile Leu Met Ser Gln Ser Val Ser Phe Arg Arg Leu
   305 310 315 320
   Ser His Phe Arg Lys Leu Val Pro Gly Tyr Gly Lys Ala Tyr Thr Ile
   325 330 335
   Leu Asn Gly Ser Leu Met Glu Thr Asn Val Tyr Tyr Lys Arg Val Asp
   340 345 350
   Lys Trp Ala Asp Ile Leu Pro Ser Lys Gly Cys Leu Lys Val Gly Gln
   355 360 365
   Gln Cys Met Glu Pro Val Lys Gly Val Leu Phe Asn Gly Ile Ile Lys
   370 375 380
   Gly Pro Asp Gly Gln Ile Leu Ile Pro Glu Met Gln Ser Glu Gln Leu
   385 390 395 400
   Lys Gln His Met Asp Leu Leu Lys Ala Ala Val Phe Pro Leu Arg His
   405 410 415
   Pro Leu Ile Ser Arg Glu Ala Val Phe Lys Lys Asp Gly Asp Ala Asp
   420 425 430
   Asp Phe Val Asp Leu His Met Pro Asp Val His Lys Ser Val Ser Asp
   435 440 445
   Val Asp Leu Gly Leu Pro His Trp Gly Phe Trp Met Leu Ile Gly Ala
   450 455 460
   Thr Ile Val Ala Phe Val Val Leu Val Cys Leu Leu Arg Val Cys Cys
   465 470 475 480
   Lys Arg Val Arg Arg Arg Arg Ser Gly Arg Ala Thr Gln Glu Ile Pro
   485 490 495
   Leu Ser Phe Pro Ser Ala Pro Val Pro Arg Ala Lys Val Val Ser Ser
   500 505 510
   Trp Glu Ser Tyr Lys Gly Leu Pro Gly Thr
   515 520
<210> 44
   <211> 1569
   <212> RNA
   <213> Mokola virus
<400> 44
   augaauauac cuugcuuugu ugugauucuc agcuuagcca cuacacauuc ucugggagaa      60
   uuccccuugu acacaauucc ugagaagaua gagaaaugga cucccauaga caugauccau     120
   cugaguugcc ccaacaaccu auuaucugag gaagaagguu gcaaugcaga gucauccuuu     180
   acuuacuuug agcucaagag ugguuaccua gcucaucaga agguuccagg guuuaccugu     240
   accggggucg ugaacgaggc agagacauau acaaacuucg ucggguacgu caccacaacc     300
   uucaaaagga agcacuuuag gccuacagua gccgccuguc gugaugccua caacuggaaa     360
   gugucaggag accccaggua cgaagaguca cuccacacuc cuuauccuga cagcaguugg     420
   uugaggacug ugacuacaac caaagaauca cuucucauaa uaucgcccag caucguggaa     480
   auggauauuu acggcaggac ucuccauucc cccauguuuc cuucaggagu auguuccaac     540
   guauaucccu cugucccauc cugugagacu aaucaugauu acacauuaug gcugccugaa     600
   gauccuaguu ugaguuuggu cugugauauc uuuacuucca gcaacggaaa gaaggccaug     660
   aacgggucac gcaucugcgg auucaaggau gaaaggggau ucuacagauc uuuaaagggc     720
   gcuugcaagc ugacauugug uggaagaccu ggaauuaggu uauucgacgg aacuuggguc     780
   ucuuuuacaa agccggacgu gcacguaugg ugcacuccca accaauugau caauauacac     840
   aaugacagac uagaugagau agaacaccug aucguggaag acaucauaaa gaaaagagaa     900
   gagugcuuag acacccugga aacaauacuu augucucaau cuguuagcuu uagaagguug     960
   agccauuucc gaaaguuagu uccaggauau gggaaggccu acacuauuuu aaacggcagc    1020
   cugauggaaa caaaugucua cuacaaaagg gucgacaagu gggcugacau cuuacccucu    1080
   aagggauguc ugaaagucgg gcaacaaugc auggaaccug ucaaaggagu ccucuucaau    1140
   gggauuauca agggcccgga uggccaaauu uugauccccg agaugcaguc agagcagcua    1200
   aagcagcaua uggaccuguu gaaggcggcu guguuuccuc uccgacaccc uuuaaucagc    1260
   cgggaggcag ucuuuaagaa agacggggau gccgaugauu uuguggaucu ccauaugccu    1320
   gauguccaca agucuguguc agaugucgac cugggucugc cucauugggg uuucuggaug    1380
   uugaucgggg caacaauagu agcauuugug gucuugguau guuuacuccg uguauguugu    1440
   aagagaguga ggaggagaag aucaggacgu gcaacucagg agaucccccu gagcuuuccc    1500
   ucugccccug uuccucgagc caaaguggug ucaucuuggg aguccuauaa agggcuucca    1560
   gguacauga                                                            1569
<210> 45
   <211> 669
   <212> PRT
   <213> Murine leukemia virus
<400> 45
   Met Glu Ser Thr Thr Leu Ser Lys Pro Phe Lys Asn Gln Val Asn Pro
   1               5                   10                  15
   Trp Gly Pro Leu Ile Val Leu Leu Ile Leu Gly Gly Val Asn Pro Val
               20                  25                  30
   Ala Leu Gly Asn Ser Pro His Gln Val Phe Asn Leu Ser Trp Glu Val
           35                  40                  45
   Thr Asn Gly Asp Arg Glu Thr Val Trp Ala Ile Thr Gly Asn His Pro
       50                  55                  60
   Leu Trp Thr Trp Trp Pro Asp Leu Thr Pro Asp Leu Cys Met Leu Ala
   65                  70                  75                  80
   Leu His Gly Pro Ser Tyr Trp Gly Leu Glu Tyr Arg Ala Pro Phe Ser
                   85                  90                  95
   Pro Pro Pro Gly Pro Pro Cys Cys Ser Gly Ser Ser Asp Ser Thr Pro
               100                 105                 110
   Gly Cys Ser Arg Asp Cys Glu Glu Pro Leu Thr Ser Tyr Thr Pro Arg
           115                 120                 125
   Cys Asn Thr Ala Trp Asn Arg Leu Lys Leu Ser Lys Val Thr His Ala
       130                 135                 140
   His Asn Glu Gly Phe Tyr Val Cys Pro Gly Pro His Arg Pro Arg Trp
   145                 150                 155                 160
   Ala Arg Leu Cys Gly Gly Pro Glu Ser Phe Tyr Cys Ala Ser Trp Gly
                   165                 170                 175
   Cys Glu Thr Thr Gly Arg Ala Ser Trp Lys Pro Ser Ser Ser Trp Asp
               180                 185                 190
   Tyr Ile Thr Val Ser Asn Asn Leu Thr Ser Asp Gln Ala Thr Pro Ala
           195                 200                 205
   Cys Lys Gly Asn Lys Trp Cys Asn Ser Leu Thr Ile Arg Phe Thr Ser
       210                 215                 220
   Phe Gly Lys Gln Ala Thr Ser Trp Val Thr Gly His Trp Trp Gly Leu
   225                 230                 235                 240
   Arg Leu Tyr Val Ser Gly His Asp Pro Gly Leu Ile Phe Gly Ile Arg
                   245                 250                 255
   Leu Lys Ile Thr Asp Leu Gly Pro Arg Val Pro Ile Gly Pro Asn Pro
               260                 265                 270
   Val Leu Ser Asp Arg Arg Pro Pro Ser Arg Pro Arg Pro Thr Arg Ser
           275                 280                 285
   Pro Pro Pro Ser Asn Ser Thr Pro Thr Glu Thr Pro Leu Thr Leu Pro
       290                 295                 300
   Glu Pro Pro Pro Ala Gly Val Glu Asn Arg Leu Leu Asn Leu Val Lys
   305                 310                 315                 320
   Gly Ala Tyr Gln Ala Leu Asn Leu Thr Ser Pro Asp Lys Thr Gln Glu
                   325                 330                 335
   Cys Trp Leu Cys Leu Val Ser Gly Pro Pro Tyr Tyr Glu Gly Val Ala
               340                 345                 350
   Val Leu Gly Thr Tyr Ser Asn His Thr Ser Ala Pro Ala Asn Cys Ser
           355                 360                 365
   Val Ala Ser Gln His Lys Leu Thr Leu Ser Glu Val Thr Gly Gln Gly
       370                 375                 380
   Leu Cys Ile Gly Ala Val Pro Lys Thr Leu Gln Val Leu Cys Asn Thr
   385 390 395 400
   Thr Gln Lys Thr Ser Ala Gly Ser Tyr Tyr Leu Ala Ala Pro Thr Gly
   405 410 415
   Thr Ile Trp Ala Cys Ser Thr Gly Leu Thr Pro Cys Ile Ser Thr Thr
   420 425 430
   Ile Leu Asn Leu Thr Thr Asp Tyr Cys Val Leu Val Glu Leu Trp Pro
   435 440 445
   Arg Val Thr Tyr His Ser Pro Ser Tyr Val Tyr His Gln Phe Glu Arg
   450 455 460
   Arg Ala Lys Tyr Lys Arg Glu Pro Val Ser Leu Thr Leu Ala Leu Leu
   465 470 475 480
   Leu Gly Gly Leu Thr Met Gly Gly Ile Ala Ala Gly Val Gly Thr Gly
   485 490 495
   Thr Thr Ala Leu Val Ala Thr Gln Gln Phe Gln Gln Leu Gln Ala Ala
   500 505 510
   Met His Asp Asp Leu Lys Glu Val Glu Lys Ser Ile Thr Asn Leu Glu
   515 520 525
   Lys Ser Leu Thr Ser Leu Ser Glu Val Val Leu Gln Asn Arg Arg Gly
   530 535 540
   Leu Asp Leu Leu Phe Leu Lys Glu Gly Gly Leu Cys Ala Ala Leu Lys
   545 550 555 560
   Glu Glu Cys Cys Phe Tyr Ala Asp His Thr Gly Leu Val Arg Asp Ser
   565 570 575
   Met Ala Lys Leu Arg Glu Arg Leu Ser Gln Arg Gln Lys Leu Phe Glu
   580 585 590
   Ser Gln Gln Gly Trp Phe Glu Gly Leu Phe Asn Arg Ser Pro Trp Phe
   595 600 605
   Thr Thr Leu Ile Ser Thr Ile Met Gly Pro Leu Ile Ile Leu Leu Leu
   610 615 620
   Ile Leu Leu Phe Gly Pro Cys Ile Leu Asn Arg Leu Val Gln Phe Ile
   625 630 635 640
   Lys Asp Arg Ile Ser Val Val Gln Ala Leu Val Leu Thr Gln Gln Tyr
   645 650 655
   His Gln Leu Lys Thr Ile Glu Asp Cys Lys Ser Arg Glu
   660 665
<210> 46
   <211> 2010
   <212> DNA
   <213> Murine leukemia virus
<400> 46
   atggagagta caacgctctc aaaacccttt aaaaatcagg ttaacccgtg gggcccccta 60
   attgtccttc tgattctcgg aggggtcaac cccgttgcgt tgggaaacag cccccaccag 120
   gtttttaacc tctcctggga agtgactaat ggagaccgag aaacggtgtg ggcaataacc 180
   ggcaatcacc ctctgtggac ttggtggcct gacctcacac cagatctctg tatgttggcc 240
   ctccacgggc cgtcctattg gggcctagaa tatcgggctc ctttttctcc tcccccgggg 300
   cccccctgct gttcaggaag cagcgactcc acgccaggct gttccagaga ttgtgaggag 360
   cccctgactt catatactcc ccggtgcaat acggcctgga acagacttaa gttatctaaa 420
   gtgacacatg cccacaatga aggattctat gtctgccccg ggccacatcg cccccggtgg 480
   gcccggttgt gtggtggtcc agaatccttc tattgtgcct cctggggctg cgaaaccaca 540
   ggccgagcat cctggaaacc atcctcgtcc tgggactaca tcacagtaag caacaatcta 600
   acctcagacc aggcaacccc agcatgcaaa ggtaataagt ggtgcaactc cttaactatc 660
   cggttcacga gctttggaaa acaggccacc tcctgggtca caggccattg gtggggattg 720
   cgcctatacg tctctggaca tgacccaggg ctcatctttg ggatccgact taaaattaca 780
   gacttggggc cccgggtccc aatagggcca aaccccgtct tgtcagaccg acgaccacct 840
   tcccggccta gacccaccag atctcccccg ccttcaaact ccaccccaac cgagacaccc 900
   ctcaccctcc ccgaaccccc gccagcggga gtcgaaaacc gattgttaaa tctagtaaaa 960
   ggagcctacc aagccctcaa cctcaccagt cctgataaaa cccaagagtg ctggttatgc 1020
   ctagtatcgg gacccccata ctacgagggg gttgccgtcc taggtaccta ctccaaccat 1080
   acttctgccc cagctaactg ctctgtggcc tctcaacaca aattgacctt gtccgaagtg 1140
   accggacagg gactctgcat aggagcggtc cctaaaaccc ttcaagtctt gtgtaatacc 1200
   acccaaaaga caagcgctgg gtcctactac ttggccgctc ccacaggaac tatctgggct 1260
   tgtagtactg gactcactcc ctgtatctca accaccatac ttaacctcac caccgattac 1320
   tgtgtcctgg tcgagctttg gccaagggtg acctaccatt cccctagtta tgtttaccac 1380
   caattcgaaa gacgagccaa atataaaaga gaacccgtct cactaactct ggccctacta 1440
   ttaggaggac tcactatggg cggaattgcc gctggagtgg gaacagggac taccgcccta 1500
   gtggccactc agcagttcca acaactccag gctgccatgc acgatgacct taaagaagtt 1560
   gaaaagtcca tcactaatct agaaaaatct ttgacctccc tgtccgaagt agtattacag 1620
   aatcgtagag gcctagatct actattccta aaagagggag gtttgtgtgc tgccttaaaa 1680
   gaagaatgct gtttctatgc cgaccacaca ggattggtac gggatagcat ggccaaactt 1740
   agagaaagat tgagtcaaag acaaaagctc tttgaatccc aacaagggtg gtttgaaggg 1800
   ctgtttaata ggtccccttg gttcaccacc ctgatatcca ccatcatggg tcccctgata 1860
   atcctcttgt taattttact ctttgggcct tgtattctca atcgcctggt ccagtttatc 1920
   aaagacagga tttcggtagt gcaggccctg gttctgactc aacaatatca tcaacttaag 1980
   acaatagaag attgtaaatc acgtgaataa 2010
<210> 47
   <211> 654
   <212> PRT
   <213> Murine leukemia virus
<400> 47
   Met Ala Arg Ser Thr Leu Ser Lys Pro Pro Lys Asp Lys Ile Asn Pro
   1               5                   10                  15
   Trp Lys Ser Leu Met Val Met Gly Val Leu Leu Arg Val Gly Met Ala
               20                  25                  30
   Glu Ser Pro His Gln Val Phe Asn Val Thr Trp Arg Val Thr Asn Leu
           35                  40                  45
   Met Thr Gly Arg Thr Ala Asn Ala Thr Ser Leu Leu Gly Thr Val Gln
       50                  55                  60
   Asp Ala Phe Pro Arg Leu Tyr Phe Asp Leu Cys Asp Leu Val Gly Glu
   65                  70                  75                  80
   Glu Trp Asp Pro Ser Asp Gln Glu Pro Tyr Val Gly Tyr Gly Cys Lys
                   85                  90                  95
   Tyr Pro Ala Gly Arg Gln Arg Thr Arg Thr Phe Asp Phe Tyr Val Cys
               100                 105                 110
   Pro Gly His Thr Val Lys Ser Gly Cys Gly Gly Pro Gly Glu Gly Tyr
           115                 120                 125
   Cys Gly Lys Trp Gly Cys Glu Thr Thr Gly Gln Ala Tyr Trp Lys Pro
       130                 135                 140
   Thr Ser Ser Trp Asp Leu Ile Ser Leu Lys Arg Gly Asn Thr Pro Trp
   145                 150                 155                 160
   Asp Thr Gly Cys Ser Lys Val Ala Cys Gly Pro Cys Tyr Asp Leu Ser
                   165                 170                 175
   Lys Val Ser Asn Ser Phe Gln Gly Ala Thr Arg Gly Gly Arg Cys Asn
               180                 185                 190
   Pro Leu Val Leu Glu Phe Thr Asp Ala Gly Lys Lys Ala Asn Trp Asp
           195                 200                 205
   Gly Pro Lys Ser Trp Gly Leu Arg Leu Tyr Arg Thr Gly Thr Asp Pro
       210                 215                 220
   Ile Thr Met Phe Ser Leu Thr Arg Gln Val Leu Asn Val Gly Pro Arg
   225                 230                 235                 240
   Val Pro Ile Gly Pro Asn Pro Val Leu Pro Asp Gln Arg Leu Pro Ser
                   245                 250                 255
   Ser Pro Val Glu Ala Ile Pro Ala Pro Gln Pro Pro Ser Pro Leu Asn
               260                 265                 270
   Thr Ser Tyr Pro Pro Ser Thr Ala Ser Thr Pro Ser Thr Ser Pro Thr
           275                 280                 285
   Ser Pro Ser Ala Pro Gln Pro Pro Pro Gly Thr Gly Asp Arg Leu Leu
       290                 295                 300
   Ala Leu Val Lys Gly Ala Tyr Gln Ala Leu Asn Leu Thr Asn Pro Asp
   305                 310                 315                 320
   Lys Thr Gln Glu Cys Trp Leu Cys Leu Val Ser Gly Pro Pro Tyr Tyr
                   325                 330                 335
   Glu Gly Val Ala Val Val Gly Thr Tyr Thr Asn His Ser Thr Ala Pro
               340                 345                 350
   Ala Asn Cys Thr Ala Thr Ser Gln His Lys Leu Thr Leu Ser Glu Val
           355                 360                 365
   Thr Gly Gln Gly Leu Cys Met Gly Ala Val Pro Lys Thr His Gln Ala
       370                 375                 380
   Leu Cys Asn Thr Thr Gln Ser Ala Gly Ser Gly Ser Tyr Tyr Leu Ala
   385                 390                 395                 400
   Ala Pro Thr Gly Thr Met Trp Ala Cys Ser Thr Gly Leu Thr Pro Cys
                   405                 410                 415
   Leu Ser Thr Thr Val Leu Asn Leu Thr Thr Asp Tyr Cys Val Leu Val
               420                 425                 430
   Glu Leu Trp Pro Arg Val Ile Tyr His Ser Pro Asp Tyr Met Tyr Gly
           435                 440                 445
   Gln Leu Glu Gln Arg Thr Lys Tyr Lys Arg Glu Pro Val Ser Leu Thr
       450                 455                 460
   Leu Ala Leu Leu Leu Gly Gly Leu Thr Met Gly Gly Ile Ala Ala Gly
   465                 470                 475                 480
   Ile Gly Thr Gly Thr Thr Ala Leu Ile Lys Thr Gln Gln Phe Glu Gln
                   485                 490                 495
   Leu His Ala Ala Ile Gln Thr Asp Leu Asn Glu Val Glu Lys Ser Ile
               500                 505                 510
   Thr Asn Leu Glu Lys Ser Leu Thr Ser Leu Ser Glu Val Val Leu Gln
           515                 520                 525
   Asn Arg Arg Gly Leu Asp Leu Leu Phe Leu Lys Glu Gly Gly Leu Cys
       530                 535                 540
   Ala Ala Leu Lys Glu Glu Cys Cys Phe Tyr Ala Asp His Thr Gly Leu
   545                 550                 555                 560
   Val Arg Asp Ser Met Ala Lys Leu Arg Glu Arg Leu Asn Gln Arg Gln
                   565                 570                 575
   Lys Leu Phe Glu Ser Gly Gln Gly Trp Phe Glu Gly Leu Phe Asn Arg
               580                 585                 590
   Ser Pro Trp Phe Thr Thr Leu Ile Ser Thr Ile Met Gly Pro Leu Ile
           595                 600                 605
   Val Leu Leu Leu Ile Leu Leu Phe Gly Pro Cys Ile Leu Asn Arg Leu
       610                 615                 620
   Val Gln Phe Val Lys Asp Arg Ile Ser Val Val Gln Ala Leu Val Leu
   625                 630                 635                 640
   Thr Gln Gln Tyr His Gln Leu Lys Pro Ile Glu Tyr Glu Pro
                   645                 650
<210> 48
   <211> 1965
   <212> DNA
   <213> Murine leukemia virus
<400> 48
   atggcgcgtt caacgctctc aaaaccccct aaagataaga ttaacccgtg gaagtcctta 60
   atggtcatgg gggtcctatt aagagtaggg atggcagaga gcccccatca ggtctttaat 120
   gtaacctgga gagtcaccaa cctgatgact gggcgtaccg ccaatgccac ctcccttttg 180
   ggaactgtac aagatgcctt cccaagatta tattttgatc tatgtgacct ggtcggagag 240
   gagtgggacc cttcagacca ggaaccatat gtcgggtatg gctgcaaata ccccgcaggg 300
   agacagcgga cccggacttt tgacttttac gtgtgccctg ggcataccgt aaaatcgggg 360
   tgtgggggac caggagaagg ctactgtggt aaatggggtt gtgaaaccac cggacaggct 420
   tactggaagc ccacatcatc atgggaccta atctccctta agcgcggtaa caccccctgg 480
   gacacgggat gctccaaagt tgcttgtggc ccctgctatg acctctccaa ggtatccaat 540
   tccttccaag gggctactcg agggggcaga tgcaaccctc tagtcctaga attcactgat 600
   gcaggaaaaa aggctaattg ggacgggccc aaatcgtggg gactgagact gtaccggaca 660
   ggaacagatc ctattaccat gttctccctg acccgacagg tccttaatgt ggggccccga 720
   gtccctatag gacccaaccc ggtattaccc gaccaaagac tcccttcctc accagtggag 780
   gctataccgg ctccacagcc acctagcccc ctcaatacca gttacccccc ttccactgcc 840
   agtacaccct caacctcccc tacaagccca agtgccccac agccaccccc aggaactgga 900
   gatagacttc tagctctagt caaaggagcc tatcaggcgc ttaacctcac caatcccgac 960
   aagacccaag aatgttggct gtgcttagtg tcgggacctc cttattacga aggagtagcg 1020
   gtcgtgggca cttataccaa tcattctacc gccccggcca actgtacggc cacttcccaa 1080
   cataagctta ccctatctga agtgacagga cagggcctat gcatgggagc agtacctaaa 1140
   actcaccagg ccttatgtaa caccacccaa agtgccggct caggatccta ctaccttgca 1200
   gcacccactg gaacaatgtg ggcttgtagc actggattga ctccctgctt gtccaccacg 1260
   gtgctcaatc taaccacaga ctattgtgta ttagttgagc tctggcccag agtaatttac 1320
   cactcccccg attatatgta tggtcagctt gaacagcgta ccaaatataa gagggagcca 1380
   gtatcgttga ccctggccct tctgctagga ggattaacca tgggagggat tgcagctgga 1440
   atagggacgg ggaccactgc cctaatcaaa acccagcagt ttgagcagct tcacgccgct 1500
   atccagacag acctcaacga agtcgaaaaa tcaattacca acctagaaaa gtcactgacc 1560
   tcgttgtctg aagtagtcct acagaaccga agaggcctag atttgctctt cctaaaagag 1620
   ggaggtctct gcgcagccct aaaagaagaa tgttgttttt atgcagacca cacgggacta 1680
   gtgagagaca gcatggccaa attaagggaa aggcttaatc agagacaaaa actatttgag 1740
   tcaggccaag gttggttcga agggctgttt aatagatccc cctggtttac caccttaatc 1800
   tccaccatca tgggacctct aatagtactc ttactgatct tactctttgg accctgcatt 1860
   ctcaatcgat tggtccaatt tgttaaagac aggatctcag tggtccaggc tctggttttg 1920
   actcaacaat atcaccagct aaaacctata gagtacgagc catga 1965
<210> 49
   <211> 535
   <212> PRT
   <213> Murine leukemia virus
<400> 49
   Met Glu Gly Pro Ala Phe Ser Lys Pro Leu Lys Asp Lys Ile Asn Pro
   1               5                   10                  15
   Trp Gly Pro Leu Ile Val Leu Gly Ile Leu Ile Arg Ala Gly Val Ser
               20                  25                  30
   Val Gln His Asp Ser Pro His Gln Val Phe Asn Val Thr Trp Arg Val
           35                  40                  45
   Thr Asn Leu Met Thr Gly Gln Thr Ala Asn Ala Thr Ser Leu Leu Gly
       50                  55                  60
   Thr Met Thr Asp Ala Phe Pro Met Leu Tyr Phe Asp Leu Cys Asp Leu
   65                  70                  75                  80
   Ile Gly Asp Asp Trp Asp Glu Thr Gly Leu Gly Cys Arg Thr Pro Gly
                   85                  90                  95
   Gly Arg Lys Arg Ala Arg Thr Phe Asp Phe Tyr Val Cys Pro Gly His
               100                 105                 110
   Thr Val Pro Thr Gly Cys Gly Gly Pro Arg Glu Gly Tyr Cys Gly Lys
           115                 120                 125
   Trp Gly Cys Glu Thr Thr Gly Gln Ala Tyr Trp Lys Pro Ser Ser Ser
       130                 135                 140
   Trp Asp Leu Ile Ser Leu Lys Arg Gly Asn Thr Pro Arg Asn Gln Gly
   145                 150                 155                 160
   Pro Cys Tyr Asp Ser Ser Val Val Ser Ser Gly Ile Gln Gly Ala Thr
                   165                 170                 175
   Pro Gly Gly Arg Cys Asn Pro Leu Val Leu Glu Phe Thr Asp Ala Gly
               180                 185                 190
   Lys Lys Ala Ser Trp Asp Gly Pro Lys Val Trp Gly Leu Arg Leu Tyr
           195                 200                 205
   Arg Ser Thr Gly Ile Asp Pro Val Thr Arg Phe Ser Leu Thr Arg Gln
       210                 215                 220
   Val Leu Asn Ile Gly Pro Arg Ile Pro Ile Gly Pro Asn Pro Val Ile
   225 230 235 240
   Thr Gly Gln Leu Pro Pro Ser Arg Pro Val Gln Ile Arg Leu Pro Arg
   245 250 255
   Pro Pro Gln Pro Pro Pro Thr Gly Ala Ala Ser Met Val Pro Gly Thr
   260 265 270
   Ala Pro Pro Ser Gln Gln Pro Gly Thr Gly Asp Arg Leu Leu Asn Leu
   275 280 285
   Val Asp Gly Ala Tyr Gln Ala Leu Asn Leu Thr Ser Pro Asp Lys Thr
   290 295 300
   Gln Glu Cys Trp Leu Cys Leu Val Ala Gly Pro Pro Tyr His Glu Gly
   305 310 315 320
   Val Ala Val Leu Gly Thr Tyr Ser Asn His Thr Ser Ala Pro Ala Asn
   325 330 335
   Cys Ser Val Ala Ser Gln His Lys Leu Thr Leu Ser Glu Val Thr Gly
   340 345 350
   Gln Gly Leu Cys Ile Gly Ala Val Pro Lys Thr His Gln Ala Leu Cys
   355 360 365
   Asn Thr Thr Gln Thr Ser Ser Arg Gly Ser Tyr Tyr Leu Val Ala Pro
   370 375 380
   Thr Gly Thr Met Trp Ala Cys Ser Thr Gly Leu Thr Pro Cys Ile Ser
   385 390 395 400
   Thr Thr Ile Leu Asn Leu Thr Thr Asp Tyr Cys Val Leu Val Glu Leu
   405 410 415
   Trp Pro Arg Val Thr Tyr His Ser Pro Ser Tyr Val Tyr Gly Leu Phe
   420 425 430
   Glu Arg Ser Asn Arg His Lys Arg Glu Pro Val Ser Leu Thr Leu Ala
   435 440 445
   Leu Leu Leu Gly Gly Leu Thr Met Gly Gly Ile Ala Ala Gly Ile Gly
   450 455 460
   Thr Gly Thr Thr Ala Leu Met Ala Thr Gln Gln Phe Gln Gln Leu Gln
   465 470 475 480
   Ala Ala Val Gln Asp Asp Leu Arg Glu Val Glu Lys Ser Ile Ser Asn
   485 490 495
   Leu Glu Lys Ser Leu Thr Ser Leu Ser Glu Val Val Leu Gln Asn Arg
   500 505 510
   Arg Gly Leu Asp Leu Leu Phe Leu Lys Glu Gly Gly Leu Cys Ala Ala
   515 520 525
   Leu Lys Glu Glu Cys Cys Phe
   530 535
<210> 50
   <211> 1926
   <212> DNA
   <213> Murine leukemia virus
<400> 50
   atggaaggtc cagcgttctc aaaatccctt aaagataaga ttaacccgtg gggccccctg 60
   atagttatgg ggatcttagt gagggcagga gcctcggtac aacgtgacag ccctcaccag 120
   gtcttcaatg ttacttggag agttaccaac ctaatgacag gacaaacagc taacgccacc 180
   tccctcctgg ggacgatgac agacaccttc cctaaactat attttgacct gtgtgattta 240
   gtaggagact actgggatga cccagatgga gatggttgcc gctctcccgg gggaagaaaa 300
   aggacaagac tgtatgactt ctatgtttgc cccggtcata ctgtaccaat agggtgtgga 360
   gggccgggag agggctactg tggcaaatgg ggatgtgaga ccactggaca ggcatactgg 420
   aagccatcat catcatggga cctaatttcc cttaagcgag gaaacactcc taaggatcag 480
   ggcccctgtt atgattcctc ggtctccagt ggcgtccagg gtgccacacc agggggtcga 540
   tgcaaccccc tagtcttaga attcactgac gcgggtagga aggccagctg ggatgccccc 600
   aaagtttggg gactaagact ctatcgatcc acaggggtcg acccggtgac ccggttctct 660
   ttgacccgcc aggtcctcaa tgtaggaccc cgcgtcccca ttgggcctaa tcccgtgatc 720
   actgaccagc tacccccatc ccaacccgtg cagatcatgc tccccaggcc tcctcatcct 780
   cctccttcag gcacggtctc tatggtacct ggggctcccc cgccttctca acaacctggg 840
   acgggagaca ggctgctaaa tctggtaaaa ggagcctacc aagcactcaa cctcaccagt 900
   cccgacagaa cccaagagtg ctggctgtgt ctagtatcgg gaccccccta ctacgaaggg 960
   gtggccgtcc tgggtactta caccaaccat acctctgccc cagctaactg ctccgtaacc 1020
   tcccaacaca agctgaccct gtccgaagtg accgggcagg gactctgcgt aggagcagtt 1080
   cccaaaaccc atcaagccct gtgtaatacc acccagaaga caagcgacgg gtcctactat 1140
   ttagcctctc ccgccgggac catttgggcc tgcagcaccg ggcttactcc ctgtatatct 1200
   accactgtgc ttaacttaac caccgattat tgtgtcctgg ttgagctctg gccaaaggta 1260
   acctaccact cccctgatta tgtttttggc cagtttgaaa agaaaactaa atataaaaga 1320
   gagccagtgt cattaactct ggccctgctg ttagggggac ttactatggg cggcatagca 1380
   gccggggtgg gaacaggaac cacggcctta gtgaaaacac aacaatttga gcagctccag 1440
   gcggctatac acacagatct cggggcacta ggaaagtctg ttagcgccct tgaaaaatct 1500
   ctgacatcgc tgtccgaagt ggtcttacag aatcgcagag ggttagactt actgtttcta 1560
   aaagaaggag gattatgtgc tgccctaaaa gaagaatgct gtttctatgc agaccacact 1620
   ggcgtagtaa gagatagcat ggctaagcta agagaaaggc taaaccagag acaaaaattg 1680
   tttgagtcag gacaagggtg gtttgaggga ctgtttaaca ggtccccatg gttcacgacc 1740
   ctgatatcca ccattatggg ccctctgata gtacttttat taatcctact cttcggaccc 1800
   tgcattctca accggttggt ccagtttgta aaagacagaa tttcagtagt acaggccctg 1860
   attctgaccc aacagtatca ccaactcaaa tcaatagatc cagaagaagt ggaatcgcgt 1920
   gaataa 1926
<210> 51
   <211> 641
   <212> PRT
   <213> Murine leukemia virus
<400> 51
   Met Glu Gly Pro Ala Phe Ser Lys Ser Leu Lys Asp Lys Ile Asn Pro
   1 5 10 15
   Trp Gly Pro Leu Ile Val Met Gly Ile Leu Val Arg Ala Gly Ala Ser
   20 25 30
   Val Gln Arg Asp Ser Pro His Gln Val Phe Asn Val Thr Trp Arg Val
   35 40 45
   Thr Asn Leu Met Thr Gly Gln Thr Ala Asn Ala Thr Ser Leu Leu Gly
   50 55 60
   Thr Met Thr Asp Thr Phe Pro Lys Leu Tyr Phe Asp Leu Cys Asp Leu
   65 70 75 80
   Val Gly Asp Tyr Trp Asp Asp Pro Asp Gly Asp Gly Cys Arg Ser Pro
   85 90 95
   Gly Gly Arg Lys Arg Thr Arg Leu Tyr Asp Phe Tyr Val Cys Pro Gly
   100 105 110
   His Thr Val Pro Ile Gly Cys Gly Gly Pro Gly Glu Gly Tyr Cys Gly
   115 120 125
   Lys Trp Gly Cys Glu Thr Thr Gly Gln Ala Tyr Trp Lys Pro Ser Ser
   130 135 140
   Ser Trp Asp Leu Ile Ser Leu Lys Arg Gly Asn Thr Pro Lys Asp Gln
   145 150 155 160
   Gly Pro Cys Tyr Asp Ser Ser Val Ser Ser Gly Val Gln Gly Ala Thr
   165 170 175
   Pro Gly Gly Arg Cys Asn Pro Leu Val Leu Glu Phe Thr Asp Ala Gly
   180 185 190
   Arg Lys Ala Ser Trp Asp Ala Pro Lys Val Trp Gly Leu Arg Leu Tyr
   195 200 205
   Arg Ser Thr Gly Val Asp Pro Val Thr Arg Phe Ser Leu Thr Arg Gln
   210 215 220
   Val Leu Asn Val Gly Pro Arg Val Pro Ile Gly Pro Asn Pro Val Ile
   225 230 235 240
   Thr Asp Gln Leu Pro Pro Ser Gln Pro Val Gln Ile Met Leu Pro Arg
   245 250 255
   Pro Pro His Pro Pro Pro Ser Gly Thr Val Ser Met Val Pro Gly Ala
   260 265 270
   Pro Pro Pro Ser Gln Gln Pro Gly Thr Gly Asp Arg Leu Leu Asn Leu
   275 280 285
   Val Lys Gly Ala Tyr Gln Ala Leu Asn Leu Thr Ser Pro Asp Arg Thr
   290 295 300
   Gln Glu Cys Trp Leu Cys Leu Val Ser Gly Pro Pro Tyr Tyr Glu Gly
   305 310 315 320
   Val Ala Val Leu Gly Thr Tyr Thr Asn His Thr Ser Ala Pro Ala Asn
   325 330 335
   Cys Ser Val Thr Ser Gln His Lys Leu Thr Leu Ser Glu Val Thr Gly
   340 345 350
   Gln Gly Leu Cys Val Gly Ala Val Pro Lys Thr His Gln Ala Leu Cys
   355 360 365
   Asn Thr Thr Gln Lys Thr Ser Asp Gly Ser Tyr Tyr Leu Ala Ser Pro
   370 375 380
   Ala Gly Thr Ile Trp Ala Cys Ser Thr Gly Leu Thr Pro Cys Ile Ser
   385 390 395 400
   Thr Thr Val Leu Asn Leu Thr Thr Asp Tyr Cys Val Leu Val Glu Leu
                   405                 410                 415
   Trp Pro Lys Val Thr Tyr His Ser Pro Asp Tyr Val Phe Gly Gln Phe
               420                 425                 430
   Glu Lys Lys Thr Lys Tyr Lys Arg Glu Pro Val Ser Leu Thr Leu Ala
           435                 440                 445
   Leu Leu Leu Gly Gly Leu Thr Met Gly Gly Ile Ala Ala Gly Val Gly
       450                 455                 460
   Thr Gly Thr Thr Ala Leu Val Lys Thr Gln Gln Phe Glu Gln Leu Gln
   465                 470                 475                 480
   Ala Ala Ile His Thr Asp Leu Gly Ala Leu Gly Lys Ser Val Ser Ala
                   485                 490                 495
   Leu Glu Lys Ser Leu Thr Ser Leu Ser Glu Val Val Leu Gln Asn Arg
               500                 505                 510
   Arg Gly Leu Asp Leu Leu Phe Leu Lys Glu Gly Gly Leu Cys Ala Ala
           515                 520                 525
   Leu Lys Glu Glu Cys Cys Phe Tyr Ala Asp His Thr Gly Val Val Arg
       530                 535                 540
   Asp Ser Met Ala Lys Leu Arg Glu Arg Leu Asn Gln Arg Gln Lys Leu
   545                 550                 555                 560
   Phe Glu Ser Gly Gln Gly Trp Phe Glu Gly Leu Phe Asn Arg Ser Pro
                   565                 570                 575
   Trp Phe Thr Thr Leu Ile Ser Thr Ile Met Gly Pro Leu Ile Val Leu
               580                 585                 590
   Leu Leu Ile Leu Leu Phe Gly Pro Cys Ile Leu Asn Arg Leu Val Gln
           595                 600                 605
   Phe Val Lys Asp Arg Ile Ser Val Val Gln Ala Leu Ile Leu Thr Gln
       610                 615                 620
   Gln Tyr His Gln Leu Lys Ser Ile Asp Pro Glu Glu Val Glu Ser Arg
   625                 630                 635                 640
   Glu
<210> 52
   <211> 1926
   <212> DNA
   <213> Murine leukemia virus
<400> 52
   atggaaggtc cagcgttctc aaaatccctt aaagataaga ttaacccgtg gggccccctg 60
   atagttatgg ggatcttagt gagggcagga gcctcggtac aacgtgacag ccctcaccag 120
   gtcttcaatg ttacttggag agttaccaac ctaatgacag gacaaacagc taacgccacc 180
   tccctcctgg ggacgatgac agacaccttc cctaaactat attttgacct gtgtgattta 240
   gtaggagact actgggatga cccagatgga gatggttgcc gctctcccgg gggaagaaaa 300
   aggacaagac tgtatgactt ctatgtttgc cccggtcata ctgtaccaat agggtgtgga 360
   gggccgggag agggctactg tggcaaatgg ggatgtgaga ccactggaca ggcatactgg 420
   aagccatcat catcatggga cctaatttcc cttaagcgag gaaacactcc taaggatcag 480
   ggcccctgtt atgattcctc ggtctccagt ggcgtccagg gtgccacacc agggggtcga 540
   tgcaaccccc tagtcttaga attcactgac gcgggtagga aggccagctg ggatgccccc 600
   aaagtttggg gactaagact ctatcgatcc acaggggtcg acccggtgac ccggttctct 660
   ttgacccgcc aggtcctcaa tgtaggaccc cgcgtcccca ttgggcctaa tcccgtgatc 720
   actgaccagc tacccccatc ccaacccgtg cagatcatgc tccccaggcc tcctcatcct 780
   cctccttcag gcacggtctc tatggtacct ggggctcccc cgccttctca acaacctggg 840
   acgggagaca ggctgctaaa tctggtaaaa ggagcctacc aagcactcaa cctcaccagt 900
   cccgacagaa cccaagagtg ctggctgtgt ctagtatcgg gaccccccta ctacgaaggg 960
   gtggccgtcc tgggtactta caccaaccat acctctgccc cagctaactg ctccgtaacc 1020
   tcccaacaca agctgaccct gtccgaagtg accgggcagg gactctgcgt aggagcagtt 1080
   cccaaaaccc atcaagccct gtgtaatacc acccagaaga caagcgacgg gtcctactat 1140
   ttagcctctc ccgccgggac catttgggcc tgcagcaccg ggcttactcc ctgtatatct 1200
   accactgtgc ttaacttaac caccgattat tgtgtcctgg ttgagctctg gccaaaggta 1260
   acctaccact cccctgatta tgtttttggc cagtttgaaa agaaaactaa atataaaaga 1320
   gagccagtgt cattaactct ggccctgctg ttagggggac ttactatggg cggcatagca 1380
   gccggggtgg gaacaggaac cacggcctta gtgaaaacac aacaatttga gcagctccag 1440
   gcggctatac acacagatct cggggcacta ggaaagtctg ttagcgccct tgaaaaatct 1500
   ctgacatcgc tgtccgaagt ggtcttacag aatcgcagag ggttagactt actgtttcta 1560
   aaagaaggag gattatgtgc tgccctaaaa gaagaatgct gtttctatgc agaccacact 1620
   ggcgtagtaa gagatagcat ggctaagcta agagaaaggc taaaccagag acaaaaattg 1680
   tttgagtcag gacaagggtg gtttgaggga ctgtttaaca ggtccccatg gttcacgacc 1740
   ctgatatcca ccattatggg ccctctgata gtacttttat taatcctact cttcggaccc 1800
   tgcattctca accggttggt ccagtttgta aaagacagaa tttcagtagt acaggccctg 1860
   attctgaccc aacagtatca ccaactcaaa tcaatagatc cagaagaagt ggaatcgcgt 1920
   gaataa 1926
<210> 53
   <211> 856
   <212> PRT
   <213> Human immunodeficiency virus type 1
<400> 53
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Gly Lys Val Gln Lys Glu Tyr Ala Phe Phe
                   165                 170                 175
   Tyr Lys Leu Asp Ile Ile Pro Ile Asp Asn Asp Thr Thr Ser Tyr Lys
               180                 185                 190
   Leu Thr Ser Cys Asn Thr Ser Val Ile Thr Gln Ala Cys Pro Lys Val
           195                 200                 205
   Ser Phe Glu Pro Ile Pro Ile His Tyr Cys Ala Pro Ala Gly Phe Ala
   210 215 220
   Ile Leu Lys Cys Asn Asn Lys Thr Phe Asn Gly Thr Gly Pro Cys Thr
   225 230 235 240
   Asn Val Ser Thr Val Gln Cys Thr His Gly Ile Arg Pro Val Val Ser
   245 250 255
   Thr Gln Leu Leu Leu Asn Gly Ser Leu Ala Glu Glu Glu Val Val Ile
   260 265 270
   Arg Ser Val Asn Phe Thr Asp Asn Ala Lys Thr Ile Ile Val Gln Leu
   275 280 285
   Asn Thr Ser Val Glu Ile Asn Cys Thr Arg Pro Asn Asn Asn Thr Arg
   290 295 300
   Lys Arg Ile Arg Ile Gln Arg Gly Pro Gly Arg Ala Phe Val Thr Ile
   305 310 315 320
   Gly Lys Ile Gly Asn Met Arg Gln Ala His Cys Asn Ile Ser Arg Ala
   325 330 335
   Lys Trp Asn Asn Thr Leu Lys Gln Ile Ala Ser Lys Leu Arg Glu Gln
   340 345 350
   Phe Gly Asn Asn Lys Thr Ile Ile Phe Lys Gln Ser Ser Gly Gly Asp
   355 360 365
   Pro Glu Ile Val Thr His Ser Phe Asn Cys Gly Gly Glu Phe Phe Tyr
   370 375 380
   Cys Asn Ser Thr Gln Leu Phe Asn Ser Thr Trp Phe Asn Ser Thr Trp
   385 390 395 400
   Ser Thr Glu Gly Ser Asn Asn Thr Glu Gly Ser Asp Thr Ile Thr Leu
   405 410 415
   Pro Cys Arg Ile Lys Gln Ile Ile Asn Met Trp Gln Lys Val Gly Lys
   420 425 430
   Ala Met Tyr Ala Pro Pro Ile Ser Gly Gln Ile Arg Cys Ser Ser Asn
   435 440 445
   Ile Thr Gly Leu Leu Leu Thr Arg Asp Gly Gly Asn Ser Asn Asn Glu
   450 455 460
   Ser Glu Ile Phe Arg Pro Gly Gly Gly Asp Met Arg Asp Asn Trp Arg
   465 470 475 480
   Ser Glu Leu Tyr Lys Tyr Lys Val Val Lys Ile Glu Pro Leu Gly Val
   485 490 495
   Ala Pro Thr Lys Ala Lys Arg Arg Val Val Gln Arg Glu Lys Arg Ala
   500 505 510
   Val Gly Ile Gly Ala Leu Phe Leu Gly Phe Leu Gly Ala Ala Gly Ser
   515 520 525
   Thr Met Gly Ala Ala Ser Met Thr Leu Thr Val Gln Ala Arg Gln Leu
   530 535 540
   Leu Ser Gly Ile Val Gln Gln Gln Asn Asn Leu Leu Arg Ala Ile Glu
   545 550 555 560
   Ala Gln Gln His Leu Leu Gln Leu Thr Val Trp Gly Ile Lys Gln Leu
   565 570 575
   Gln Ala Arg Ile Leu Ala Val Glu Arg Tyr Leu Lys Asp Gln Gln Leu
   580 585 590
   Leu Gly Ile Trp Gly Cys Ser Gly Lys Leu Ile Cys Thr Thr Ala Val
   595 600 605
   Pro Trp Asn Ala Ser Trp Ser Asn Lys Ser Leu Glu Gln Ile Trp Asn
   610 615 620
   His Thr Thr Trp Met Glu Trp Asp Arg Glu Ile Asn Asn Tyr Thr Ser
   625 630 635 640
   Leu Ile His Ser Leu Ile Glu Glu Ser Gln Asn Gln Gln Glu Lys Asn
   645 650 655
   Glu Gln Glu Leu Leu Glu Leu Asp Lys Trp Ala Ser Leu Trp Asn Trp
   660 665 670
   Phe Asn Ile Thr Asn Trp Leu Trp Tyr Ile Lys Leu Phe Ile Met Ile
   675 680 685
   Val Gly Gly Leu Val Gly Leu Arg Ile Val Phe Ala Val Leu Ser Ile
   690 695 700
   Val Asn Arg Val Arg Gln Gly Tyr Ser Pro Leu Ser Phe Gln Thr His
   705 710 715 720
   Leu Pro Thr Pro Arg Gly Pro Asp Arg Pro Glu Gly Ile Glu Glu Glu
   725 730 735
   Gly Gly Glu Arg Asp Arg Asp Arg Ser Ile Arg Leu Val Asn Gly Ser
               740                 745                 750
   Leu Ala Leu Ile Trp Asp Asp Leu Arg Ser Leu Cys Leu Phe Ser Tyr
           755                 760                 765
   His Arg Leu Arg Asp Leu Leu Leu Ile Val Thr Arg Ile Val Glu Leu
       770                 775                 780
   Leu Gly Arg Arg Gly Trp Glu Ala Leu Lys Tyr Trp Trp Asn Leu Leu
   785                 790                 795                 800
   Gln Tyr Trp Ser Gln Glu Leu Lys Asn Ser Ala Val Ser Leu Leu Asn
                   805                 810                 815
   Ala Thr Ala Ile Ala Val Ala Glu Gly Thr Asp Arg Val Ile Glu Val
               820                 825                 830
   Val Gln Gly Ala Cys Arg Ala Ile Arg His Ile Pro Arg Arg Ile Arg
           835                 840                 845
   Gln Gly Leu Glu Arg Ile Leu Leu
       850                 855
<210> 54
   <211> 2571
   <212> RNA
   <213> Human immunodeficiency virus type 1
<400> 54
   augagaguga aggagaaaua ucagcacuug uggagauggg gguggagaug gggcaccaug 60
   cuccuuggga uguugaugau cuguagugcu acagaaaaau ugugggucac agucuauuau 120
   gggguaccug uguggaagga agcaaccacc acucuauuuu gugcaucaga ugcuaaagca 180
   uaugauacag agguacauaa uguuugggcc acacaugccu guguacccac agaccccaac 240
   ccacaagaag uaguauuggu aaaugugaca gaaaauuuua acauguggaa aaaugacaug 300
   guagaacaga ugcaugagga uauaaucagu uuaugggauc aaagccuaaa gccaugugua 360
   aaauuaaccc cacucugugu uaguuuaaag ugcacugauu ugaagaauga uacuaauacc 420
   aauaguagua gcgggagaau gauaauggag aaaggagaga uaaaaaacug cucuuucaau 480
   aucagcacaa gcauaagagg uaaggugcag aaagaauaug cauuuuuuua uaaacuugau 540
   auaauaccaa uagauaauga uacuaccagc uauaaguuga caaguuguaa caccucaguc 600
   auuacacagg ccuguccaaa gguauccuuu gagccaauuc ccauacauua uugugccccg 660
   gcugguuuug cgauucuaaa auguaauaau aagacguuca auggaacagg accauguaca 720
   aaugucagca caguacaaug uacacaugga auuaggccag uaguaucaac ucaacugcug 780
   uuaaauggca gucuagcaga agaagaggua guaauuagau cugucaauuu cacggacaau 840
   gcuaaaacca uaauaguaca gcugaacaca ucuguagaaa uuaauuguac aagacccaac 900
   aacaauacaa gaaaaagaau ccguauccag agaggaccag ggagagcauu uguuacaaua 960
   ggaaaaauag gaaauaugag acaagcacau uguaacauua guagagcaaa auggaauaac 1020
   acuuuaaaac agauagcuag caaauuaaga gaacaauuug gaaauaauaa aacaauaauc 1080
   uuuaagcaau ccucaggagg ggacccagaa auuguaacgc acaguuuuaa uuguggaggg 1140
   gaauuuuucu acuguaauuc aacacaacug uuuaauagua cuugguuuaa uaguacuugg 1200
   aguacugaag ggucaaauaa cacugaagga agugacacaa ucacccuccc augcagaaua 1260
   aaacaaauua uaaacaugug gcagaaagua ggaaaagcaa uguaugcccc ucccaucagu 1320
   ggacaaauua gauguucauc aaauauuaca gggcugcuau uaacaagaga uggugguaau 1380
   agcaacaaug aguccgagau cuucagaccu ggaggaggag auaugaggga caauuggaga 1440
   agugaauuau auaaauauaa aguaguaaaa auugaaccau uaggaguagc acccaccaag 1500
   gcaaagagaa gaguggugca gagagaaaaa agagcagugg gaauaggagc uuuguuccuu 1560
   ggguucuugg gagcagcagg aagcacuaug ggcgcagccu caaugacgcu gacgguacag 1620
   gccagacaau uauugucugg uauagugcag cagcagaaca auuugcugag ggcuauugag 1680
   gcgcaacagc aucuguugca acucacaguc uggggcauca agcagcucca ggcaagaauc 1740
   cuggcugugg aaagauaccu aaaggaucaa cagcuccugg ggauuugggg uugcucugga 1800
   aaacucauuu gcaccacugc ugugccuugg aaugcuaguu ggaguaauaa aucucuggaa 1860
   cagauuugga aucacacgac cuggauggag ugggacagag aaauuaacaa uuacacaagc 1920
   uuaauacacu ccuuaauuga agaaucgcaa aaccagcaag aaaagaauga acaagaauua 1980
   uuggaauuag auaaaugggc aaguuugugg aauugguuua acauaacaaa uuggcugugg 2040
   uauauaaaau uauucauaau gauaguagga ggcuugguag guuuaagaau aguuuuugcu 2100
   guacuuucua uagugaauag aguuaggcag ggauauucac cauuaucguu ucagacccac 2160
   cucccaaccc cgaggggacc cgacaggccc gaaggaauag aagaagaagg uggagagaga 2220
   gacagagaca gauccauucg auuagugaac ggauccuugg cacuuaucug ggacgaucug 2280
   cggagccugu gccucuucag cuaccaccgc uugagagacu uacucuugau uguaacgagg 2340
   auuguggaac uucugggacg cagggggugg gaagcccuca aauauuggug gaaucuccua 2400
   caguauugga gucaggaacu aaagaauagu gcuguuagcu ugcucaaugc cacagccaua 2460
   gcaguagcug aggggacaga uaggguuaua gaaguaguac aaggagcuug uagagcuauu 2520
   cgccacauac cuagaagaau aagacagggc uuggaaagga uuuugcuaua a 2571
<210> 55
   <211> 860
   <212> PRT
   <213> Human immunodeficiency virus type 2
<400> 55
   Met Glu Pro Gly Arg Asn Gln Leu Phe Val Val Ile Leu Leu Thr Ser
   1               5                   10                  15
   Ala Cys Leu Val Tyr Cys Ser Gln Tyr Val Thr Val Phe Tyr Gly Ile
               20                  25                  30
   Pro Ala Trp Lys Asn Ala Ser Ile Pro Leu Phe Cys Ala Thr Lys Asn
           35                  40                  45
   Arg Asp Thr Trp Gly Thr Ile Gln Cys Leu Pro Asp Asn Asp Asp Tyr
       50                  55                  60
   Gln Glu Ile Ile Leu Asn Val Thr Glu Ala Phe Asp Ala Trp Asn Asn
   65                  70                  75                  80
   Thr Val Thr Glu Gln Ala Val Glu Asp Val Trp His Leu Phe Glu Thr
                   85                  90                  95
   Ser Ile Lys Pro Cys Val Lys Leu Thr Pro Leu Cys Val Ala Met Asn
               100                 105                 110
   Cys Ser Arg Val Gln Gly Asn Thr Thr Thr Pro Asn Pro Arg Thr Ser
           115                 120                 125
   Ser Ser Thr Thr Ser Arg Pro Pro Thr Ser Ala Ala Ser Ile Ile Asn
       130                 135                 140
   Glu Thr Ser Asn Cys Ile Glu Asn Asn Thr Cys Ala Gly Leu Gly Tyr
   145                 150                 155                 160
   Glu Glu Met Met Gln Cys Glu Phe Asn Met Lys Gly Leu Glu Gln Asp
                   165                 170                 175
   Lys Lys Arg Arg Tyr Lys Asp Thr Trp Tyr Leu Glu Asp Val Val Cys
               180                 185                 190
   Asp Asn Thr Thr Ala Gly Thr Cys Tyr Met Arg His Cys Asn Thr Ser
           195                 200                 205
   Ile Ile Lys Glu Ser Cys Asp Lys His Tyr Trp Asp Ala Met Arg Phe
       210                 215                 220
   Arg Tyr Cys Ala Pro Pro Gly Phe Ala Leu Leu Arg Cys Asn Asp Thr
   225                 230                 235                 240
   Asn Tyr Ser Gly Phe Glu Pro Lys Cys Thr Lys Val Val Ala Ala Ser
                   245                 250                 255
   Cys Thr Arg Met Met Glu Thr Gln Thr Ser Thr Trp Phe Gly Phe Asn
               260                 265                 270
   Gly Thr Arg Ala Glu Asn Arg Thr Tyr Ile Tyr Trp His Gly Arg Asp
           275                 280                 285
   Asn Arg Thr Ile Ile Ser Leu Asn Lys Tyr Tyr Asn Leu Thr Met Arg
       290                 295                 300
   Cys Lys Arg Pro Gly Asn Lys Thr Val Leu Pro Ile Thr Leu Met Ser
   305                 310                 315                 320
   Gly Leu Val Phe His Ser Gln Pro Ile Asn Thr Arg Pro Arg Gln Ala
                   325                 330                 335
   Trp Cys Arg Phe Gly Gly Arg Trp Arg Glu Ala Met Gln Glu Val Lys
               340                 345                 350
   Gln Thr Leu Val Gln His Pro Arg Tyr Lys Gly Ile Asn Asp Thr Gly
           355                 360                 365
   Lys Ile Asn Phe Thr Lys Pro Gly Ala Gly Ser Asp Pro Glu Val Ala
       370                 375                 380
   Phe Met Trp Thr Asn Cys Arg Gly Glu Phe Leu Tyr Cys Asn Met Thr
   385                 390                 395                 400
   Trp Phe Leu Asn Trp Val Glu Asp Lys Asn Gln Thr Arg Arg Asn Tyr
                   405                 410                 415
   Cys His Ile Lys Gln Ile Ile Asn Thr Trp His Lys Val Gly Lys Asn
               420                 425                 430
   Val Tyr Leu Pro Pro Arg Glu Gly Glu Leu Ala Cys Glu Ser Thr Val
           435                 440                 445
   Thr Ser Ile Ile Ala Asn Ile Asp Ile Asp Lys Asn Arg Thr His Thr
       450                 455                 460
   Asn Ile Thr Phe Ser Ala Glu Val Ala Glu Leu Tyr Arg Leu Glu Leu
   465                 470                 475                 480
   Gly Asp Tyr Lys Leu Ile Glu Ile Thr Pro Ile Gly Phe Ala Pro Thr
                   485                 490                 495
   Asp Gln Arg Arg Tyr Ser Ser Thr Pro Val Arg Asn Lys Arg Gly Val
               500                 505                 510
   Phe Val Leu Gly Phe Leu Gly Phe Leu Ala Thr Ala Gly Ser Ala Met
               515                 520                 525
   Gly Ala Arg Ser Leu Thr Leu Ser Ala Gln Ser Arg Thr Leu Leu Ala
       530                 535                 540
   Gly Ile Val Gln Gln Gln Gln Gln Leu Leu Asp Val Val Lys Arg Gln
   545                 550                 555                 560
   Gln Glu Met Leu Arg Leu Thr Val Trp Gly Thr Lys Asn Leu Gln Ala
                   565                 570                 575
   Arg Val Thr Ala Ile Glu Lys Tyr Leu Lys His Gln Ala Gln Leu Asn
               580                 585                 590
   Ser Trp Gly Cys Ala Phe Arg Gln Val Cys His Thr Thr Val Pro Trp
           595                 600                 605
   Val Asn Asp Ser Leu Ser Pro Asp Trp Lys Asn Met Thr Trp Gln Glu
       610                 615                 620
   Trp Glu Lys Gln Val Arg Tyr Leu Glu Ala Asn Ile Ser Gln Ser Leu
   625                 630                 635                 640
   Glu Glu Ala Gln Ile Gln Gln Glu Lys Asn Met Tyr Glu Leu Gln Lys
                   645                 650                 655
   Leu Asn Ser Trp Asp Ile Leu Gly Asn Trp Phe Asp Leu Thr Ser Trp
               660                 665                 670
   Val Lys Tyr Ile Gln Tyr Gly Val His Ile Val Val Gly Ile Ile Ala
           675                 680                 685
   Leu Arg Ile Ala Ile Tyr Val Val Gln Leu Leu Ser Arg Phe Arg Lys
       690                 695                 700
   Gly Tyr Arg Pro Val Phe Ser Ser Pro Pro Gly Tyr Leu Gln Gln Ile
   705                 710                 715                 720
   His Ile His Lys Asp Arg Gly Gln Pro Ala Asn Glu Gly Thr Glu Glu
                   725                 730                 735
   Asp Val Gly Gly Asp Ser Gly Tyr Asp Leu Trp Pro Trp Pro Ile Asn
               740                 745                 750
   Tyr Val Gln Phe Leu Ile His Leu Leu Thr Arg Leu Leu Ile Gly Leu
           755                 760                 765
   Tyr Asn Ile Cys Arg Asp Leu Leu Ser Lys Asn Ser Pro Thr Arg Arg
   770 775 780
   Leu Ile Ser Gln Ser Leu Thr Ala Ile Arg Asp Trp Leu Arg Leu Lys
   785 790 795 800
   Ala Ala Gln Leu Gln Tyr Gly Cys Glu Trp Ile Gln Glu Ala Phe Gln
   805 810 815
   Ala Phe Ala Arg Thr Thr Arg Glu Thr Leu Ala Gly Ala Trp Gly Trp
   820 825 830
   Leu Trp Glu Ala Ala Arg Arg Ile Gly Arg Gly Ile Leu Ala Val Pro
   835 840 845
   Arg Arg Ile Arg Gln Gly Ala Glu Leu Ala Leu Leu
   850 855 860
<210> 56
   <211> 2583
   <212> RNA
   <213> Human immunodeficiency virus type 2
<400> 56
   auggagccug guaggaauca gcuguuuguu gucauuuuac uaacaagugc uugcuuagua      60
   uauuguagcc aguaugugac uguuuucuau ggcauacccg cguggaaaaa ugcaucuauu     120
   cccuuauuuu gugcaacuaa aaauagagac acuuggggga ccauacagug cuugccagac     180
   aaugaugauu aucaggaaau aauuuuaaau gugacagagg cuuuugaugc auggaauaau     240
   acagugacag aacaagcagu agaagauguc uggcaucuau uugagacauc aauaaaacca     300
   ugugucaagc uaacaccucu auguguggca augaauugua gcaggguuca agggaauacc     360
   acgaccccga aucccaggac cucgaguucc acaaccucga gaccacccac auccgcagcc     420
   uccauaauaa augaaacuuc uaacugcaua gaaaacaaca caugcgcagg auuaggguau     480
   gaggagauga ugcaauguga guucaauaug aagggguuag aacaagauaa gaaaaggagg     540
   uauaaggaca caugguauuu agaagaugug guuugugaca acacaacagc uggcacaugu     600
   uacaugagac auugcaacac aucaaucauc aaagagucau gugauaagca cuauugggau     660
   gcuaugaggu uuagauacug ugcaccaccg ggcuuugccc uauuaagaug uaaugauacc     720
   aacuauucag gcuuugaacc uaagugcacu aaaguaguag cugcuucaug cacaaggaug     780
   auggaaacgc aaacuucuac uugguuuggc uuuaauggca cuagagcaga aaauagaaca     840
   uauaucuauu ggcauggcag agauaauagg acuaucauua gcuuaaacaa guauuauaau     900
   cucacaaugc guuguaagag accaggaaau aagacaguuu uaccaauaac acuuauguca     960
   ggauuagugu uucacucuca gccaaucaac acaaggccua ggcaggcaug gugccgguuu    1020
   ggaggcagau ggagggaagc caugcaggag gugaagcaaa cccuuguaca acaucccaga 1080
   uacaaaggaa ucaaugauac agggaaaauu aacuuuacga aaccgggagc aggcucagac 1140
   ccggaagugg cauuuaugug gacuaacugc agaggagaau uucucuacug uaacaugacu 1200
   ugguuccuca auuggguaga agacaagaac caaacacggc gcaacuauug ccauauaaag 1260
   cagauaauua auaccuggca uaaaguaggg aaaaauguau auuugccucc uagggaaggg 1320
   gaguuggccu gugaaucaac aguaaccagc auaauugcua acauugacau agauaaaaau 1380
   cggacucaua ccaacauuac cuuuagugca gaaguggcag aacuguaccg auuagaacug 1440
   ggagacuaca aauuaauaga aauaacacca auuggcuucg caccuacaga ucagagaagg 1500
   uacuccucaa cuccagugag gaacaaaaga gguguguucg ugcuaggguu cuuggguuuu 1560
   cucgcgacag cagguucugc aaugggcgcg cggucccuga cgcugucagc ccagucccgg 1620
   acuuuacugg ccgggauagu gcagcaacag caacagcugu uggacguagu caagagacaa 1680
   caagaaaugu ugcgacugac cgucugggga acgaaaaacc uccaggcaag agucacugcu 1740
   aucgagaagu accuaaagca ucaggcacag cuaaauucau ggggaugugc guuuagacag 1800
   gucugccaca cuacuguacc guggguaaau gacucuuuau cgccugacug gaaaaauaug 1860
   acauggcagg agugggagaa acaaguccgc uaccuagagg caaauaucag ucaaaguuua 1920
   gaagaagccc aaauucaaca agaaaagaau auguaugaau uacaaaaauu aaauagcugg 1980
   gauauucuug gcaacugguu ugacuuaacc uccuggguca aguauauuca auauggagug 2040
   cauauaguag ugggaauaau agcuuuaaga auagcaaucu auguagugca auuguuaagu 2100
   agauuuagaa agggcuauag gccuguuuuc ucuucccccc ccgguuaucu ccaacagauc 2160
   cauauccaca aggaccgggg acagccagcc aacgaaggaa cagaagaaga cgucggaggc 2220
   gacagugguu acgacuugug gccuuggcca auaaacuaug ugcaguuccu gauccaccua 2280
   cugacucgcc ucuugaucgg gcuauacaac aucugcagag acuuacuauc caagaacucc 2340
   ccgacccgcc gacugaucuc ccagagucua acagcaauca gggacuggcu gagacuuaag 2400
   gcggcccaac ugcaauaugg gugcgagugg auccaagaag cuuuccaagc auucgcgagg 2460
   acuacgagag agacucuugc gggcgcgugg ggaugguuau gggaagcagc gcgacgcauc 2520
   gggaggggaa uacucgcagu uccaagaaga aucaggcagg gagcagaacu cgcccuccug 2580
   uga 2583
<210> 57
   <211> 854
   <212> PRT
   <213> Simian immunodeficiency virus
<400> 57
   Met Arg Val Lys Glu Lys Tyr Gln His Leu Trp Arg Trp Gly Trp Arg
   1               5                   10                  15
   Trp Gly Thr Met Leu Leu Gly Met Leu Met Ile Cys Ser Ala Thr Glu
               20                  25                  30
   Lys Leu Trp Val Thr Val Tyr Tyr Gly Val Pro Val Trp Arg Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Gly Asn Val Thr Glu Asn Phe Asn Met Trp
                   85                  90                  95
   Lys Asn Asn Met Val Asp Gln Met His Glu Asp Ile Ile Ser Leu Trp
               100                 105                 110
   Asp Glu Ser Leu Lys Pro Cys Val Lys Leu Thr Pro Leu Cys Val Thr
           115                 120                 125
   Leu Asn Cys Thr Asn Leu Asn Ile Thr Lys Asn Thr Thr Asn Pro Thr
       130                 135                 140
   Ser Ser Ser Trp Gly Met Met Glu Lys Gly Glu Ile Lys Asn Cys Ser
   145                 150                 155                 160
   Phe Tyr Ile Thr Thr Ser Ile Arg Asn Lys Val Lys Lys Glu Tyr Ala
                   165                 170                 175
   Leu Phe Asn Arg Leu Asp Val Val Pro Ile Glu Asn Thr Asn Asn Thr
               180                 185                 190
   Lys Tyr Arg Leu Ile Ser Cys Asn Thr Ser Val Ile Thr Gln Ala Cys
           195                 200                 205
   Pro Lys Val Ser Phe Gln Pro Ile Pro Ile His Tyr Cys Val Pro Ala
       210                 215                 220
   Gly Phe Ala Met Leu Lys Cys Asn Asn Lys Thr Phe Asn Gly Ser Gly
   225                 230                 235                 240
   Pro Cys Thr Asn Val Ser Thr Val Gln Cys Thr His Gly Ile Arg Pro
                   245                 250                 255
   Val Val Ser Thr Gln Leu Leu Leu Asn Gly Ser Leu Ala Glu Glu Asp
               260                 265                 270
   Ile Val Ile Arg Ser Glu Asn Phe Thr Asp Asn Ala Lys Thr Ile Ile
           275                 280                 285
   Val Gln Leu Asn Glu Ser Val Val Ile Asn Cys Thr Arg Pro Asn Asn
       290                 295                 300
   Asn Thr Arg Arg Arg Leu Ser Ile Gly Pro Gly Arg Ala Phe Tyr Ala
   305                 310                 315                 320
   Arg Arg Asn Ile Ile Gly Asp Ile Arg Gln Ala His Cys Asn Ile Ser
                   325                 330                 335
   Arg Ala Lys Trp Asn Asn Thr Leu Gln Gln Ile Val Ile Lys Leu Arg
               340                 345                 350
   Glu Lys Phe Arg Asn Lys Thr Ile Ala Phe Asn Gln Ser Ser Gly Gly
           355                 360                 365
   Asp Pro Glu Ile Val Met His Ser Phe Asn Cys Gly Gly Glu Phe Phe
       370                 375                 380
   Tyr Cys Asn Thr Ala Gln Leu Phe Asn Ser Thr Trp Asn Val Thr Gly
   385                 390                 395                 400
   Gly Thr Asn Gly Thr Glu Gly Asn Asp Ile Ile Thr Leu Gln Cys Arg
                   405                 410                 415
   Ile Lys Gln Ile Ile Asn Met Trp Gln Lys Val Gly Lys Ala Met Tyr
               420                 425                 430
   Ala Pro Pro Ile Thr Gly Gln Ile Arg Cys Ser Ser Asn Ile Thr Gly
           435                 440                 445
   Leu Leu Leu Thr Arg Asp Gly Gly Asn Ser Thr Glu Thr Glu Thr Glu
       450                 455                 460
   Ile Phe Arg Pro Gly Gly Gly Asp Met Arg Asp Asn Trp Arg Ser Glu
   465                 470                 475                 480
   Leu Tyr Lys Tyr Lys Val Val Arg Ile Glu Pro Ile Gly Val Ala Pro
                   485                 490                 495
   Thr Arg Ala Lys Arg Arg Thr Val Gln Arg Glu Lys Arg Ala Val Gly
               500                 505                 510
   Ile Gly Ala Val Phe Leu Gly Phe Leu Gly Ala Ala Gly Ser Thr Met
           515                 520                 525
   Gly Ala Ala Ser Val Thr Leu Thr Val Gln Ala Arg Leu Leu Leu Ser
       530                 535                 540
   Gly Ile Val Gln Gln Gln Asn Asn Leu Leu Arg Ala Ile Glu Ala Gln
   545                 550                 555                 560
   Gln His Met Leu Gln Leu Thr Val Trp Gly Ile Lys Gln Leu Gln Ala
                   565                 570                 575
   Arg Val Leu Ala Leu Glu Arg Tyr Leu Arg Asp Gln Gln Leu Met Gly
               580                 585                 590
   Ile Trp Gly Cys Ser Gly Lys Leu Ile Cys Thr Thr Ser Val Pro Trp
           595                 600                 605
   Asn Val Ser Trp Ser Asn Lys Ser Val Asp Asp Ile Trp Asn Asn Met
       610                 615                 620
   Thr Trp Met Glu Trp Glu Arg Glu Ile Asp Asn Tyr Thr Asp Tyr Ile
   625                 630                 635                 640
   Tyr Asp Leu Leu Glu Lys Ser Gln Thr Gln Gln Glu Lys Asn Glu Lys
                   645                 650                 655
   Glu Leu Leu Glu Leu Asp Lys Trp Ala Ser Leu Trp Asn Trp Phe Asp
               660                 665                 670
   Ile Thr Asn Trp Leu Trp Tyr Ile Arg Leu Phe Ile Met Ile Val Gly
           675                 680                 685
   Gly Leu Ile Gly Leu Arg Ile Val Phe Ala Val Leu Ser Ile Val Asn
       690                 695                 700
   Arg Val Arg Gln Gly Tyr Ser Pro Leu Ser Phe Gln Thr Leu Leu Pro
   705                 710                 715                 720
   Ala Ser Arg Gly Pro Asp Arg Pro Glu Gly Thr Glu Glu Glu Gly Gly
                   725                 730                 735
   Glu Arg Asp Arg Asp Arg Ser Gly Pro Leu Val Asn Gly Ser Leu Ala
               740                 745                 750
   Leu Ile Trp Asp Asp Leu Arg Ser Leu Cys Leu Phe Ser Tyr His Arg
           755                 760                 765
   Leu Arg Asp Leu Leu Leu Ile Val Thr Arg Ile Val Glu Leu Leu Gly
       770                 775                 780
   Arg Arg Gly Trp Glu Ala Leu Lys Tyr Trp Trp Asn Leu Leu Gln Tyr
   785                 790                 795                 800
   Trp Ser Gln Glu Leu Lys Asn Ser Ala Val Ser Leu Leu Asn Ala Thr
   805 810 815
   Ala Ile Ala Val Ala Glu Gly Thr Asp Arg Val Ile Glu Val Val Gln
   820 825 830
   Gly Ala Cys Arg Ala Ile Arg His Ile Pro Arg Arg Ile Arg Gln Gly
   835 840 845
   Leu Glu Arg Ile Leu Leu
   850
<210> 58
   <211> 2565
   <212> DNA
   <213> Simian immunodeficiency virus
<400> 58
   atgagagtga aggagaaata tcagcacttg tggagatggg ggtggagatg gggcaccatg      60
   ctccttggga tgttgatgat ctgtagtgct acagaaaaat tgtgggtcac agtctattat     120
   ggggtacctg tgtggagaga agcaaccacc actctatttt gtgcatcaga tgctaaagcc     180
   tatgatacag aggtacataa tgtttgggcc acacatgcct gtgtacccac agaccccaac     240
   ccacaagaag tagtattggg aaatgtgaca gaaaatttta acatgtggaa aaataacatg     300
   gtagatcaga tgcatgagga tataatcagt ttatgggatg aaagcctaaa gccatgtgta     360
   aaattaaccc cactctgtgt tactttaaat tgcactaatt tgaatatcac taagaatact     420
   actaatccca ctagtagcag ctggggaatg atggagaaag gagaaataaa aaattgctct     480
   ttctatatca ccacaagcat aagaaataag gtaaagaaag aatatgcact ttttaataga     540
   cttgatgtag taccaataga aaatactaat aatactaagt ataggttaat aagttgtaac     600
   acctcagtca ttacacaggc ctgtccaaag gtatcctttc agccaattcc catacattat     660
   tgtgtcccgg ctgggtttgc gatgctaaag tgtaacaata agacattcaa tggatcagga     720
   ccatgcacaa atgtcagcac agtacaatgt acacatggaa ttaggccagt ggtgtcaact     780
   caactgctgt taaatggcag tctagcagaa gaagacatag taattagatc tgaaaatttc     840
   acagacaatg ctaaaaccat aatagtacag ctaaatgaat ctgtagtaat taattgtaca     900
   agacccaaca acaatacaag aagaaggtta tctataggac cagggagagc attttatgca     960
   agaagaaaca taataggaga tataagacaa gcacattgta acattagtag agcaaaatgg    1020
   aataacactt tacaacagat agttataaaa ttaagagaaa aatttaggaa taaaacaata    1080
   gcctttaatc aatcctcagg aggggaccca gaaattgtaa tgcacagttt taattgtgga    1140
   ggggaatttt tctactgtaa tacagcacaa ctgtttaata gtacttggaa tgttactgga    1200
   gggacaaatg gcactgaagg aaatgacata atcacactcc aatgcagaat aaaacaaatt    1260
   ataaatatgt ggcagaaagt aggaaaagca atgtatgccc ctcccatcac aggacaaatt 1320
   agatgttcat caaatattac agggctgcta ctaacaagag atggaggtaa tagtactgag 1380
   actgagactg agatcttcag acctggagga ggagatatga gggacaattg gagaagtgaa 1440
   ttatataaat ataaagtagt aagaattgaa ccaataggag tagcacccac cagggcaaag 1500
   agaagaacag tgcaaagaga aaaaagagca gtgggaatag gagctgtgtt ccttgggttc 1560
   ttgggagcag caggaagcac tatgggcgca gcgtcagtga cgctgacggt acaggccagg 1620
   ctattattgt ctggtatagt gcagcagcag aacaatctgc tgagggctat tgaggcgcaa 1680
   cagcatatgt tgcaactcac agtctggggc atcaagcagc tccaggcaag agtcctggct 1740
   ctggaaagat acctaaggga tcaacagctc atgggaattt ggggttgctc tggaaaactc 1800
   atttgcacca cttctgtgcc ttggaatgtt agttggagta ataaatctgt ggatgatatt 1860
   tggaataaca tgacctggat ggagtgggaa agagaaattg acaattacac agactatata 1920
   tatgacttac ttgaaaaatc gcaaacccaa caagaaaaga atgaaaaaga attattggaa 1980
   ttggataaat gggcaagttt gtggaattgg tttgacataa caaactggct gtggtatata 2040
   agattattca taatgatagt aggaggcttg ataggtttaa gaatagtttt tgctgtactt 2100
   tctatagtaa atagagttag gcagggatat tcaccattat cgtttcagac cctcctccca 2160
   gcctcgaggg gacccgacag gcccgaagga acagaagaag aaggtggaga gagagacaga 2220
   gacagatccg gtccattagt gaacggatcc ttggcactta tctgggacga tctgcggagc 2280
   ctgtgcctct tcagctacca ccgcttgaga gacttactct tgattgtaac gaggattgtg 2340
   gaacttctgg gacgcagggg gtgggaagcc ctcaaatatt ggtggaatct cctacagtat 2400
   tggagtcagg aactaaagaa tagtgctgtt agcttgctca atgccacagc catagcagta 2460
   gctgagggga cagatagggt tatagaagta gtacaaggag cttgtagagc tattcgccac 2520
   atacctagaa gaataagaca gggcttggaa aggattttgc tataa 2565
<210> 59
   <211> 488
   <212> PRT
   <213> Human T-cell lymphotropic virus type 1
<400> 59
   Met Gly Lys Phe Leu Ala Thr Leu Ile Leu Phe Phe Gln Phe Cys Pro
   1               5                   10                  15
   Leu Ile Leu Gly Asp Tyr Ser Pro Ser Cys Cys Thr Leu Thr Val Gly
               20                  25                  30
   Val Ser Ser Tyr His Ser Lys Pro Cys Asn Pro Ala Gln Pro Val Cys
           35                  40                  45
   Ser Trp Thr Leu Asp Leu Leu Ala Leu Ser Ala Asp Gln Ala Leu Gln
       50                  55                  60
   Pro Pro Cys Pro Asn Leu Val Ser Tyr Ser Ser Tyr His Ala Thr Tyr
   65                  70                  75                  80
   Ser Leu Tyr Leu Phe Pro His Trp Ile Lys Lys Pro Asn Arg Asn Gly
                   85                  90                  95
   Gly Gly Tyr Tyr Ser Ala Ser Tyr Ser Asp Pro Cys Ser Leu Lys Cys
               100                 105                 110
   Pro Tyr Leu Gly Cys Gln Ser Trp Thr Cys Pro Tyr Thr Gly Ala Val
           115                 120                 125
   Ser Ser Pro Tyr Trp Lys Phe Gln Gln Asp Val Asn Phe Thr Gln Glu
       130                 135                 140
   Val Ser His Leu Asn Ile Asn Leu His Phe Ser Lys Cys Gly Phe Ser
   145                 150                 155                 160
   Phe Ser Leu Leu Val Asp Ala Pro Gly Tyr Asp Pro Ile Trp Phe Leu
                   165                 170                 175
   Asn Thr Glu Pro Ser Gln Leu Pro Pro Thr Ala Pro Pro Leu Leu Ser
               180                 185                 190
   His Ser Asn Leu Asp His Ile Leu Glu Pro Ser Ile Pro Trp Lys Ser
           195                 200                 205
   Lys Leu Leu Thr Leu Val Gln Leu Thr Leu Gln Ser Thr Asn Tyr Thr
       210                 215                 220
   Cys Ile Val Cys Ile Asp Arg Ala Ser Leu Ser Thr Trp His Val Leu
   225                 230                 235                 240
   Tyr Ser Pro Asn Val Ser Val Pro Ser Pro Ser Ser Thr Pro Leu Leu
                   245                 250                 255
   Tyr Pro Ser Leu Ala Leu Pro Ala Pro His Leu Thr Leu Pro Phe Asn
               260                 265                 270
   Trp Thr His Cys Phe Asp Pro Gln Ile Gln Ala Ile Val Ser Ser Pro
           275                 280                 285
   Cys His Asn Ser Leu Ile Leu Pro Pro Phe Ser Leu Ser Pro Val Pro
       290                 295                 300
   Thr Leu Gly Ser Arg Ser Arg Arg Ala Val Pro Val Ala Val Trp Leu
   305                 310                 315                 320
   Val Ser Ala Leu Ala Met Gly Ala Gly Val Ala Gly Arg Ile Thr Gly
                   325                 330                 335
   Ser Met Ser Leu Ala Ser Gly Lys Ser Leu Leu His Glu Val Asp Lys
               340                 345                 350
   Asp Ile Ser Gln Leu Thr Gln Ala Ile Val Lys Asn His Lys Asn Leu
           355                 360                 365
   Leu Lys Ile Ala Gln Tyr Ala Ala Gln Asn Arg Arg Gly Leu Asp Leu
       370                 375                 380
   Leu Phe Trp Glu Gln Gly Gly Leu Cys Lys Ala Leu Gln Glu Gln Cys
   385                 390                 395                 400
   Cys Phe Leu Asn Ile Thr Asn Ser His Val Ser Ile Leu Gln Glu Arg
                   405                 410                 415
   Pro Pro Leu Glu Asn Arg Val Leu Thr Gly Trp Gly Leu Asn Trp Asp
               420                 425                 430
   Leu Gly Leu Ser Gln Trp Ala Arg Glu Ala Leu Gln Thr Gly Ile Thr
           435                 440                 445
   Leu Val Ala Leu Leu Leu Leu Val Ile Leu Ala Gly Pro Cys Ile Leu
       450                 455                 460
   Arg Gln Leu Arg His Leu Pro Ser Arg Val Arg Tyr Pro His Tyr Ser
   465                 470                 475                 480
   Leu Ile Asn Pro Glu Ser Ser Leu
                   485
<210> 60
   <211> 1467
   <212> DNA
   <213> Human T-cell lymphotropic virus type 1
<400> 60
   atgggtaagt ttctcgccac tttgatttta ttcttccagt tctgccccct catcctcggt      60
   gattacagcc ccagctgctg tactctcaca gttggagtct cctcatacca ctctaaaccc     120
   tgcaatcctg cccagccagt ttgttcatgg accctcgacc tgctggccct ttcagcagat     180
   caggccctac agccaccctg ccctaatcta gtaagttact ccagctacca tgccacctat     240
   tccctatatc tattccctca ttggatcaaa aagccaaacc gaaatggcgg aggctattat     300
   tcagcctctt attcagaccc ttgttcctta aaatgcccat acctagggtg ccaatcatgg     360
   acctgcccct atacaggagc cgtctccagc ccctactgga aatttcagca agatgtcaat     420
   tttactcaag aagtttcaca cctcaatatt aatctccatt tttcaaaatg cggtttttcc     480
   ttctcccttc tagtcgacgc tccaggatat gaccccatct ggttccttaa taccgaaccc     540
   agccaactgc ctcccaccgc ccctcctcta ctctcccact ctaacctaga ccatatcctc     600
   gagccctcta taccatggaa atcaaaactc ctgactcttg tccagttaac cctacaaagc     660
   actaattata cttgcattgt ctgtatcgat cgtgccagcc tatccacttg gcacgtccta     720
   tactctccca acgtctctgt tccatcccct tcttctaccc ccctccttta cccatcgtta     780
   gcgcttccag ccccccacct gacgttacca tttaactgga cccactgctt tgacccccag     840
   attcaagcta tagtctcctc cccctgtcat aactccctca tcctgccccc cttttccttg     900
   tcacctgttc ccacgctagg atcccgctcc cgccgagcag taccggtggc ggtctggctt     960
   gtctccgccc tggccatggg agccggagtg gctggcagga ttaccggctc catgtccctc    1020
   gcctcaggaa agagcctcct acatgaggtg gacaaagata tttcccaatt aactcaagca    1080
   atagtcaaaa accacaaaaa tctgctcaaa attgcacagt atgctgccca gaacagacga    1140
   ggccttgatc tcctgttctg ggagcaagga ggattatgca aagcattaca agaacagtgc    1200
   tgttttctaa atattactaa ttcccatgtc tcaatactac aagagagacc cccccttgaa    1260
   aatcgagtcc tgactggctg gggccttaac tgggaccttg gcctctcaca gtgggctcga    1320
   gaagccttac aaactggaat cacccttgtc gcgctactcc ttcttgttat ccttgcagga    1380
   ccatgcatcc tccgtcagct acgacacctc ccctcgcgcg tcagataccc ccattactct    1440
   cttataaacc ctgagtcatc cctgtaa                                        1467
<210> 61
   <211> 486
   <212> PRT
   <213> Human T-cell lymphotropic virus type 2
<400> 61
   Met Gly Asn Val Phe Phe Leu Leu Leu Phe Ser Leu Thr His Phe Pro
   1               5                   10                  15
   Leu Ala Gln Gln Ser Arg Cys Thr Leu Thr Ile Gly Ile Ser Ser Tyr
               20                  25                  30
   His Ser Ser Pro Cys Ser Pro Thr Gln Pro Val Cys Thr Trp Asn Leu
           35                  40                  45
   Asp Leu Asn Ser Leu Thr Thr Asp Gln Arg Leu His Pro Pro Cys Pro
       50                  55                  60
   Asn Leu Ile Thr Tyr Ser Gly Phe His Lys Thr Tyr Ser Leu Tyr Leu
   65                  70                  75                  80
   Phe Pro His Trp Ile Lys Lys Pro Asn Arg Gln Gly Leu Gly Tyr Tyr
                   85                  90                  95
   Ser Pro Ser Tyr Asn Asp Pro Cys Ser Leu Gln Cys Pro Tyr Leu Gly
               100                 105                 110
   Cys Gln Ala Trp Thr Ser Ala Tyr Thr Gly Pro Val Ser Ser Pro Ser
           115                 120                 125
   Trp Lys Phe His Ser Asp Val Asn Phe Thr Gln Glu Val Ser Gln Val
       130                 135                 140
   Ser Leu Arg Leu His Phe Ser Lys Cys Gly Ser Ser Met Thr Leu Leu
   145                 150                 155                 160
   Val Asp Ala Pro Gly Tyr Asp Pro Leu Trp Phe Ile Thr Ser Glu Pro
                   165                 170                 175
   Thr Gln Pro Pro Pro Thr Ser Pro Pro Leu Val His Asp Ser Asp Leu
               180                 185                 190
   Glu His Val Leu Thr Pro Ser Thr Ser Trp Thr Thr Lys Ile Leu Lys
           195                 200                 205
   Phe Ile Gln Leu Thr Leu Gln Ser Thr Asn Tyr Ser Cys Met Val Cys
       210                 215                 220
   Val Asp Arg Ser Ser Leu Ser Ser Trp His Val Leu Tyr Thr Pro Asn
   225                 230                 235                 240
   Ile Ser Ile Pro Gln Gln Thr Ser Ser Arg Thr Ile Leu Phe Pro Ser
                   245                 250                 255
   Leu Ala Leu Pro Ala Pro Pro Ser Gln Pro Phe Pro Trp Thr His Cys
               260                 265                 270
   Tyr Gln Pro Arg Leu Gln Ala Ile Thr Thr Asp Asn Cys Asn Asn Ser
           275                 280                 285
   Ile Ile Leu Pro Pro Phe Ser Leu Ala Pro Val Pro Pro Pro Ala Thr
       290                 295                 300
   Arg Arg Arg Arg Ala Val Pro Ile Ala Val Trp Leu Val Ser Ala Leu
   305                 310                 315                 320
   Ala Ala Gly Thr Gly Ile Ala Gly Gly Val Thr Gly Ser Leu Ser Leu
                   325                 330                 335
   Ala Ser Ser Lys Ser Leu Leu Leu Glu Val Asp Lys Asp Ile Ser His
   340 345 350
   Leu Thr Gln Ala Ile Val Lys Asn His Gln Asn Ile Leu Arg Val Ala
   355 360 365
   Gln Tyr Ala Ala Gln Asn Arg Arg Gly Leu Asp Leu Leu Phe Trp Glu
   370 375 380
   Gln Gly Gly Leu Cys Lys Ala Ile Gln Glu Gln Cys Cys Phe Leu Asn
   385 390 395 400
   Ile Ser Asn Thr His Val Ser Val Leu Gln Glu Arg Pro Pro Leu Glu
   405 410 415
   Lys Arg Val Ile Thr Gly Trp Gly Leu Asn Trp Asp Leu Gly Leu Ser
   420 425 430
   Gln Trp Ala Arg Glu Ala Leu Gln Thr Gly Ile Thr Ile Leu Ala Leu
   435 440 445
   Leu Leu Leu Val Ile Leu Phe Gly Pro Cys Ile Leu Arg Gln Ile Gln
   450 455 460
   Ala Leu Pro Gln Arg Leu Gln Asn Arg His Asn Gln Tyr Ser Leu Ile
   465 470 475 480
   Asn Pro Glu Thr Met Leu
   485
<210> 62
   <211> 1461
   <212> RNA
   <213> Human T-cell lymphotropic virus type 2
<400> 62
   auggguaaug uuuucuuccu acuuuuauuc agucucacac auuuuccacu agcccagcag      60
   agccgaugca cacucacgau ugguaucucc uccuaccacu ccagccccug uagcccaacc     120
   caacccgucu gcacguggaa ccucgaccuu aauucccuaa caacggacca acgacuacac     180
   ccccccugcc cuaaccuaau uacuuacucu ggcuuccaua agacuuauuc cuuauacuua     240
   uucccacauu ggauaaaaaa gccaaacaga cagggccuag gguacuacuc gccuuccuac     300
   aaugacccuu gcucgcuaca augccccuac uugggcugcc aagcauggac auccgcauac     360
   acgggccccg ucuccagucc auccuggaag uuucauucag auguaaauuu cacccaggaa     420
   gucagccaag ugucccuucg acuacacuuc ucuaagugcg gcuccuccau gacccuccua     480
   guagaugccc cuggauauga uccuuuaugg uucaucaccu cagaacccac ucagccucca     540
   ccaacuucuc ccccauuggu ccaugacucc gaccuugaac auguccuaac ccccuccacg     600
   uccuggacga ccaaaauacu caaauuuauc cagcugaccu uacagagcac caauuacucc     660
   ugcaugguuu gcguggauag auccagccuc ucauccuggc auguacucua cacccccaac     720
   aucuccauuc cccaacaaac cuccucccga accauccucu uuccuucccu ugcccugccc     780
   gcuccuccau cccaacccuu cccuuggacc cauugcuacc aaccucgccu acaggcgaua     840
   acaacagaua acugcaacaa cuccauuauc cuccccccuu uuucccucgc ucccguaccu     900
   ccuccggcga caagacgccg ccgugccguu ccaauagcag uguggcuugu cuccgcccua     960
   gcggccggaa cagguaucgc ugguggagua acaggcuccc uaucucuggc uuccaguaaa    1020
   agccuucucc ucgagguuga caaagacauc ucccaccuua cccaggccau agucaaaaau    1080
   caucaaaaca uccuccgggu ugcacaguau gcagcccaaa auagacgagg auuagaccuc    1140
   cuauucuggg aacaaggggg uuugugcaag gccauacagg agcaauguug cuuccucaac    1200
   aucaguaaca cucauguauc cguccuccag gaacggcccc cucuugaaaa acgugucauc    1260
   accggcuggg gacuaaacug ggaucuugga cugucccaau gggcacgaga agcccuccag    1320
   acaggcauaa ccauucucgc ucuacuccuc cucgucauau uguuuggccc cuguauccuc    1380
   cgccaaaucc aggcccuucc acagcgguua caaaaccgac auaaccagua uucccuuauc    1440
   aacccagaaa ccaugcuaua a                                              1461
<210> 63
   <211> 485
   <212> PRT
   <213> Human T-cell lymphotropic virus type 4
<400> 63
   Met Gly Asn Val Leu Phe Leu Thr Leu Leu Ala Thr Leu Gly Ile Pro
   1 5 10 15
   Val Leu Gln Ala Ser Arg Cys Thr Ile Thr Val Gly Ile Ser Ser Tyr
   20 25 30
   His Ser Ser Pro Cys Ser Pro Ala Gln Pro Leu Cys Thr Trp Ala Leu
   35 40 45
   Asp Leu Val Ser Ile Thr Lys Asp Gln Leu Leu Tyr Pro Pro Cys Gln
   50 55 60
   Asn Leu Ile Thr Tyr Ser Asn Tyr His Lys Thr Tyr Ser Leu Tyr Leu
   65 70 75 80
   Phe Pro His Trp Val Gln Lys Pro Leu Arg Arg Gly Leu Gly Tyr Tyr
   85 90 95
   Ser Ala Ser Tyr Ser Asp Pro Cys Ser Leu Gln Cys Pro Tyr Leu Gly
   100 105 110
   Ser Gln Ser Trp Thr Cys Pro Tyr Thr Gly Pro Val Ser Ser Pro Thr
           115                 120                 125
   Trp Arg Phe Ser Thr Asp Val Asn Phe Thr Gln Glu Val Ser Arg Val
       130                 135                 140
   Ser Leu Lys Leu His Phe Ser Lys Cys Gly Ser Ser Leu Thr Leu Leu
   145                 150                 155                 160
   Ile Asp Ala Pro Gly Tyr Asp Pro Leu Trp Tyr Leu Thr Ser Glu Pro
                   165                 170                 175
   Thr Gln Glu Pro Pro Thr Pro Pro Pro Leu Val Ser Asp Ser Asp Leu
               180                 185                 190
   Glu His Val Leu Thr Pro Ser Ala Ser Trp Ala Ser Lys Met Leu Thr
           195                 200                 205
   Leu Ile His Leu Thr Leu Gln Ser Thr Asn Tyr Ser Cys Met Val Cys
       210                 215                 220
   Ile Asp Arg Ala Ser Leu Ser Ser Trp His Val Leu Tyr Thr Pro Asn
   225                 230                 235                 240
   Ile Ser Ser Asn Ala Pro Ser Lys Pro Ile Val Arg Pro Ser Leu Ala
                   245                 250                 255
   Leu Ser Ala Pro Arg Pro Gln Pro Phe Pro Trp Thr His Cys Tyr Gln
               260                 265                 270
   Pro Gln Val Gln Ala Val Thr Thr Ala Lys Cys Asn Asn Ser Ile Ile
           275                 280                 285
   Leu Pro Pro Phe Ser Leu Ser Pro Leu Pro Gly Ala Pro Leu Thr Arg
       290                 295                 300
   Arg Arg Arg Ala Val Pro Val Ala Val Trp Leu Val Ser Ala Leu Ala
   305                 310                 315                 320
   Ala Gly Thr Gly Ile Ala Gly Gly Val Thr Gly Ser Leu Ser Leu Ala
                   325                 330                 335
   Ser Ser Arg Ser Leu Leu Ser Glu Val Asp Lys Asp Ile Ser His Leu
               340                 345                 350
   Thr Arg Ala Ile Val Lys Asn His Gln Asn Ile Leu Arg Val Ala Gln
           355                 360                 365
   Tyr Ala Ala Gln Asn Arg Arg Gly Leu Asp Leu Leu Phe Trp Glu Gln
       370                 375                 380
   Gly Gly Leu Cys Lys Ala Ile Gln Glu Gln Cys Cys Phe Leu Asn Ile
   385                 390                 395                 400
   Ser Asn Thr His Ile Ser Val Leu Gln Glu Arg Pro Pro Leu Glu Thr
                   405                 410                 415
   Arg Val Thr Thr Gly Trp Gly Leu Asn Trp Asp Leu Gly Leu Ser Gln
               420                 425                 430
   Trp Ala Arg Glu Ala Leu Gln Thr Gly Ile Thr Leu Leu Ala Leu Leu
           435                 440                 445
   Leu Leu Ile Ile Ile Leu Gly Pro Cys Ile Ile Arg Gln Leu Gln Ala
       450                 455                 460
   Leu Pro Gln Arg Leu Gln Gln Arg Pro Asp Gln Tyr Pro Leu Leu Asn
   465                 470                 475                 480
   Pro Glu Thr Pro Leu
                   485
<210> 64
   <211> 1458
   <212> DNA
   <213> Human T-cell lymphotropic virus type 4
<400> 64
   atgggtaacg tactcttctt aactttattg gccaccctgg gcatcccagt acttcaggcc      60
   agccggtgta caatcacggt aggtatctcc tcctaccact ccagcccctg cagcccagcc     120
   cagcctttat gtacctgggc cctcgacctt gtgtccatca ctaaggacca gctcctctac     180
   cccccctgcc aaaacctgat cacctattcc aactaccaca agacctactc cctgtatctc    240
   ttcccacact gggtacaaaa gccactccgc cgggggcttg gatactactc agcctcctac     300
   tctgatcctt gctccctaca atgtccctac ctaggaagtc aatcatggac ttgcccctat     360
   actggccctg tctcgagccc aacttggaga ttctccacag atgtaaattt cacccaagaa     420
   gtcagccgtg tctccctaaa acttcatttc tccaaatgtg gttcctcctt aactctgtta     480
   atagatgccc ccggttacga tccgctgtgg tacctcacat ccgagcctac tcaggaaccc     540
   ccaacccctc cgccactagt cagcgactca gacctagagc atgtcctgac tccttcggcc     600
   tcctgggcct ccaagatgct gaccctcatc cacctaacct tgcagagcac caactattcc     660
   tgtatggtct gtattgaccg cgccagcctc tcttcctggc acgtattata cactcccaac     720
   atctctagta atgccccctc aaaacccatc gtccgccctt cccttgccct atccgccccg      780
   cgaccacagc ccttcccctg gacccattgc tatcaaccac aggtgcaagc tgtaaccacc     840
   gcaaagtgca ataattccat catacttccc ccattttctc tctctccctt gcctggtgcc     900
   cctctcacta ggcgacgccg ggccgtccca gtggcggtct ggctcgtttc cgctttggcc     960
   gcagggacag gaatagcagg aggtgtcacc gggtccttat ccctggcctc cagtagaagt    1020
   ctcctgtccg aagtggacaa ggatatttcc cacctcacac gggccattgt aaaaaaccac    1080
   caaaacattc ttcgagtggc ccaatatgcc gcccaaaaca ggcgagggtt agacctcctg    1140
   ttctgggaac aaggggggct gtgtaaagcg atacaagaac aatgctgctt cctcaacatc   1200
   agcaataccc atatttcagt cttacaagag cgaccccctc tagaaactcg ggtaactact   1260
   ggatggggct taaattggga tctaggactc tcccagtggg cccgtgaggc tctccagact    1320
   ggtattaccc ttttggccct ccttctgtta atcatcatcc tcgggccctg cattattcgc    1380
   cagctgcaag ccctccccca gaggctacag cagcgacctg accagtaccc tctcctcaac    1440
   cctgagaccc ctttataa                                                  1458
<210> 65
   <211> 565
   <212> PRT
   <213> Endogenous feline virus
<400> 65
   Met Lys Leu Pro Thr Gly Met Val Ile Leu Cys Ser Leu Ile Ile Val
   1               5                   10                  15
   Arg Ala Gly Phe Asp Asp Pro Arg Lys Ala Ile Ala Leu Val Gln Lys
               20                  25                  30
   Gln His Gly Lys Pro Cys Glu Cys Ser Gly Gly Gln Val Ser Glu Ala
           35                  40                  45
   Pro Pro Asn Ser Ile Gln Gln Val Thr Cys Pro Gly Lys Thr Ala Tyr
       50                  55                  60
   Leu Met Thr Asn Gln Lys Trp Lys Cys Arg Val Thr Pro Lys Asn Leu
   65                  70                  75                  80
   Thr Pro Ser Gly Gly Glu Leu Gln Asn Cys Pro Cys Asn Thr Phe Gln
                   85                  90                  95
   Asp Ser Met His Ser Ser Cys Tyr Thr Glu Tyr Arg Gln Cys Arg Ala
               100                 105                 110
   Asn Asn Lys Thr Tyr Tyr Thr Ala Thr Leu Leu Lys Ile Arg Ser Gly
           115                 120                 125
   Ser Leu Asn Glu Val Gln Ile Leu Gln Asn Pro Asn Gln Leu Leu Gln
   130 135 140
   Ser Pro Cys Arg Gly Ser Ile Asn Gln Pro Val Cys Trp Ser Ala Thr
   145 150 155 160
   Ala Pro Ile His Ile Ser Asp Gly Gly Gly Pro Leu Asp Thr Lys Arg
   165 170 175
   Val Trp Thr Val Gln Lys Arg Leu Glu Gln Ile His Lys Ala Met His
   180 185 190
   Pro Glu Leu Gln Tyr His Pro Leu Ala Leu Pro Lys Val Arg Asp Asp
   195 200 205
   Leu Ser Leu Asp Ala Arg Thr Phe Asp Ile Leu Asn Thr Thr Phe Arg
   210 215 220
   Leu Leu Gln Met Ser Asn Phe Ser Leu Ala Gln Asp Cys Trp Leu Cys
   225 230 235 240
   Leu Lys Leu Gly Thr Pro Thr Pro Leu Ala Ile Pro Thr Pro Ser Leu
   245 250 255
   Thr Tyr Ser Leu Ala Asp Ser Leu Ala Asn Ala Ser Cys Gln Ile Ile
   260 265 270
   Pro Pro Leu Leu Val Gln Pro Met Gln Phe Ser Asn Ser Ser Cys Leu
   275 280 285
   Ser Ser Pro Phe Ile Asn Asp Thr Glu Gln Ile Asp Leu Gly Ala Val
   290 295 300
   Thr Phe Thr Asn Cys Thr Ser Val Ala Asn Val Ser Ser Pro Leu Cys
   305 310 315 320
   Ala Leu Asn Gly Ser Val Phe Leu Cys Gly Asn Asn Met Ala Tyr Thr
   325 330 335
   Tyr Leu Pro Gln Asn Trp Thr Gly Leu Cys Val Gln Ala Ser Leu Leu
   340 345 350
   Pro Asp Ile Asp Ile Ile Pro Gly Asp Glu Pro Val Pro Ile Pro Ala
   355 360 365
   Ile Asp His Tyr Ile His Arg Pro Lys Arg Ala Val Gln Phe Ile Pro
   370 375 380
   Leu Leu Ala Gly Leu Gly Ile Thr Ala Ala Phe Thr Thr Gly Ala Thr
   385 390 395 400
   Gly Leu Gly Val Ser Val Thr Gln Tyr Thr Lys Leu Ser His Gln Leu
   405 410 415
   Ile Ser Asp Val Gln Val Leu Ser Gly Thr Ile Gln Asp Leu Gln Asp
   420 425 430
   Gln Val Asp Ser Leu Ala Glu Val Val Leu Gln Asn Arg Arg Gly Leu
   435 440 445
   Asp Leu Leu Thr Ala Glu Gln Gly Gly Ile Cys Leu Ala Leu Gln Glu
   450 455 460
   Lys Cys Cys Phe Tyr Ala Asn Lys Ser Gly Ile Val Arg Asn Lys Ile
   465 470 475 480
   Arg Thr Leu Gln Glu Glu Leu Gln Lys Arg Arg Glu Ser Leu Ala Ser
   485 490 495
   Asn Pro Leu Trp Thr Gly Leu Gln Gly Phe Leu Pro Tyr Leu Leu Pro
   500 505 510
   Leu Leu Gly Pro Leu Leu Thr Leu Leu Leu Ile Leu Thr Ile Gly Pro
   515 520 525
   Cys Val Phe Ser Arg Leu Met Ala Phe Ile Asn Asp Arg Leu Asn Val
   530 535 540
   Val His Ala Met Val Leu Ala Gln Gln Tyr Gln Ala Leu Lys Ala Glu
   545 550 555 560
   Glu Glu Ala Gln Asp
   565
<210> 66
   <211> 1698
   <212> DNA
   <213> Endogenous feline virus
<400> 66
   atgaaactcc caacaggaat ggtcatttta tgtagcctaa taatagttcg ggcagggttt 60
   gacgaccccc gcaaggctat cgcattagta caaaaacaac atggtaaacc atgcgaatgc 120
   agcggagggc aggtatccga ggccccaccg aactccatcc aacaggtaac ttgcccaggc 180
   aagacggcct acttaatgac caaccaaaaa tggaaatgca gagtcactcc aaaaaatctc 240
   acccctagcg ggggagaact ccagaactgc ccctgtaaca ctttccagga ctcgatgcac 300
   agttcttgtt atactgaata ccggcaatgc agggcgaata ataagacata ctacacggcc 360
   accttgctta aaatacggtc tgggagcctc aacgaggtac agatattaca aaaccccaat 420
   cagctcctac agtccccttg taggggctct ataaatcagc ccgtttgctg gagtgccaca 480
   gcccccatcc atatctccga tggtggagga cccctcgata ctaagagagt gtggacagtc 540
   caaaaaaggc tagaacaaat tcataaggct atgcatcctg aacttcaata ccacccctta 600
   gccctgccca aagtcagaga tgaccttagc cttgatgcac ggacttttga tatcctgaat 660
   accactttta ggttactcca gatgtccaat tttagccttg cccaagattg ttggctctgt 720
   ttaaaactag gtacccctac ccctcttgcg atacccactc cctctttaac ctactcccta 780
   gcagactccc tagcgaatgc ctcctgtcag attatacctc ccctcttggt tcaaccgatg 840
   cagttctcca actcgtcctg tttatcttcc cctttcatta acgatacgga acaaatagac 900
   ttaggtgcag tcacctttac taactgcacc tctgtagcca atgtcagtag tcctttatgt 960
   gccctaaacg ggtcagtctt cctctgtgga aataacatgg catacaccta tttaccccaa 1020
   aactggacag gactttgcgt ccaagcctcc ctcctccccg acattgacat catcccgggg 1080
   gatgagccag tccccattcc tgccattgat cattatatac atagacctaa acgagctgta 1140
   cagttcatcc ctttactagc tggactggga atcaccgcag cattcaccac cggagctaca 1200
   ggcctaggtg tctccgtcac ccagtataca aaattatccc atcagttaat atctgatgtc 1260
   caagtcttat ccggtaccat acaagattta caagaccagg tagactcgtt agctgaagta 1320
   gttctccaaa ataggagggg actggaccta ctaacggcag aacaaggagg aatttgttta 1380
   gccttacaag aaaaatgctg tttttatgct aacaagtcag gaattgtgag aaacaaaata 1440
   agaaccctac aagaagaatt acaaaaacgc agggaaagcc tggcatccaa ccctctctgg 1500
   accgggctgc agggctttct tccgtacctc ctacctctcc tgggacccct actcaccctc 1560
   ctactcatac taaccattgg gccatgcgtt ttcagtcgcc tcatggcctt cattaatgat 1620
   agacttaatg ttgtacatgc catggtgctg gcccagcaat accaagcact caaagctgag 1680
   gaagaagctc aggattga 1698
<210> 67
   <211> 520
   <212> PRT
   <213> Gibbon leukemia virus
<400> 67
   Met Gly Gln Asp Asn Ser Thr Pro Ile Ser Leu Thr Leu Asn His Trp
   1               5                   10                  15
   Arg Asp Val Arg Thr Arg Ala His Asn Leu Ser Val Glu Ile Lys Lys
               20                  25                  30
   Gly Lys Trp Gln Thr Phe Cys Ser Ser Glu Trp Pro Thr Phe Gly Val
           35                  40                  45
   Gly Trp Pro Pro Glu Gly Thr Phe Asn Leu Ser Val Ile Phe Ala Val
   50 55 60
   Lys Lys Ile Val Phe Gln Glu Asn Gly Gly His Pro Asp Gln Val Pro
   65 70 75 80
   Tyr Ile Val Val Trp Gln Asp Leu Ala Gln Asn Pro Pro Pro Trp Val
   85 90 95
   Pro Ala Ser Ala Lys Val Ala Val Val Ser Asp Thr Arg Arg Pro Val
   100 105 110
   Ala Gly Arg Pro Ser Ala Pro Pro Arg Pro Pro Ile Tyr Pro Ala Thr
   115 120 125
   Asp Asp Leu Leu Leu Leu Ser Glu Pro Thr Pro Pro Pro Tyr Pro Ala
   130 135 140
   Ala Leu Pro Pro Pro Leu Ala Pro Gln Ala Ile Gly Pro Pro Ser Gly
   145 150 155 160
   Gln Met Pro Asp Ser Ser Asp Pro Glu Gly Pro Ala Ala Gly Thr Arg
   165 170 175
   Ser Arg Arg Ala Arg Ser Pro Ala Asp Asn Ser Gly Pro Asp Ser Thr
   180 185 190
   Val Ile Leu Pro Leu Arg Ala Ile Gly Pro Pro Ala Glu Pro Asn Gly
   195 200 205
   Leu Val Pro Leu Gln Tyr Trp Pro Phe Ser Ser Ala Asp Leu Tyr Asn
   210 215 220
   Trp Lys Ser Asn His Pro Ser Phe Ser Glu Asn Pro Ala Gly Leu Thr
   225 230 235 240
   Gly Leu Leu Glu Ser Leu Met Phe Ser His Gln Pro Thr Trp Asp Asp
   245 250 255
   Cys Gln Gln Leu Leu Gln Ile Leu Phe Thr Thr Glu Glu Arg Glu Arg
   260 265 270
   Ile Leu Leu Glu Ala Arg Lys Asn Val Leu Gly Asp Asn Gly Ala Pro
   275 280 285
   Thr Gln Leu Glu Asn Leu Ile Asn Glu Ala Phe Pro Leu Asn Arg Pro
   290 295 300
   His Trp Asp Tyr Asn Thr Ala Ala Gly Arg Glu Arg Leu Leu Val Tyr
   305                 310                 315                 320
   Arg Arg Thr Leu Val Ala Gly Leu Lys Gly Ala Ala Arg Arg Pro Thr
                   325                 330                 335
   Asn Leu Ala Lys Val Arg Glu Val Leu Gln Gly Pro Ala Glu Pro Pro
               340                 345                 350
   Ser Val Phe Leu Glu Arg Leu Met Glu Ala Tyr Arg Arg Tyr Thr Pro
           355                 360                 365
   Phe Asp Pro Ser Ser Glu Gly Gln Gln Ala Ala Val Ala Met Ala Phe
       370                 375                 380
   Ile Gly Gln Ser Ala Pro Asp Ile Lys Lys Lys Leu Gln Arg Leu Glu
   385                 390                 395                 400
   Gly Leu Gln Asp Tyr Ser Leu Gln Asp Leu Val Lys Glu Ala Glu Lys
                   405                 410                 415
   Val Tyr His Lys Arg Glu Thr Glu Glu Glu Arg Gln Glu Arg Glu Lys
               420                 425                 430
   Lys Glu Ala Glu Glu Lys Glu Arg Arg Arg Asp Arg Pro Lys Lys Lys
           435                 440                 445
   Asn Leu Thr Lys Ile Leu Ala Ala Val Val Ser Arg Glu Gly Ser Thr
       450                 455                 460
   Gly Arg Gln Thr Gly Asn Leu Ser Asn Gln Ala Lys Lys Thr Pro Arg
   465                 470                 475                 480
   Asp Gly Arg Pro Pro Leu Asp Lys Asp Gln Cys Ala Tyr Cys Lys Glu
                   485                 490                 495
   Lys Gly His Trp Ala Arg Glu Cys Pro Arg Lys Lys His Val Arg Glu
               500                 505                 510
   Ala Lys Val Leu Ala Leu Asp Asn
           515                 520
<210> 68
   <211> 1563
   <212> RNA
   <213> Gibbon leukemia virus
<400> 68
   augggacaag auaauucuac cccuaucucc cucacucuaa aucacuggag agaugugaga 60
   acaagggcuc acaaucuauc cguggaaauc aaaaagggaa aauggcagac uuucuguucc 120
   uccgaguggc ccacauucgg cguggggugg ccaccggagg gaacuuuuaa ucucucuguc 180
   auuuuugcag uuaaaaagau ugucuuucag gagaacgggg gacauccgga ccaaguucca 240
   uauaucgugg uauggcagga ccucgcccag aaucccccac caugggugcc agccuccgcc 300
   aaggucgcug uugucucuga uacccgaaga ccaguugcgg ggaggccauc agcuccuccc 360
   cgacccccca ucuacccggc aacagacgac uuacuccucc ucucugaacc cacgcccccg 420
   cccuauccgg cggcacugcc acccccucug gccccucagg cgaucggacc gccgucaggc 480
   cagaugcccg auaguagcga uccugagggg ccagccgcug ggaccaggag ucgccgugcc 540
   cgcaguccag cagacaacuc ggguccugac uccacuguga uuuugccccu ccgagccaua 600
   ggacccccgg ccgagcccaa uggccugguc ccucuacaau auuggccuuu uuccucagca 660
   gaucuuuaua auuggaaauc uaaucauccc ucuuuuucug aaaacccagc aggucucacg 720
   gggcuccuug agucucuuau guucucccau cagcccacuu gggacgauug ccaacagcuc 780
   cuacagauuc uuuucaccac ugaggaacgg gaaagaauuc uccuggaggc ccgcaaaaau 840
   guccuugggg acaauggggc cccuacacag cucgagaacc ucauuaauga ggccuucccc 900
   cucaaucgac cucacuggga uuacaacaca gccgcaggua gggagcgucu ucuggucuac 960
   cgccggacuc uaguggcagg ucucaaaggg gcagcucggc guccuaccaa uuuggcuaag 1020
   guaagagagg ucuugcaggg accggcagaa cccccuucgg uuuucuuaga acgccugaug 1080
   gaggccuaua ggagauacac uccguuugau cccucuucug agggacaaca ggcugcgguc 1140
   gccauggccu uuaucggaca gucagcccca gauaucaaga aaaaguuaca gaggcuagag 1200
   gggcuccagg acuauuccuu acaagauuua guaaaagagg cagaaaaggu guaccauaag 1260
   agagagacag aagaagaaag acaagaaaga gaaaaaaagg aggcagaaga aaaggagagg 1320
   cggcgcgaua ggccgaagaa aaaaaacuug acuaaaauuc uggccgcagu aguaaguaga 1380
   gaagggucca cagguaggca gacagggaac cugagcaacc aggcaaagaa gacaccuagg 1440
   gauggaagac cuccacuaga caaagaccag ugcgcauacu guaaagagaa gggccauugg 1500
   gcaagagaau guccccgaaa aaaacacguc agagaagcca agguucuagc ccuagauaac 1560
   uag 1563
<210> 69
   <211> 522
   <212> PRT
   <213> Measles virus
<400> 69
   Met Asn Ile Pro Cys Phe Val Val Ile Leu Ser Leu Ala Thr Thr His
   1               5                   10                  15
   Ser Leu Gly Glu Phe Pro Leu Tyr Thr Ile Pro Glu Lys Ile Glu Lys
               20                  25                  30
   Trp Thr Pro Ile Asp Met Ile His Leu Ser Cys Pro Asn Asn Leu Leu
   35 40 45
   Ser Glu Glu Glu Gly Cys Asn Ala Glu Ser Ser Phe Thr Tyr Phe Glu
   50 55 60
   Leu Lys Ser Gly Tyr Leu Ala His Gln Lys Val Pro Gly Phe Thr Cys
   65 70 75 80
   Thr Gly Val Val Asn Glu Ala Glu Thr Tyr Thr Asn Phe Val Gly Tyr
   85 90 95
   Val Thr Thr Thr Phe Lys Arg Lys His Phe Arg Pro Thr Val Ala Ala
   100 105 110
   Cys Arg Asp Ala Tyr Asn Trp Lys Val Ser Gly Asp Pro Arg Tyr Glu
   115 120 125
   Glu Ser Leu His Thr Pro Tyr Pro Asp Ser Ser Trp Leu Arg Thr Val
   130 135 140
   Thr Thr Thr Lys Glu Ser Leu Leu Ile Ile Ser Pro Ser Ile Val Glu
   145 150 155 160
   Met Asp Ile Tyr Gly Arg Thr Leu His Ser Pro Met Phe Pro Ser Gly
   165 170 175
   Val Cys Ser Asn Val Tyr Pro Ser Val Pro Ser Cys Glu Thr Asn His
   180 185 190
   Asp Tyr Thr Leu Trp Leu Pro Glu Asp Pro Ser Leu Ser Leu Val Cys
   195 200 205
   Asp Ile Phe Thr Ser Ser Asn Gly Lys Lys Ala Met Asn Gly Ser Arg
   210 215 220
   Ile Cys Gly Phe Lys Asp Glu Arg Gly Phe Tyr Arg Ser Leu Lys Gly
   225 230 235 240
   Ala Cys Lys Leu Thr Leu Cys Gly Arg Pro Gly Ile Arg Leu Phe Asp
   245 250 255
   Gly Thr Trp Val Ser Phe Thr Lys Pro Asp Val His Val Trp Cys Thr
   260 265 270
   Pro Asn Gln Leu Ile Asn Ile His Asn Asp Arg Leu Asp Glu Ile Glu
   275 280 285
   His Leu Ile Val Glu Asp Ile Ile Lys Lys Arg Glu Glu Cys Leu Asp
       290                 295                 300
   Thr Leu Glu Thr Ile Leu Met Ser Gln Ser Val Ser Phe Arg Arg Leu
   305                 310                 315                 320
   Ser His Phe Arg Lys Leu Val Pro Gly Tyr Gly Lys Ala Tyr Thr Ile
                   325                 330                 335
   Leu Asn Gly Ser Leu Met Glu Thr Asn Val Tyr Tyr Lys Arg Val Asp
               340                 345                 350
   Lys Trp Ala Asp Ile Leu Pro Ser Lys Gly Cys Leu Lys Val Gly Gln
           355                 360                 365
   Gln Cys Met Glu Pro Val Lys Gly Val Leu Phe Asn Gly Ile Ile Lys
       370                 375                 380
   Gly Pro Asp Gly Gln Ile Leu Ile Pro Glu Met Gln Ser Glu Gln Leu
   385                 390                 395                 400
   Lys Gln His Met Asp Leu Leu Lys Ala Ala Val Phe Pro Leu Arg His
                   405                 410                 415
   Pro Leu Ile Ser Arg Glu Ala Val Phe Lys Lys Asp Gly Asp Ala Asp
               420                 425                 430
   Asp Phe Val Asp Leu His Met Pro Asp Val His Lys Ser Val Ser Asp
           435                 440                 445
   Val Asp Leu Gly Leu Pro His Trp Gly Phe Trp Met Leu Ile Gly Ala
       450                 455                 460
   Thr Ile Val Ala Phe Val Val Leu Val Cys Leu Leu Arg Val Cys Cys
   465                 470                 475                 480
   Lys Arg Val Arg Arg Arg Arg Ser Gly Arg Ala Thr Gln Glu Ile Pro
                   485                 490                 495
   Leu Ser Phe Pro Ser Ala Pro Val Pro Arg Ala Lys Val Val Ser Ser
               500                 505                 510
   Trp Glu Ser Tyr Lys Gly Leu Pro Gly Thr
           515                 520
<210> 70
   <211> 1653
   <212> DNA
   <213> Measles virus
<400> 70
   atgggtctca aggtgaacgt ctctgccata ttcatggcag tactgttaac tctccaaaca      60
   cccaccggtc aaatccattg gggcaatctc tctaagatag gggtggtagg gataggaagt     120
   gcaagctaca aagttatgac tcgttccagc catcaatcat tggtcataaa attaatgccc     180
   aatataactc tcctcaataa ctgcacgagg gtagagatcg cagaatacag gagactactg     240
   agaacagttt tggaaccaat tagagatgca cttaatgcaa tgacccagaa tataagaccg     300
   gttcagagtg tagcttcaag taggagacac aagagatttg cgggagttgt cctggcaggt     360
   gcggccctag gcgttgccac agctgctcag ataacagccg gcattgcact tcaccagtcc     420
   atgctgaact ctcaagccat cgacaatctg agagcaagcc tggaaactac taatcaggca     480
   attgaggcaa tcagacaagc agggcaggag atgatattgg ctgttcaggg tgtccaagac     540
   tacatcaata atgagctgat accgtctatg aaccaactat cttgtgattt aatcggccag     600
   aagctagggc tcaaattgct cagatactat acagaaatcc tgtcattatt tggccccagc     660
   ttacgggacc ccatatctgc ggagatatcc atccaggctt tgagctatgc gcttggagga     720
   gatatcaata aggtattaga aaagctcgga tacagtggag gtgatttact gggcatctta     780
   gagagcagag gaataaaggc ccggataact cacgtcgaca cagagtccta cttcattgta     840
   ctcagtatag cctatccgac gctgtccgag attaaggggg tgattgtcca ccggctagag     900
   ggggtctcgt acaatatagg ctctcaagag tggtatacca ctgtgcccaa gtatgttgca     960
   acccaagggt accttatctc gaattttgat gagtcatcgt gtactttcat gccagagggg    1020
   actgtgtgca gccaaaatgc cttgtacccg atgagtcctc tgctccaaga atgcctccgg    1080
   gggtccacca agtcctgtgc tcgtacactc gtatccgggt cttttgggaa ccggttcatt    1140
   ttatcacaag ggaacctaat agccaattgt gcatcaatcc tctgcaagtg ttacacaaca    1200
   ggaacgatca ttaatcaaga ccctgacaag atcctaacat acattgctgc cgatcactgc    1260
   ccggtggtcg aggtgaacgg tgtgaccatc caagtcggga gcaggaggta tccggacgcg    1320
   gtgtacctgc acagaattga cctcggtcct cccatatcat tggagaggtt ggacgtaggg    1380
   acaaatctgg ggaatgcaat tgctaagttg gaggatgcca aggaattgtt ggagtcatcg    1440
   gaccagatat tgaggagtat gaaaggttta tcgagcacta gcatagttta catcctgatt    1500
   gcagtgtgtc ttggagggtt gatagggatc cccgctttaa tatgttgctg cagggggcgc   1560
   tgtaacaaaa agggagaaca agttggtatg tcaagaccag gcctaaagcc tgatcttaca    1620
   gggacatcaa aatcctatgt aaggtcgctc tga                                 1653
<210> 71
   <211> 617
   <212> PRT
   <213> Measles virus
<400> 71
   Met Ser Pro Gln Arg Asp Arg Ile Asn Ala Phe Tyr Lys Asp Asn Pro
   1               5                   10                  15
   His Pro Lys Gly Ser Arg Ile Val Ile Asn Arg Glu His Leu Met Ile
               20                  25                  30
   Asp Arg Pro Tyr Val Leu Leu Ala Val Leu Phe Val Met Phe Leu Ser
           35                  40                  45
   Leu Ile Gly Leu Leu Ala Ile Ala Gly Ile Arg Leu His Arg Ala Ala
       50                  55                  60
   Ile Tyr Thr Ala Glu Ile His Lys Ser Leu Ser Thr Asn Leu Asp Val
   65                  70                  75                  80
   Thr Asn Ser Ile Glu His Gln Val Lys Asp Val Leu Thr Pro Leu Phe
                   85                  90                  95
   Lys Ile Ile Gly Asp Glu Val Gly Leu Arg Thr Pro Gln Arg Phe Thr
               100                 105                 110
   Asp Leu Val Lys Phe Ile Ser Asp Lys Ile Lys Phe Leu Asn Pro Asp
           115                 120                 125
   Arg Glu Tyr Asp Phe Arg Asp Leu Thr Trp Cys Ile Asn Pro Pro Glu
       130                 135                 140
   Arg Ile Lys Leu Asp Tyr Asp Gln Tyr Cys Ala Asp Val Ala Ala Glu
   145                 150                 155                 160
   Glu Leu Met Asn Ala Leu Val Asn Ser Thr Leu Leu Glu Ala Arg Ala
                   165                 170                 175
   Thr Asn Gln Phe Leu Ala Val Ser Lys Gly Asn Cys Ser Gly Pro Thr
               180                 185                 190
   Thr Ile Arg Gly Gln Phe Ser Asn Met Ser Leu Ser Leu Leu Asp Leu
           195                 200                 205
   Tyr Leu Ser Arg Gly Tyr Asn Val Ser Ser Ile Val Thr Met Thr Ser
       210                 215                 220
   Gln Gly Met Tyr Gly Gly Thr Tyr Leu Val Gly Lys Pro Asn Leu Ser
   225                 230                 235                 240
   Ser Lys Gly Ser Glu Leu Ser Gln Leu Ser Met His Arg Val Phe Glu
                   245                 250                 255
   Val Gly Val Ile Arg Asn Pro Gly Leu Gly Ala Pro Val Phe His Met
               260                 265                 270
   Thr Asn Tyr Phe Glu Gln Pro Val Ser Asn Asp Phe Ser Asn Cys Met
           275                 280                 285
   Val Ala Leu Gly Glu Leu Lys Phe Ala Ala Leu Cys His Arg Glu Asp
       290                 295                 300
   Ser Ile Thr Ile Pro Tyr Gln Gly Ser Gly Lys Gly Val Ser Phe Gln
   305                 310                 315                 320
   Leu Val Lys Leu Gly Val Trp Lys Ser Pro Thr Asp Met Arg Ser Trp
                   325                 330                 335
   Val Pro Leu Ser Thr Asp Asp Pro Val Ile Asp Arg Leu Tyr Leu Ser
               340                 345                 350
   Ser His Arg Gly Val Ile Ala Asp Asn Gln Ala Lys Trp Ala Val Pro
           355                 360                 365
   Thr Thr Arg Thr Asp Asp Lys Leu Arg Met Glu Thr Cys Phe Gln Gln
       370                 375                 380
   Ala Cys Lys Gly Lys Asn Gln Ala Leu Cys Glu Asn Pro Glu Trp Ala
   385                 390                 395                 400
   Pro Leu Lys Asp Asn Arg Ile Pro Ser Tyr Gly Val Leu Ser Val Asn
                   405                 410                 415
   Leu Ser Leu Thr Val Glu Leu Lys Ile Lys Ile Ala Ser Gly Phe Gly
               420                 425                 430
   Pro Leu Ile Thr His Gly Ser Gly Met Asp Leu Tyr Lys Thr Asn His
           435                 440                 445
   Asn Asn Val Tyr Trp Leu Thr Ile Pro Pro Met Lys Asn Leu Ala Leu
       450                 455                 460
   Gly Val Ile Asn Thr Leu Glu Trp Ile Pro Arg Phe Lys Val Ser Pro
   465                 470                 475                 480
   Asn Leu Phe Thr Val Pro Ile Lys Glu Ala Gly Glu Asp Cys His Ala
                   485                 490                 495
   Pro Thr Tyr Leu Pro Ala Glu Val Asp Gly Asp Val Lys Leu Ser Ser
               500                 505                 510
   Asn Leu Val Ile Leu Pro Gly Gln Asp Leu Gln Tyr Val Leu Ala Thr
           515                 520                 525
   Tyr Asp Thr Ser Arg Val Glu His Ala Val Val Tyr Tyr Val Tyr Ser
   530 535 540
   Pro Ser Arg Ser Phe Ser Tyr Phe Tyr Pro Phe Arg Leu Pro Ile Lys
   545 550 555 560
   Gly Val Pro Ile Glu Leu Gln Val Glu Cys Phe Thr Trp Asp Lys Lys
   565 570 575
   Leu Trp Cys Arg His Phe Cys Val Leu Ala Asp Ser Glu Ser Gly Gly
   580 585 590
   His Ile Thr His Ser Gly Met Val Gly Met Gly Val Ser Cys Thr Val
   595 600 605
   Thr Arg Glu Asp Gly Thr Asn Arg Arg
   610 615
<210> 72
   <211> 1854
   <212> DNA
   <213> Measles virus
<400> 72
   atgtcaccac aacgagaccg aataaatgcc ttctacaaag acaacccaca tcctaaggga 60
   agtaggatag ttattaacag agaacatctt atgattgata gaccttatgt tttgctggct 120
   gttctattcg tcatgtttct gagcttgatc gggttgctag ccattgcagg cattagactc 180
   catcgtgcag ccatctacac cgcagagatc cataagagcc tcagcaccaa tctagatgta 240
   actaactcga tcgagcatca ggtcaaggac gtgctgacac cactcttcaa gatcattggt 300
   gatgaagtgg gcctgaggac acctcagaga ttcactgacc tagtgaaatt catctctgac 360
   aaaattaaat tccttaatcc ggatagggag tacgacttca gagatctcac ttggtgtatc 420
   aacccgccag agagaatcaa attggattat gatcaatact gtgcagatgt ggctgctgaa 480
   gaactcatga atgcattggt gaactcaact ctactggagg ccagggcaac caatcagttc 540
   ctagctgtct caaagggaaa ctgctcaggg cccactacaa tcagaggtca attctcaaac 600
   atgtcgctgt ccctgttgga cttgtattta agtcgaggtt acaatgtgtc atctatagtc 660
   actatgacat cccagggaat gtacggggga acttacctag tgggaaagcc taatctgagc 720
   agtaaagggt cagagttgtc acaactgagc atgcaccgag tgtttgaagt aggggttatc 780
   agaaatccgg gtttgggggc tccggtgttc catatgacaa actattttga gcaaccagtc 840
   agtaatgatt tcagcaactg catggtggct ttgggggagc ttaaattcgc agccctctgt 900
   cacagggaag attctatcac aattccctat caggggtcag ggaaaggtgt cagcttccag 960
   ctcgtcaagc taggtgtctg gaaatcccca accgacatgc gatcctgggt ccccctatca 1020
   acggatgatc cagtgataga taggctttac ctctcatctc acagaggtgt tatcgctgac 1080
   aatcaagcaa aatgggctgt cccgacaaca cggacagatg acaagttgcg aatggagaca 1140
   tgcttccagc aggcgtgtaa gggtaaaaac caagcactct gcgagaatcc cgagtgggca 1200
   ccattgaagg ataacaggat tccttcatac ggggtcttgt ctgttaatct gagtctgaca 1260
   gttgagctta aaatcaaaat tgcttcagga ttcgggccat tgatcacaca cggttcaggg 1320
   atggacctat acaaaaccaa ccacaacaat gtgtattggc tgactatccc gccaatgaag 1380
   aacctagcct taggtgtaat caacacattg gagtggatac cgagattcaa ggttagtccc 1440
   aacctcttca ctgttccaat caaggaagca ggcgaggact gccatgcccc aacataccta 1500
   cctgcggagg tggatggtga tgtcaaactc agttccaatc tggtaattct acctggtcag 1560
   gatctccaat atgttttggc aacctacgat acttccaggg ttgaacatgc tgtggtttat 1620
   tatgtttaca gcccaagccg ctcattttct tacttttatc cttttaggtt gcctataaag 1680
   ggggtcccaa tcgaattaca agtggaatgc ttcacatggg acaaaaaact ctggtgccgt 1740
   cacttctgtg tgcttgcgga ctcagaatct ggtggacata tcactcactc tgggatggtg 1800
   ggcatgggag tcagctgcac agtcactcgg gaagatggaa ccaatcgcag atag 1854
<210> 73
   <211> 546
   <212> PRT
   <213> Nipah virus
<400> 73
   Met Val Val Ile Leu Asp Lys Arg Cys Tyr Cys Asn Leu Leu Ile Leu
   1               5                   10                  15
   Ile Leu Met Ile Ser Glu Cys Ser Val Gly Ile Leu His Tyr Glu Lys
               20                  25                  30
   Leu Ser Lys Ile Gly Leu Val Lys Gly Val Thr Arg Lys Tyr Lys Ile
           35                  40                  45
   Lys Ser Asn Pro Leu Thr Lys Asp Ile Val Ile Lys Met Ile Pro Asn
       50                  55                  60
   Val Ser Asn Met Ser Gln Cys Thr Gly Ser Val Met Glu Asn Tyr Lys
   65                  70                  75                  80
   Thr Arg Leu Asn Gly Ile Leu Thr Pro Ile Lys Gly Ala Leu Glu Ile
                   85                  90                  95
   Tyr Lys Asn Asn Thr His Asp Leu Val Gly Asp Val Arg Leu Ala Gly
               100                 105                 110
   Val Ile Met Ala Gly Val Ala Ile Gly Ile Ala Thr Ala Ala Gln Ile
   115 120 125
   Thr Ala Gly Val Ala Leu Tyr Glu Ala Met Lys Asn Ala Asp Asn Ile
   130 135 140
   Asn Lys Leu Lys Ser Ser Ile Glu Ser Thr Asn Glu Ala Val Val Lys
   145 150 155 160
   Leu Gln Glu Thr Ala Glu Lys Thr Val Tyr Val Leu Thr Ala Leu Gln
   165 170 175
   Asp Tyr Ile Asn Thr Asn Leu Val Pro Thr Ile Asp Lys Ile Ser Cys
   180 185 190
   Lys Gln Thr Glu Leu Ser Leu Asp Leu Ala Leu Ser Lys Tyr Leu Ser
   195 200 205
   Asp Leu Leu Phe Val Phe Gly Pro Asn Leu Gln Asp Pro Val Ser Asn
   210 215 220
   Ser Met Thr Ile Gln Ala Ile Ser Gln Ala Phe Gly Gly Asn Tyr Glu
   225 230 235 240
   Thr Leu Leu Arg Thr Leu Gly Tyr Ala Thr Glu Asp Phe Asp Asp Leu
   245 250 255
   Leu Glu Ser Asp Ser Ile Thr Gly Gln Ile Ile Tyr Val Asp Leu Ser
   260 265 270
   Ser Tyr Tyr Ile Ile Val Arg Val Tyr Phe Pro Ile Leu Thr Glu Ile
   275 280 285
   Gln Gln Ala Tyr Ile Gln Glu Leu Leu Pro Val Ser Phe Asn Asn Asp
   290 295 300
   Asn Ser Glu Trp Ile Ser Ile Val Pro Asn Phe Ile Leu Val Arg Asn
   305 310 315 320
   Thr Leu Ile Ser Asn Ile Glu Ile Gly Phe Cys Leu Ile Thr Lys Arg
   325 330 335
   Ser Val Ile Cys Asn Gln Asp Tyr Ala Thr Pro Met Thr Asn Asn Met
   340 345 350
   Arg Glu Cys Leu Thr Gly Ser Thr Glu Lys Cys Pro Arg Glu Leu Val
   355 360 365
   Val Ser Ser His Val Pro Arg Phe Ala Leu Ser Asn Gly Val Leu Phe
       370                 375                 380
   Ala Asn Cys Ile Ser Val Thr Cys Gln Cys Gln Thr Thr Gly Arg Ala
   385                 390                 395                 400
   Ile Ser Gln Ser Gly Glu Gln Thr Leu Leu Met Ile Asp Asn Thr Thr
                   405                 410                 415
   Cys Pro Thr Ala Val Leu Gly Asn Val Ile Ile Ser Leu Gly Lys Tyr
               420                 425                 430
   Leu Gly Ser Val Asn Tyr Asn Ser Glu Gly Ile Ala Ile Gly Pro Pro
           435                 440                 445
   Val Phe Thr Asp Lys Val Asp Ile Ser Ser Gln Ile Ser Ser Met Asn
       450                 455                 460
   Gln Ser Leu Gln Gln Ser Lys Asp Tyr Ile Lys Glu Ala Gln Arg Leu
   465                 470                 475                 480
   Leu Asp Thr Val Asn Pro Ser Leu Ile Ser Met Leu Ser Met Ile Ile
                   485                 490                 495
   Leu Tyr Val Leu Ser Ile Ala Ser Leu Cys Ile Gly Leu Ile Thr Phe
               500                 505                 510
   Ile Ser Phe Ile Ile Val Glu Lys Lys Arg Asn Thr Tyr Ser Arg Leu
           515                 520                 525
   Glu Asp Arg Arg Val Arg Pro Thr Ser Ser Gly Asp Leu Tyr Tyr Ile
       530                 535                 540
   Gly Thr
   545
<210> 74
   <211> 1641
   <212> RNA
   <213> Nipah virus
<400> 74
   augguaguua uacuugacaa gagauguuau uguaaucuuu uaauauugau uuugaugauc 60
   ucggagugua guguugggau ucuacauuau gagaaauuga guaaaauugg acuugucaaa 120
   ggaguaacaa gaaaauacaa gauuaaaagc aauccucuca caaaagacau uguuauaaaa 180
   augauuccga augugucgaa caugucucag ugcacaggga gugucaugga aaauuauaaa 240
   acacgauuaa acgguaucuu aacaccuaua aagggagcgu uagagaucua caaaaacaac 300
   acucaugacc uugucgguga ugugagauua gccggaguua uaauggcagg aguugcuauu 360
   gggauugcaa ccgcagcuca aaucacugca gguguagcac uauaugaggc aaugaagaau 420
   gcugacaaca ucaacaaacu caaaagcagc auugaaucaa cuaaugaagc ugucguuaaa 480
   cuucaagaga cugcagaaaa gacagucuau gugcugacug cucuacagga uuacauuaau 540
   acuaauuuag uaccgacaau ugacaagaua agcugcaaac agacagaacu cucacuagau 600
   cuggcauuau caaaguaccu cucugauuug cuuuuuguau uuggccccaa ccuucaagac 660
   ccaguuucua auucaaugac uauacaggcu auaucucagg cauucggugg aaauuaugaa 720
   acacugcuaa gaacauuggg uuacgcuaca gaagacuuug augaucuucu agaaagugac 780
   agcauaacag gucaaaucau cuauguugau cuaaguagcu acuauauaau ugucaggguu 840
   uauuuuccua uucugacuga aauucaacag gccuauaucc aagaguuguu accagugagc 900
   uucaacaaug auaauucaga auggaucagu auugucccaa auuucauauu gguaaggaau 960
   acauuaauau caaauauaga gauuggauuu ugccuaauua caaagaggag cgugaucugc 1020
   aaccaagauu augccacacc uaugaccaac aacaugagag aauguuuaac gggaucgacu 1080
   gagaaguguc cucgagagcu gguuguuuca ucacauguuc ccagauuugc acuaucuaac 1140
   gggguucugu uugccaauug cauaaguguu acaugucagu gucaaacaac aggcagggca 1200
   aucucacaau caggagaaca aacucugcug augauugaca acaccaccug uccuacagcc 1260
   guacucggua augugauuau cagcuuaggg aaauaucugg ggucaguaaa uuauaauucu 1320
   gaaggcauug cuaucggucc uccagucuuu acagauaaag uugauauauc aagucagaua 1380
   uccagcauga aucaguccuu acaacagucu aaggacuaua ucaaagaggc ucaacgacuc 1440
   cuugauacug uuaauccauc auuaauaagc auguugucua ugaucauacu guauguauua 1500
   ucgaucgcau cguuguguau aggguugauu acauuuauca guuuuaucau uguugagaaa 1560
   aagagaaaca ccuacagcag auuagaggau aggagaguca gaccuacaag caguggggau 1620
   cucuacuaca uugggacaua g 1641
<210> 75
   <211> 602
   <212> PRT
   <213> Nipah virus
<400> 75
   Met Pro Ala Glu Asn Lys Lys Val Arg Phe Glu Asn Thr Thr Ser Asp
   1               5                   10                  15
   Lys Gly Lys Ile Pro Ser Lys Val Ile Lys Ser Tyr Tyr Gly Thr Met
               20                  25                  30
   Asp Ile Lys Lys Ile Asn Glu Gly Leu Leu Asp Ser Lys Ile Leu Ser
           35                  40                  45
   Ala Phe Asn Thr Val Ile Ala Leu Leu Gly Ser Ile Val Ile Ile Val
   50 55 60
   Met Asn Ile Met Ile Ile Gln Asn Tyr Thr Arg Ser Thr Asp Asn Gln
   65 70 75 80
   Ala Val Ile Lys Asp Ala Leu Gln Gly Ile Gln Gln Gln Ile Lys Gly
   85 90 95
   Leu Ala Asp Lys Ile Gly Thr Glu Ile Gly Pro Lys Val Ser Leu Ile
   100 105 110
   Asp Thr Ser Ser Thr Ile Thr Ile Pro Ala Asn Ile Gly Leu Leu Gly
   115 120 125
   Ser Lys Ile Ser Gln Ser Thr Ala Ser Ile Asn Glu Asn Val Asn Glu
   130 135 140
   Lys Cys Lys Phe Thr Leu Pro Pro Leu Lys Ile His Glu Cys Asn Ile
   145 150 155 160
   Ser Cys Pro Asn Pro Leu Pro Phe Arg Glu Tyr Arg Pro Gln Thr Glu
   165 170 175
   Gly Val Ser Asn Leu Val Gly Leu Pro Asn Asn Ile Cys Leu Gln Lys
   180 185 190
   Thr Ser Asn Gln Ile Leu Lys Pro Lys Leu Ile Ser Tyr Thr Leu Pro
   195 200 205
   Val Val Gly Gln Ser Gly Thr Cys Ile Thr Asp Pro Leu Leu Ala Met
   210 215 220
   Asp Glu Gly Tyr Phe Ala Tyr Ser His Leu Glu Arg Ile Gly Ser Cys
   225 230 235 240
   Ser Arg Gly Val Ser Lys Gln Arg Ile Ile Gly Val Gly Glu Val Leu
   245 250 255
   Asp Arg Gly Asp Glu Val Pro Ser Leu Phe Met Thr Asn Val Trp Thr
   260 265 270
   Pro Pro Asn Pro Asn Thr Val Tyr His Cys Ser Ala Val Tyr Asn Asn
   275 280 285
   Glu Phe Tyr Tyr Val Leu Cys Ala Val Ser Thr Val Gly Asp Pro Ile
   290 295 300
   Leu Asn Ser Thr Tyr Trp Ser Gly Ser Leu Met Met Thr Arg Leu Ala
   305 310 315 320
   Val Lys Pro Lys Ser Asn Gly Gly Gly Tyr Asn Gln His Gln Leu Ala
   325 330 335
   Leu Arg Ser Ile Glu Lys Gly Arg Tyr Asp Lys Val Met Pro Tyr Gly
   340 345 350
   Pro Ser Gly Ile Lys Gln Gly Asp Thr Leu Tyr Phe Pro Ala Val Gly
   355 360 365
   Phe Leu Val Arg Thr Glu Phe Lys Tyr Asn Asp Ser Asn Cys Pro Ile
   370 375 380
   Thr Lys Cys Gln Tyr Ser Lys Pro Glu Asn Cys Arg Leu Ser Met Gly
   385 390 395 400
   Ile Arg Pro Asn Ser His Tyr Ile Leu Arg Ser Gly Leu Leu Lys Tyr
   405 410 415
   Asn Leu Ser Asp Gly Glu Asn Pro Lys Val Val Phe Ile Glu Ile Ser
   420 425 430
   Asp Gln Arg Leu Ser Ile Gly Ser Pro Ser Lys Ile Tyr Asp Ser Leu
   435 440 445
   Gly Gln Pro Val Phe Tyr Gln Ala Ser Phe Ser Trp Asp Thr Met Ile
   450 455 460
   Lys Phe Gly Asp Val Leu Thr Val Asn Pro Leu Val Val Asn Trp Arg
   465 470 475 480
   Asn Asn Thr Val Ile Ser Arg Pro Gly Gln Ser Gln Cys Pro Arg Phe
   485 490 495
   Asn Thr Cys Pro Glu Ile Cys Trp Glu Gly Val Tyr Asn Asp Ala Phe
   500 505 510
   Leu Ile Asp Arg Ile Asn Trp Ile Ser Ala Gly Val Phe Leu Asp Ser
   515 520 525
   Asn Gln Thr Ala Glu Asn Pro Val Phe Thr Val Phe Lys Asp Asn Glu
   530 535 540
   Ile Leu Tyr Arg Ala Gln Leu Ala Ser Glu Asp Thr Asn Ala Gln Lys
   545 550 555 560
   Thr Ile Thr Asn Cys Phe Leu Leu Lys Asn Lys Ile Trp Cys Ile Ser
   565 570 575
   Leu Val Glu Ile Tyr Asp Thr Gly Asp Asn Val Ile Arg Pro Lys Leu
   580 585 590
   Phe Ala Val Lys Ile Pro Glu Gln Cys Thr
   595 600
<210> 76
   <211> 1809
   <212> RNA
   <213> Nipah virus
<400> 76
   augccggcag aaaacaagaa aguuagauuc gaaaauacua cuucagacaa agggaaaauu      60
   ccuaguaaag uuauuaagag cuacuacgga accauggaca uuaagaaaau aaaugaagga     120
   uuauuggaca gcaaaauauu aagugcuuuc aacacaguaa uagcauugcu uggaucuauc     180
   gugaucauag ugaugaauau aaugaucauc caaaauuaca caagaucaac agacaaucag     240
   gccgugauca aagaugcguu gcaggguauc caacagcaga ucaaagggcu ugcugacaaa     300
   aucggcacag agauagggcc caaaguauca cugauugaca cauccaguac cauuacuauc     360
   ccagcuaaca uugggcuguu agguucaaag aucagccagu cgacugcaag uauaaaugag     420
   aaugugaaug aaaaaugcaa auucacacug ccucccuuga aaauccacga auguaacauu     480
   ucuuguccua acccacuccc uuuuagagag uauaggccac agacagaagg ggugagcaau     540
   cuaguaggau uaccuaauaa uauuugccug caaaagacau cuaaucagau auugaagcca     600
   aagcugauuu cauacacuuu acccguaguc ggucaaagug guaccuguau cacagaccca     660
   uugcuggcua uggacgaggg cuauuuugca uauagccacc uggaaagaau cggaucaugu     720
   ucaagagggg ucuccaaaca aagaauaaua ggaguuggag agguacuaga cagaggugau     780
   gaaguuccuu cuuuauuuau gaccaauguc uggaccccac caaauccaaa caccguuuac     840
   cacuguagug cuguauacaa caaugaauuc uauuauguac uuugugcagu gucaacuguu     900
   ggagacccua uucugaauag caccuacugg uccggaucuc uaaugaugac ccgucuagcu     960
   gugaaaccca agaguaaugg uggggguuac aaucaacauc aacuugcccu acgaaguauc     1020
   gagaaaggga gguaugauaa aguuaugccg uauggaccuu caggcaucaa acagggugac    1080
   acccuguauu uuccugcugu aggauuuuug gucaggacag aguuuaaaua caaugauuca    1140
   aauuguccca ucacgaagug ucaauacagu aaaccugaaa auugcaggcu aucuaugggg     1200
   auuagaccaa acagccauua uauccuucga ucuggacuau uaaaauacaa ucuaucagau    1260
   ggggagaacc ccaaaguugu auucauugaa auaucugauc aaagauuauc uauuggaucu    1320
   ccuagcaaaa ucuaugauuc uuugggucaa ccuguuuucu accaagcguc auuuucaugg    1380
   gauacuauga uuaaauuugg agauguucua acagucaacc cucugguugu caauuggcgu    1440
   aauaacacgg uaauaucaag acccgggcaa ucacaaugcc cuagauucaa uacaugucca    1500
   gagaucugcu gggaaggagu uuauaaugau gcauuccuaa uugacagaau caauuggaua    1560
   agcgcgggug uauuccuuga cagcaaucag accgcagaaa auccuguuuu uacuguauuc    1620
   aaagauaaug aaauacuuua uagggcacaa cuggcuucug aggacaccaa ugcacaaaaa    1680
   acaauaacua auuguuuucu cuugaagaau aagauuuggu gcauaucauu gguugagaua    1740
   uaugacacag gagacaaugu cauaagaccc aaacuauucg cgguuaagau accagagcaa    1800
   uguacauaa                                                            1809
<210> 77
   <211> 563
   <212> PRT
   <213> Influenza A virus
<400> 77
   Met Asn Thr Gln Ile Leu Val Phe Ala Leu Val Ala Val Ile Pro Thr
   1               5                   10                  15
   Asn Ala Asp Lys Ile Cys Leu Gly His His Ala Val Ser Asn Gly Thr
               20                  25                  30
   Lys Val Asn Thr Leu Thr Glu Arg Gly Val Glu Val Val Asn Ala Thr
           35                  40                  45
   Glu Thr Val Glu Arg Thr Asn Ile Pro Lys Ile Cys Ser Lys Gly Lys
       50                  55                  60
   Arg Thr Thr Asp Leu Gly Gln Cys Gly Leu Leu Gly Thr Ile Thr Gly
   65                  70                  75                  80
   Pro Pro Gln Cys Asp Gln Phe Leu Glu Phe Ser Ala Asp Leu Ile Ile
                   85                  90                  95
   Glu Arg Arg Glu Gly Asn Asp Val Cys Tyr Pro Gly Lys Phe Val Asn
               100                 105                 110
   Glu Glu Ala Leu Arg Gln Ile Leu Arg Gly Ser Gly Gly Ile Asp Lys
           115                 120                 125
   Glu Thr Met Gly Phe Thr Tyr Ser Gly Ile Arg Thr Asn Gly Thr Thr
       130                 135                 140
   Ser Ala Cys Arg Arg Ser Gly Ser Ser Phe Tyr Ala Glu Met Glu Trp
   145                 150                 155                 160
   Leu Leu Ser Asn Thr Asp Asn Ala Ala Phe Pro Gln Met Thr Lys Ser
                   165                 170                 175
   Tyr Lys Asn Thr Arg Arg Glu Ser Ala Leu Ile Val Trp Gly Ile His
   180 185 190
   His Ser Gly Ser Thr Thr Glu Gln Thr Lys Leu Tyr Gly Ser Gly Asn
   195 200 205
   Lys Leu Ile Thr Val Gly Ser Ser Lys Tyr His Gln Ser Phe Val Pro
   210 215 220
   Ser Pro Gly Thr Arg Pro Gln Ile Asn Gly Gln Ser Gly Arg Ile Asp
   225 230 235 240
   Phe His Trp Leu Ile Leu Asp Pro Asn Asp Thr Val Thr Phe Ser Phe
   245 250 255
   Asn Gly Ala Phe Ile Ala Pro Asn Arg Ala Ser Phe Leu Arg Gly Lys
   260 265 270
   Ser Met Gly Ile Gln Ser Asp Val Gln Val Asp Ala Asn Cys Glu Gly
   275 280 285
   Glu Cys Tyr His Ser Gly Gly Thr Ile Thr Ser Arg Leu Pro Phe Gln
   290 295 300
   Asn Ile Asn Ser Arg Ala Val Gly Lys Cys Pro Arg Tyr Val Lys Gln
   305 310 315 320
   Glu Ser Leu Leu Leu Ala Thr Gly Met Lys Asn Val Pro Glu Pro Ser
   325 330 335
   Lys Lys Arg Lys Lys Arg Gly Leu Phe Gly Ala Ile Ala Gly Phe Ile
   340 345 350
   Glu Asn Gly Trp Glu Gly Leu Val Asp Gly Trp Tyr Gly Phe Arg His
   355 360 365
   Gln Asn Ala Gln Gly Glu Gly Thr Ala Ala Asp Tyr Lys Ser Thr Gln
   370 375 380
   Ser Ala Ile Asp Gln Ile Thr Gly Lys Leu Asn Arg Leu Ile Glu Lys
   385 390 395 400
   Thr Asn Gln Gln Phe Glu Leu Ile Asp Asn Glu Phe Thr Glu Val Glu
   405 410 415
   Lys Gln Ile Gly Asn Leu Ile Asn Trp Thr Lys Asp Ser Ile Thr Glu
   420 425 430
   Val Trp Ser Tyr Asn Ala Glu Leu Leu Val Ala Met Glu Asn Gln His
   435 440 445
   Thr Ile Asp Leu Ala Asp Ser Glu Met Asn Lys Leu Tyr Glu Arg Val
   450 455 460
   Arg Lys Gln Leu Arg Glu Asn Ala Glu Glu Asp Gly Thr Gly Cys Phe
   465 470 475 480
   Glu Ile Phe His Lys Cys Asp Asp Asp Cys Met Ala Ser Ile Arg Asn
   485 490 495
   Asn Thr Tyr Asp His Ser Lys Tyr Arg Glu Glu Ala Met Gln Asn Arg
   500 505 510
   Ile Gln Ile Asp Pro Val Lys Leu Ser Ser Gly Tyr Lys Asp Val Ile
   515 520 525
   Leu Trp Phe Ser Phe Gly Ala Ser Cys Phe Leu Leu Leu Ala Ile Ala
   530 535 540
   Met Gly Leu Val Phe Ile Cys Val Lys Asn Gly Asn Met Arg Cys Thr
   545 550 555 560
   Ile Cys Ile
<210> 78
   <211> 1692
   <212> DNA
   <213> Influenza A virus
<400> 78
   atgaacactc aaatcctggt tttcgccctt gtggcagtca tccccacaaa tgcagacaaa      60
   atttgtcttg gacatcatgc tgtatcaaat ggcaccaaag taaacacact cactgagaga     120
   ggagtagaag ttgtcaatgc aacggaaaca gtggagcgga caaacatccc caaaatttgc     180
   tcaaaaggga aaagaaccac tgatcttggc caatgcggac tgttagggac cattaccgga     240
   ccacctcaat gcgaccaatt tctagaattt tcagctgatc taataatcga gagacgagaa     300
   ggaaatgatg tttgttaccc ggggaagttt gttaatgaag aggcattgcg acaaatcctc     360
   agaggatcag gtgggattga caaagaaaca atgggattca catatagtgg aataaggacc     420
   aacggaacaa ctagtgcatg tagaagatca gggtcttcat tctatgcaga aatggagtgg     480
   ctcctgtcaa atacagacaa tgctgctttc ccacaaatga caaaatcata caaaaacaca     540
   aggagagaat cagctctgat agtctgggga atccaccatt caggatcaac caccgaacag     600
   accaaactat atgggagtgg aaataaactg ataacagtcg ggagttccaa atatcatcaa     660
   tcttttgtgc cgagtccagg aacacgaccg cagataaatg gccagtccgg acggattgat     720
   tttcattggt tgatcttgga tcccaatgat acagttactt ttagtttcaa tggggctttc     780
   atagctccaa atcgtgccag cttcttgagg ggaaagtcca tggggatcca gagcgatgtg     840
   caggttgatg ccaattgcga aggggaatgc taccacagtg gagggactat aacaagcaga     900
   ttgccttttc aaaacatcaa tagcagagca gttggcaaat gcccaagata tgtaaaacag     960
   gaaagtttat tattggcaac tgggatgaag aacgttcccg aaccttccaa aaaaaggaaa    1020
   aaaagaggcc tgtttggcgc tatagcaggg tttattgaaa atggttggga aggtctggtc    1080
   gacgggtggt acggtttcag gcatcagaat gcacaaggag aaggaactgc agcagactac    1140
   aaaagcaccc aatcggcaat tgatcagata accggaaagt taaatagact cattgagaaa    1200
   accaaccagc aatttgagct aatagataat gaattcactg aggtggaaaa gcagattggc    1260
   aatttaatta actggaccaa agactccatc acagaagtat ggtcttacaa tgctgaactt    1320
   cttgtggcaa tggaaaacca gcacactatt gatttggctg attcagagat gaacaagctg    1380
   tatgagcgag tgaggaaaca attaagggaa aatgctgaag aggatggcac tggttgcttt    1440
   gaaatttttc ataaatgtga cgatgattgt atggctagta taaggaacaa tacttatgat    1500
   cacagcaaat acagagaaga agcgatgcaa aatagaatac aaattgaccc agtcaaattg    1560
   agtagtggct acaaagatgt gatactttgg tttagcttcg ggcatcatgg ctttttgctt    1620
   cttgccattg caatgggcct tgttttcata tgtgtgaaga acggaaacat gcggtgcact    1680
   atttgtatat aa                                                        1692
<210> 79
   <211> 447
   <212> PRT
   <213> Influenza A virus
<400> 79
   Met Asn Pro Asn Gln Lys Ile Ile Thr Ile Gly Ser Ile Cys Met Gly
   1               5                   10                  15
   Ile Gly Ile Ile Ser Leu Ile Leu Gln Ile Gly Asn Ile Ile Ser Met
               20                  25                  30
   Trp Val Ser His Ser Ile Gln Thr Glu Asn Gln Asn His His Glu Ala
           35                  40                  45
   Cys Asn Pro Ser Ile Ala Gly Gln Asp Ala Ala Ser Val Ala Leu Ala
       50                  55                  60
   Gly Asn Ser Ser Leu Cys Pro Ile Ser Gly Trp Ala Ile Tyr Ser Lys
   65                  70                  75                  80
   Asp Asn Gly Ile Arg Ile Gly Ser Lys Gly Asp Val Phe Val Ile Arg
                   85                  90                  95
   Glu Pro Phe Ile Ser Cys Ser His Leu Glu Cys Arg Thr Phe Phe Leu
               100                 105                 110
   Thr Gln Gly Ala Leu Leu Asn Asp Lys His Ser Asn Gly Thr Val Lys
           115                 120                 125
   Asp Arg Ser Pro Tyr Arg Thr Leu Met Ser Cys Pro Val Gly Glu Ala
       130                 135                 140
   Pro Ser Pro Tyr Asn Ser Arg Phe Glu Ser Val Ala Trp Ser Ala Ser
   145                 150                 155                 160
   Ala Cys His Asp Gly Met Gly Trp Leu Thr Ile Gly Ile Ser Gly Pro
                   165                 170                 175
   Asp Asn Gly Ala Val Ala Val Leu Lys Tyr Asn Gly Ile Ile Thr Asp
               180                 185                 190
   Thr Ile Lys Ser Trp Lys Asn Asn Ile Leu Arg Thr Gln Glu Ser Glu
           195                 200                 205
   Cys Ala Cys Ile Asn Gly Ser Cys Phe Thr Ile Met Thr Asp Gly Pro
       210                 215                 220
   Ser Asn Gly Gln Ala Ser Tyr Lys Ile Phe Lys Ile Glu Lys Gly Lys
   225                 230                 235                 240
   Val Val Lys Ser Ser Glu Leu Asn Ala Pro Asn Tyr His Tyr Glu Glu
                   245                 250                 255
   Cys Ser Cys Tyr Pro Asp Ala Gly Glu Val Met Cys Val Cys Arg Asp
               260                 265                 270
   Asn Trp His Gly Ser Asn Arg Pro Trp Val Ser Phe Asn Lys Asn Leu
           275                 280                 285
   Asp Tyr Gln Ile Gly Tyr Ile Cys Ser Gly Val Phe Gly Asp Asn Pro
       290                 295                 300
   Arg Pro Asn Asp Gly Thr Gly Ser Cys Gly Pro Val Ser Ser Asn Gly
   305                 310                 315                 320
   Ala Tyr Gly Ile Lys Gly Phe Ser Phe Lys Tyr Gly Asn Gly Val Trp
                   325                 330                 335
   Ile Gly Arg Thr Lys Ser Thr Ser Ser Arg Ser Gly Phe Glu Met Ile
               340                 345                 350
   Trp Asp Pro Asn Gly Trp Thr Glu Thr Asp Ser Ser Phe Ser Val Lys
   355 360 365
   Gln Asp Ile Val Ala Ile Thr Asp Trp Ser Gly Tyr Ser Gly Ser Phe
   370 375 380
   Val Gln His Pro Glu Leu Thr Gly Leu Asp Cys Met Arg Pro Cys Phe
   385 390 395 400
   Trp Val Glu Leu Ile Arg Gly Arg Pro Lys Glu Asn Thr Ile Trp Thr
   405 410 415
   Ser Gly Ser Ser Ile Ser Phe Cys Gly Val Asn Ser Asp Thr Val Gly
   420 425 430
   Trp Ser Trp Pro Asp Gly Ala Glu Leu Pro Phe Thr Ile Asp Lys
   435 440 445
<210> 80
   <211> 1344
   <212> DNA
   <213> Influenza A virus
<400> 80
   atgaatccaa atcagaaaat aataaccatt gggtcaatct gtatggggat cggaataatc 60
   agcctaatat tacaaattgg aaacataatc tcaatgtggg ttagtcattc aattcagact 120
   gaaaatcaaa atcaccatga agcatgcaac ccaagcattg ctggacagga tgcagcttca 180
   gtggcactag caggcaattc ctctctttgt cccattagtg ggtgggctat atacagtaaa 240
   gacaatggta taagaattgg ctccaaagga gacgtatttg tcataagaga gccatttatt 300
   tcatgctctc acttggaatg caggaccttt tttctgactc agggcgcctt gttgaatgac 360
   aaacactcaa atggaaccgt taaagacaga agcccttata gaaccttgat gagctgtcct 420
   gttggtgaag ctccttctcc ctacaattca aggttcgagt cggttgcatg gtcagcaagt 480
   gcctgccatg atggcatggg ttggctaaca atcggaattt ctggtccaga taatggagca 540
   gtggctgtat taaaatacaa tggtataata acagacacca tcaaaagttg gaaaaataac 600
   atattgagaa cgcaagagtc tgaatgtgcc tgtataaatg gttcatgttt cactataatg 660
   accgatggcc caagtaatgg gcaggcctcg tacaaaattt tcaagataga aaaggggaag 720
   gtagtcaaat caagtgaatt aaatgcacct aattaccact acgaggaatg ttcttgttat 780
   cctgatgcag gtgaagtaat gtgtgtatgc agggataatt ggcatggttc gaatcgacca 840
   tgggtgtctt tcaataaaaa ccttgattat caaataggtt acatctgcag tggggttttt 900
   ggtgataatc cacgacccaa tgatggaaca ggcagctgtg gtccagtgtc ttctaatgga 960
   gcatatggga taaaggggtt ctcatttaag tatggtaatg gtgtttggat agggagaacc 1020
   aaaagcacta gttccagaag cgggtttgag atgatttggg atcccaatgg atggacagaa 1080
   actgacagca gtttctctgt gaagcaagat attgtagcaa taactgattg gtcgggatat 1140
   agcgggagtt tcgtccaaca ccctgaatta acagggttgg actgcatgag gccttgtttc 1200
   tgggttgaac tgatcagggg acggcccaag gagaatacga tctggactag tgggagcagc 1260
   atttccttct gtggtgtgaa cagcgatact gtaggttggt cttggccaga cggtgctgag 1320
   ttgccattca ccattgacaa gtag 1344
<210> 81
   <211> 400
   <212> PRT
   <213> Hepatitis B virus
<400> 81
   Met Gly Gly Trp Ser Ser Lys Pro Arg Gln Gly Met Gly Thr Asn Leu
   1               5                   10                  15
   Ser Val Pro Asn Pro Leu Gly Phe Phe Pro Asp His Gln Leu Asp Pro
               20                  25                  30
   Ala Phe Gly Ala Asn Ser Asn Asn Pro Asp Trp Asp Phe Asn Pro Asn
           35                  40                  45
   Lys Asp His Trp Pro Glu Ala Asn Gln Val Gly Ala Gly Ala Phe Gly
       50                  55                  60
   Pro Gly Phe Thr Pro Pro His Gly Gly Leu Leu Gly Trp Ser Pro Gln
   65                  70                  75                  80
   Ala Gln Gly Ile Leu Thr Thr Leu Pro Ala Ala Pro Pro Pro Ala Ser
                   85                  90                  95
   Thr Asn Arg Gln Ser Gly Arg Gln Pro Thr Pro Ile Ser Pro Pro Leu
               100                 105                 110
   Arg Asp Ser His Pro Gln Ala Met Gln Trp Asn Ser Thr Thr Phe His
           115                 120                 125
   Gln Ala Leu Leu Asp Pro Arg Val Arg Gly Leu Tyr Phe Pro Ala Gly
       130                 135                 140
   Gly Ser Ser Ser Gly Thr Val Asn Pro Val Pro Thr Thr Ala Ser Pro
   145                 150                 155                 160
   Ile Ser Ser Ile Phe Ser Arg Thr Gly Asp Pro Ala Pro Asn Met Glu
                   165                 170                 175
   Ser Thr Thr Ser Gly Phe Leu Gly Pro Leu Leu Val Leu Gln Ala Gly
               180                 185                 190
   Phe Phe Leu Leu Thr Arg Ile Leu Thr Ile Pro Gln Ser Leu Asp Ser
           195                 200                 205
   Trp Trp Thr Ser Leu Asn Phe Leu Gly Gly Ala Pro Thr Cys Pro Gly
       210                 215                 220
   Gln Asn Ser Gln Ser Pro Thr Ser Asn His Ser Pro Thr Ser Cys Pro
   225                 230                 235                 240
   Pro Thr Cys Pro Gly Tyr Arg Trp Met Cys Leu Arg Arg Phe Ile Ile
                   245                 250                 255
   Phe Leu Phe Ile Leu Leu Leu Cys Leu Ile Phe Leu Leu Val Leu Leu
               260                 265                 270
   Asp Tyr Gln Gly Met Leu Pro Val Cys Pro Leu Leu Pro Gly Thr Ser
           275                 280                 285
   Thr Thr Ser Thr Gly Pro Cys Arg Thr Cys Thr Ile Pro Ala Gln Gly
       290                 295                 300
   Thr Ser Met Phe Pro Ser Cys Cys Cys Thr Lys Pro Ser Asp Gly Asn
   305                 310                 315                 320
   Cys Thr Cys Ile Pro Ile Pro Ser Ser Trp Ala Phe Ala Arg Phe Leu
                   325                 330                 335
   Trp Glu Trp Ala Ser Val Arg Phe Ser Trp Leu Ser Leu Leu Val Pro
               340                 345                 350
   Phe Val Gln Trp Phe Val Gly Leu Ser Pro Thr Val Trp Leu Ser Ala
           355                 360                 365
   Ile Trp Met Met Trp Tyr Trp Gly Pro Ser Leu Tyr Asn Ile Leu Ser
       370                 375                 380
   Pro Phe Leu Pro Leu Leu Pro Ile Phe Phe Cys Leu Trp Val Tyr Ile
   385                 390                 395                 400
<210> 82
   <211> 1203
   <212> DNA
   <213> Hepatitis B virus
<400> 82
   atgggaggtt ggtcttccaa acctcgacaa ggcatgggga cgaatctttc tgttcccaat      60
   cctctgggat tctttcccga tcaccagttg gaccctgcgt tcggagccaa ctcaaacaat     120
   ccagattggg acttcaaccc caacaaggat cactggccag aggcaaatca ggtaggagcg     180
   ggagcatttg gtccagggtt caccccacca cacggaggcc ttttggggtg gagccctcag     240
   gctcagggca tattgacaac actgccagca gcacctcctc ctgcctccac caatcggcag     300
   tcaggaagac agcctactcc catctctcca cctctaagag acagtcatcc tcaggccatg     360
   cagtggaact ccacaacatt ccaccaagct ctgctagatc ccagagtgag gggcctatat     420
   tttcctgctg gtggctccag ttccggaaca gtaaaccctg ttccgactac tgcctcaccc     480
   atatcgtcaa tcttctcgag gactggggac cctgcaccga acatggagag cacaacatca     540
   ggattcctag gacccctgct cgtgttacag gcggggtttt tcttgttgac aagaatcctc     600
   acaataccac agagtctaga ctcgtggtgg acttctctca attttctagg gggagcaccc     660
   acgtgtcctg gccaaaattc gcagtcccca acctccaatc actcaccaac ctcttgtcct     720
   ccaacttgtc ctggctatcg ctggatgtgt ctgcggcgtt ttatcatatt cctcttcatc     780
   ctgctgctat gcctcatctt cttgttggtt cttctggact accaaggtat gttgcccgtt     840
   tgtcctctac ttccaggaac atcaactacc agcacgggac catgcagaac ctgcacgatt     900
   cctgctcaag gaacctctat gtttccctct tgttgctgta caaaaccttc ggacggaaac     960
   tgcacttgta ttcccatccc atcatcctgg gctttcgcaa gattcctatg ggagtgggcc    1020
   tcagtccgtt tctcctggct cagtttacta gtgccatttg ttcagtggtt cgtagggctt    1080
   tcccccactg tttggctttc agctatatgg atgatgtggt attgggggcc aagtctgtac    1140
   aacatcttga gtcccttttt acctctatta ccaattttct tttgtctttg ggtatacatt    1200
   tga                                                                  1203
<210> 83
   <211> 281
   <212> PRT
   <213> Hepatitis B virus
<400> 83
   Met Gln Trp Asn Ser Thr Thr Phe His Gln Ala Leu Leu Asp Pro Arg
   1               5                   10                  15
   Val Arg Gly Leu Tyr Phe Pro Ala Gly Gly Ser Ser Ser Gly Thr Val
               20                  25                  30
   Asn Pro Val Pro Thr Thr Ala Ser Pro Ile Ser Ser Ile Phe Ser Arg
           35                  40                  45
   Thr Gly Asp Pro Ala Pro Asn Met Glu Ser Thr Thr Ser Gly Phe Leu
       50                  55                  60
   Gly Pro Leu Leu Val Leu Gln Ala Gly Phe Phe Leu Leu Thr Arg Ile
   65                  70                  75                  80
   Leu Thr Ile Pro Gln Ser Leu Asp Ser Trp Trp Thr Ser Leu Asn Phe
                   85                  90                  95
   Leu Gly Gly Ala Pro Thr Cys Pro Gly Gln Asn Ser Gln Ser Pro Thr
               100                 105                 110
   Ser Asn His Ser Pro Thr Ser Cys Pro Pro Thr Cys Pro Gly Tyr Arg
           115                 120                 125
   Trp Met Cys Leu Arg Arg Phe Ile Ile Phe Leu Phe Ile Leu Leu Leu
       130                 135                 140
   Cys Leu Ile Phe Leu Leu Val Leu Leu Asp Tyr Gln Gly Met Leu Pro
   145                 150                 155                 160
   Val Cys Pro Leu Leu Pro Gly Thr Ser Thr Thr Ser Thr Gly Pro Cys
                   165                 170                 175
   Arg Thr Cys Thr Ile Pro Ala Gln Gly Thr Ser Met Phe Pro Ser Cys
               180                 185                 190
   Cys Cys Thr Lys Pro Ser Asp Gly Asn Cys Thr Cys Ile Pro Ile Pro
           195                 200                 205
   Ser Ser Trp Ala Phe Ala Arg Phe Leu Trp Glu Trp Ala Ser Val Arg
       210                 215                 220
   Phe Ser Trp Leu Ser Leu Leu Val Pro Phe Val Gln Trp Phe Val Gly
   225                 230                 235                 240
   Leu Ser Pro Thr Val Trp Leu Ser Ala Ile Trp Met Met Trp Tyr Trp
                   245                 250                 255
   Gly Pro Ser Leu Tyr Asn Ile Leu Ser Pro Phe Leu Pro Leu Leu Pro
               260                 265                 270
   Ile Phe Phe Cys Leu Trp Val Tyr Ile
           275                 280
<210> 84
   <211> 846
   <212> DNA
   <213> Hepatitis B virus
<400> 84
   atgcagtgga actccacaac attccaccaa gctctgctag atcccagagt gaggggccta      60
   tattttcctg ctggtggctc cagttccgga acagtaaacc ctgttccgac tactgcctca     120
   cccatatcgt caatcttctc gaggactggg gaccctgcac cgaacatgga gagcacaaca     180
   tcaggattcc taggacccct gctcgtgtta caggcggggt ttttcttgtt gacaagaatc     240
   ctcacaatac cacagagtct agactcgtgg tggacttctc tcaattttct agggggagca     300
   cccacgtgtc ctggccaaaa ttcgcagtcc ccaacctcca atcactcacc aacctcttgt     360
   cctccaactt gtcctggcta tcgctggatg tgtctgcggc gttttatcat attcctcttc     420
   atcctgctgc tatgcctcat cttcttgttg gttcttctgg actaccaagg tatgttgccc     480
   gtttgtcctc tacttccagg aacatcaact accagcacgg gaccatgcag aacctgcacg     540
   attcctgctc aaggaacctc tatgtttccc tcttgttgct gtacaaaacc ttcggacgga     600
   aactgcactt gtattcccat cccatcatcc tgggctttcg caagattcct atgggagtgg     660
   gcctcagtcc gtttctcctg gctcagttta ctagtgccat ttgttcagtg gttcgtaggg     720
   ctttccccca ctgtttggct ttcagctata tggatgatgt ggtattgggg gccaagtctg     780
   tacaacatct tgagtccctt tttacctcta ttaccaattt tcttttgtct ttgggtatac     840
   atttga                                                                846
<210> 85
   <211> 226
   <212> PRT
   <213> Hepatitis B virus
<400> 85
   Met Glu Ser Thr Thr Ser Gly Phe Leu Gly Pro Leu Leu Val Leu Gln
   1               5                   10                  15
   Ala Gly Phe Phe Leu Leu Thr Arg Ile Leu Thr Ile Pro Gln Ser Leu
               20                  25                  30
   Asp Ser Trp Trp Thr Ser Leu Asn Phe Leu Gly Gly Ala Pro Thr Cys
           35                  40                  45
   Pro Gly Gln Asn Ser Gln Ser Pro Thr Ser Asn His Ser Pro Thr Ser
       50                  55                  60
   Cys Pro Pro Thr Cys Pro Gly Tyr Arg Trp Met Cys Leu Arg Arg Phe
   65                  70                  75                  80
   Ile Ile Phe Leu Phe Ile Leu Leu Leu Cys Leu Ile Phe Leu Leu Val
                   85                  90                  95
   Leu Leu Asp Tyr Gln Gly Met Leu Pro Val Cys Pro Leu Leu Pro Gly
               100                 105                 110
   Thr Ser Thr Thr Ser Thr Gly Pro Cys Arg Thr Cys Thr Ile Pro Ala
           115                 120                 125
   Gln Gly Thr Ser Met Phe Pro Ser Cys Cys Cys Thr Lys Pro Ser Asp
       130                 135                 140
   Gly Asn Cys Thr Cys Ile Pro Ile Pro Ser Ser Trp Ala Phe Ala Arg
   145                 150                 155                 160
   Phe Leu Trp Glu Trp Ala Ser Val Arg Phe Ser Trp Leu Ser Leu Leu
                   165                 170                 175
   Val Pro Phe Val Gln Trp Phe Val Gly Leu Ser Pro Thr Val Trp Leu
               180                 185                 190
   Ser Ala Ile Trp Met Met Trp Tyr Trp Gly Pro Ser Leu Tyr Asn Ile
           195                 200                 205
   Leu Ser Pro Phe Leu Pro Leu Leu Pro Ile Phe Phe Cys Leu Trp Val
       210                 215                 220
   Tyr Ile
   225
<210> 86
   <211> 681
   <212> DNA
   <213> Hepatitis B virus
<400> 86
   atggagagca caacatcagg attcctagga cccctgctcg tgttacaggc ggggtttttc      60
   ttgttgacaa gaatcctcac aataccacag agtctagact cgtggtggac ttctctcaat     120
   tttctagggg gagcacccac gtgtcctggc caaaattcgc agtccccaac ctccaatcac     180
   tcaccaacct cttgtcctcc aacttgtcct ggctatcgct ggatgtgtct gcggcgtttt     240
   atcatattcc tcttcatcct gctgctatgc ctcatcttct tgttggttct tctggactac     300
   caaggtatgt tgcccgtttg tcctctactt ccaggaacat caactaccag cacgggacca     360
   tgcagaacct gcacgattcc tgctcaagga acctctatgt ttccctcttg ttgctgtaca     420
   aaaccttcgg acggaaactg cacttgtatt cccatcccat catcctgggc tttcgcaaga     480
   ttcctatggg agtgggcctc agtccgtttc tcctggctca gtttactagt gccatttgtt     540
   cagtggttcg tagggctttc ccccactgtt tggctttcag ctatatggat gatgtggtat     600
   tggggggcaa gtctgtacaa catcttgagt ccctttttac ctctattacc aattttcttt     660
   tgtctttggg tatacatttg a                                               681
<210> 87
   <211> 192
   <212> PRT
   <213> Hepatitis C virus
<400> 87
   Tyr Gln Val Arg Asn Ser Ser Gly Leu Tyr His Val Thr Asn Asp Cys
   1               5                   10                  15
   Pro Asn Ser Ser Ile Val Tyr Glu Ala Ala Asp Ala Ile Leu His Thr
               20                  25                  30
   Pro Gly Cys Val Pro Cys Val Arg Glu Gly Asn Ala Ser Arg Cys Trp
           35                  40                  45
   Val Ala Val Thr Pro Thr Val Ala Thr Arg Asp Gly Lys Leu Pro Thr
       50                  55                  60
   Thr Gln Leu Arg Arg His Ile Asp Leu Leu Val Gly Ser Ala Thr Leu
   65                  70                  75                  80
   Cys Ser Ala Leu Tyr Val Gly Asp Leu Cys Gly Ser Val Phe Leu Val
                   85                  90                  95
   Gly Gln Leu Phe Thr Phe Ser Pro Arg Arg His Trp Thr Thr Gln Asp
               100                 105                 110
   Cys Asn Cys Ser Ile Tyr Pro Gly His Ile Thr Gly His Arg Met Ala
           115                 120                 125
   Trp Asp Met Met Met Asn Trp Ser Pro Thr Ala Ala Leu Val Val Ala
       130                 135                 140
   Gln Leu Leu Arg Ile Pro Gln Ala Ile Met Asp Met Ile Ala Gly Ala
   145                 150                 155                 160
   His Trp Gly Val Leu Ala Gly Ile Ala Tyr Phe Ser Met Val Gly Asn
                   165                 170                 175
   Trp Ala Lys Val Leu Val Val Leu Leu Leu Phe Ala Gly Val Asp Ala
               180                 185                 190
<210> 88
   <211> 576
   <212> RNA
   <213> Hepatitis C virus
<400> 88
<210> 89
   <211> 363
   <212> PRT
   <213> Hepatitis C virus
<400> 89
   Glu Thr His Val Thr Gly Gly Ser Ala Gly Arg Thr Thr Ala Gly Leu
   1               5                   10                  15
   Val Gly Leu Leu Thr Pro Gly Ala Lys Gln Asn Ile Gln Leu Ile Asn
               20                  25                  30
   Thr Asn Gly Ser Trp His Ile Asn Ser Thr Ala Leu Asn Cys Asn Glu
           35                  40                  45
   Ser Leu Asn Thr Gly Trp Leu Ala Gly Leu Phe Tyr Gln His Lys Phe
       50                  55                  60
   Asn Ser Ser Gly Cys Pro Glu Arg Leu Ala Ser Cys Arg Arg Leu Thr
   65                  70                  75                  80
   Asp Phe Ala Gln Gly Trp Gly Pro Ile Ser Tyr Ala Asn Gly Ser Gly
                   85                  90                  95
   Leu Asp Glu Arg Pro Tyr Cys Trp His Tyr Pro Pro Arg Pro Cys Gly
               100                 105                 110
   Ile Val Pro Ala Lys Ser Val Cys Gly Pro Val Tyr Cys Phe Thr Pro
           115                 120                 125
   Ser Pro Val Val Val Gly Thr Thr Asp Arg Ser Gly Ala Pro Thr Tyr
       130                 135                 140
   Ser Trp Gly Ala Asn Asp Thr Asp Val Phe Val Leu Asn Asn Thr Arg
   145                 150                 155                 160
   Pro Pro Leu Gly Asn Trp Phe Gly Cys Thr Trp Met Asn Ser Thr Gly
                   165                 170                 175
   Phe Thr Lys Val Cys Gly Ala Pro Pro Cys Val Ile Gly Gly Val Gly
               180                 185                 190
   Asn Asn Thr Leu Leu Cys Pro Thr Asp Cys Phe Arg Lys His Pro Glu
           195                 200                 205
   Ala Thr Tyr Ser Arg Cys Gly Ser Gly Pro Trp Ile Thr Pro Arg Cys
   210 215 220
   Met Val Asp Tyr Pro Tyr Arg Leu Trp His Tyr Pro Cys Thr Ile Asn
   225 230 235 240
   Tyr Thr Ile Phe Lys Val Arg Met Tyr Val Gly Gly Val Glu His Arg
   245 250 255
   Leu Glu Ala Ala Cys Asn Trp Thr Arg Gly Glu Arg Cys Asp Leu Glu
   260 265 270
   Asp Arg Asp Arg Ser Glu Leu Ser Pro Leu Leu Leu Ser Thr Thr Gln
   275 280 285
   Trp Gln Val Leu Pro Cys Ser Phe Thr Thr Leu Pro Ala Leu Ser Thr
   290 295 300
   Gly Leu Ile His Leu His Gln Asn Ile Val Asp Val Gln Tyr Leu Tyr
   305 310 315 320
   Gly Val Gly Ser Ser Ile Ala Ser Trp Ala Ile Lys Trp Glu Tyr Val
   325 330 335
   Val Leu Leu Phe Leu Leu Leu Ala Asp Ala Arg Val Cys Ser Cys Leu
   340 345 350
   Trp Met Met Leu Leu Ile Ser Gln Ala Glu Ala
   355 360
<210> 90
   <211> 1089
   <212> RNA
   <213> Hepatitis C virus
<400> 90
   gaaacccacg ucaccggggg aagugccggc cgcaccacgg cugggcuugu uggucuccuu 60
   acaccaggcg ccaagcagaa cauccaacug aucaacacca acggcaguug gcacaucaau 120
   agcacggccu ugaacugcaa ugaaagccuu aacaccggcu gguuagcagg gcucuucuau 180
   cagcacaaau ucaacucuuc aggcuguccu gagagguugg ccagcugccg acgccuuacc 240
   gauuuugccc agggcugggg uccuaucagu uaugccaacg gaagcggccu cgacgaacgc 300
   cccuacugcu ggcacuaccc uccaagaccu uguggcauug ugcccgcaaa gagcgugugu 360
   ggcccgguau auugcuucac ucccagcccc gugguggugg gaacgaccga caggucgggc 420
   gcgccuaccu acagcugggg ugcaaaugau acggaugucu ucguccuuaa caacaccagg 480
   ccaccgcugg gcaauugguu cgguuguacc uggaugaacu caacuggauu caccaaagug 540
   ugcggagcgc ccccuugugu caucggaggg gugggcaaca acaccuugcu cugccccacu 600
   gauuguuucc gcaagcaucc ggaagccaca uacucucggu gcggcuccgg ucccuggauu 660
   acacccaggu gcauggucga cuacccguau aggcuuuggc acuauccuug uaccaucaau 720
   uacaccauau ucaaagucag gauguacgug ggaggggucg agcacaggcu ggaagcggcc 780
   ugcaacugga cgcggggcga acgcugugau cuggaagaca gggacagguc cgagcucagc 840
   ccauugcugc uguccaccac acaguggcag guccuuccgu guucuuucac gacccugcca 900
   gccuugucca ccggccucau ccaccuccac cagaacauug uggacgugca guacuuguac 960
   gggguagggu caagcaucgc guccugggcc auuaaguggg aguacgucgu ucuccuguuc 1020
   cuccugcuug cagacgcgcg cgucugcucc ugcuugugga ugauguuacu cauaucccaa 1080
   gcggaggcg 1089
<210> 91
   <211> 498
   <212> PRT
   <213> Lymphocytic choriomeningitis virus
<400> 91
   Met Gly Gln Ile Val Thr Met Phe Glu Ala Leu Pro His Ile Ile Asp
   1               5                   10                  15
   Glu Val Ile Asn Ile Val Ile Ile Val Leu Ile Val Ile Thr Gly Ile
               20                  25                  30
   Lys Ala Val Tyr Asn Phe Ala Thr Cys Gly Ile Phe Ala Leu Ile Ser
           35                  40                  45
   Phe Leu Leu Leu Ala Gly Arg Ser Cys Gly Met Tyr Gly Leu Lys Gly
       50                  55                  60
   Pro Asp Ile Tyr Lys Gly Val Tyr Gln Phe Lys Ser Val Glu Phe Asp
   65                  70                  75                  80
   Met Ser His Leu Asn Leu Thr Met Pro Asn Ala Cys Ser Ala Asn Asn
                   85                  90                  95
   Ser His His Tyr Ile Ser Met Gly Thr Ser Gly Leu Glu Leu Thr Phe
               100                 105                 110
   Thr Asn Asp Ser Ile Ile Ser His Asn Phe Cys Asn Leu Thr Ser Ala
           115                 120                 125
   Phe Asn Lys Lys Thr Phe Asp His Thr Leu Met Ser Ile Val Ser Ser
       130                 135                 140
   Leu His Leu Ser Ile Arg Gly Asn Ser Asn Tyr Lys Ala Val Ser Cys
   145 150 155 160
   Asp Phe Asn Asn Gly Ile Thr Ile Gln Tyr Asn Leu Thr Phe Ser Asp
   165 170 175
   Arg Gln Ser Ala Gln Ser Gln Cys Arg Thr Phe Arg Gly Arg Val Leu
   180 185 190
   Asp Met Phe Arg Thr Ala Phe Gly Gly Lys Tyr Met Arg Ser Gly Trp
   195 200 205
   Gly Trp Thr Gly Ser Asp Gly Lys Thr Thr Trp Cys Ser Gln Thr Ser
   210 215 220
   Tyr Gln Tyr Leu Ile Ile Gln Asn Arg Thr Trp Glu Asn His Cys Thr
   225 230 235 240
   Tyr Ala Gly Pro Phe Gly Met Ser Arg Ile Leu Leu Ser Gln Glu Lys
   245 250 255
   Thr Lys Phe Phe Thr Arg Arg Leu Ala Gly Thr Phe Thr Trp Thr Leu
   260 265 270
   Ser Asp Ser Ser Gly Val Glu Asn Pro Gly Gly Tyr Cys Leu Thr Lys
   275 280 285
   Trp Met Ile Leu Ala Ala Glu Leu Lys Cys Phe Gly Asn Thr Ala Val
   290 295 300
   Ala Lys Cys Asn Val Asn His Asp Ala Glu Phe Cys Asp Met Leu Arg
   305 310 315 320
   Leu Ile Asp Tyr Asn Lys Ala Ala Leu Ser Lys Phe Lys Glu Asp Val
   325 330 335
   Glu Ser Ala Leu His Leu Phe Lys Thr Thr Val Asn Ser Leu Ile Ser
   340 345 350
   Asp Gln Leu Leu Met Arg Asn His Leu Arg Asp Leu Met Gly Val Pro
   355 360 365
   Tyr Cys Asn Tyr Ser Lys Phe Trp Tyr Leu Glu His Ala Lys Thr Gly
   370 375 380
   Glu Thr Ser Val Pro Lys Cys Trp Leu Val Thr Asn Gly Ser Tyr Leu
   385 390 395 400
   Asn Glu Thr His Phe Ser Asp Gln Ile Glu Gln Glu Ala Asp Asn Met
   405 410 415
   Ile Thr Glu Met Leu Arg Lys Asp Tyr Ile Lys Arg Gln Gly Ser Thr
   420 425 430
   Pro Leu Ala Leu Met Asp Leu Leu Met Phe Ser Thr Ser Ala Tyr Leu
   435 440 445
   Val Ser Ile Phe Leu His Leu Val Lys Ile Pro Thr His Arg His Ile
   450 455 460
   Lys Gly Gly Ser Cys Pro Lys Pro His Arg Leu Thr Asn Lys Gly Ile
   465 470 475 480
   Cys Ser Cys Gly Ala Phe Lys Val Pro Gly Val Lys Thr Val Trp Lys
   485 490 495
   Arg Arg
<210> 92
   <211> 1497
   <212> RNA
   <213> Lymphocytic choriomeningitis virus
<400> 92
   augggucaga uugugacaau guuugaggcu cugccucaca ucaucgauga ggugaucaac      60
   auugucauua uugugcuuau cgugaucacg gguaucaagg cugucuacaa uuuugccacc     120
   ugugggauau ucgcauugau caguuuccua cuucuggcug gcagguccug uggcauguac     180
   ggucuuaagg gacccgacau uuacaaagga guuuaccaau uuaagucagu ggaguuugau     240
   augucacauc ugaaccugac caugcccaac gcauguucag ccaacaacuc ccaccauuac     300
   aucaguaugg ggacuucugg acuagaauug accuucacca augauuccau caucagucac     360
   aacuuuugca aucugaccuc ugccuucaac aaaaagaccu uugaccacac acucaugagu     420
   auaguuucga gccuacaccu caguaucaga gggaacucca acuauaaggc aguauccugc     480
   gacuucaaca auggcauaac cauccaauac aacuugacau ucucagaucg acaaagugcu     540
   cagagccagu guagaaccuu cagagguaga guccuagaua uguuuagaac ugccuucggg     600
   gggaaauaca ugaggagugg cuggggcugg acaggcucag auggcaagac caccuggugu     660
   agccagacga guuaccaaua ccugauuaua caaaauagaa ccugggaaaa ccacugcaca     720
   uaugcagguc cuuuugggau guccaggauu cuccuuuccc aagagaagac uaaguucuuc     780
   acuaggagac uagcgggcac auucaccugg acuuugucag acucuucagg gguggagaau     840
   ccaggugguu auugccugac caaauggaug auucuugcug cagagcuuaa guguuucggg     900
   aacacagcag uugcgaaaug caauguaaau caugaugccg aauucuguga caugcugcga     960
   cuaauugacu acaacaaggc ugcuuugagu aaguucaaag aggacguaga aucugccuug    1020
   cacuuauuca aaacaacagu gaauucuuug auuucagauc aacuacugau gaggaaccac    1080
   uugagagauc ugaugggggu gccauauugc aauuacucaa aguuuuggua ccuagaacau    1140
   gcaaagaccg gcgaaacuag uguccccaag ugcuggcuug ucaccaaugg uucuuacuua    1200
   aaugagaccc acuucaguga ucaaaucgaa caggaagccg auaacaugau uacagagaug    1260
   uugaggaagg auuacauaaa gaggcagggg aguacccccc uagcauugau ggaccuucug    1320
   auguuuucca caucugcaua ucuagucagc aucuuccugc accuugucaa aauaccaaca    1380
   cacaggcaca uaaaaggugg cucaugucca aagccacacc gauuaaccaa caaaggaauu    1440
   uguaguugug gugcauuuaa ggugccuggu guaaaaaccg ucuggaaaag acgcuga        1497
<210> 93
   <211> 639
   <212> PRT
   <213> Artificial Sequence
<220>
   <223> fusion protein of vesicular stomatitis virus derived glycoprotein and a rapamycin-binding heterodimerizable protein domain
<400> 93
   Met Val Ser Arg Gly Val Gln Val Glu Thr Ile Ser Pro Gly Asp Gly
   1               5                   10                  15
   Arg Thr Phe Pro Lys Arg Gly Gln Thr Cys Val Val His Tyr Thr Gly
               20                  25                  30
   Met Leu Glu Asp Gly Lys Lys Phe Asp Ser Ser Arg Asp Arg Asn Lys
           35                  40                  45
   Pro Phe Lys Phe Met Leu Gly Lys Gln Glu Val Ile Arg Gly Trp Glu
       50                  55                  60
   Glu Gly Val Ala Gln Met Ser Val Gly Gln Arg Ala Lys Leu Thr Ile
   65                  70                  75                  80
   Ser Pro Asp Tyr Ala Tyr Gly Ala Thr Gly His Pro Gly Ile Ile Pro
                   85                  90                  95
   Pro His Ala Thr Leu Val Phe Asp Val Glu Leu Leu Lys Leu Glu Thr
               100                 105                 110
   Ser Gly Ser Gly Gly Gly Gly Ser Gly Gly Gly Gly Ser Gly Gly Gly
           115                 120                 125
   Ala Ser Cys Leu Leu Tyr Leu Ala Phe Leu Phe Ile Gly Val Asn Cys
       130                 135                 140
   Lys Phe Thr Ile Val Phe Pro His Asn Gln Lys Gly Asn Trp Lys Asn
   145                 150                 155                 160
   Val Pro Ser Asn Tyr His Tyr Cys Pro Ser Ser Ser Asp Leu Asn Trp
                   165                 170                 175
   His Asn Asp Leu Ile Gly Thr Ala Leu Gln Val Lys Met Pro Lys Ser
               180                 185                 190
   His Lys Ala Ile Gln Ala Asp Gly Trp Met Cys His Ala Ser Lys Trp
           195                 200                 205
   Val Thr Thr Cys Asp Phe Arg Trp Tyr Gly Pro Lys Tyr Ile Thr Gln
       210                 215                 220
   Ser Ile Arg Ser Phe Thr Pro Ser Val Glu Gln Cys Lys Glu Ser Ile
   225                 230                 235                 240
   Glu Gln Thr Lys Gln Gly Thr Trp Leu Asn Pro Gly Phe Pro Pro Gln
                   245                 250                 255
   Ser Cys Gly Tyr Ala Thr Val Thr Asp Ala Glu Ala Val Ile Val Gln
               260                 265                 270
   Val Thr Pro His His Val Leu Val Asp Glu Tyr Thr Gly Glu Trp Val
           275                 280                 285
   Asp Ser Gln Phe Ile Asn Gly Lys Cys Ser Asn Tyr Ile Cys Pro Thr
       290                 295                 300
   Val His Asn Ser Thr Thr Trp His Ser Asp Tyr Lys Val Lys Gly Leu
   305                 310                 315                 320
   Cys Asp Ser Asn Leu Ile Ser Met Asp Ile Thr Phe Phe Ser Glu Asp
                   325                 330                 335
   Gly Glu Leu Ser Ser Leu Gly Lys Glu Gly Thr Gly Phe Arg Ser Asn
               340                 345                 350
   Tyr Phe Ala Tyr Glu Thr Gly Gly Lys Ala Cys Lys Met Gln Tyr Cys
           355                 360                 365
   Lys His Trp Gly Val Arg Leu Pro Ser Gly Val Trp Phe Glu Met Ala
       370                 375                 380
   Asp Lys Asp Leu Phe Ala Ala Ala Arg Phe Pro Glu Cys Pro Glu Gly
   385                 390                 395                 400
   Ser Ser Ile Ser Ala Pro Ser Gln Thr Ser Val Asp Val Ser Leu Ile
                   405                 410                 415
   Gln Asp Val Glu Arg Ile Leu Asp Tyr Ser Leu Cys Gln Glu Thr Trp
               420                 425                 430
   Ser Lys Ile Arg Ala Gly Leu Pro Ile Ser Pro Val Asp Leu Ser Tyr
           435                 440                 445
   Leu Ala Pro Lys Asn Pro Gly Thr Gly Pro Ala Phe Thr Ile Ile Asn
       450                 455                 460
   Gly Thr Leu Lys Tyr Phe Glu Thr Arg Tyr Ile Arg Val Asp Ile Ala
   465                 470                 475                 480
   Ala Pro Ile Leu Ser Arg Met Val Gly Met Ile Ser Gly Thr Thr Thr
                   485                 490                 495
   Glu Arg Glu Leu Trp Asp Asp Trp Ala Pro Tyr Glu Asp Val Glu Ile
               500                 505                 510
   Gly Pro Asn Gly Val Leu Arg Thr Ser Ser Gly Tyr Lys Phe Pro Leu
           515                 520                 525
   Tyr Met Ile Gly His Gly Met Leu Asp Ser Asp Leu His Leu Ser Ser
       530                 535                 540
   Lys Ala Gln Val Phe Glu His Pro His Ile Gln Asp Ala Ala Ser Gln
   545                 550                 555                 560
   Leu Pro Asp Asp Glu Ser Leu Phe Phe Gly Asp Thr Gly Leu Ser Lys
                   565                 570                 575
   Asn Pro Ile Glu Leu Val Glu Gly Trp Phe Ser Ser Trp Lys Ser Ser
               580                 585                 590
   Ile Ala Ser Phe Phe Phe Ile Ile Gly Leu Ile Ile Gly Leu Phe Leu
           595                 600                 605
   Val Leu Arg Val Gly Ile His Leu Cys Ile Lys Leu Lys His Thr Lys
       610                 615                 620
   Lys Arg Gln Ile Tyr Thr Asp Ile Glu Met Asn Arg Leu Gly Lys
   625                 630                 635
<210> 94
   <211> 1920
   <212> DNA
   <213> Artificial Sequence
<220>
   <223> polynucleotide encoding for a fusion protein of vesicular stomatitis virus derived glycoprotein and a rapamycin-binding heterodimerizable protein domain
<400> 94
   atggtgtcta gaggagtgca ggtggaaacc atctccccag gagacgggcg caccttcccc 60
   aagcgcggcc agacctgcgt ggtgcactac accgggatgc ttgaagatgg aaagaaattt 120
   gattcctccc gggacagaaa caagcccttt aagtttatgc taggcaagca ggaggtgatc 180
   cgaggctggg aagaaggggt tgcccagatg agtgtgggtc agagagccaa actgactata 240
   tctccagatt atgcctatgg tgccactggg cacccaggca tcatcccacc acatgccact 300
   ctcgtcttcg atgtggagct tctaaaactg gaaactagtg gatccggagg cggcggcagc 360
   ggcgggggcg ggagcggcgg cggggctagc tgccttttgt acttagcctt tttattcatt 420
   ggggtgaatt gcaagttcac catagttttt ccacacaacc aaaaaggaaa ctggaaaaat 480
   gttccttcta attaccatta ttgcccgtca agctcagatt taaattggca taatgactta 540
   ataggcacag ccttacaagt caaaatgccc aagagtcaca aggctattca agcagacggt 600
   tggatgtgtc atgcttccaa atgggtcact acttgtgatt tccgctggta tggaccgaag 660
   tatataacac agtccatccg atccttcact ccatctgtag aacaatgcaa ggaaagcatt 720
   gaacaaacga aacaaggaac ttggctgaat ccaggcttcc ctcctcaaag ttgtggatat 780
   gcaactgtga cggatgccga agcagtgatt gtccaggtga ctcctcacca tgtgctggtt 840
   gatgaataca caggagaatg ggttgattca cagttcatca acggaaaatg cagcaattac 900
   atatgcccca ctgtccataa ctctacaacc tggcattctg actataaggt caaagggcta 960
   tgtgattcta acctcatttc catggacatc accttcttct cagaggacgg agagctatca 1020
   tccctgggaa aggagggcac agggttcaga agtaactact ttgcttatga aactggaggc 1080
   aaggcctgca aaatgcaata ctgcaagcat tggggagtca gactcccatc aggtgtctgg 1140
   ttcgagatgg ctgataagga tctctttgct gcagccagat tccctgaatg cccagaaggg 1200
   tcaagtatct ctgctccatc tcagacctca gtggatgtaa gtctaattca ggacgttgag 1260
   aggatcttgg attattccct ctgccaagaa acctggagca aaatcagagc gggtcttcca 1320
   atctctccag tggatctcag ctatcttgct cctaaaaacc caggaaccgg tcctgctttc 1380
   accataatca atggtaccct aaaatacttt gagaccagat acatcagagt cgatattgct 1440
   gctccaatcc tctcaagaat ggtcggaatg atcagtggaa ctaccacaga aagggaactg 1500
   tgggatgact gggcaccata tgaagacgtg gaaattggac ccaatggagt tctgaggacc 1560
   agttcaggat ataagtttcc tttatacatg attggacatg gtatgttgga ctccgatctt 1620
   catcttagct caaaggctca ggtgttcgaa catcctcaca ttcaagacgc tgcttcgcaa 1680
   cttcctgatg atgagagttt attttttggt gatactgggc tatccaaaaa tccaatcgag 1740
   cttgtagaag gttggttcag tagttggaaa agctctattg cctctttttt ctttatcata 1800
   gggttaatca ttggactatt cttggttctc cgagttggta tccatctttg cattaaatta     1860
   aagcacacca agaaaagaca gatttataca gacatagaga tgaaccgact tggaaagtaa     1920
<210> 95
   <211> 625
   <212> PRT
   <213> Artificial Sequence
<220>
   <223> fusion protein of vesicular stomatitis virus derived glycoprotein and a rapamycin-binding heterodimerizable protein domain
<400> 95
   Met Val Ser Arg Ile Leu Trp His Glu Met Trp His Glu Gly Leu Glu
   1               5                   10                  15
   Glu Ala Ser Arg Leu Tyr Phe Gly Glu Arg Asn Val Lys Gly Met Phe
               20                  25                  30
   Glu Val Leu Glu Pro Leu His Ala Met Met Glu Arg Gly Pro Gln Thr
           35                  40                  45
   Leu Lys Glu Thr Ser Phe Asn Gln Ala Tyr Gly Arg Asp Leu Met Glu
       50                  55                  60
   Ala Gln Glu Trp Cys Arg Lys Tyr Met Lys Ser Gly Asn Val Lys Asp
   65                  70                  75                  80
   Leu Leu Gln Ala Trp Asp Leu Tyr Tyr His Val Phe Arg Arg Ile Ser
                   85                  90                  95
   Lys Thr Ser Gly Ser Gly Gly Gly Gly Ser Gly Gly Gly Gly Ser Gly
               100                 105                 110
   Gly Gly Ala Ser Cys Leu Leu Tyr Leu Ala Phe Leu Phe Ile Gly Val
           115                 120                 125
   Asn Cys Lys Phe Thr Ile Val Phe Pro His Asn Gln Lys Gly Asn Trp
       130                 135                 140
   Lys Asn Val Pro Ser Asn Tyr His Tyr Cys Pro Ser Ser Ser Asp Leu
   145                 150                 155                 160
   Asn Trp His Asn Asp Leu Ile Gly Thr Ala Leu Gln Val Lys Met Pro
                   165                 170                 175
   Lys Ser His Lys Ala Ile Gln Ala Asp Gly Trp Met Cys His Ala Ser
               180                 185                 190
   Lys Trp Val Thr Thr Cys Asp Phe Arg Trp Tyr Gly Pro Lys Tyr Ile
   195 200 205
   Thr Gln Ser Ile Arg Ser Phe Thr Pro Ser Val Glu Gln Cys Lys Glu
   210 215 220
   Ser Ile Glu Gln Thr Lys Gln Gly Thr Trp Leu Asn Pro Gly Phe Pro
   225 230 235 240
   Pro Gln Ser Cys Gly Tyr Ala Thr Val Thr Asp Ala Glu Ala Val Ile
   245 250 255
   Val Gln Val Thr Pro His His Val Leu Val Asp Glu Tyr Thr Gly Glu
   260 265 270
   Trp Val Asp Ser Gln Phe Ile Asn Gly Lys Cys Ser Asn Tyr Ile Cys
   275 280 285
   Pro Thr Val His Asn Ser Thr Thr Trp His Ser Asp Tyr Lys Val Lys
   290 295 300
   Gly Leu Cys Asp Ser Asn Leu Ile Ser Met Asp Ile Thr Phe Phe Ser
   305 310 315 320
   Glu Asp Gly Glu Leu Ser Ser Leu Gly Lys Glu Gly Thr Gly Phe Arg
   325 330 335
   Ser Asn Tyr Phe Ala Tyr Glu Thr Gly Gly Lys Ala Cys Lys Met Gln
   340 345 350
   Tyr Cys Lys His Trp Gly Val Arg Leu Pro Ser Gly Val Trp Phe Glu
   355 360 365
   Met Ala Asp Lys Asp Leu Phe Ala Ala Ala Arg Phe Pro Glu Cys Pro
   370 375 380
   Glu Gly Ser Ser Ile Ser Ala Pro Ser Gln Thr Ser Val Asp Val Ser
   385 390 395 400
   Leu Ile Gln Asp Val Glu Arg Ile Leu Asp Tyr Ser Leu Cys Gln Glu
   405 410 415
   Thr Trp Ser Lys Ile Arg Ala Gly Leu Pro Ile Ser Pro Val Asp Leu
   420 425 430
   Ser Tyr Leu Ala Pro Lys Asn Pro Gly Thr Gly Pro Ala Phe Thr Ile
   435 440 445
   Ile Asn Gly Thr Leu Lys Tyr Phe Glu Thr Arg Tyr Ile Arg Val Asp
   450 455 460
   Ile Ala Ala Pro Ile Leu Ser Arg Met Val Gly Met Ile Ser Gly Thr
   465 470 475 480
   Thr Thr Glu Arg Glu Leu Trp Asp Asp Trp Ala Pro Tyr Glu Asp Val
   485 490 495
   Glu Ile Gly Pro Asn Gly Val Leu Arg Thr Ser Ser Gly Tyr Lys Phe
   500 505 510
   Pro Leu Tyr Met Ile Gly His Gly Met Leu Asp Ser Asp Leu His Leu
   515 520 525
   Ser Ser Lys Ala Gln Val Phe Glu His Pro His Ile Gln Asp Ala Ala
   530 535 540
   Ser Gln Leu Pro Asp Asp Glu Ser Leu Phe Phe Gly Asp Thr Gly Leu
   545 550 555 560
   Ser Lys Asn Pro Ile Glu Leu Val Glu Gly Trp Phe Ser Ser Trp Lys
   565 570 575
   Ser Ser Ile Ala Ser Phe Phe Phe Ile Ile Gly Leu Ile Ile Gly Leu
   580 585 590
   Phe Leu Val Leu Arg Val Gly Ile His Leu Cys Ile Lys Leu Lys His
   595 600 605
   Thr Lys Lys Arg Gln Ile Tyr Thr Asp Ile Glu Met Asn Arg Leu Gly
   610 615 620
   Lys
   625
<210> 96
   <211> 1878
   <212> DNA
   <213> Artificial Sequence
<220>
   <223> polynucleotide encoding for a fusion protein of vesicular stomatitis virus derived glycoprotein and a rapamycin-binding heterodimerizable protein domain
<400> 96
   atggtgtcta gaatcctctg gcatgagatg tggcatgaag gcctggaaga ggcatctcgt 60
   ttgtactttg gggaaaggaa cgtgaaaggc atgtttgagg tgctggagcc cttgcatgct 120
   atgatggaac ggggccccca gactctgaag gaaacatcct ttaatcaggc ctatggtcga 180
   gatttaatgg aggcccaaga gtggtgcagg aagtacatga aatcagggaa tgtcaaggac 240
   ctcctccaag cctgggacct ctattatcat gtgttccgac gaatctcaaa gactagtgga 300
   tccggaggcg gcggcagcgg cgggggcggg agcggcggcg gggctagctg ccttttgtac 360
   ttagcctttt tattcattgg ggtgaattgc aagttcacca tagtttttcc acacaaccaa 420
   aaaggaaact ggaaaaatgt tccttctaat taccattatt gcccgtcaag ctcagattta 480
   aattggcata atgacttaat aggcacagcc ttacaagtca aaatgcccaa gagtcacaag 540
   gctattcaag cagacggttg gatgtgtcat gcttccaaat gggtcactac ttgtgatttc 600
   cgctggtatg gaccgaagta tataacacag tccatccgat ccttcactcc atctgtagaa 660
   caatgcaagg aaagcattga acaaacgaaa caaggaactt ggctgaatcc aggcttccct 720
   cctcaaagtt gtggatatgc aactgtgacg gatgccgaag cagtgattgt ccaggtgact 780
   cctcaccatg tgctggttga tgaatacaca ggagaatggg ttgattcaca gttcatcaac 840
   ggaaaatgca gcaattacat atgccccact gtccataact ctacaacctg gcattctgac 900
   tataaggtca aagggctatg tgattctaac ctcatttcca tggacatcac cttcttctca 960
   gaggacggag agctatcatc cctgggaaag gagggcacag ggttcagaag taactacttt 1020
   gcttatgaaa ctggaggcaa ggcctgcaaa atgcaatact gcaagcattg gggagtcaga 1080
   ctcccatcag gtgtctggtt cgagatggct gataaggatc tctttgctgc agccagattc 1140
   cctgaatgcc cagaagggtc aagtatctct gctccatctc agacctcagt ggatgtaagt 1200
   ctaattcagg acgttgagag gatcttggat tattccctct gccaagaaac ctggagcaaa 1260
   atcagagcgg gtcttccaat ctctccagtg gatctcagct atcttgctcc taaaaaccca 1320
   ggaaccggtc ctgctttcac cataatcaat ggtaccctaa aatactttga gaccagatac 1380
   atcagagtcg atattgctgc tccaatcctc tcaagaatgg tcggaatgat cagtggaact 1440
   accacagaaa gggaactgtg ggatgactgg gcaccatatg aagacgtgga aattggaccc 1500
   aatggagttc tgaggaccag ttcaggatat aagtttcctt tatacatgat tggacatggt 1560
   atgttggact ccgatcttca tcttagctca aaggctcagg tgttcgaaca tcctcacatt 1620
   caagacgctg cttcgcaact tcctgatgat gagagtttat tttttggtga tactgggcta 1680
   tccaaaaatc caatcgagct tgtagaaggt tggttcagta gttggaaaag ctctattgcc 1740
   tcttttttct ttatcatagg gttaatcatt ggactattct tggttctccg agttggtatc 1800
   catctttgca ttaaattaaa gcacaccaag aaaagacaga tttatacaga catagagatg 1860
   aaccgacttg gaaagtaa 1878
<210> 97
   <211> 624
   <212> PRT
   <213> Artificial Sequence
<220>
   <223> fusion protein of vesicular stomatitis virus derived glycoprotein and a rapamycin-binding heterodimerizable protein domain
<400> 97
   Met Lys Cys Leu Leu Tyr Leu Ala Phe Leu Phe Ile Gly Val Asn Cys
   1               5                   10                  15
   Lys Phe Thr Ile Val Phe Pro His Asn Gln Lys Gly Asn Trp Lys Asn
               20                  25                  30
   Val Pro Ser Asn Tyr His Tyr Cys Pro Ser Ser Ser Asp Leu Asn Trp
           35                  40                  45
   His Asn Asp Leu Ile Gly Thr Ala Leu Gln Val Lys Met Pro Lys Ser
       50                  55                  60
   His Lys Ala Ile Gln Ala Asp Gly Trp Met Cys His Ala Ser Lys Trp
   65                  70                  75                  80
   Val Thr Thr Cys Asp Phe Arg Trp Tyr Gly Pro Lys Tyr Ile Thr Gln
                   85                  90                  95
   Ser Ile Arg Ser Phe Thr Pro Ser Val Glu Gln Cys Lys Glu Ser Ile
               100                 105                 110
   Glu Gln Thr Lys Gln Gly Thr Trp Leu Asn Pro Gly Phe Pro Pro Gln
           115                 120                 125
   Ser Cys Gly Tyr Ala Thr Val Thr Asp Ala Glu Ala Val Ile Val Gln
       130                 135                 140
   Val Thr Pro His His Val Leu Val Asp Glu Tyr Thr Gly Glu Trp Val
   145                 150                 155                 160
   Asp Ser Gln Phe Ile Asn Gly Lys Cys Ser Asn Tyr Ile Cys Pro Thr
                   165                 170                 175
   Val His Asn Ser Thr Thr Trp His Ser Asp Tyr Lys Val Lys Gly Leu
               180                 185                 190
   Cys Asp Ser Asn Leu Ile Ser Met Asp Ile Thr Phe Phe Ser Glu Asp
           195                 200                 205
   Gly Glu Leu Ser Ser Leu Gly Lys Glu Gly Thr Gly Phe Arg Ser Asn
       210                 215                 220
   Tyr Phe Ala Tyr Glu Thr Gly Gly Lys Ala Cys Lys Met Gln Tyr Cys
   225                 230                 235                 240
   Lys His Trp Gly Val Arg Leu Pro Ser Gly Val Trp Phe Glu Met Ala
                   245                 250                 255
   Asp Lys Asp Leu Phe Ala Ala Ala Arg Phe Pro Glu Cys Pro Glu Gly
               260                 265                 270
   Ser Ser Ile Ser Ala Pro Ser Gln Thr Ser Val Asp Val Ser Leu Ile
           275                 280                 285
   Gln Asp Val Glu Arg Ile Leu Asp Tyr Ser Leu Cys Gln Glu Thr Trp
       290                 295                 300
   Ser Lys Ile Arg Ala Gly Leu Pro Ile Ser Pro Val Asp Leu Ser Tyr
   305                 310                 315                 320
   Leu Ala Pro Lys Asn Pro Gly Thr Gly Pro Ala Phe Thr Ile Ile Asn
                   325                 330                 335
   Gly Thr Leu Lys Tyr Phe Glu Thr Arg Tyr Ile Arg Val Asp Ile Ala
               340                 345                 350
   Ala Pro Ile Leu Ser Arg Met Val Gly Met Ile Ser Gly Thr Thr Thr
           355                 360                 365
   Glu Arg Glu Leu Trp Asp Asp Trp Ala Pro Tyr Glu Asp Val Glu Ile
       370                 375                 380
   Gly Pro Asn Gly Val Leu Arg Thr Ser Ser Gly Tyr Lys Phe Pro Leu
   385                 390                 395                 400
   Tyr Met Ile Gly His Gly Met Leu Asp Ser Asp Leu His Leu Ser Ser
                   405                 410                 415
   Lys Ala Gln Val Phe Glu His Pro His Ile Gln Asp Ala Ala Ser Gln
               420                 425                 430
   Leu Pro Asp Asp Glu Ser Leu Phe Phe Gly Asp Thr Gly Leu Ser Lys
           435                 440                 445
   Asn Pro Ile Glu Leu Val Glu Gly Trp Phe Ser Ser Trp Lys Ser Ser
       450                 455                 460
   Ile Ala Ser Phe Phe Phe Ile Ile Gly Leu Ile Ile Gly Leu Phe Leu
   465                 470                 475                 480
   Val Leu Arg Val Gly Ile His Leu Cys Ile Lys Leu Lys His Thr Lys
                   485                 490                 495
   Lys Arg Gln Ile Tyr Thr Asp Ile Glu Met Asn Arg Leu Gly Lys Ala
               500                 505                 510
   Ser Ser Arg Gly Val Gln Val Glu Thr Ile Ser Pro Gly Asp Gly Arg
   515 520 525
   Thr Phe Pro Lys Arg Gly Gln Thr Cys Val Val His Tyr Thr Gly Met
   530 535 540
   Leu Glu Asp Gly Lys Lys Phe Asp Ser Ser Arg Asp Arg Asn Lys Pro
   545 550 555 560
   Phe Lys Phe Met Leu Gly Lys Gln Glu Val Ile Arg Gly Trp Glu Glu
   565 570 575
   Gly Val Ala Gln Met Ser Val Gly Gln Arg Ala Lys Leu Thr Ile Ser
   580 585 590
   Pro Asp Tyr Ala Tyr Gly Ala Thr Gly His Pro Gly Ile Ile Pro Pro
   595 600 605
   His Ala Thr Leu Val Phe Asp Val Glu Leu Leu Lys Leu Glu Thr Ser
   610 615 620
<210> 98
   <211> 1875
   <212> DNA
   <213> Artificial Sequence
<220>
   <223> polynucleotide encoding for a fusion protein of vesicular stomatitis virus derived glycoprotein and a rapamycin-binding heterodimerizable protein domain
<400> 98
   atgaagtgcc ttttgtactt agccttttta ttcattgggg tgaattgcaa gttcaccata 60
   gtttttccac acaaccaaaa aggaaactgg aaaaatgttc cttctaatta ccattattgc 120
   ccgtcaagct cagatttaaa ttggcataat gacttaatag gcacagcctt acaagtcaaa 180
   atgcccaaga gtcacaaggc tattcaagca gacggttgga tgtgtcatgc ttccaaatgg 240
   gtcactactt gtgatttccg ctggtatgga ccgaagtata taacacagtc catccgatcc 300
   ttcactccat ctgtagaaca atgcaaggaa agcattgaac aaacgaaaca aggaacttgg 360
   ctgaatccag gcttccctcc tcaaagttgt ggatatgcaa ctgtgacgga tgccgaagca 420
   gtgattgtcc aggtgactcc tcaccatgtg ctggttgatg aatacacagg agaatgggtt 480
   gattcacagt tcatcaacgg aaaatgcagc aattacatat gccccactgt ccataactct 540
   acaacctggc attctgacta taaggtcaaa gggctatgtg attctaacct catttccatg 600
   gacatcacct tcttctcaga ggacggagag ctatcatccc tgggaaagga gggcacaggg 660
   ttcagaagta actactttgc ttatgaaact ggaggcaagg cctgcaaaat gcaatactgc 720
   aagcattggg gagtcagact cccatcaggt gtctggttcg agatggctga taaggatctc 780
   tttgctgcag ccagattccc tgaatgccca gaagggtcaa gtatctctgc tccatctcag 840
   acctcagtgg atgtaagtct aattcaggac gttgagagga tcttggatta ttccctctgc 900
   caagaaacct ggagcaaaat cagagcgggt cttccaatct ctccagtgga tctcagctat 960
   cttgctccta aaaacccagg aaccggtcct gctttcacca taatcaatgg taccctaaaa 1020
   tactttgaga ccagatacat cagagtcgat attgctgctc caatcctctc aagaatggtc 1080
   ggaatgatca gtggaactac cacagaaagg gaactgtggg atgactgggc accatatgaa 1140
   gacgtggaaa ttggacccaa tggagttctg aggaccagtt caggatataa gtttccttta 1200
   tacatgattg gacatggtat gttggactcc gatcttcatc ttagctcaaa ggctcaggtg 1260
   ttcgaacatc ctcacattca agacgctgct tcgcaacttc ctgatgatga gagtttattt 1320
   tttggtgata ctgggctatc caaaaatcca atcgagcttg tagaaggttg gttcagtagt 1380
   tggaaaagct ctattgcctc ttttttcttt atcatagggt taatcattgg actattcttg 1440
   gttctccgag ttggtatcca tctttgcatt aaattaaagc acaccaagaa aagacagatt 1500
   tatacagaca tagagatgaa ccgacttgga aaggctagct ctagaggagt gcaggtggaa 1560
   accatctccc caggagacgg gcgcaccttc cccaagcgcg gccagacctg cgtggtgcac 1620
   tacaccggga tgcttgaaga tggaaagaaa tttgattcct cccgggacag aaacaagccc 1680
   tttaagttta tgctaggcaa gcaggaggtg atccgaggct gggaagaagg ggttgcccag 1740
   atgagtgtgg gtcagagagc caaactgact atatctccag attatgccta tggtgccact 1800
   gggcacccag gcatcatccc accacatgcc actctcgtct tcgatgtgga gcttctaaaa 1860
   ctggaaacta gttag 1875
<210> 99
   <211> 610
   <212> PRT
   <213> Artificial Sequence
<220>
   <223> fusion protein of vesicular stomatitis virus derived glycoprotein and a rapamycin-binding heterodimerizable protein domain
<400> 99
   Met Lys Cys Leu Leu Tyr Leu Ala Phe Leu Phe Ile Gly Val Asn Cys
   1               5                   10                  15
   Lys Phe Thr Ile Val Phe Pro His Asn Gln Lys Gly Asn Trp Lys Asn
               20                  25                  30
   Val Pro Ser Asn Tyr His Tyr Cys Pro Ser Ser Ser Asp Leu Asn Trp
           35                  40                  45
   His Asn Asp Leu Ile Gly Thr Ala Leu Gln Val Lys Met Pro Lys Ser
   50 55 60
   His Lys Ala Ile Gln Ala Asp Gly Trp Met Cys His Ala Ser Lys Trp
   65 70 75 80
   Val Thr Thr Cys Asp Phe Arg Trp Tyr Gly Pro Lys Tyr Ile Thr Gln
   85 90 95
   Ser Ile Arg Ser Phe Thr Pro Ser Val Glu Gln Cys Lys Glu Ser Ile
   100 105 110
   Glu Gln Thr Lys Gln Gly Thr Trp Leu Asn Pro Gly Phe Pro Pro Gln
   115 120 125
   Ser Cys Gly Tyr Ala Thr Val Thr Asp Ala Glu Ala Val Ile Val Gln
   130 135 140
   Val Thr Pro His His Val Leu Val Asp Glu Tyr Thr Gly Glu Trp Val
   145 150 155 160
   Asp Ser Gln Phe Ile Asn Gly Lys Cys Ser Asn Tyr Ile Cys Pro Thr
   165 170 175
   Val His Asn Ser Thr Thr Trp His Ser Asp Tyr Lys Val Lys Gly Leu
   180 185 190
   Cys Asp Ser Asn Leu Ile Ser Met Asp Ile Thr Phe Phe Ser Glu Asp
   195 200 205
   Gly Glu Leu Ser Ser Leu Gly Lys Glu Gly Thr Gly Phe Arg Ser Asn
   210 215 220
   Tyr Phe Ala Tyr Glu Thr Gly Gly Lys Ala Cys Lys Met Gln Tyr Cys
   225 230 235 240
   Lys His Trp Gly Val Arg Leu Pro Ser Gly Val Trp Phe Glu Met Ala
   245 250 255
   Asp Lys Asp Leu Phe Ala Ala Ala Arg Phe Pro Glu Cys Pro Glu Gly
   260 265 270
   Ser Ser Ile Ser Ala Pro Ser Gln Thr Ser Val Asp Val Ser Leu Ile
   275 280 285
   Gln Asp Val Glu Arg Ile Leu Asp Tyr Ser Leu Cys Gln Glu Thr Trp
   290 295 300
   Ser Lys Ile Arg Ala Gly Leu Pro Ile Ser Pro Val Asp Leu Ser Tyr
   305 310 315 320
   Leu Ala Pro Lys Asn Pro Gly Thr Gly Pro Ala Phe Thr Ile Ile Asn
   325 330 335
   Gly Thr Leu Lys Tyr Phe Glu Thr Arg Tyr Ile Arg Val Asp Ile Ala
   340 345 350
   Ala Pro Ile Leu Ser Arg Met Val Gly Met Ile Ser Gly Thr Thr Thr
   355 360 365
   Glu Arg Glu Leu Trp Asp Asp Trp Ala Pro Tyr Glu Asp Val Glu Ile
   370 375 380
   Gly Pro Asn Gly Val Leu Arg Thr Ser Ser Gly Tyr Lys Phe Pro Leu
   385 390 395 400
   Tyr Met Ile Gly His Gly Met Leu Asp Ser Asp Leu His Leu Ser Ser
   405 410 415
   Lys Ala Gln Val Phe Glu His Pro His Ile Gln Asp Ala Ala Ser Gln
   420 425 430
   Leu Pro Asp Asp Glu Ser Leu Phe Phe Gly Asp Thr Gly Leu Ser Lys
   435 440 445
   Asn Pro Ile Glu Leu Val Glu Gly Trp Phe Ser Ser Trp Lys Ser Ser
   450 455 460
   Ile Ala Ser Phe Phe Phe Ile Ile Gly Leu Ile Ile Gly Leu Phe Leu
   465 470 475 480
   Val Leu Arg Val Gly Ile His Leu Cys Ile Lys Leu Lys His Thr Lys
   485 490 495
   Lys Arg Gln Ile Tyr Thr Asp Ile Glu Met Asn Arg Leu Gly Lys Ala
   500 505 510
   Ser Ser Arg Ile Leu Trp His Glu Met Trp His Glu Gly Leu Glu Glu
   515 520 525
   Ala Ser Arg Leu Tyr Phe Gly Glu Arg Asn Val Lys Gly Met Phe Glu
   530 535 540
   Val Leu Glu Pro Leu His Ala Met Met Glu Arg Gly Pro Gln Thr Leu
   545 550 555 560
   Lys Glu Thr Ser Phe Asn Gln Ala Tyr Gly Arg Asp Leu Met Glu Ala
   565 570 575
   Gln Glu Trp Cys Arg Lys Tyr Met Lys Ser Gly Asn Val Lys Asp Leu
               580                 585                 590
   Leu Gln Ala Trp Asp Leu Tyr Tyr His Val Phe Arg Arg Ile Ser Lys
           595                 600                 605
   Thr Ser
       610
<210> 100
   <211> 1833
   <212> DNA
   <213> Artificial Sequence
<220>
   <223> polynucleotide encoding for a fusion protein of vesicular stomatitis virus derived glycoprotein and a rapamycin-binding heterodimerizable protein domain
<400> 100
   atgaagtgcc ttttgtactt agccttttta ttcattgggg tgaattgcaa gttcaccata 60
   gtttttccac acaaccaaaa aggaaactgg aaaaatgttc cttctaatta ccattattgc 120
   ccgtcaagct cagatttaaa ttggcataat gacttaatag gcacagcctt acaagtcaaa 180
   atgcccaaga gtcacaaggc tattcaagca gacggttgga tgtgtcatgc ttccaaatgg 240
   gtcactactt gtgatttccg ctggtatgga ccgaagtata taacacagtc catccgatcc 300
   ttcactccat ctgtagaaca atgcaaggaa agcattgaac aaacgaaaca aggaacttgg 360
   ctgaatccag gcttccctcc tcaaagttgt ggatatgcaa ctgtgacgga tgccgaagca 420
   gtgattgtcc aggtgactcc tcaccatgtg ctggttgatg aatacacagg agaatgggtt 480
   gattcacagt tcatcaacgg aaaatgcagc aattacatat gccccactgt ccataactct 540
   acaacctggc attctgacta taaggtcaaa gggctatgtg attctaacct catttccatg 600
   gacatcacct tcttctcaga ggacggagag ctatcatccc tgggaaagga gggcacaggg 660
   ttcagaagta actactttgc ttatgaaact ggaggcaagg cctgcaaaat gcaatactgc 720
   aagcattggg gagtcagact cccatcaggt gtctggttcg agatggctga taaggatctc 780
   tttgctgcag ccagattccc tgaatgccca gaagggtcaa gtatctctgc tccatctcag 840
   acctcagtgg atgtaagtct aattcaggac gttgagagga tcttggatta ttccctctgc 900
   caagaaacct ggagcaaaat cagagcgggt cttccaatct ctccagtgga tctcagctat 960
   cttgctccta aaaacccagg aaccggtcct gctttcacca taatcaatgg taccctaaaa 1020
   tactttgaga ccagatacat cagagtcgat attgctgctc caatcctctc aagaatggtc 1080
   ggaatgatca gtggaactac cacagaaagg gaactgtggg atgactgggc accatatgaa 1140
   gacgtggaaa ttggacccaa tggagttctg aggaccagtt caggatataa gtttccttta 1200
   tacatgattg gacatggtat gttggactcc gatcttcatc ttagctcaaa ggctcaggtg 1260
   ttcgaacatc ctcacattca agacgctgct tcgcaacttc ctgatgatga gagtttattt 1320
   tttggtgata ctgggctatc caaaaatcca atcgagcttg tagaaggttg gttcagtagt 1380
   tggaaaagct ctattgcctc tttttctttt atcatagggt taatcattgg actattcttg 1440
   gttctccgag ttggtatcca tctttgcatt aaattaaagc acaccaagaa aagacagatt 1500
   tatacagaca tagagatgaa ccgacttgga aaggctagct ctagaatcct ctggcatgag 1560
   atgtggcatg aaggcctgga agaggcatct cgtttgtact ttggggaaag gaacgtgaaa 1620
   ggcatgtttg aggtgctgga gcccttgcat gctatgatgg aacggggccc ccagactctg 1680
   aaggaaacat cctttaatca ggcctatggt cgagatttaa tggaggccca agagtggtgc 1740
   aggaagtaca tgaaatcagg gaatgtcaag gacctcctcc aagcctggga cctctattat 1800
   catgtgttcc gacgaatctc aaagactagt tag 1833
<210> 101
   <211> 136
   <212> PRT
   <213> Human immunodeficiency virus type 1
<400> 101
   Met Gly Ala Arg Ala Ser Val Leu Ser Gly Gly Glu Leu Asp Arg Trp
   1               5                   10                  15
   Glu Lys Ile Arg Leu Arg Pro Gly Gly Lys Lys Lys Tyr Lys Leu Lys
               20                  25                  30
   His Ile Val Trp Ala Ser Arg Glu Leu Glu Arg Phe Ala Val Asn Pro
           35                  40                  45
   Gly Leu Leu Glu Thr Ser Glu Gly Cys Arg Gln Ile Leu Gly Gln Leu
       50                  55                  60
   Gln Pro Ser Leu Gln Thr Gly Ser Glu Glu Leu Arg Ser Leu Tyr Asn
   65                  70                  75                  80
   Thr Val Ala Thr Leu Tyr Cys Val His Gln Arg Ile Glu Ile Lys Asp
                   85                  90                  95
   Thr Lys Glu Ala Leu Asp Lys Ile Glu Glu Glu Gln Asn Lys Ser Lys
               100                 105                 110
   Lys Lys Ala Gln Gln Ala Ala Ala Asp Thr Gly His Ser Ser Gln Val
           115                 120                 125
   Ser Gln Asn Tyr Pro Ile Val Gln
       130                 135
<210> 102
   <211> 408
   <212> DNA
   <213> Human immunodeficiency virus type 1
<400> 102
   atgggcgcca gagccagcgt gctgagcggc ggcgagctgg acaggtggga gaagatcagg       60
   ctgaggcccg gcggcaagaa gaagtataag ctgaagcaca tcgtgtgggc cagcagggag      120
   ctggagaggt tcgccgtgaa ccccggcctg ctggagacca gcgagggctg caggcagatc      180
   ctgggccagc tgcagcccag cctgcagacc ggcagcgagg agctgaggag cctgtacaac      240
   accgtggcca ccctgtactg cgtgcaccag aggatcgaga tcaaggacac caaggaggcc      300
   ctggacaaga tcgaggagga gcagaacaag tccaagaaga aggcccagca ggccgccgcc      360
   gacaccggcc acagcagcca ggtgagccag aactacccca tcgtgcag                   408
<210> 103
   <211> 135
   <212> PRT
   <213> Human immunodeficiency virus type 2
<400> 103
   Met Gly Ala Arg Asn Ser Val Leu Arg Gly Lys Lys Ala Asp Glu Leu
   1               5                   10                  15
   Glu Lys Val Arg Leu Arg Pro Gly Gly Lys Lys Lys Tyr Arg Leu Lys
               20                  25                  30
   His Ile Val Trp Ala Ala Asn Glu Leu Asp Lys Phe Gly Leu Ala Glu
           35                  40                  45
   Ser Leu Leu Glu Ser Lys Glu Gly Cys Gln Lys Ile Leu Arg Val Leu
       50                  55                  60
   Asp Pro Leu Val Pro Thr Gly Ser Glu Asn Leu Lys Ser Leu Phe Asn
   65                  70                  75                  80
   Thr Val Cys Val Ile Trp Cys Leu His Ala Glu Glu Lys Val Lys Asp
                   85                  90                  95
   Thr Glu Glu Ala Lys Lys Leu Ala Gln Arg His Leu Val Ala Glu Thr
               100                 105                 110
   Gly Thr Ala Glu Lys Met Pro Asn Thr Ser Arg Pro Thr Ala Pro Pro
           115                 120                 125
   Ser Gly Lys Arg Gly Asn Tyr
       130                 135
<210> 104
   <211> 405
   <212> RNA
   <213> Human immunodeficiency virus type 2
<400> 104
   augggcgcga gaaacuccgu cuugagaggg aaaaaagcag acgaauuaga aaaaguuagg       60
   uuacggcccg gcggaaagaa aaaguacagg uuaaaacaua uugugugggc agcgaaugaa      120
   uuggauaaau ucggauuggc agagagccug uuggagucaa aagaagguug ccaaaagauu      180
   cucagaguuu uagauccauu aguaccaaca gggucagaaa auuuaaaaag ccuuuuuaau      240
   accgucugcg ucauuuggug cuugcacgca gaagagaaag ugaaagauac ugaggaagca      300
   aagaaacuag cacagagaca ucuaguggca gaaacuggaa cugcagagaa aaugccaaau      360
   acaaguagac caacagcacc accuaguggg aaaagaggaa acuac                      405
<210> 105
   <211> 92
   <212> PRT
   <213> Human T-cell lymphotropic virus type 1
<400> 105
   Met Gly Gln Ile Phe Ser Arg Ser Ala Ser Pro Ile Pro Arg Pro Pro
   1               5                   10                  15
   Arg Gly Leu Ala Ala His His Trp Leu Asn Phe Leu Gln Ala Ala Tyr
               20                  25                  30
   Arg Leu Glu Pro Gly Pro Ser Ser Tyr Asp Phe His Gln Leu Lys Lys
           35                  40                  45
   Phe Leu Lys Ile Ala Leu Glu Thr Pro Val Trp Ile Cys Pro Ile Asn
       50                  55                  60
   Tyr Ser Leu Leu Ala Ser Leu Leu Pro Lys Gly Tyr Pro Gly Arg Val
   65                  70                  75                  80
   Asn Glu Ile Leu His Ile Leu Ile Gln Thr Gln Ala
                   85                  90
<210> 106
   <211> 276
   <212> DNA
   <213> Human T-cell lymphotropic virus type 1
<400> 106
   atgggccaaa tcttttcccg tagcgctagc cctattccgc ggccgccccg ggggctggcc 60
   gctcatcact ggcttaactt cctccaggcg gcatatcgcc tagaacccgg tccctccagt 120
   tacgatttcc accagttaaa aaaatttctt aaaatagctt tagaaacacc ggtctggatc 180
   tgccccatta actactccct cctagccagc ctactcccaa aaggataccc cggccgggtg 240
   aatgaaattt tacacatact catccaaacc caagcc 276
<210> 107
   <211> 91
   <212> PRT
   <213> Human T-cell lymphotropic virus type 2
<400> 107
   Met Gly Gln Ile His Gly Leu Ser Pro Thr Pro Ile Pro Lys Ala Pro
   1 5 10 15
   Arg Gly Leu Ser Thr His His Trp Leu Asn Phe Leu Gln Ala Ala Tyr
   20 25 30
   Arg Leu Gln Pro Arg Pro Ser Asp Phe Asp Phe Gln Gln Leu Arg Arg
   35 40 45
   Phe Leu Lys Leu Ala Leu Lys Thr Pro Ile Trp Leu Asn Pro Ile Asp
   50 55 60
   Tyr Ser Leu Leu Ala Ser Leu Ile Pro Lys Gly Tyr Pro Gly Arg Val
   65 70 75 80
   Val Glu Ile Ile Asn Ile Leu Val Lys Asn Gln
   85 90
<210> 108
   <211> 273
   <212> RNA
   <213> Human T-cell lymphotropic virus type 2
<400> 108
   augggacaaa uccacgggcu uuccccaacu ccaauaccca aagcccccag ggggcuauca 60
   acccaccacu ggcuuaacuu ucuccaggcu gcuuaccgcu ugcagccuag gcccuccgau 120
   uucgacuucc agcagcuacg acgcuuucua aaacuagccc uuaaaacgcc cauuuggcua 180
   aauccuauug acuacucgcu uuuagcuagc cuuaucccca agggauaucc aggaagggug 240
   guagagauua uaaauauccu ugucaaaaau caa 273
<210> 109
   <211> 90
   <212> PRT
   <213> Human T-cell lymphotropic virus type 4
<400> 109
   Met Gly Gln Thr His Thr Ser Ser Pro Val Pro Lys Ala Pro Arg Gly
   1               5                   10                  15
   Leu Ser Thr His His Trp Leu Asn Phe Leu Gln Ala Ala Tyr Arg Leu
               20                  25                  30
   Gln Pro Gly Pro Ser Glu Phe Asp Phe His Gln Leu Arg Arg Phe Leu
           35                  40                  45
   Lys Leu Ala Leu Gln Thr Pro Val Trp Leu Asn Pro Ile Asp Tyr Ser
       50                  55                  60
   Leu Leu Ala Gly Leu Ile Pro Lys Gly Tyr Pro Gly Arg Val Thr Glu
   65                  70                  75                  80
   Ile Val Asn Ile Leu Leu Arg Ala His Pro
                   85                  90
<210> 110
   <211> 270
   <212> DNA
   <213> Human T-cell lymphotropic virus type 4
<400> 110
   atgggtcaga cccacacatc cagtcccgtc cctaaggccc ccagggggct ctccacccac      60
   cactggctta atttcctgca ggcggcttac cgcctgcaac ctggaccctc cgaattcgat     120
   tttcaccagt taagacgatt ccttaagcta gcgctccaaa ccccagtctg gttaaaccct     180
   atcgattact ccctcctagc cggcctaatc cccaaggggt accccggtcg ggtgaccgag     240
   atcgttaata tcctcctccg cgctcatcca                                       270
<210> 111
   <211> 144
   <212> PRT
   <213> Simian immunodeficiency virus
<400> 111
   Met Gly Val Arg Asn Ser Val Leu Ser Gly Lys Lys Ala Asp Glu Leu
   1               5                   10                  15
   Glu Lys Ile Arg Leu Arg Pro Asn Gly Lys Lys Lys Tyr Met Leu Lys
               20                  25                  30
   His Val Val Trp Ala Ala Asn Glu Leu Asp Arg Phe Gly Leu Ala Glu
           35                  40                  45
   Ser Leu Leu Glu Asn Lys Glu Gly Cys Gln Lys Ile Leu Ser Val Leu
       50                  55                  60
   Ala Pro Leu Val Pro Thr Gly Ser Glu Asn Leu Lys Ser Leu Tyr Asn
   65                  70                  75                  80
   Thr Val Cys Val Ile Trp Cys Ile His Ala Glu Glu Lys Val Lys His
                   85                  90                  95
   Thr Glu Glu Ala Lys Gln Ile Val Gln Arg His Leu Val Val Glu Thr
               100                 105                 110
   Gly Thr Thr Glu Thr Met Pro Lys Thr Ser Arg Pro Thr Ala Pro Ser
           115                 120                 125
   Ser Gly Arg Gly Gly Asn Tyr Pro Val Gln Gln Ile Gly Gly Asn Tyr
       130                 135                 140
<210> 112
   <211> 432
   <212> DNA
   <213> Simian immunodeficiency virus
<400> 112
   atgggcgtga gaaactccgt cttgtcaggg aagaaagcag atgaattaga aaaaattagg 60
   ctacgaccca acggaaagaa aaagtacatg ttgaagcatg tagtatgggc agcaaatgaa 120
   ttagatagat ttggattagc agaaagcctg ttggagaaca aagaaggatg tcaaaaaata 180
   ctttcggtct tagctccatt agtgccaaca ggctcagaaa atttaaaaag cctttataat 240
   actgtctgcg tcatctggtg cattcacgca gaagagaaag tgaaacacac tgaggaagca 300
   aaacagatag tgcagagaca cctagtggtg gaaacaggaa caacagaaac tatgccaaaa 360
   acaagtagac caacagcacc atctagcggc agaggaggaa attacccagt acaacaaata 420
   ggtggtaact at 432
<210> 113
   <211> 131
   <212> PRT
   <213> Murine leukemia virus
<400> 113
   Met Gly Gln Thr Val Thr Thr Pro Leu Ser Leu Thr Leu Gly His Trp
   1 5 10 15
   Lys Asp Val Glu Arg Ile Ala His Asn Gln Ser Val Asp Val Lys Lys
   20 25 30
   Arg Arg Trp Val Thr Phe Cys Ser Ala Glu Trp Pro Thr Phe Asn Val
   35 40 45
   Gly Trp Pro Arg Asp Gly Thr Phe Asn Arg Asp Leu Ile Thr Gln Val
   50 55 60
   Lys Ile Lys Val Phe Ser Pro Gly Pro His Gly His Pro Asp Gln Val
   65 70 75 80
   Pro Tyr Ile Val Thr Trp Glu Ala Leu Ala Phe Asp Pro Pro Pro Trp
   85 90 95
   Val Lys Pro Phe Val His Pro Lys Pro Pro Pro Pro Leu Pro Pro Ser
   100 105 110
   Ala Pro Ser Leu Pro Leu Glu Pro Pro Arg Ser Thr Pro Pro Arg Ser
   115 120 125
   Ser Leu Tyr
   130
<210> 114
   <211> 393
   <212> RNA
   <213> Murine leukemia virus
<400> 114
   augggccaga cuguuaccac ucccuuaagu uugaccuuag gucacuggaa agaugucgag 60
   cggaucgcuc acaaccaguc gguagauguc aagaagagac guuggguuac cuucugcucu 120
   gcagaauggc caaccuuuaa cgucggaugg ccgcgagacg gcaccuuuaa ccgagaccuc 180
   aucacccagg uuaagaucaa ggucuuuuca ccuggcccgc auggacaccc agaccagguc 240
   cccuacaucg ugaccuggga agccuuggcu uuugaccccc cucccugggu caagcccuuu 300
   guacacccua agccuccgcc uccucuuccu ccauccgccc cgucucuccc ccuugaaccu 360
   ccucguucga ccccgccucg auccucccuu uau 393
<210> 115
   <211> 108
   <212> PRT
   <213> Mason-Pfizer monkey virus
<400> 115
   Met Gly Gln Glu Leu Ser Gln His Glu Arg Tyr Val Glu Gln Leu Lys
   1               5                   10                  15
   Gln Ala Leu Lys Thr Arg Gly Val Lys Val Lys Tyr Ala Asp Leu Leu
               20                  25                  30
   Lys Phe Phe Asp Phe Val Lys Asp Thr Cys Pro Trp Phe Pro Gln Glu
           35                  40                  45
   Gly Thr Ile Asp Ile Lys Arg Trp Arg Arg Val Gly Asp Cys Phe Gln
       50                  55                  60
   Asp Tyr Tyr Asn Thr Phe Gly Pro Glu Lys Val Pro Val Thr Ala Phe
   65                  70                  75                  80
   Ser Tyr Trp Asn Leu Ile Lys Glu Leu Ile Asp Lys Lys Glu Val Asn
                   85                  90                  95
   Pro Gln Val Met Ala Ala Val Ala Gln Thr Thr Gly
               100                 105
<210> 116
   <211> 325
   <212> DNA
   <213> Mason-Pfizer monkey virus
<400> 116
   atgggccagg aactgagcca gcacgagaga tacgtggagc agctgaagca ggccctgaaa      60
   accaggggcg tgaaggtgaa atacgccgac ctgctgaagt tcttcgactt cgtgaaggac     120
   acctgcccat ggtttccaca ggaaggcacc atcgacatca agcggtggcg gagagtgggc     180
   gactgcttcc aggactacta caacaccttc ggccccgaga aggtgcccgt gaccgccttc     240
   agctactgga acctgatcaa agagctgatc gacaagaaag aggtgaaccc ccaggtcatg     300
   gccgccgtgg cccagaccac cggtt                                           325
<210> 117
   <211> 155
   <212> PRT
   <213> Avian leukosis virus
<400> 117
   Met Glu Ala Val Ile Lys Val Ile Ser Ser Ala Cys Lys Thr Tyr Cys
   1               5                   10                  15
   Gly Lys Ile Ser Pro Ser Lys Lys Glu Ile Gly Ala Met Leu Ser Leu
               20                  25                  30
   Leu Gln Lys Glu Gly Leu Leu Met Ser Pro Ser Asp Leu Tyr Ser Pro
           35                  40                  45
   Gly Ser Trp Asp Pro Ile Thr Ala Ala Leu Ser Gln Arg Ala Met Val
       50                  55                  60
   Leu Gly Lys Ser Gly Glu Leu Lys Thr Trp Gly Leu Val Leu Gly Ala
   65                  70                  75                  80
   Leu Lys Ala Ala Arg Glu Glu Gln Val Thr Ser Glu Gln Ala Lys Phe
                   85                  90                  95
   Trp Leu Gly Leu Gly Gly Gly Arg Val Ser Pro Pro Gly Pro Glu Cys
               100                 105                 110
   Ile Glu Lys Pro Ala Thr Glu Arg Arg Ile Asp Lys Gly Glu Glu Val
           115                 120                 125
   Gly Glu Thr Thr Ala Gln Arg Asp Ala Lys Met Ala Pro Glu Lys Met
       130                 135                 140
   Ala Thr Pro Lys Thr Val Gly Thr Ser Cys Tyr
   145                 150                 155
<210> 118
   <211> 465
   <212> RNA
   <213> Avian leukosis virus
<400> 118
   auggaagccg ucauuaaggu gauuucgucc gcguguaaaa ccuauugcgg gaaaaucucu      60
   ccuucuaaga aggaaauagg ggccauguug ucccuguuac aaaaggaagg guugcuuaug     120
   ucucccucag auuuauauuc cccggggucc ugggauccca ucacugcggc gcucucccag      180
   cgggcaaugg uacuugggaa aucgggagag uuaaaaaccu ggggauuggu uuugggggca     240
   uugaaggcgg cucgagagga acagguuaca ucugagcaag caaaguuuug guugggauua     300
   gggggaggga gggucucucc cccagguccg gagugcaucg agaaaccagc aacggagcgg     360
   cgaaucgaca aaggggagga agugggagaa acaacugcgc agcgagaugc gaagauggcg     420
   ccggagaaaa uggccacacc uaaaaccguu ggcacauccu gcuau                     465
<210> 119
   <211> 92
   <212> PRT
   <213> Mouse mammary tumor virus
<400> 119
   Met Gly Val Ser Gly Ser Lys Gly Gln Lys Leu Phe Val Ser Val Leu
   1               5                   10                  15
   Gln Arg Leu Leu Ser Glu Arg Gly Leu His Val Lys Glu Ser Ser Ala
               20                  25                  30
   Ile Glu Phe Tyr Gln Phe Leu Ile Lys Val Ser Pro Trp Phe Pro Glu
           35                  40                  45
   Glu Gly Gly Leu Asn Leu Gln Asp Trp Lys Arg Val Gly Arg Glu Met
       50                  55                  60
   Lys Arg Tyr Ala Ala Glu His Gly Thr Asp Ser Ile Pro Lys Gln Ala
   65                  70                  75                  80
   Tyr Pro Ile Trp Leu Gln Leu Arg Glu Ile Leu Thr
                   85                  90
<210> 120
   <211> 276
   <212> DNA
   <213> Mouse mammary tumor virus
<400> 120
   atgggggtct cgggatcaaa agggcagaaa ctctttgttt ctgttctaca aagactcctc      60
   tcagagaggg gtcttcatgt gaaagagagc agtgcaatag agttttatca gttcctaata     120
   aaggtctctc cttggtttcc cgaagaagga ggattaaatt tacaagattg gaaaagggtg     180
   ggaagagaga tgaagaggta cgcagcagaa catgggacgg atagtatacc aaagcaggct     240
   taccccattt ggcttcagtt gagagagata ctgaca                               276
<210> 121
   <211> 90
   <212> PRT
   <213> Jaagsiekte sheep retrovirus
<400> 121
   Met Gly His Thr His Ser Arg Gln Leu Phe Val His Met Leu Ser Val
   1               5                   10                  15
   Met Leu Lys His Arg Gly Ile Thr Val Ser Lys Thr Lys Leu Ile Asn
               20                  25                  30
   Phe Leu Ser Phe Ile Glu Glu Val Cys Pro Trp Phe Pro Arg Glu Gly
           35                  40                  45
   Thr Val Asn Leu Glu Thr Trp Lys Lys Val Gly Glu Gln Ile Arg Thr
       50                  55                  60
   His Tyr Thr Leu His Gly Pro Glu Lys Val Pro Val Glu Thr Leu Ser
   65                  70                  75                  80
   Phe Trp Thr Leu Ile Arg Asp Cys Leu Asp
                   85                  90
<210> 122
   <211> 270
   <212> RNA
   <213> Jaagsiekte sheep retrovirus
<400> 122
   auaugggaca cacgcauagu cgucaguugu uugugcauau guuaucugua auguuaaaac      60
   auagggggau cacuguuucu aaaacuaaau uaauuaauuu ucuuucauuu auugaggaag     120
   uuugcccuug guuuccuagg gaaggcacag ugaauuugga gacauggaag aaggugggag     180
   agcaaauucg gacgcauuau accuuacacg gcccugaaaa ggucccuguc gagacauuau     240
   cuuuuuggac auugauucgu gacugccugg                                      270
<210> 123
   <211> 125
   <212> PRT
   <213> Gibbon leukemia virus
<400> 123
   Met Gly Gln Asp Asn Ser Thr Pro Ile Ser Leu Thr Leu Asn His Trp
   1               5                   10                  15
   Arg Asp Val Arg Thr Arg Ala His Asn Leu Ser Val Glu Ile Lys Lys
               20                  25                  30
   Gly Lys Trp Gln Thr Phe Cys Ser Ser Glu Trp Pro Thr Phe Gly Val
           35                  40                  45
   Gly Trp Pro Pro Glu Gly Thr Phe Asn Leu Ser Val Ile Phe Ala Val
       50                  55                  60
   Lys Lys Ile Val Phe Gln Glu Asn Gly Gly His Pro Asp Gln Val Pro
   65                  70                  75                  80
   Tyr Ile Val Val Trp Gln Asp Leu Ala Gln Asn Pro Pro Pro Trp Val
                   85                  90                  95
   Pro Ala Ser Ala Lys Val Ala Val Val Ser Asp Thr Arg Arg Pro Val
               100                 105                 110
   Ala Gly Arg Pro Ser Ala Pro Pro Arg Pro Pro Ile Tyr
           115                 120                 125
<210> 124
   <211> 375
   <212> RNA
   <213> Gibbon leukemia virus
<400> 124
   augggacaag auaauucuac cccuaucucc cucacucuaa aucacuggag agaugugaga      60
   acaagggcuc acaaucuauc cguggaaauc aaaaagggaa aauggcagac uuucuguucc     120
   uccgaguggc ccacauucgg cguggggugg ccaccggagg gaacuuuuaa ucucucuguc     180
   auuuuugcag uuaaaaagau ugucuuucag gagaacgggg gacauccgga ccaaguucca     240
   uauaucgugg uauggcagga ccucgcccag aaucccccac caugggugcc agccuccgcc     300
   aaggucgcug uugucucuga uacccgaaga ccaguugcgg ggaggccauc agcuccuccc     360
   cgacccccca ucuac                                                       375
<210> 125
   <211> 10
   <212> PRT
   <213> Homo sapiens
<400> 125
   Met Gly Ser Asn Lys Ser Lys Pro Lys Asp
   1 5 10
<210> 126
   <211> 10
   <212> PRT
   <213> Homo sapiens
<400> 126
   Met Gly Cys Val Gln Cys Lys Asp Lys Glu
   1 5 10
<210> 127
   <211> 10
   <212> PRT
   <213> Homo sapiens
<400> 127
   Met Gly Cys Gly Cys Ser Ser His Pro Glu
   1 5 10
<210> 128
   <211> 263
   <212> PRT
   <213> Artificial Sequence
<220>
   <223> fusion protein of HIV-1 derived membrane targeting domain and a rapamycin-binding heterodimerizable protein domain
<400> 128
   Met Gly Ala Arg Ala Ser Val Leu Ser Gly Gly Glu Leu Asp Arg Trp
   1               5                   10                  15
   Glu Lys Ile Arg Leu Arg Pro Gly Gly Lys Lys Lys Tyr Lys Leu Lys
               20                  25                  30
   His Ile Val Trp Ala Ser Arg Glu Leu Glu Arg Phe Ala Val Asn Pro
           35                  40                  45
   Gly Leu Leu Glu Thr Ser Glu Gly Cys Arg Gln Ile Leu Gly Gln Leu
       50                  55                  60
   Gln Pro Ser Leu Gln Thr Gly Ser Glu Glu Leu Arg Ser Leu Tyr Asn
   65                  70                  75                  80
   Thr Val Ala Thr Leu Tyr Cys Val His Gln Arg Ile Glu Ile Lys Asp
                   85                  90                  95
   Thr Lys Glu Ala Leu Asp Lys Ile Glu Glu Glu Gln Asn Lys Ser Lys
               100                 105                 110
   Lys Lys Ala Gln Gln Ala Ala Ala Asp Thr Gly His Ser Ser Gln Val
           115                 120                 125
   Ser Gln Asn Tyr Pro Ile Val Gln Ser Gly Gly Gly Gly Ser Gly Gly
       130                 135                 140
   Gly Gly Ser Gly Gly Gly Ala Ser Ser Arg Gly Val Gln Val Glu Thr
   145                 150                 155                 160
   Ile Ser Pro Gly Asp Gly Arg Thr Phe Pro Lys Arg Gly Gln Thr Cys
                   165                 170                 175
   Val Val His Tyr Thr Gly Met Leu Glu Asp Gly Lys Lys Phe Asp Ser
               180                 185                 190
   Ser Arg Asp Arg Asn Lys Pro Phe Lys Phe Met Leu Gly Lys Gln Glu
           195                 200                 205
   Val Ile Arg Gly Trp Glu Glu Gly Val Ala Gln Met Ser Val Gly Gln
       210                 215                 220
   Arg Ala Lys Leu Thr Ile Ser Pro Asp Tyr Ala Tyr Gly Ala Thr Gly
   225                 230                 235                 240
   His Pro Gly Ile Ile Pro Pro His Ala Thr Leu Val Phe Asp Val Glu
                   245                 250                 255
   Leu Leu Lys Leu Glu Thr Ser
               260
<210> 129
   <211> 792
   <212> DNA
   <213> Artificial Sequence
<220>
   <223> polynucleotide encoding for a fusion protein of HIV-1 derived membrane targeting domain and a rapamycin-binding heterodimerizable protein domain
<400> 129
   atgggcgcca gagccagcgt gctgagcggc ggcgagctgg acaggtggga gaagatcagg      60
   ctgaggcccg gcggcaagaa gaagtataag ctgaagcaca tcgtgtgggc cagcagggag     120
   ctggagaggt tcgccgtgaa ccccggcctg ctggagacca gcgagggctg caggcagatc     180
   ctgggccagc tgcagcccag cctgcagacc ggcagcgagg agctgaggag cctgtacaac     240
   accgtggcca ccctgtactg cgtgcaccag aggatcgaga tcaaggacac caaggaggcc     300
   ctggacaaga tcgaggagga gcagaacaag tccaagaaga aggcccagca ggccgccgcc     360
   gacaccggcc acagcagcca ggtgagccag aactacccca tcgtgcagtc cggaggcggc     420
   ggcagcggcg ggggcgggag cggcggcggg gctagctcta gaggagtgca ggtggaaacc      480
   atctccccag gagacgggcg caccttcccc aagcgcggcc agacctgcgt ggtgcactac     540
   accgggatgc ttgaagatgg aaagaaattt gattcctccc gggacagaaa caagcccttt     600
   aagtttatgc taggcaagca ggaggtgatc cgaggctggg aagaaggggt tgcccagatg     660
   agtgtgggtc agagagccaa actgactata tctccagatt atgcctatgg tgccactggg     720
   cacccaggca tcatcccacc acatgccact ctcgtcttcg atgtggagct tctaaaactg     780
   gaaactagtt ag                                                         792
<210> 130
   <211> 249
   <212> PRT
   <213> Artificial Sequence
<220>
   <223> fusion protein of a HIV-1 derived membrane targeting domain and a rapamycin-binding heterodimerizable protein domain
<400> 130
   Met Gly Ala Arg Ala Ser Val Leu Ser Gly Gly Glu Leu Asp Arg Trp
   1               5                   10                  15
   Glu Lys Ile Arg Leu Arg Pro Gly Gly Lys Lys Lys Tyr Lys Leu Lys
               20                  25                  30
   His Ile Val Trp Ala Ser Arg Glu Leu Glu Arg Phe Ala Val Asn Pro
           35                  40                  45
   Gly Leu Leu Glu Thr Ser Glu Gly Cys Arg Gln Ile Leu Gly Gln Leu
       50                  55                  60
   Gln Pro Ser Leu Gln Thr Gly Ser Glu Glu Leu Arg Ser Leu Tyr Asn
   65                  70                  75                  80
   Thr Val Ala Thr Leu Tyr Cys Val His Gln Arg Ile Glu Ile Lys Asp
                   85                  90                  95
   Thr Lys Glu Ala Leu Asp Lys Ile Glu Glu Glu Gln Asn Lys Ser Lys
               100                 105                 110
   Lys Lys Ala Gln Gln Ala Ala Ala Asp Thr Gly His Ser Ser Gln Val
           115                 120                 125
   Ser Gln Asn Tyr Pro Ile Val Gln Ser Gly Gly Gly Gly Ser Gly Gly
       130                 135                 140
   Gly Gly Ser Gly Gly Gly Ala Ser Ser Arg Ile Leu Trp His Glu Met
   145                 150                 155                 160
   Trp His Glu Gly Leu Glu Glu Ala Ser Arg Leu Tyr Phe Gly Glu Arg
                   165                 170                 175
   Asn Val Lys Gly Met Phe Glu Val Leu Glu Pro Leu His Ala Met Met
               180                 185                 190
   Glu Arg Gly Pro Gln Thr Leu Lys Glu Thr Ser Phe Asn Gln Ala Tyr
           195                 200                 205
   Gly Arg Asp Leu Met Glu Ala Gln Glu Trp Cys Arg Lys Tyr Met Lys
       210                 215                 220
   Ser Gly Asn Val Lys Asp Leu Leu Gln Ala Trp Asp Leu Tyr Tyr His
   225                 230                 235                 240
   Val Phe Arg Arg Ile Ser Lys Thr Ser
                   245
<210> 131
   <211> 750
   <212> DNA
   <213> Artificial Sequence
<220>
   <223> polynucleotide encoding for a fusion protein of a HIV-1 derived membrane targeting domain and a rapamycin-binding heterodimerizable protein domain
<400> 131
   atgggcgcca gagccagcgt gctgagcggc ggcgagctgg acaggtggga gaagatcagg      60
   ctgaggcccg gcggcaagaa gaagtataag ctgaagcaca tcgtgtgggc cagcagggag     120
   ctggagaggt tcgccgtgaa ccccggcctg ctggagacca gcgagggctg caggcagatc     180
   ctgggccagc tgcagcccag cctgcagacc ggcagcgagg agctgaggag cctgtacaac     240
   accgtggcca ccctgtactg cgtgcaccag aggatcgaga tcaaggacac caaggaggcc     300
   ctggacaaga tcgaggagga gcagaacaag tccaagaaga aggcccagca ggccgccgcc     360
   gacaccggcc acagcagcca ggtgagccag aactacccca tcgtgcagtc cggaggcggc     420
   ggcagcggcg ggggcgggag cggcggcggg gctagctcta gaatcctctg gcatgagatg      480
   tggcatgaag gcctggaaga ggcatctcgt ttgtactttg gggaaaggaa cgtgaaaggc     540
   atgtttgagg tgctggagcc cttgcatgct atgatggaac ggggccccca gactctgaag     600
   gaaacatcct ttaatcaggc ctatggtcga gatttaatgg aggcccaaga gtggtgcagg     660
   aagtacatga aatcagggaa tgtcaaggac ctcctccaag cctgggacct ctattatcat     720
   gtgttccgac gaatctcaaa gactagttag                                      750
<210> 132
   <211> 147
   <212> PRT
   <213> Artificial Sequence
<220>
   <223> fusion protein of an Avian sarcoma virus derived membrane targeting domain and a rapamycin-binding heterodimerizable protein domain
<400> 132
   Met Gly Ser Ser Lys Ser Lys Pro Lys Asp Thr Gly Tyr Pro Tyr Asp
   1 5 10 15
   Val Pro Asp Tyr Ala Gly Gly Gly Gly Ser Gly Gly Gly Gly Ser Gly
   20 25 30
   Gly Gly Ala Ser Ser Arg Gly Val Gln Val Glu Thr Ile Ser Pro Gly
   35 40 45
   Asp Gly Arg Thr Phe Pro Lys Arg Gly Gln Thr Cys Val Val His Tyr
   50 55 60
   Thr Gly Met Leu Glu Asp Gly Lys Lys Phe Asp Ser Ser Arg Asp Arg
   65 70 75 80
   Asn Lys Pro Phe Lys Phe Met Leu Gly Lys Gln Glu Val Ile Arg Gly
   85 90 95
   Trp Glu Glu Gly Val Ala Gln Met Ser Val Gly Gln Arg Ala Lys Leu
   100 105 110
   Thr Ile Ser Pro Asp Tyr Ala Tyr Gly Ala Thr Gly His Pro Gly Ile
   115 120 125
   Ile Pro Pro His Ala Thr Leu Val Phe Asp Val Glu Leu Leu Lys Leu
   130 135 140
   Glu Thr Ser
   145
<210> 133
   <211> 444
   <212> DNA
   <213> Artificial Sequence
<220>
   <223> polynucleotide encoding for a fusion protein of an Avian sarcoma virus derived membrane targeting domain and a rapamycin-binding heterodimerizable protein domain
<400> 133
   atgggcagca gcaagagcaa gcccaaggac accggttacc cctacgacgt gcctgactac 60
   gccggaggcg gcggcagcgg cgggggcggg agcggcggcg gggctagctc tagaggagtg 120
   caggtggaaa ccatctcccc aggagacggg cgcaccttcc ccaagcgcgg ccagacctgc 180
   gtggtgcact acaccgggat gcttgaagat ggaaagaaat ttgattcctc ccgggacaga 240
   aacaagccct ttaagtttat gctaggcaag caggaggtga tccgaggctg ggaagaaggg      300
   gttgcccaga tgagtgtggg tcagagagcc aaactgacta tatctccaga ttatgcctat      360
   ggtgccactg ggcacccagg catcatccca ccacatgcca ctctcgtctt cgatgtggag      420
   cttctaaaac tggaaactag ttag                                             444
<210> 134
   <211> 133
   <212> PRT
   <213> Artificial Sequence
<220>
   <223> fusion protein of an Avian sarcoma virus derived membrane targeting domain and a rapamycin-binding heterodimerizable protein domain
<400> 134
   Met Gly Ser Ser Lys Ser Lys Pro Lys Asp Thr Gly Tyr Pro Tyr Asp
   1               5                   10                  15
   Val Pro Asp Tyr Ala Gly Gly Gly Gly Ser Gly Gly Gly Gly Ser Gly
               20                  25                  30
   Gly Gly Ala Ser Ser Arg Ile Leu Trp His Glu Met Trp His Glu Gly
           35                  40                  45
   Leu Glu Glu Ala Ser Arg Leu Tyr Phe Gly Glu Arg Asn Val Lys Gly
       50                  55                  60
   Met Phe Glu Val Leu Glu Pro Leu His Ala Met Met Glu Arg Gly Pro
   65                  70                  75                  80
   Gln Thr Leu Lys Glu Thr Ser Phe Asn Gln Ala Tyr Gly Arg Asp Leu
                   85                  90                  95
   Met Glu Ala Gln Glu Trp Cys Arg Lys Tyr Met Lys Ser Gly Asn Val
               100                 105                 110
   Lys Asp Leu Leu Gln Ala Trp Asp Leu Tyr Tyr His Val Phe Arg Arg
           115                 120                 125
   Ile Ser Lys Thr Ser
       130
<210> 135
   <211> 402
   <212> DNA
   <213> Artificial Sequence
<220>
   <223> polynucleotide encoding for a fusion protein of an Avian sarcoma virus derived membrane targeting domain and a rapamycin-binding heterodimerizable protein domain
<400> 135
   atgggcagca gcaagagcaa gcccaaggac accggttacc cctacgacgt gcctgactac      60
   gccggaggcg gcggcagcgg cgggggcggg agcggcggcg gggctagctc tagaatcctc     120
   tggcatgaga tgtggcatga aggcctggaa gaggcatctc gtttgtactt tggggaaagg     180
   aacgtgaaag gcatgtttga ggtgctggag cccttgcatg ctatgatgga acggggcccc     240
   cagactctga aggaaacatc ctttaatcag gcctatggtc gagatttaat ggaggcccaa     300
   gagtggtgca ggaagtacat gaaatcaggg aatgtcaagg acctcctcca agcctgggac     360
   ctctattatc atgtgttccg acgaatctca aagactagtt ag                        402
<210> 136
   <211> 491
   <212> PRT
   <213> Lassa virus
<400> 136
   Met Gly Gln Ile Val Thr Phe Phe Gln Glu Val Pro His Val Ile Glu
   1               5                   10                  15
   Glu Val Met Asn Ile Val Leu Ile Ala Leu Ser Val Leu Ala Val Leu
               20                  25                  30
   Lys Gly Leu Tyr Asn Phe Ala Thr Cys Gly Leu Val Gly Leu Val Thr
           35                  40                  45
   Phe Leu Leu Leu Cys Gly Arg Ser Cys Thr Thr Ser Leu Tyr Lys Gly
       50                  55                  60
   Val Tyr Glu Leu Gln Thr Leu Glu Leu Asn Met Glu Thr Leu Asn Met
   65                  70                  75                  80
   Thr Met Pro Leu Ser Cys Thr Lys Asn Asn Ser His His Tyr Ile Met
                   85                  90                  95
   Val Gly Asn Glu Thr Gly Leu Glu Leu Thr Leu Thr Asn Thr Ser Ile
               100                 105                 110
   Ile Asn His Lys Phe Cys Asn Leu Ser Asp Ala His Lys Lys Asn Leu
           115                 120                 125
   Tyr Asp His Ala Leu Met Ser Ile Ile Ser Thr Phe His Leu Ser Ile
       130                 135                 140
   Pro Asn Phe Asn Gln Tyr Glu Ala Met Ser Cys Asp Phe Asn Gly Gly
   145                 150                 155                 160
   Lys Ile Ser Val Gln Tyr Asn Leu Ser His Ser Tyr Ala Gly Asp Ala
                   165                 170                 175
   Ala Asn His Cys Gly Thr Val Ala Asn Gly Val Leu Gln Thr Phe Met
               180                 185                 190
   Arg Met Ala Trp Gly Gly Ser Tyr Ile Ala Leu Asp Ser Gly Arg Gly
           195                 200                 205
   Asn Trp Asp Cys Ile Met Thr Ser Tyr Gln Tyr Leu Ile Ile Gln Asn
       210                 215                 220
   Thr Thr Trp Glu Asp His Cys Gln Phe Ser Arg Pro Ser Pro Ile Gly
   225                 230                 235                 240
   Tyr Leu Gly Leu Leu Ser Gln Arg Thr Arg Asp Ile Tyr Ile Ser Arg
                   245                 250                 255
   Arg Leu Leu Gly Thr Phe Thr Trp Thr Leu Ser Asp Ser Glu Gly Lys
               260                 265                 270
   Asp Thr Pro Gly Gly Tyr Cys Leu Thr Arg Trp Met Leu Ile Glu Ala
           275                 280                 285
   Glu Leu Lys Cys Phe Gly Asn Thr Ala Val Ala Lys Cys Asn Glu Lys
       290                 295                 300
   His Asp Glu Glu Phe Cys Asp Met Leu Arg Leu Phe Asp Phe Asn Lys
   305                 310                 315                 320
   Gln Ala Ile Gln Arg Leu Lys Ala Glu Ala Gln Met Ser Ile Gln Leu
                   325                 330                 335
   Ile Asn Lys Ala Val Asn Ala Leu Ile Asn Asp Gln Leu Ile Met Lys
               340                 345                 350
   Asn His Leu Arg Asp Ile Met Gly Ile Pro Tyr Cys Asn Tyr Ser Lys
           355                 360                 365
   Tyr Trp Tyr Leu Asn His Thr Thr Thr Gly Arg Thr Ser Leu Pro Lys
       370                 375                 380
   Cys Trp Leu Val Ser Asn Gly Ser Tyr Leu Asn Glu Thr His Phe Ser
   385                 390                 395                 400
   Asp Asp Ile Glu Gln Gln Ala Asp Asn Met Ile Thr Glu Met Leu Gln
                   405                 410                 415
   Lys Glu Tyr Met Glu Arg Gln Gly Lys Thr Pro Leu Gly Leu Val Asp
   420 425 430
   Leu Phe Val Phe Ser Thr Ser Phe Tyr Leu Ile Ser Ile Phe Leu His
   435 440 445
   Leu Val Lys Ile Pro Thr His Arg His Ile Val Gly Lys Ser Cys Pro
   450 455 460
   Lys Pro His Arg Leu Asn His Met Gly Ile Cys Ser Cys Gly Leu Tyr
   465 470 475 480
   Lys Gln Pro Gly Val Pro Val Lys Trp Lys Arg
   485 490
<210> 137
   <211> 1476
   <212> DNA
   <213> Lassa virus
<400> 137
   atgggacaaa tagtgacatt cttccaggaa gtgcctcatg taatagaaga ggtgatgaac      60
   attgttctca ttgcactgtc tgtactagca gtgctgaaag gtctgtacaa ttttgcaacg     120
   tgtggccttg ttggtttggt cactttcctc ctgttgtgtg gtaggtcttg cacaaccagt     180
   ctttataaag gggtttatga gcttcagact ctggaactaa acatggagac actcaatatg     240
   accatgcctc tctcctgcac aaagaacaac agtcatcatt atataatggt gggcaatgag     300
   acaggactag aactgacctt gaccaacacg agcattatta atcacaaatt ttgcaatctg     360
   tctgatgccc acaaaaagaa cctctatgac cacgctctta tgagcataat ctcaactttc     420
   cacttgtcca tccccaactt caatcagtat gaggcaatga gctgcgattt taatggggga      480
   aagattagtg tgcagtacaa cctgagtcac agctatgctg gggatgcagc caaccattgt     540
   ggtactgttg caaatggtgt gttacagact tttatgagga tggcttgggg tgggagctac     600
   attgctcttg actcaggccg tggcaactgg gactgtatta tgactagtta tcaatatctg     660
   ataatccaaa atacaacctg ggaagatcac tgccaattct cgagaccatc tcccatcggt     720
   tatctcgggc tcctctcaca aaggactaga gatatttata ttagtagaag attgctaggc     780
   acattcacat ggacactgtc agattctgaa ggtaaagaca caccaggggg atattgtctg     840
   accaggtgga tgctaattga ggctgaacta aaatgcttcg ggaacacagc tgtggcaaaa     900
   tgtaatgaga agcatgatga ggaattttgt gacatgctga ggctgtttga cttcaacaaa     960
   caagccattc aaaggttgaa agctgaagca caaatgagca ttcagttgat caacaaagca    1020
   gtaaatgctt tgataaatga ccaacttata atgaagaacc atctacggga catcatggga    1080
   attccatact gtaattacag caagtattgg tacctcaacc acacaactac tgggagaaca    1140
   tcactgccca aatgttggct tgtatcaaat ggttcatact tgaacgagac ccacttttct    1200
   gatgatattg aacaacaagc tgacaatatg atcactgaga tgttacagaa ggagtatatg    1260
   gagaggcagg ggaagacacc attgggtcta gttgacctct ttgtgttcag tacaagtttc    1320
   tatcttatta gcatcttcct tcacctagtc aaaataccaa ctcataggca tattgtaggc    1380
   aagtcgtgtc ccaaacctca cagattgaat catatgggca tttgttcctg tggactctac    1440
   aaacagcctg gtgtgcctgt gaaatggaag agatga                              1476
<210> 138
   <211> 602
   <212> PRT
   <213> Sendai virus
<400> 138
   Met Pro Ala Glu Asn Lys Lys Val Arg Phe Glu Asn Thr Thr Ser Asp
   1               5                   10                  15
   Lys Gly Lys Ile Pro Ser Lys Val Ile Lys Ser Tyr Tyr Gly Thr Met
               20                  25                  30
   Asp Ile Lys Lys Ile Asn Glu Gly Leu Leu Asp Ser Lys Ile Leu Ser
           35                  40                  45
   Ala Phe Asn Thr Val Ile Ala Leu Leu Gly Ser Ile Val Ile Ile Val
       50                  55                  60
   Met Asn Ile Met Ile Ile Gln Asn Tyr Thr Arg Ser Thr Asp Asn Gln
   65                  70                  75                  80
   Ala Val Ile Lys Asp Ala Leu Gln Gly Ile Gln Gln Gln Ile Lys Gly
                   85                  90                  95
   Leu Ala Asp Lys Ile Gly Thr Glu Ile Gly Pro Lys Val Ser Leu Ile
               100                 105                 110
   Asp Thr Ser Ser Thr Ile Thr Ile Pro Ala Asn Ile Gly Leu Leu Gly
           115                 120                 125
   Ser Lys Ile Ser Gln Ser Thr Ala Ser Ile Asn Glu Asn Val Asn Glu
       130                 135                 140
   Lys Cys Lys Phe Thr Leu Pro Pro Leu Lys Ile His Glu Cys Asn Ile
   145                 150                 155                 160
   Ser Cys Pro Asn Pro Leu Pro Phe Arg Glu Tyr Arg Pro Gln Thr Glu
                   165                 170                 175
   Gly Val Ser Asn Leu Val Gly Leu Pro Asn Asn Ile Cys Leu Gln Lys
               180                 185                 190
   Thr Ser Asn Gln Ile Leu Lys Pro Lys Leu Ile Ser Tyr Thr Leu Pro
           195                 200                 205
   Val Val Gly Gln Ser Gly Thr Cys Ile Thr Asp Pro Leu Leu Ala Met
       210                 215                 220
   Asp Glu Gly Tyr Phe Ala Tyr Ser His Leu Glu Arg Ile Gly Ser Cys
   225                 230                 235                 240
   Ser Arg Gly Val Ser Lys Gln Arg Ile Ile Gly Val Gly Glu Val Leu
                   245                 250                 255
   Asp Arg Gly Asp Glu Val Pro Ser Leu Phe Met Thr Asn Val Trp Thr
               260                 265                 270
   Pro Pro Asn Pro Asn Thr Val Tyr His Cys Ser Ala Val Tyr Asn Asn
           275                 280                 285
   Glu Phe Tyr Tyr Val Leu Cys Ala Val Ser Thr Val Gly Asp Pro Ile
       290                 295                 300
   Leu Asn Ser Thr Tyr Trp Ser Gly Ser Leu Met Met Thr Arg Leu Ala
   305                 310                 315                 320
   Val Lys Pro Lys Ser Asn Gly Gly Gly Tyr Asn Gln His Gln Leu Ala
                   325                 330                 335
   Leu Arg Ser Ile Glu Lys Gly Arg Tyr Asp Lys Val Met Pro Tyr Gly
               340                 345                 350
   Pro Ser Gly Ile Lys Gln Gly Asp Thr Leu Tyr Phe Pro Ala Val Gly
           355                 360                 365
   Phe Leu Val Arg Thr Glu Phe Lys Tyr Asn Asp Ser Asn Cys Pro Ile
       370                 375                 380
   Thr Lys Cys Gln Tyr Ser Lys Pro Glu Asn Cys Arg Leu Ser Met Gly
   385                 390                 395                 400
   Ile Arg Pro Asn Ser His Tyr Ile Leu Arg Ser Gly Leu Leu Lys Tyr
                   405                 410                 415
   Asn Leu Ser Asp Gly Glu Asn Pro Lys Val Val Phe Ile Glu Ile Ser
               420                 425                 430
   Asp Gln Arg Leu Ser Ile Gly Ser Pro Ser Lys Ile Tyr Asp Ser Leu
           435                 440                 445
   Gly Gln Pro Val Phe Tyr Gln Ala Ser Phe Ser Trp Asp Thr Met Ile
       450                 455                 460
   Lys Phe Gly Asp Val Leu Thr Val Asn Pro Leu Val Val Asn Trp Arg
   465                 470                 475                 480
   Asn Asn Thr Val Ile Ser Arg Pro Gly Gln Ser Gln Cys Pro Arg Phe
                   485                 490                 495
   Asn Thr Cys Pro Glu Ile Cys Trp Glu Gly Val Tyr Asn Asp Ala Phe
               500                 505                 510
   Leu Ile Asp Arg Ile Asn Trp Ile Ser Ala Gly Val Phe Leu Asp Ser
           515                 520                 525
   Asn Gln Thr Ala Glu Asn Pro Val Phe Thr Val Phe Lys Asp Asn Glu
       530                 535                 540
   Ile Leu Tyr Arg Ala Gln Leu Ala Ser Glu Asp Thr Asn Ala Gln Lys
   545                 550                 555                 560
   Thr Ile Thr Asn Cys Phe Leu Leu Lys Asn Lys Ile Trp Cys Ile Ser
                   565                 570                 575
   Leu Val Glu Ile Tyr Asp Thr Gly Asp Asn Val Ile Arg Pro Lys Leu
               580                 585                 590
   Phe Ala Val Lys Ile Pro Glu Gln Cys Thr
           595                 600
<210> 139
   <211> 1809
   <212> RNA
   <213> Sendai virus
<400> 139
   augccggcag aaaacaagaa aguuagauuc gaaaauacua cuucagacaa agggaaaauu       60
   ccuaguaaag uuauuaagag cuacuacgga accauggaca uuaagaaaau aaaugaagga      120
   uuauuggaca gcaaaauauu aagugcuuuc aacacaguaa uagcauugcu uggaucuauc      180
   gugaucauag ugaugaauau aaugaucauc caaaauuaca caagaucaac agacaaucag      240
   gccgugauca aagaugcguu gcaggguauc caacagcaga ucaaagggcu ugcugacaaa      300
   aucggcacag agauagggcc caaaguauca cugauugaca cauccaguac cauuacuauc      360
   ccagcuaaca uugggcuguu agguucaaag aucagccagu cgacugcaag uauaaaugag      420
   aaugugaaug aaaaaugcaa auucacacug ccucccuuga aaauccacga auguaacauu      480
   ucuuguccua acccacuccc uuuuagagag uauaggccac agacagaagg ggugagcaau      540
   cuaguaggau uaccuaauaa uauuugccug caaaagacau cuaaucagau auugaagcca      600
   aagcugauuu cauacacuuu acccguaguc ggucaaagug guaccuguau cacagaccca      660
   uugcuggcua uggacgaggg cuauuuugca uauagccacc uggaaagaau cggaucaugu      720
   ucaagagggg ucuccaaaca aagaauaaua ggaguuggag agguacuaga cagaggugau      780
   gaaguuccuu cuuuauuuau gaccaauguc uggaccccac caaauccaaa caccguuuac      840
   cacuguagug cuguauacaa caaugaauuc uauuauguac uuugugcagu gucaacuguu      900
   ggagacccua uucugaauag caccuacugg uccggaucuc uaaugaugac ccgucuagcu      960
   gugaaaccca agaguaaugg uggggguuac aaucaacauc aacuugcccu acgaaguauc     1020
   gagaaaggga gguaugauaa aguuaugccg uauggaccuu caggcaucaa acagggugac     1080
   acccuguauu uuccugcugu aggauuuuug gucaggacag aguuuaaaua caaugauuca      1140
   aauuguccca ucacgaagug ucaauacagu aaaccugaaa auugcaggcu aucuaugggg      1200
   auuagaccaa acagccauua uauccuucga ucuggacuau uaaaauacaa ucuaucagau     1260
   ggggagaacc ccaaaguugu auucauugaa auaucugauc aaagauuauc uauuggaucu     1320
   ccuagcaaaa ucuaugauuc uuugggucaa ccuguuuucu accaagcguc auuuucaugg     1380
   gauacuauga uuaaauuugg agauguucua acagucaacc cucugguugu caauuggcgu     1440
   aauaacacgg uaauaucaag acccgggcaa ucacaaugcc cuagauucaa uacaugucca     1500
   gagaucugcu gggaaggagu uuauaaugau gcauuccuaa uugacagaau caauuggaua     1560
   agcgcgggug uauuccuuga cagcaaucag accgcagaaa auccuguuuu uacuguauuc     1620
   aaagauaaug aaauacuuua uagggcacaa cuggcuucug aggacaccaa ugcacaaaaa     1680
   acaauaacua auuguuuucu cuugaagaau aagauuuggu gcauaucauu gguugagaua     1740
   uaugacacag gagacaaugu cauaagaccc aaacuauucg cgguuaagau accagagcaa     1800
   uguacauaa                                                             1809
<210> 140
   <211> 565
   <212> PRT
   <213> Sendai virus
<400> 140
   Met Ala Thr Tyr Ile Gln Arg Val Gln Cys Ile Ser Ala Leu Leu Ser
   1               5                   10                  15
   Val Val Leu Thr Thr Leu Val Ser Cys Gln Ile Pro Arg Asp Arg Leu
               20                  25                  30
   Ser Asn Ile Gly Val Ile Val Asp Glu Gly Lys Ser Leu Lys Ile Ala
           35                  40                  45
   Gly Ser His Glu Ser Arg Tyr Ile Val Leu Ser Leu Val Pro Gly Ile
       50                  55                  60
   Asp Leu Glu Asn Gly Cys Gly Thr Ala Gln Val Ile Gln Tyr Lys Ser
   65 70 75 80
   Leu Leu Asn Arg Leu Leu Ile Pro Leu Arg Asp Ala Leu Asp Leu Gln
   85 90 95
   Glu Ala Leu Ile Thr Val Thr Asn Asp Thr Met Thr Gly Ala Asp Val
   100 105 110
   Pro Gln Ser Arg Phe Phe Gly Ala Val Ile Gly Thr Ile Ala Leu Gly
   115 120 125
   Val Ala Thr Ser Ala Gln Ile Thr Ala Gly Ile Ala Leu Ala Glu Ala
   130 135 140
   Arg Glu Ala Lys Arg Asp Ile Ala Leu Ile Lys Glu Ser Met Thr Lys
   145 150 155 160
   Thr His Lys Ser Ile Glu Leu Leu Gln Asn Ala Val Gly Glu Gln Ile
   165 170 175
   Leu Ala Leu Lys Thr Leu Gln Asp Phe Val Asn Asp Glu Ile Lys Pro
   180 185 190
   Ala Ile Ser Glu Leu Gly Cys Glu Thr Ala Ala Leu Arg Leu Gly Ile
   195 200 205
   Lys Leu Thr Gln His Tyr Ser Glu Leu Leu Thr Ala Phe Gly Ser Asn
   210 215 220
   Phe Gly Thr Ile Gly Glu Lys Ser Leu Thr Leu Gln Ala Leu Ser Ser
   225 230 235 240
   Leu Tyr Ser Ala Asn Ile Thr Glu Ile Met Thr Thr Ile Arg Thr Gly
   245 250 255
   Gln Ser Asn Ile Tyr Asp Val Ile Tyr Thr Glu Gln Ile Lys Gly Thr
   260 265 270
   Val Ile Asp Val Asp Leu Glu Arg Tyr Met Val Thr Leu Ser Val Lys
   275 280 285
   Ile Pro Ile Leu Ser Glu Val Pro Gly Val Leu Ile His Lys Ala Ser
   290 295 300
   Ser Ile Ser Tyr Asn Ile Asp Gly Glu Glu Trp Tyr Val Thr Val Pro
   305 310 315 320
   Ser His Ile Leu Ser Arg Ala Ser Phe Leu Gly Gly Ala Asn Ile Ala
                   325                 330                 335
   Asp Cys Val Glu Ser Arg Leu Thr Tyr Ile Cys Pro Arg Asp Pro Ala
               340                 345                 350
   Gln Leu Ile Pro Asp Ser Gln Gln Lys Cys Ile Leu Gly Asp Thr Thr
           355                 360                 365
   Arg Cys Pro Val Thr Lys Val Val Asp Asn Ile Ile Pro Lys Phe Ala
       370                 375                 380
   Phe Val Asn Gly Gly Val Val Ala Asn Cys Ile Ala Ser Thr Cys Thr
   385                 390                 395                 400
   Cys Gly Thr Gly Arg Arg Pro Ile Ser Gln Asp Arg Ser Lys Gly Val
                   405                 410                 415
   Val Phe Leu Thr His Asp Asn Cys Gly Leu Ile Gly Val Asn Gly Ile
               420                 425                 430
   Glu Leu Tyr Ala Asn Arg Lys Gly His Asp Ala Thr Trp Gly Val Gln
           435                 440                 445
   Asn Leu Thr Val Gly Pro Ala Ile Ala Ile Arg Pro Val Asp Ile Ser
       450                 455                 460
   Leu Asn Leu Ala Ala Ala Thr Asp Phe Leu Gln Asp Ser Arg Ala Glu
   465                 470                 475                 480
   Leu Glu Lys Ala Arg Lys Ile Leu Ser Glu Val Gly Arg Trp Tyr Asn
                   485                 490                 495
   Ser Gly Ala Thr Leu Ile Thr Ile Ile Val Val Met Ile Val Val Leu
               500                 505                 510
   Val Val Ile Ile Val Ile Val Ile Val Leu Tyr Arg Leu Arg Arg Ser
           515                 520                 525
   Met Leu Met Ser Asn Pro Ala Gly Arg Ile Ser Arg Asp Thr Tyr Thr
       530                 535                 540
   Leu Glu Pro Lys Ile Arg His Met Tyr Thr Asn Gly Gly Phe Asp Ala
   545                 550                 555                 560
   Met Thr Glu Lys Arg
                   565
<210> 141
   <211> 1698
   <212> DNA
   <213> Sendai virus
<400> 141
   atggcgactt atatccagag ggtacagtgc atatctgcat tattgtcagt tgtgctcaca      60
   acactggtct cgtgtcaaat tcctagagat aggctctcta atataggggt catagtcgac     120
   gaagggaaat cactgaagat tgccgggtcc cacgaatcta ggtacatcgt attaagtctg     180
   gtcccaggga tagacctcga aaatgggtgc ggaacagctc aggttatcca gtataagagc     240
   ctactgaacc ggttgttaat cccattgaga gatgccttag atcttcagga ggctctgata     300
   actgtcacta atgacacgat gaccggtgct gatgtcccac agtcaagatt cttcggcgcc     360
   gtgattggga ccatagcact tggagtggca acatcagcac agataaccgc agggatcgcg     420
   ctagctgagg cgagggaggc caagagagac atagcactca taaaagaatc aatgacaaag     480
   acacacaaat ctatagaact gctgcaaaac gctgtggggg aacaaattct tgctctaaag     540
   acgctccagg atttcgtgaa tgatgagatc aaacccgcga taagcgaatt gggttgtgag     600
   actgccgctt taagactggg gataaaactg acacaacatt actccgagct gctgactgca     660
   tttggatcta actttggaac catcggagag aagagcctca cactgcaggc actgtcttca     720
   ctctactctg ctaacatcac tgagattatg accacgatca ggacagggca gtccaacatc     780
   tatgacgtca tttatacgga gcagatcaag ggaactgtga ttgatgtaga tctagagaga     840
   tacatggtta ccctgtctgt aaaaatcccc attctttctg aagtcccagg tgtgcttata     900
   cacaaagcat cgtctatttc ttacaatata gacggggagg agtggtatgt gactgtcccc     960
   agtcacatac tcagtcgtgc ctccttcctg gggggtgcaa acatagctga ttgtgtagag    1020
   tccagattga cctacatatg ccctagggat cctgcacaat tgatacctga cagccagcaa    1080
   aaatgtatct tgggggacac aacaaggtgt cctgtcacaa aggttgtgga taacatcatc    1140
   cccaaatttg cctttgtaaa tggaggtgtc gttgcgaact gcatagcatc cacatgtacc    1200
   tgtgggacag gccgaaggcc aatcagtcag gatcgctcta aaggtgtagt attcttaact    1260
   catgataact gtggactcat aggagtcaat gggatagaat tgtatgctaa taggaaaggg    1320
   catgatgcca cttggggggt ccagaatttg acagtcggtc ctgcaatcgc tatcagaccc    1380
   gtggacattt ctctcaacct tgccgctgct actgacttcc tgcaagactc tagagctgaa    1440
   cttgagaagg cacgaaaaat cctctctgaa gtaggtagat ggtacaattc aggggcgact    1500
   ttgattacga tcatagtagt catgattgta gtattggtgg tcattatagt gattgtcatt    1560
   gtgctctaca gactcaggag atcaatgcta atgagtaatc cagccggtcg gatatcaaga    1620
   gacacatata cattagagcc gaagataagg catatgtata ctaacggtgg gttcgatgct    1680
   atgactgaga aaagatga                                                  1698
<210> 142
   <211> 676
   <212> PRT
   <213> Ebola virus
<400> 142
   Met Gly Val Thr Gly Ile Leu Gln Leu Pro Arg Asp Arg Phe Lys Arg
   1               5                   10                  15
   Thr Ser Phe Phe Leu Trp Val Ile Ile Leu Phe Gln Arg Thr Phe Ser
               20                  25                  30
   Ile Pro Leu Gly Val Ile His Asn Ser Thr Leu Gln Val Ser Asp Val
           35                  40                  45
   Asp Lys Leu Val Cys Arg Asp Lys Leu Ser Ser Thr Asn Gln Leu Arg
       50                  55                  60
   Ser Val Gly Leu Asn Leu Glu Gly Asn Gly Val Ala Thr Asp Val Pro
   65                  70                  75                  80
   Ser Ala Thr Lys Arg Trp Gly Phe Arg Ser Gly Val Pro Pro Lys Val
                   85                  90                  95
   Val Asn Tyr Glu Ala Gly Glu Trp Ala Glu Asn Cys Tyr Asn Leu Glu
               100                 105                 110
   Ile Lys Lys Pro Asp Gly Ser Glu Cys Leu Pro Ala Ala Pro Asp Gly
           115                 120                 125
   Ile Arg Gly Phe Pro Arg Cys Arg Tyr Val His Lys Val Ser Gly Thr
       130                 135                 140
   Gly Pro Cys Ala Gly Asp Phe Ala Phe His Lys Glu Gly Ala Phe Phe
   145                 150                 155                 160
   Leu Tyr Asp Arg Leu Ala Ser Thr Val Ile Tyr Arg Gly Thr Thr Phe
                   165                 170                 175
   Ala Glu Gly Val Val Ala Phe Leu Ile Leu Pro Gln Ala Lys Lys Asp
               180                 185                 190
   Phe Phe Ser Ser His Pro Leu Arg Glu Pro Val Asn Ala Thr Glu Asp
           195                 200                 205
   Pro Ser Ser Gly Tyr Tyr Ser Thr Thr Ile Arg Tyr Gln Ala Thr Gly
       210                 215                 220
   Phe Gly Thr Asn Glu Thr Glu Tyr Leu Phe Glu Val Asp Asn Leu Thr
   225                 230                 235                 240
   Tyr Val Gln Leu Glu Ser Arg Phe Thr Pro Gln Phe Leu Leu Gln Leu
   245 250 255
   Asn Glu Thr Ile Tyr Thr Ser Gly Lys Arg Ser Asn Thr Thr Gly Lys
   260 265 270
   Leu Ile Trp Lys Val Asn Pro Glu Ile Asp Thr Thr Ile Gly Glu Trp
   275 280 285
   Ala Phe Trp Glu Thr Lys Lys Asn Leu Thr Arg Lys Ile Arg Ser Glu
   290 295 300
   Glu Leu Ser Phe Thr Val Val Ser Asn Gly Ala Lys Asn Ile Ser Gly
   305 310 315 320
   Gln Ser Pro Ala Arg Thr Ser Ser Asp Pro Gly Thr Asn Thr Thr Thr
   325 330 335
   Glu Asp His Lys Ile Met Ala Ser Glu Asn Ser Ser Ala Met Val Gln
   340 345 350
   Val His Ser Gln Gly Arg Glu Ala Ala Val Ser His Leu Thr Thr Leu
   355 360 365
   Ala Thr Ile Ser Thr Ser Pro Gln Ser Leu Thr Thr Lys Pro Gly Pro
   370 375 380
   Asp Asn Ser Thr His Asn Thr Pro Val Tyr Lys Leu Asp Ile Ser Glu
   385 390 395 400
   Ala Thr Gln Val Glu Gln His His Arg Arg Thr Asp Asn Asp Ser Thr
   405 410 415
   Ala Ser Asp Thr Pro Ser Ala Thr Thr Ala Ala Gly Pro Pro Lys Ala
   420 425 430
   Glu Asn Thr Asn Thr Ser Lys Ser Thr Asp Phe Leu Asp Pro Ala Thr
   435 440 445
   Thr Thr Ser Pro Gln Asn His Ser Glu Thr Ala Gly Asn Asn Asn Thr
   450 455 460
   His His Gln Asp Thr Gly Glu Glu Ser Ala Ser Ser Gly Lys Leu Gly
   465 470 475 480
   Leu Ile Thr Asn Thr Ile Ala Gly Val Ala Gly Leu Ile Thr Gly Gly
   485 490 495
   Arg Arg Thr Arg Arg Glu Ala Ile Val Asn Ala Gln Pro Lys Cys Asn
   500 505 510
   Pro Asn Leu His Tyr Trp Thr Thr Gln Asp Glu Gly Ala Ala Ile Gly
   515 520 525
   Leu Ala Trp Ile Pro Tyr Phe Gly Pro Ala Ala Glu Gly Ile Tyr Ile
   530 535 540
   Glu Gly Leu Met His Asn Gln Asp Gly Leu Ile Cys Gly Leu Arg Gln
   545 550 555 560
   Leu Ala Asn Glu Thr Thr Gln Ala Leu Gln Leu Phe Leu Arg Ala Thr
   565 570 575
   Thr Glu Leu Arg Thr Phe Ser Ile Leu Asn Arg Lys Ala Ile Asp Phe
   580 585 590
   Leu Leu Gln Arg Trp Gly Gly Thr Cys His Ile Leu Gly Pro Asp Cys
   595 600 605
   Cys Ile Glu Pro His Asp Trp Thr Lys Asn Ile Thr Asp Lys Ile Asp
   610 615 620
   Gln Ile Ile His Asp Phe Val Asp Lys Thr Leu Pro Asp Gln Gly Asp
   625 630 635 640
   Asn Asp Asn Trp Trp Thr Gly Trp Arg Gln Trp Ile Pro Ala Gly Ile
   645 650 655
   Gly Val Thr Gly Val Ile Ile Ala Val Ile Ala Leu Phe Cys Ile Cys
   660 665 670
   Lys Phe Val Phe
   675
<210> 143
   <211> 2031
   <212> DNA
   <213> Ebola virus
<400> 143
   atgggcgtta caggaatatt gcagttacct cgtgatcgat tcaagaggac atcattcttt      60
   ctttgggtaa ttatcctttt ccaaagaaca ttttccatcc cacttggagt catccacaat     120
   agcacattac aggttagtga tgtcgacaaa ctagtttgtc gtgacaaact gtcatccaca     180
   aatcaattga gatcagttgg actgaatctc gaagggaatg gagtggcaac tgacgtgcca     240
   tctgcaacta aaagatgggg cttcaggtcc ggtgtcccac caaaggtggt caattatgaa     300
   gctggtgaat gggctgaaaa ctgctacaat cttgaaatca aaaaacctga cgggagtgag 360
   tgtctaccag cagcgccaga cgggattcgg ggcttccccc ggtgccggta tgtgcacaaa 420
   gtatcaggaa cgggaccgtg tgccggagac tttgccttcc ataaagaggg tgctttcttc 480
   ctgtatgatc gacttgcttc cacagttatc taccgaggaa cgactttcgc tgaaggtgtc 540
   gttgcatttc tgatactgcc ccaagctaag aaggacttct tcagctcaca ccccttgaga 600
   gagccggtca atgcaacgga ggacccgtct agtggctact attctaccac aattagatat 660
   caggctaccg gttttggaac caatgagaca gagtacttgt tcgaggttga caatttgacc 720
   tacgtccaac ttgaatcaag attcacacca cagtttctgc tccagctgaa tgagacaata 780
   tatacaagtg ggaaaaggag caataccacg ggaaaactaa tttggaaggt caaccccgaa 840
   attgatacaa caatcgggga gtgggccttc tgggaaacta aaaaaaacct cactagaaaa 900
   attcgcagtg aagagttgtc tttcacagtt gtatcaaacg gagccaaaaa catcagtggt 960
   cagagtccgg cgcgaacttc ttccgaccca gggaccaaca caacaactga agaccacaaa 1020
   atcatggctt cagaaaattc ctctgcaatg gttcaagtgc acagtcaagg aagggaagct 1080
   gcagtgtcgc atctaacaac ccttgccaca atctccacga gtccccaatc cctcacaacc 1140
   aaaccaggtc cggacaacag cacccataat acacccgtgt ataaacttga catctctgag 1200
   gcaactcaag ttgaacaaca tcaccgcaga acagacaacg acagcacagc ctccgacact 1260
   ccctctgcca cgaccgcagc cggaccccca aaagcagaga acaccaacac gagcaagagc 1320
   actgacttcc tggaccccgc caccacaaca agtccccaaa accacagcga gaccgctggc 1380
   aacaacaaca ctcatcacca agataccgga gaagagagtg ccagcagcgg gaagctaggc 1440
   ttaattacca atactattgc tggagtcgca ggactgatca caggcgggag aagaactcga 1500
   agagaagcaa ttgtcaatgc tcaacccaaa tgcaacccta atttacatta ctggactact 1560
   caggatgaag gtgctgcaat cggactggcc tggataccat atttcgggcc agcagccgag 1620
   ggaatttaca tagaggggct aatgcacaat caagatggtt taatctgtgg gttgagacag 1680
   ctggccaacg agacgactca agctcttcaa ctgttcctga gagccacaac tgagctacgc 1740
   accttttcaa tcctcaaccg taaggcaatt gatttcttgc tgcagcgatg gggcggcaca 1800
   tgccacattc tgggaccgga ctgctgtatc gaaccacatg attggaccaa gaacataaca 1860
   gacaaaattg atcagattat tcatgatttt gttgataaaa cccttccgga ccagggggac 1920
   aatgacaatt ggtggacagg atggagacaa tggataccgg caggtattgg agttacaggc 1980
   gttataattg cagttatcgc tttattctgt atatgcaaat ttgtctttta g 2031
<210> 144
   <211> 681
   <212> PRT
   <213> Marburg virus
<400> 144
   Met Lys Thr Thr Cys Phe Leu Ile Ser Leu Ile Leu Ile Gln Gly Thr
   1               5                   10                  15
   Lys Asn Leu Pro Ile Leu Glu Ile Ala Ser Asn Asn Gln Pro Gln Asn
               20                  25                  30
   Val Asp Ser Val Cys Ser Gly Thr Leu Gln Lys Thr Glu Asp Val His
           35                  40                  45
   Leu Met Gly Phe Thr Leu Ser Gly Gln Lys Val Ala Asp Ser Pro Leu
       50                  55                  60
   Glu Ala Ser Lys Arg Trp Ala Phe Arg Thr Gly Val Pro Pro Lys Asn
   65                  70                  75                  80
   Val Glu Tyr Thr Glu Gly Glu Glu Ala Lys Thr Cys Tyr Asn Ile Ser
                   85                  90                  95
   Val Thr Asp Pro Ser Gly Lys Ser Leu Leu Leu Asp Pro Pro Thr Asn
               100                 105                 110
   Ile Arg Asp Tyr Pro Lys Cys Lys Thr Ile His His Ile Gln Gly Gln
           115                 120                 125
   Asn Pro His Ala Gln Gly Ile Ala Leu His Leu Trp Gly Ala Phe Phe
       130                 135                 140
   Leu Tyr Asp Arg Ile Ala Ser Thr Thr Met Tyr Arg Gly Lys Val Phe
   145                 150                 155                 160
   Thr Glu Gly Asn Ile Ala Ala Met Ile Val Asn Lys Thr Val His Lys
                   165                 170                 175
   Met Ile Phe Ser Arg Gln Gly Gln Gly Tyr Arg His Met Asn Leu Thr
               180                 185                 190
   Ser Thr Asn Lys Tyr Trp Thr Ser Ser Asn Gly Thr Gln Thr Asn Asp
           195                 200                 205
   Thr Gly Cys Phe Gly Ala Leu Gln Glu Tyr Asn Ser Thr Lys Asn Gln
       210                 215                 220
   Thr Cys Ala Pro Ser Lys Ile Pro Pro Pro Leu Pro Thr Ala Arg Pro
   225                 230                 235                 240
   Glu Ile Lys Leu Thr Ser Thr Pro Thr Asp Ala Thr Lys Leu Asn Thr
                   245                 250                 255
   Thr Asp Pro Ser Ser Asp Asp Glu Asp Leu Ala Thr Ser Gly Ser Gly
   260 265 270
   Ser Gly Glu Arg Glu Pro His Thr Thr Ser Asp Ala Val Thr Lys Gln
   275 280 285
   Gly Leu Ser Ser Thr Met Pro Pro Thr Pro Ser Pro Gln Pro Ser Thr
   290 295 300
   Pro Gln Gln Gly Gly Asn Asn Thr Asn His Ser Gln Asp Ala Val Thr
   305 310 315 320
   Glu Leu Asp Lys Asn Asn Thr Thr Ala Gln Pro Ser Met Pro Pro His
   325 330 335
   Asn Thr Thr Thr Ile Ser Thr Asn Asn Thr Ser Lys His Asn Phe Ser
   340 345 350
   Thr Leu Ser Ala Pro Leu Gln Asn Thr Thr Asn Asp Asn Thr Gln Ser
   355 360 365
   Thr Ile Thr Glu Asn Glu Gln Thr Ser Ala Pro Ser Ile Thr Thr Leu
   370 375 380
   Pro Pro Thr Gly Asn Pro Thr Thr Ala Lys Ser Thr Ser Ser Lys Lys
   385 390 395 400
   Gly Pro Ala Thr Thr Ala Pro Asn Thr Thr Asn Glu His Phe Thr Ser
   405 410 415
   Pro Pro Pro Thr Pro Ser Ser Thr Ala Gln His Leu Val Tyr Phe Arg
   420 425 430
   Arg Lys Arg Ser Ile Leu Trp Arg Glu Gly Asp Met Phe Pro Phe Leu
   435 440 445
   Asp Gly Leu Ile Asn Ala Pro Ile Asp Phe Asp Pro Val Pro Asn Thr
   450 455 460
   Lys Thr Ile Phe Asp Glu Ser Ser Ser Ser Gly Ala Ser Ala Glu Glu
   465 470 475 480
   Asp Gln His Ala Ser Pro Asn Ile Ser Leu Thr Leu Ser Tyr Phe Pro
   485 490 495
   Asn Ile Asn Glu Asn Thr Ala Tyr Ser Gly Glu Asn Glu Asn Asp Cys
   500 505 510
   Asp Ala Glu Leu Arg Ile Trp Ser Val Gln Glu Asp Asp Leu Ala Ala
   515 520 525
   Gly Leu Ser Trp Ile Pro Phe Phe Gly Pro Gly Ile Glu Gly Leu Tyr
   530 535 540
   Thr Ala Val Leu Ile Lys Asn Gln Asn Asn Leu Val Cys Arg Leu Arg
   545 550 555 560
   Arg Leu Ala Asn Gln Thr Ala Lys Ser Leu Glu Leu Leu Leu Arg Val
   565 570 575
   Thr Thr Glu Glu Arg Thr Phe Ser Leu Ile Asn Arg His Ala Ile Asp
   580 585 590
   Phe Leu Leu Thr Arg Trp Gly Gly Thr Cys Lys Val Leu Gly Pro Asp
   595 600 605
   Cys Cys Ile Gly Ile Glu Asp Leu Ser Lys Asn Ile Ser Glu Gln Ile
   610 615 620
   Asp Gln Ile Lys Lys Asp Glu Gln Lys Glu Gly Thr Gly Trp Gly Leu
   625 630 635 640
   Gly Gly Lys Trp Trp Thr Ser Asp Trp Gly Val Leu Thr Asn Leu Gly
   645 650 655
   Ile Leu Leu Leu Leu Ser Ile Ala Val Leu Ile Ala Leu Ser Cys Ile
   660 665 670
   Cys Arg Ile Phe Thr Lys Tyr Ile Gly
   675 680
<210> 145
   <211> 2046
   <212> DNA
   <213> Marburg virus
<400> 145
   atgaagacca catgtttcct tatcagtctt atcttaattc aagggacaaa aaatctcccc 60
   attttagaga tagctagtaa taatcaaccc caaaatgtgg attcggtatg ctccggaact 120
   ctccagaaga cagaagacgt ccatctgatg ggattcacac tgagtgggca aaaagttgct 180
   gattcccctt tggaggcatc caagcgatgg gctttcagga caggtgtacc tcccaagaat 240
   gttgagtaca cagaggggga ggaagccaaa acatgctaca atataagtgt aacggatccc 300
   tctggaaaat ccttgctgtt agatcctcct accaacatcc gtgactatcc taaatgcaaa 360
   actatccatc atattcaagg tcaaaaccct catgcacagg ggatcgccct tcatttatgg 420
   ggagcatttt ttctgtatga tcgcattgcc tccacaacaa tgtaccgagg caaagtcttc 480
   actgaaggga acatagcagc tatgattgtc aataagacag tgcacaaaat gattttctcg 540
   cggcaaggac aagggtaccg tcatatgaat ctgacttcta ctaataaata ttggacaagt 600
   agtaacggaa cgcaaacgaa tgacactgga tgtttcggcg ctcttcaaga atacaattct 660
   acaaagaacc aaacatgtgc tccgtccaaa atacctccac cactgcccac agcccgtccg 720
   gagatcaaac tcacaagcac cccaactgat gccaccaaac tcaataccac ggacccaagc 780
   agtgatgatg aggacctcgc aacatccggc tcagggtccg gagaacgaga accccacaca 840
   acttctgatg cggtcaccaa gcaagggctt tcatcaacaa tgccacccac tccctcacca 900
   caaccaagca cgccacagca aggaggaaac aacacaaacc attcccaaga tgctgtgact 960
   gaactagaca aaaataacac aactgcacaa ccgtccatgc cccctcataa cactaccaca 1020
   atctctacta acaacacctc caaacacaac ttcagcactc tctctgcacc attacaaaac 1080
   accaccaatg acaacacaca gagcacaatc actgaaaatg agcaaaccag tgccccctcg 1140
   ataacaaccc tgcctccaac gggaaatccc accacagcaa agagcaccag cagcaaaaaa 1200
   ggccccgcca caacggcacc aaacacgaca aatgagcatt tcaccagtcc tccccccacc 1260
   cccagctcga ctgcacaaca tcttgtatat ttcagaagaa agcgaagtat cctctggagg 1320
   gaaggcgaca tgttcccttt tctggatggg ttaataaatg ctccaattga ttttgaccca 1380
   gttccaaata caaaaacaat ctttgatgaa tcctctagtt ctggtgcctc ggctgaggaa 1440
   gatcaacatg cctcccccaa tattagttta actttatctt attttcctaa tataaatgag 1500
   aacactgcct actctggaga aaatgagaat gattgtgatg cagagttaag aatttggagc 1560
   gttcaggagg atgacctggc cgcagggctc agttggatac cgttttttgg ccctggaatt 1620
   gaaggacttt acactgctgt tttaattaaa aatcaaaaca atttggtctg caggttgagg 1680
   cgtctagcca atcaaactgc caaatccttg gaactcttat tgagagtcac aactgaggaa 1740
   agaacattct ccttaatcaa tagacatgct attgactttc tactcacaag atggggagga 1800
   acatgcaaag tgcttggacc tgattgttgc atcgggatag aagacttgtc caaaaatatt 1860
   tcagagcaaa ttgaccaaat taaaaaggac gaacaaaaag aggggactgg ttggggtctg 1920
   ggtggtaaat ggtggacatc cgactggggt gttcttacta acttgggcat tttgctacta 1980
   ttatccatag ctgtcttgat tgctctatcc tgtatttgtc gtatctttac taaatatatc 2040
   ggataa 2046
<210> 146
   <211> 413
   <212> PRT
   <213> Homo sapiens
<400> 146
<210> 147
   <211> 532
   <212> PRT
   <213> Homo sapiens
<400> 147
   Met Ala Pro Ser Ser Pro Arg Pro Ala Leu Pro Ala Leu Leu Val Leu
   1               5                   10                  15
   Leu Gly Ala Leu Phe Pro Gly Pro Gly Asn Ala Gln Thr Ser Val Ser
               20                  25                  30
   Pro Ser Lys Val Ile Leu Pro Arg Gly Gly Ser Val Leu Val Thr Cys
           35                  40                  45
   Ser Thr Ser Cys Asp Gln Pro Lys Leu Leu Gly Ile Glu Thr Pro Leu
       50                  55                  60
   Pro Lys Lys Glu Leu Leu Leu Pro Gly Asn Asn Arg Lys Val Tyr Glu
   65                  70                  75                  80
   Leu Ser Asn Val Gln Glu Asp Ser Gln Pro Met Cys Tyr Ser Asn Cys
                   85                  90                  95
   Pro Asp Gly Gln Ser Thr Ala Lys Thr Phe Leu Thr Val Tyr Trp Thr
               100                 105                 110
   Pro Glu Arg Val Glu Leu Ala Pro Leu Pro Ser Trp Gln Pro Val Gly
           115                 120                 125
   Lys Asn Leu Thr Leu Arg Cys Gln Val Glu Gly Gly Ala Pro Arg Ala
       130                 135                 140
   Asn Leu Thr Val Val Leu Leu Arg Gly Glu Lys Glu Leu Lys Arg Glu
   145                 150                 155                 160
   Pro Ala Val Gly Glu Pro Ala Glu Val Thr Thr Thr Val Leu Val Arg
                   165                 170                 175
   Arg Asp His His Gly Ala Asn Phe Ser Cys Arg Thr Glu Leu Asp Leu
               180                 185                 190
   Arg Pro Gln Gly Leu Glu Leu Phe Glu Asn Thr Ser Ala Pro Tyr Gln
           195                 200                 205
   Leu Gln Thr Phe Val Leu Pro Ala Thr Pro Pro Gln Leu Val Ser Pro
       210                 215                 220
   Arg Val Leu Glu Val Asp Thr Gln Gly Thr Val Val Cys Ser Leu Asp
   225                 230                 235                 240
   Gly Leu Phe Pro Val Ser Glu Ala Gln Val His Leu Ala Leu Gly Asp
                   245                 250                 255
   Gln Arg Leu Asn Pro Thr Val Thr Tyr Gly Asn Asp Ser Phe Ser Ala
               260                 265                 270
   Lys Ala Ser Val Ser Val Thr Ala Glu Asp Glu Gly Thr Gln Arg Leu
           275                 280                 285
   Thr Cys Ala Val Ile Leu Gly Asn Gln Ser Gln Glu Thr Leu Gln Thr
       290                 295                 300
   Val Thr Ile Tyr Ser Phe Pro Ala Pro Asn Val Ile Leu Thr Lys Pro
   305                 310                 315                 320
   Glu Val Ser Glu Gly Thr Glu Val Thr Val Lys Cys Glu Ala His Pro
                   325                 330                 335
   Arg Ala Lys Val Thr Leu Asn Gly Val Pro Ala Gln Pro Leu Gly Pro
               340                 345                 350
   Arg Ala Gln Leu Leu Leu Lys Ala Thr Pro Glu Asp Asn Gly Arg Ser
   355 360 365
   Phe Ser Cys Ser Ala Thr Leu Glu Val Ala Gly Gln Leu Ile His Lys
   370 375 380
   Asn Gln Thr Arg Glu Leu Arg Val Leu Tyr Gly Pro Arg Leu Asp Glu
   385 390 395 400
   Arg Asp Cys Pro Gly Asn Trp Thr Trp Pro Glu Asn Ser Gln Gln Thr
   405 410 415
   Pro Met Cys Gln Ala Trp Gly Asn Pro Leu Pro Glu Leu Lys Cys Leu
   420 425 430
   Lys Asp Gly Thr Phe Pro Leu Pro Ile Gly Glu Ser Val Thr Val Thr
   435 440 445
   Arg Asp Leu Glu Gly Thr Tyr Leu Cys Arg Ala Arg Ser Thr Gln Gly
   450 455 460
   Glu Val Thr Arg Lys Val Thr Val Asn Val Leu Ser Pro Arg Tyr Glu
   465 470 475 480
   Ile Val Ile Ile Thr Val Val Ala Ala Ala Val Ile Met Gly Thr Ala
   485 490 495
   Gly Leu Ser Thr Tyr Leu Tyr Asn Arg Gln Arg Lys Ile Lys Lys Tyr
   500 505 510
   Arg Leu Gln Gln Ala Gln Lys Gly Thr Pro Met Lys Pro Asn Thr Gln
   515 520 525
   Ala Thr Pro Pro
   530
<210> 148
   <211> 220
   <212> PRT
   <213> Homo sapiens
<400> 148
   Met Leu Arg Leu Leu Leu Ala Leu Asn Leu Phe Pro Ser Ile Gln Val
   1               5                   10                  15
   Thr Gly Asn Lys Ile Leu Val Lys Gln Ser Pro Met Leu Val Ala Tyr
               20                  25                  30
   Asp Asn Ala Val Asn Leu Ser Cys Lys Tyr Ser Tyr Asn Leu Phe Ser
           35                  40                  45
   Arg Glu Phe Arg Ala Ser Leu His Lys Gly Leu Asp Ser Ala Val Glu
       50                  55                  60
   Val Cys Val Val Tyr Gly Asn Tyr Ser Gln Gln Leu Gln Val Tyr Ser
   65                  70                  75                  80
   Lys Thr Gly Phe Asn Cys Asp Gly Lys Leu Gly Asn Glu Ser Val Thr
                   85                  90                  95
   Phe Tyr Leu Gln Asn Leu Tyr Val Asn Gln Thr Asp Ile Tyr Phe Cys
               100                 105                 110
   Lys Ile Glu Val Met Tyr Pro Pro Pro Tyr Leu Asp Asn Glu Lys Ser
           115                 120                 125
   Asn Gly Thr Ile Ile His Val Lys Gly Lys His Leu Cys Pro Ser Pro
       130                 135                 140
   Leu Phe Pro Gly Pro Ser Lys Pro Phe Trp Val Leu Val Val Val Gly
   145                 150                 155                 160
   Gly Val Leu Ala Cys Tyr Ser Leu Leu Val Thr Val Ala Phe Ile Ile
                   165                 170                 175
   Phe Trp Val Arg Ser Lys Arg Ser Arg Leu Leu His Ser Asp Tyr Met
               180                 185                 190
   Asn Met Thr Pro Arg Arg Pro Gly Pro Thr Arg Lys His Tyr Gln Pro
           195                 200                 205
   Tyr Ala Pro Pro Arg Asp Phe Ala Ala Tyr Arg Ser
       210                 215                 220
<210> 149
   <211> 288
   <212> PRT
   <213> Homo sapiens
<400> 149
   Met Gly His Thr Arg Arg Gln Gly Thr Ser Pro Ser Lys Cys Pro Tyr
   1               5                   10                  15
   Leu Asn Phe Phe Gln Leu Leu Val Leu Ala Gly Leu Ser His Phe Cys
               20                  25                  30
   Ser Gly Val Ile His Val Thr Lys Glu Val Lys Glu Val Ala Thr Leu
           35                  40                  45
   Ser Cys Gly His Asn Val Ser Val Glu Glu Leu Ala Gln Thr Arg Ile
   50 55 60
   Tyr Trp Gln Lys Glu Lys Lys Met Val Leu Thr Met Met Ser Gly Asp
   65 70 75 80
   Met Asn Ile Trp Pro Glu Tyr Lys Asn Arg Thr Ile Phe Asp Ile Thr
   85 90 95
   Asn Asn Leu Ser Ile Val Ile Leu Ala Leu Arg Pro Ser Asp Glu Gly
   100 105 110
   Thr Tyr Glu Cys Val Val Leu Lys Tyr Glu Lys Asp Ala Phe Lys Arg
   115 120 125
   Glu His Leu Ala Glu Val Thr Leu Ser Val Lys Ala Asp Phe Pro Thr
   130 135 140
   Pro Ser Ile Ser Asp Phe Glu Ile Pro Thr Ser Asn Ile Arg Arg Ile
   145 150 155 160
   Ile Cys Ser Thr Ser Gly Gly Phe Pro Glu Pro His Leu Ser Trp Leu
   165 170 175
   Glu Asn Gly Glu Glu Leu Asn Ala Ile Asn Thr Thr Val Ser Gln Asp
   180 185 190
   Pro Glu Thr Glu Leu Tyr Ala Val Ser Ser Lys Leu Asp Phe Asn Met
   195 200 205
   Thr Thr Asn His Ser Phe Met Cys Leu Ile Lys Tyr Gly His Leu Arg
   210 215 220
   Val Asn Gln Thr Phe Asn Trp Asn Thr Thr Lys Gln Glu His Phe Pro
   225 230 235 240
   Asp Asn Leu Leu Pro Ser Trp Ala Ile Thr Leu Ile Ser Val Asn Gly
   245 250 255
   Ile Phe Val Ile Cys Cys Leu Thr Tyr Cys Phe Ala Pro Arg Cys Arg
   260 265 270
   Glu Arg Arg Arg Asn Glu Arg Leu Arg Arg Glu Ser Val Arg Pro Val
   275 280 285
<210> 150
   <211> 365
   <212> PRT
   <213> Homo sapiens
<400> 150
<210> 151
   <211> 10
   <212> PRT
   <213> Avian sarcoma virus
<400> 151
   Met Gly Ser Ser Lys Ser Lys Pro Lys Asp
   1 5 10
<210> 152
   <211> 647
   <212> PRT
   <213> Simian foamy virus
<400> 152
   Met Ala Ala Ile Glu Gly Asp Leu Asp Val Gln Ala Leu Ala Asn Leu
   1 5 10 15
   Phe Asn Asp Leu Gly Ile Asn Arg Asn Pro Arg His Arg Glu Val Ile
   20 25 30
   Ala Leu Arg Met Thr Gly Gly Trp Trp Gly Pro Ala Thr Arg Tyr Asn
   35 40 45
   Leu Val Ser Leu Leu Leu Gln Asp Asp Gln Gly Gln Pro Leu Pro Gln
   50 55 60
   Pro Arg Trp Arg Ala Glu Gly Arg Ala Ala Asn Pro Ala Val Met Phe
   65 70 75 80
   Thr Leu Glu Ala Pro Trp Gln Asp Leu Arg Leu Ala Phe Asp Asn Ile
   85 90 95
   Asp Val Gly Glu Gly Thr Leu Arg Phe Gly Pro Leu Ala Asn Gly Asn
   100 105 110
   Tyr Ile Pro Gly Asp Glu Phe Ser Leu Glu Phe Leu Pro Pro Ala Met
   115 120 125
   Gln Glu Ile Thr Gln Met Gln Arg Asp Glu Leu Glu Glu Val Leu Asp
   130 135 140
   Val Val Gly Gln Ile Thr Met Gln Met Asn Asp Leu Ile Gly Met Gln
   145 150 155 160
   Asp Ala Gln Ile Arg Gly Leu Glu Gly Gln Leu Arg Gly Leu Arg Gly
   165 170 175
   Asn Leu Pro Val Ala Gly Thr Pro Pro Pro Pro Pro Pro Ser Leu Asp
   180 185 190
   Leu Gln Pro Ala Ala Ala Ser Ser Pro Tyr Val Ala Pro Ala Pro Ser
   195 200 205
   Ala Pro Ala Ala Ser Ala Ala Ala Ala Asp Leu Gly Trp Phe Ala Gly
   210 215 220
   Gly Pro Gly Pro Gly Ser Leu Asp Pro Arg Leu Ala Arg Val Ala Tyr
   225 230 235 240
   Asn Pro Phe Leu Pro Gly Pro Ser Asp Gly Ser Gly Ala Ala Pro Ala
   245 250 255
   Gln Pro Ser Ala Pro Pro Val Ala Ser Pro Leu Pro Ser Leu Leu Pro
   260 265 270
   Ala Gln Pro Met Gln Pro Val Ile Gln Tyr Val His Pro Pro Pro Ile
   275 280 285
   Asn Pro Ala Gln Gln Val Ile Pro Ile Gln His Ile Arg Ala Val Thr
   290 295 300
   Gly Asn Ala Pro Ser Asn Pro Arg Glu Ile Pro Met Trp Ile Gly Arg
   305 310 315 320
   Asn Ala Ser Ala Ile Glu Gly Val Phe Pro Ile Pro Thr Ser Asp Ile
   325 330 335
   Arg Ser Arg Val Ile Asn Ala Leu Leu Gly Arg Gln Leu Gly Leu Asn
               340                 345                 350
   Leu Asp Pro Gln His Cys Ile Thr Trp Ala Ser Ala Ile Ala Thr Leu
           355                 360                 365
   Tyr Val Arg Thr His Gly Ser Tyr Pro Leu His Gln Leu Ala Glu Val
       370                 375                 380
   Leu Arg Arg Val Ser Asn Ser Glu Gly Ala Ala Ala Ala Trp Gln Leu
   385                 390                 395                 400
   Gly Met Met Leu Thr Asn Gln Asp Tyr Asn Leu Val Trp Gly Met Val
                   405                 410                 415
   Arg Pro Leu Leu Pro Gly Gln Ala Val Val Thr Ala Met Gln His Arg
               420                 425                 430
   Leu Asp Gln Glu Val Ser Asp Ala Ala Arg Ile Val Ser Phe Val Asn
           435                 440                 445
   His Leu Asn Ala Val Tyr Glu Leu Leu Gly Leu Asn Ala Arg Gly Gln
       450                 455                 460
   Asn Leu Arg Val Ser Thr Gly Gly Gln Thr Thr Ala Arg Thr Ser Ala
   465                 470                 475                 480
   Gly Arg Gly Ala Arg Gly Arg Arg Ser Gln Gln Gly Thr Pro Gly Arg
                   485                 490                 495
   Gln Ser Ser Gly Gln Ala Pro Pro Gln Gly Arg Arg Ser Ser Gln Gly
               500                 505                 510
   Gln Gln Pro Arg Gln Ser Glu Ser Gly Asp Gln Asn Asn Gln Arg Gln
           515                 520                 525
   Leu Gln Gly Gly Asn Asn Arg Gly Gly Tyr Asn Leu Arg Pro Arg Thr
       530                 535                 540
   Tyr Gln Pro Gln Arg Tyr Gly Gly Gly Arg Gly Arg Arg Trp Asn Asp
   545                 550                 555                 560
   Gln Thr Ala Arg Ala Asp Asn Gln Gln Arg Ser Gln Ser Gln Gln Pro
                   565                 570                 575
   Gln Ser Glu Ala Arg Gly Glu Gln Ser Arg Thr Ser Gly Ala Gly Arg
               580                 585                 590
   Glu Gln Gly Gly Arg Gly Asn Gln Asn Arg Asn Gln Arg Ser Ala Gly
   595 600 605
   Glu Asn Thr Asp Arg Ser Val Asn Thr Val Thr Ala Thr Ser Ala Ser
   610 615 620
   Ile Ser Ala Ser Gly Gln Asn Gly Ser Ser Thr Thr Pro Pro Ala Ser
   625 630 635 640
   Gly Ser Gly Asn Gln Gly Asn
   645
<210> 153
   <211> 1666
   <212> DNA
   <213> Artificial Sequence
<220>
   <223> expression optimized polynucleotide encoding for a simian foamy virus derived capsid protein
<400> 153
   atggcagcaa tcgagggaga cctggatgtg caggctctgg ctaatctgtt caacgacctg 60
   ggaatcaacc gaaacccccg acaccgcgaa gtgatcgcac tgcgaatgac cggaggatgg 120
   tggggaccag ctacaagata caacctggtc tccctgctgc tgcaggacga tcagggacag 180
   ccactgccac agcctagatg gagggcagag ggacgagcag ctaatccagc cgtgatgttc 240
   actctggaag ctccttggca ggacctgagg ctggccttcg acaacattga tgtgggggag 300
   ggaaccctgc gctttggccc tctggctaac gggaattata ttccaggaga tgagttctct 360
   ctggaatttc tgccccctgc catgcaggag atcacacaga tgcagcggga cgaactggag 420
   gaagtgctgg atgtggtcgg gcagatcact atgcagatga atgacctgat tggaatgcag 480
   gatgcccaga tcagagggct ggaaggacag ctgaggggcc tgcgaggaaa cctgccagtg 540
   gcaggcacac caccacctcc accaccttcc ctggacctgc agccagcagc cgctagctcc 600
   ccttacgtgg ctccagcacc atccgcacct gcagcatctg ctgcagccgc tgacctggga 660
   tggttcgctg gaggacctgg accaggcagc ctggaccccc gactggctcg ggtggcatat 720
   aatccctttc tgccaggacc tagcgatggc agcggagcag cacctgcaca gccaagcgca 780
   ccaccagtgg cttcaccact gcccagcctg ctgccagcac agcctatgca gccagtgatt 840
   cagtacgtcc accctccacc catcaatcca gcccagcagg tcatccccat tcagcatatt 900
   agggctgtca ccggcaatgc accctctaac cctcgggaga tccccatgtg gattgggaga 960
   aacgccagtg ctatcgaagg agtgttccct atcccaactc ccgacattcg gagcagagtg 1020
   atcaacgcac tgctgggagg acagctgggc ctgaacctgg acccccagca ctgcattacc 1080
   tgggcaagcg ccatcgctac actgtacgtg cggacccacg gcagctatcc tctgcatcag 1140
   ctggccgagg tgctgagagg cgtgagcaac tcagaaggag ctgcagccgc ttggcagctg 1200
   ggaatgatgc tgacaaatca ggactacaac ctggtgtggg gaatggtccg cccactgctg 1260
   ccaggacagg cagtggtcac tgcaatgcag caccgactgg accaggaggt gagtgatgca 1320
   gcccgcattg tgtcatttgt caatcatctg aacgcagtgt atgaactgct gggcctgaat 1380
   gccagaggcc agaacctgcg ggtgagcact ggaggacaga ccacagctgg aaccagtgca 1440
   ggaagaggag ccaggggccg gagatcacag cagggaaccc ctggaaggca gtctagtgga 1500
   caggcacctc cacagggaag gcgctcaagc cagggacagc agccacgaca gagcgagagc 1560
   ggcgaccaga acaatcagag gcagctgcag ggaggcaaca atcgcggggg atataatctg 1620
   cggcctagaa cctaccagcc acagagatat ggagggggac gcggcc 1666
<210> 154
   <211> 643
   <212> PRT
   <213> Simian foamy virus
<400> 154
   Met Gly Asp His Asn Leu Asn Val Gln Glu Leu Leu Asn Leu Phe Gln
   1               5                   10                  15
   Asn Leu Gly Ile Pro Arg Gln Pro Asn His Arg Glu Val Ile Gly Leu
               20                  25                  30
   Arg Met Leu Gly Gly Trp Trp Gly Pro Gly Thr Arg Tyr Ile Leu Val
           35                  40                  45
   Ser Ile Phe Leu Gln Asp Asp Ser Gly Gln Pro Leu Gln Gln Pro Arg
       50                  55                  60
   Trp Arg Pro Glu Gly Arg Pro Val Asn Pro Leu Val His Asn Thr Ile
   65                  70                  75                  80
   Glu Ala Pro Trp Gly Glu Leu Arg Gln Ala Phe Glu Asp Leu Asp Val
                   85                  90                  95
   Ala Glu Gly Thr Leu Arg Phe Gly Pro Leu Ala Asn Gly Asn Trp Ile
               100                 105                 110
   Pro Gly Asp Glu Tyr Ser Met Glu Phe Gln Pro Pro Leu Ala Gln Glu
           115                 120                 125
   Ile Ala Gln Met Gln Arg Asp Glu Leu Glu Glu Ile Leu Asp Ile Thr
       130                 135                 140
   Gly Gln Ile Cys Ala Gln Val Ile Asp Leu Val Asp Met Gln Asp Ala
   145                 150                 155                 160
   Gln Ile Arg Gly Leu Glu Arg Arg Ile Gln Asp Arg Leu Gly Leu Arg
   165 170 175
   Asp Asn Leu Pro Val Ala Gly Ile Gln Ala Pro Pro Ser Ser Pro Ile
   180 185 190
   Gly Gln Pro Ile Ala Ser Ser Ser Leu Gln Pro Ile Pro Gly Ser Ser
   195 200 205
   Ser Ser Pro Ala Asp Leu Asp Gly Ile Trp Thr Pro Arg Gln Ile Asp
   210 215 220
   Pro Arg Leu Ser Arg Val Ala Tyr Asn Pro Phe Leu Pro Gly Ser Ser
   225 230 235 240
   Asp Gly Ser Gly Gly Ser Ile Pro Val Gln Pro Ser Ala Pro Pro Ala
   245 250 255
   Val Leu Pro Ser Leu Pro Ser Leu Pro Ala Pro Val Ser Gln Pro Ile
   260 265 270
   Ile Gln Tyr Val Ala Gln Pro Pro Val Pro Ala Pro Gln Ala Ile Pro
   275 280 285
   Ile Gln His Ile Arg Ala Val Thr Gly Asn Thr Pro Thr Asn Pro Arg
   290 295 300
   Asp Ile Pro Met Trp Leu Gly Arg His Ser Ala Ala Ile Glu Gly Val
   305 310 315 320
   Phe Pro Met Thr Thr Pro Asp Leu Arg Cys Arg Val Val Asn Ala Leu
   325 330 335
   Ile Gly Gly Ser Leu Gly Leu Ser Leu Glu Pro Ile His Cys Val Asn
   340 345 350
   Trp Ala Ala Val Val Ala Ala Leu Tyr Val Arg Thr His Gly Ser Tyr
   355 360 365
   Pro Ile His Glu Leu Ala Asn Val Leu Arg Ala Val Val Thr Gln Glu
   370 375 380
   Gly Val Ala Thr Gly Phe Gln Leu Gly Ile Met Leu Ser Asn Gln Asp
   385 390 395 400
   Tyr Asn Leu Val Trp Gly Ile Leu Arg Pro Leu Leu Pro Gly Gln Ala
   405 410 415
   Val Val Thr Ala Met Gln Gln Arg Leu Asp Gln Glu Val Asn Asp Ala
   420 425 430
   Ala Arg Ile Thr Ser Phe Asn Gly His Leu Asn Asp Ile Tyr Gln Leu
   435 440 445
   Leu Gly Leu Asn Ala Arg Gly Gln Ser Ile Ala Arg Ala Gln Ser Ala
   450 455 460
   Ser Thr Ser Gly Asn Ser Ala Ser Ala Gly Arg Gly Arg Arg Gly Gln
   465 470 475 480
   Arg Thr Gln Gln Gln Ala Gly Arg Gln Gln Gln Gln Gln Thr Arg Arg
   485 490 495
   Thr Asn Gln Gly Asn Gln Gly Gln Arg Asp Asn Asn Gln Arg Gln Ser
   500 505 510
   Ser Gly Gly Asn Gln Gly Gln Arg Gly Gln Gly Gly Tyr Asp Leu Arg
   515 520 525
   Pro Arg Thr Tyr Gln Pro Gln Arg Tyr Gly Gly Gly Arg Gly Arg Arg
   530 535 540
   Trp Asn Asp Asn Gln Gln Gln Gln Gln Ala Gln Pro Gly Arg Ser Ser
   545 550 555 560
   Asp Gln Pro Arg Ser Gln Ser Gln Gln Pro Gln Pro Glu Ala Arg Gly
   565 570 575
   Asp Gln Ser Arg Thr Ser Gly Ala Gly Arg Gly Gln Gln Gly Arg Gly
   580 585 590
   Asn Gln Asn Arg Asn Gln Arg Arg Ala Asp Ala Asn Asn Thr Arg Asn
   595 600 605
   Val Asp Thr Val Thr Ala Thr Thr Thr Ser Ser Ser Thr Ala Ser Ser
   610 615 620
   Gly Gln Asn Gly Ser Ser Thr Thr Pro Pro Ala Ser Gly Ser Arg Asn
   625 630 635 640
   Gln Gly Asp
<210> 155
   <211> 1945
   <212> DNA
   <213> Artificial Sequence
<220>
   <223> expression optimized polynoucleotide encoding for a simian foamy virus derived capsid protein
<400> 155
   atgggcgatc ataatctgaa cgtccaggag ctgctgaacc tgtttcagaa cctgggcatt 60
   ccaaggcagc caaatcatag agaagtcatc ggcctgcgaa tgctgggagg atggtggggg 120
   cctggaaccc ggtacatcct ggtgagcatt ttcctgcagg acgatagcgg acagccactg 180
   cagcagcctc gatggagacc agagggaagg cccgtgaatc ctctggtcca caacaccatc 240
   gaggcaccat ggggagaact gagacaggca ttcgaggacc tggatgtggc cgaaggcaca 300
   ctgaggtttg ggcctctggc taacggaaat tggattcccg gcgacgagta ttccatggaa 360
   tttcagcccc ctctggccca ggagattgct cagatgcaga gagatgaact ggaggaaatc 420
   ctggacatta cagggcagat ctgcgcacag gtcatcgacc tggtcgatat gcaggacgcc 480
   cagatcaggg gactggaacg gagaattcag gatcgcctgg gactgcgaga caatctgcct 540
   gtggcaggca tccaggcacc acccagctcc cccatcggac agcctattgc atctagttca 600
   ctgcagccta ttccagggag ctcctctagt ccagctgacc tggatggaat ctggacccct 660
   cgccagattg atccaagact gagcagggtg gcctacaacc cattcctgcc agggtcaagc 720
   gacggcagcg gaggctctat ccctgtgcag ccatccgcac ctccagcagt cctgcccagt 780
   ctgccttcac tgcccgctcc tgtgtctcag cccatcattc agtatgtggc acagccacct 840
   gtcccagctc cacaggcaat ccctattcag cacattagag ccgtgacagg caatacacca 900
   actaaccccc gcgatatccc aatgtggctg ggacgacata gcgcagctat tgagggcgtg 960
   tttccaatga ccacacccga cctgaggtgc cgcgtggtca atgccctgat cggaggatct 1020
   ctgggcctga gcctggagcc aattcactgt gtgaactggg cagccgtggt cgctgcactg 1080
   tacgtccgca ctcacgggtc ctatcccatc catgagctgg ccaatgtgct gcgggctgtg 1140
   gtcacacagg aaggcgtcgc cactgggttc cagctgggaa tcatgctgtc taatcaggat 1200
   tacaacctgg tgtgggggat tctgcggcct ctgctgccag gacaggctgt ggtcacagca 1260
   atgcagcaga gactggatca ggaggtgaac gacgccgcta ggatcactag ctttaatggc 1320
   catctgaatg acatctacca gctgctgggg ctgaatgccc gaggacagag catcgcacgg 1380
   gcccagtctg ctagtacttc aggcaacagc gcttccgcag gaagagggag gcgaggacag 1440
   aggacccagc agcaggccgg gcgccagcag cagcagcaga cccggcggac caatcagggg 1500
   aaccagggac agcgggataa caatcagaga cagtcctctg gaggaaatca gggacagcga 1560
   ggacagggag gatacgacct gcgaccccgg acttaccagc ctcagagata tggaggaggc 1620
   cgcggaagaa ggtggaacga taatcagcag cagcagcagg cacagccagg acgcagttca 1680
   gaccagccac ggagccagag ccagcagcct cagccagagg cccgaggcga tcagagccgg 1740
   acctccggag ctggaagagg acagcaggga aggggcaacc agaatcgcaa ccagcgccga 1800
   gccgatgcta acaataccag aaacgtggac accgtcacag ctactaccac aagctcctct 1860
   acagcaagtt caggacagaa tggcagctcc actaccccac cagcatcagg cagcaggaac 1920
   cagggggact gatcaagggg actaa 1945
<210> 156
   <211> 644
   <212> PRT
   <213> Simian foamy virus
<400> 156
   Met Ala Ser Asn Ser Asn Val Glu Glu Tyr Leu Leu Asp Val Glu Ala
   1               5                   10                  15
   Leu Val Asp Ile Leu Arg Glu Arg His Ile Pro Arg Asn Pro Met His
               20                  25                  30
   Gln Glu Ile Ile Gly Leu Arg Met Thr Glu Gly Trp Trp Gly Gln Met
           35                  40                  45
   Glu Arg Phe Gln Met Thr Arg Leu Ile Leu Gln Asp Asp Asp Gly Glu
       50                  55                  60
   Pro Leu Gln Gln Pro Arg Tyr Glu Val Val Pro Arg Ala Val Asn Pro
   65                  70                  75                  80
   Arg Thr Gln Phe Val Ile Ser Gly Thr Leu Ala Glu Leu Arg Leu Ala
                   85                  90                  95
   Phe Leu Glu Leu Asp Leu Pro Glu Gly Ala Leu Arg Tyr Gly Pro Leu
               100                 105                 110
   Ala Asn Gly His Tyr Ile Gln Gly Asp Pro Tyr Ser Leu Ser Tyr Arg
           115                 120                 125
   Pro Val Thr Met Ala Glu Thr Ala His Met Thr Arg Asp Glu Leu Glu
       130                 135                 140
   Asp Val Leu Asn Val Gln Ser Glu Ile Gly Ile Gln Leu Ile Asn Ile
   145                 150                 155                 160
   Leu Glu Leu Gln Glu Val Glu Val Arg Ala Leu Arg Arg Gln Leu Ala
                   165                 170                 175
   Glu Asn Ser Ser Val Arg Arg Gly Gly Met Ser Pro Gly Ala Pro Ile
               180                 185                 190
   Pro Arg Pro Pro Val Ser Ser Tyr Ala Gly Leu Pro Ser Leu Pro Pro
           195                 200                 205
   Ala Pro Gly Ile Phe Pro Ser Ala Pro Ser Pro Pro Gln Ala Ser Ser
       210                 215                 220
   Thr Pro Gly Gln Arg Pro Trp Ser Leu Gly Asn Asp Leu Asp Gln Ser
   225                 230                 235                 240
   Val Ile Pro Gly Pro Ser Gln Pro Arg Val Ser Phe His Pro Ser Asn
                   245                 250                 255
   Pro Phe Gln Ile Asp Ser Val Pro Arg Pro Arg Ser Ala Ser Arg Glu
               260                 265                 270
   Arg Pro Ala Val Glu Thr Gln Pro Ser Ala Pro Pro Leu Ile Gln Tyr
           275                 280                 285
   Val Pro Val Pro Pro Pro Val Phe Pro Pro Ser Val Asn Thr Val Ile
       290                 295                 300
   Pro Ile Gln His Ile Arg Ala Val Thr Gly Glu Pro Pro Arg Asn Pro
   305                 310                 315                 320
   Arg Glu Ile Pro Ile Trp Leu Gly Arg Asn Ala Pro Ala Ile Glu Gly
                   325                 330                 335
   Val Phe Pro Val Asn Ser Pro Glu Val Arg Cys Arg Val Ile Asn Ala
               340                 345                 350
   Ile Ile Gly Gly Asn Leu Gly Leu Ala Leu Thr Pro Thr Glu Cys Ala
           355                 360                 365
   Thr Trp Asp Ser Ala Val Ala Thr Leu Phe Ile Arg Thr His Gly Thr
       370                 375                 380
   Tyr Pro Met His Gln Leu Gly Asn Val Ile Lys Gly Ile Val Asp Gln
   385                 390                 395                 400
   Glu Gly Val Ala Thr Gly Tyr Thr Leu Gly Met Met Leu Ser Gly Gln
                   405                 410                 415
   Asn Phe Pro Leu Val Tyr Gly Ile Ile Arg Gly Phe Leu Pro Gly Gln
               420                 425                 430
   Ala Val Val Thr Ala Ile Gln Gln Arg Leu Asp Gln Glu Val Asp Asp
           435                 440                 445
   Gln Thr Arg Ser Asp Thr Phe Ile Gln His Pro Asn Ala Val Tyr Glu
       450                 455                 460
   Ile Leu Gly Leu Asn Ala Arg Gly Gln Ser Ile Arg Thr Gly Ala Asn
   465 470 475 480
   Ser Thr Pro Arg Pro Thr Arg Gly Arg Gly Arg Gly Ile Ser Arg Gln
   485 490 495
   Ser Gln Ser Ala Pro Gly Ala Gly Arg Gly Arg Gln Arg Ser Asp Gly
   500 505 510
   Asn Leu Gln Asp Asn Asn Pro Ser Ser Gln Asn Gln Gly Gln Ser Asn
   515 520 525
   Ser Asn Gln Arg Gly Tyr Asn Leu Arg Pro Arg Thr Tyr Gln Pro Ala
   530 535 540
   Arg Tyr Gly Gly Gly Arg Gly Arg Arg Trp Ser Asp Asn Thr Gln Thr
   545 550 555 560
   Asn Arg Gln Thr Glu Ser Ser Thr Gln Asn Pro Arg Gln Ala Gln Thr
   565 570 575
   Gly Gln Gly Val Arg Gly Asp Gln Ser Arg Ala Pro Arg Ser Gly Ala
   580 585 590
   Ser Arg Gly Gly Arg Gly Asn Gln Asn Arg Asn Gln Arg Pro Ser Gly
   595 600 605
   Ser Gly Asp Ser His Thr Val Asn Thr Val Thr Gln Ala Val Thr Ser
   610 615 620
   Ser Thr Thr Asp Glu Ser Ser Thr Val Ala Ser Ala Pro Thr Ser Gly
   625 630 635 640
   Ser Gln Gly Glu
<210> 157
   <211> 1935
   <212> DNA
   <213> Artificial Sequence
<220>
   <223> expression optimized polynoucleotide encoding for a simian foamy virus derived capsid protein
<400> 157
   atggcatcaa acagtaatgt ggaagaatac ctgctggacg tggaagccct ggtggacatc 60
   ctgagagaac ggcatatccc tcggaatccc atgcaccagg agatcattgg cctgaggatg 120
   acagaggggt ggtggggaca gatggaacgc ttccagatga cccggctgat cctgcaggac 180
   gatgacggag agccactgca gcagcctcga tacgaagtgg tcccacgggc cgtcaacccc 240
   agaactcagt tcgtgatctc cggaaccctg gcagagctgc ggctggcttt tctggagctg 300
   gatctgcccg aaggagctct gagatacggc cctctggcaa atgggcacta tatccaggga 360
   gacccttact cactgagcta taggccagtg accatggccg aaacagctca tatgacccgg 420
   gacgagctgg aagacgtgct gaatgtccag agcgagatcg ggattcagct gatcaacatt 480
   ctggaactgc aggaggtgga agtcagggct ctgcggagac agctggcaga gaacagctcc 540
   gtgaggcgag gaggaatgag ccccggagca cccatccctc gcccccctgt gtctagttac 600
   gcaggcctgc cctcactgcc accagcacct ggaattttcc catccgcacc ctctcctcca 660
   caggcttcaa gcaccccagg acagcgacca tggagcctgg gaaacgatct ggaccagtct 720
   gtcatcccag gccccagtca gcccagagtg agcttccacc cttccaatcc atttcagatt 780
   gattccgtcc ctagaccaag gtccgcatct cgcgagcgac cagcagtgga aacccagcca 840
   agcgctccac ctctgatcca gtatgtgcct gtcccacccc ctgtgtttcc accctccgtg 900
   aatactgtca tcccaattca gcatattagg gcagtgaccg gcgagcctcc acggaatcct 960
   agagaaatcc caatttggct gggacgcaac gcccctgcta tcgagggcgt gttccccgtc 1020
   aattctcctg aagtgcggtg cagagtgatc aacgccatca ttggaggaaa cctgggactg 1080
   gcactgaccc ccaccgagtg tgctacatgg gatagcgcag tggccactct gtttatccgg 1140
   acccacggca cctatccaat gcatcagctg ggcaacgtga tcaaggggat tgtcgaccag 1200
   gaaggagtgg ccacaggcta cactctgggg atgatgctgt ctggccagaa tttcccactg 1260
   gtgtatggaa tcattagagg ctttctgccc gggcaggctg tggtcacagc aatccagcag 1320
   cgcctggatc aggaggtgga tgaccagacc cgaagcgaca ccttcatcca gcaccccaat 1380
   gctgtgtacg aaattctggg actgaacgca aggggccagt caatccgaac cggagcaaac 1440
   agcactcccc gacctaccag gggccgcggg cgaggaattt ccagacagag ccagtcagca 1500
   ccaggagcag gacggggaag acagaggtct gatggcaatc tgcaggacaa caatccctcc 1560
   tctcagaacc aggggcagag caactccaat cagcggggat ataacctgcg ccctcgaaca 1620
   taccagccag ctcgatatgg aggaggccgg ggccgacgat ggagcgataa tacacagact 1680
   aacagacaga cagagagttc aactcagaat cccagacagg cacagaccgg acagggggtg 1740
   aggggggacc agtccagggc acctcgaagt ggagcctcac gagggggacg gggaaaccag 1800
   aatagaaacc agaggccctc tggaagtggc gattctcata ccgtgaacac cgtcacacag 1860
   gccgtcacaa gctccaccac agacgagagc agtaccgtgg ctagtgcacc tacatcaggc 1920
   agccaggggg aatga 1935
<210> 158
   <211> 624
   <212> PRT
   <213> Simian foamy virus
<400> 158
   Met Ala Ala Gln Asn Phe Glu Leu Asp Val Gln Glu Leu Leu Asn Leu
   1               5                   10                  15
   Phe Gln Asp Asn Gly Val Thr Arg Asn Pro Arg His Leu Glu Thr Ile
               20                  25                  30
   Gly Leu Arg Met Leu Gly Gly Trp Trp Gly Glu Gln Glu Arg Tyr Gln
           35                  40                  45
   Ser Ala Arg Ile Ile Leu Gln Asp Asp Asp Gly Glu Pro Leu Gln Val
       50                  55                  60
   Pro Arg Trp Glu Glu Val Leu Arg Pro Val Asn Pro Leu Ala His Phe
   65                  70                  75                  80
   Val Ile Ser Ala Pro Trp Asp Gln Leu Arg Arg Ala Phe His Asp Leu
                   85                  90                  95
   Asp Val Gly Asn Gly Ala Leu Arg Phe Gly Pro Leu Ala Asn Gly Asn
               100                 105                 110
   Tyr Ile Pro Glu Asp Pro Tyr Ser Thr Ser Tyr Arg Pro Val Asn Pro
           115                 120                 125
   Gln Glu Met Ala Gln Met Gln Arg Asp Glu Leu Glu Glu Val Leu Glu
       130                 135                 140
   Val Gln Gly Glu Ile Glu Leu Gln Met Ile Asp Leu Ile Glu Met Gln
   145                 150                 155                 160
   Thr Ile Glu Ile Arg Gly Leu Arg Gln Leu Val Asn Glu Leu Gln Arg
                   165                 170                 175
   Glu Arg Asp Ser Gly Arg Gly Ala Ser Ile Pro Gly Ala Ser Ser Ser
               180                 185                 190
   Pro Pro Pro Gln Ser Tyr Val Gly Leu Pro Gly Met Pro Ser Val Ser
           195                 200                 205
   Gly Leu Ser Ser Leu Gln Pro Glu Ala Ser Ser Thr Pro Gly Gly Arg
       210                 215                 220
   Ala Pro Arg Val Ser Phe His Pro Ser Asn Pro Phe Val Arg Pro Pro
   225                 230                 235                 240
   Ser Pro Glu Arg Pro Arg Ala Arg Ser Ser Glu Arg Ile Pro Leu Pro
                   245                 250                 255
   Leu Gln Pro Pro Val Ile Gln Tyr Val Pro Val Pro Gln Pro Val Leu
               260                 265                 270
   Ala Pro Ala Pro Ala Pro Ser Pro Val Ile Pro Ile Gln His Ile Arg
           275                 280                 285
   Ala Val Thr Gly Glu Val Pro Asn Asn Pro Arg Asp Ile Pro Met Trp
       290                 295                 300
   Ile Gly Arg Asn Ala Pro Ala Ile Glu Gly Val Tyr Pro Val Thr Thr
   305                 310                 315                 320
   Pro Asp Leu Arg Ala Arg Ile Ile Asn Ala Leu Ile Gly Gly Lys Ser
                   325                 330                 335
   Gly Ile His Leu Thr Ala Pro Glu Ala Val Thr Trp Ala Ser Ala Val
               340                 345                 350
   Ala Ala Ile Phe Thr Arg Thr His Gly Ser Phe Pro Met His Asn Leu
           355                 360                 365
   Ser Ala Ile Leu Thr Gly Ile Ala Asn Gly Glu Gly Val Glu Ser Ala
       370                 375                 380
   Tyr Asn Leu Gly Met Met Leu Ser Asn Gly Asp Phe Asn Leu Val Tyr
   385                 390                 395                 400
   Gly Ile Val Arg Gly Leu Leu Pro Gly Gln Ala Ala Val Ala Tyr Met
                   405                 410                 415
   Gln Gln Arg Leu Asp Ala Glu Pro Ser Asp Ala Leu Arg Ala Gln Asn
               420                 425                 430
   Phe Ile Gln His Leu His Leu Val Tyr Glu Ile Leu Gly Leu Asn His
           435                 440                 445
   Arg Gly Gln Ser Ile Arg Thr Ser Leu Pro Thr Ser Thr Arg Pro Arg
       450                 455                 460
   Gly Gln Gly Arg Gly Arg Gly Gln Gly Gln Gly Ser Ile Pro Ser Thr
   465                 470                 475                 480
   Pro Arg Arg Pro Gln Ser Gly Arg Gly Leu Ser Thr Pro Asn Arg Gly
                   485                 490                 495
   Ser Asn Asn Ala Asn Asn Asn Thr Gln Ser Asn Val Gln Thr Glu Thr
               500                 505                 510
   Pro Arg Arg Ser Phe Gly Gly Tyr Asn Leu Arg Pro Asn Thr Phe Arg
   515 520 525
   Pro Gln Arg Tyr Gly Gly Gly Gln Gly Gln Arg Arg Asp Ser Gln Pro
   530 535 540
   Asp Arg Arg Ser Gln Gly Ser Ser Gln Asn Asn Arg Pro Ser Ala Pro
   545 550 555 560
   Leu Glu Ser Arg Gly Glu Gln Ser Arg Gly Pro Gly Gly Gly Gly Arg
   565 570 575
   Ala Gly Gly Arg Arg Asn Gln Asn Arg Asn Ser Gly Gln Gly Asn Glu
   580 585 590
   Ser Ser Ser His Ala Val Asn Ala Val Thr Gln Ser Ala Val Thr Glu
   595 600 605
   Gln Gln Asn Glu Ser Pro Thr Pro Pro Pro Thr Ser Gly Gly Arg Ser
   610 615 620
<210> 159
   <211> 1875
   <212> DNA
   <213> Artificial Sequence
<220>
   <223> expression optimized polynoucleotide encoding for a simian foamy virus derived capsid protein
<400> 159
   atggccgccc agaactttga actggacgtg caggaactgc tgaatctgtt tcaggacaat       60
   ggagtgacaa ggaacccccg acatctggag accatcggac tgagaatgct gggaggatgg      120
   tggggagagc aggaacgata tcagtctgcc cggatcattc tgcaggacga tgacggagaa      180
   ccactgcagg tcccccgatg ggaggaagtg ctgagacctg tcaacccact ggcccacttc      240
   gtgatcagcg ctccatggga tcagctgcgg agagctttcc atgatctgga cgtggggaat      300
   ggagctctga ggtttgggcc cctggcaaat ggaaactaca ttcccgagga cccttactca      360
   actagctata gaccagtgaa cccccaggaa atggcccaga tgcagaggga tgagctggag      420
   gaagtgctgg aagtccaggg cgagatcgaa ctgcagatga tcgacctgat tgagatgcag      480
   accatcgaaa ttcgcggcct gcgacagctg gtgaatgagc tgcagaggga acgagattct      540
   ggccgcggag caagtattcc aggagccagc tcctctcccc ctccacagtc ctatgtggga      600
   ctgcctggca tgccatccgt ctctggcctg agttcactgc agcctgaggc cagctccaca      660
   cccggaggcc gggctcctag agtgagcttc cacccctcca acccttttgt ccgcccccct      720
   agcccagagc gaccccgggc tagatctagt gaaagaatcc ccctgccact gcagccaccc      780
   gtgattcagt atgtgcccgt ccctcagcca gtcctggctc cagcacctgc accatccccc      840
   gtgatcccca ttcagcacat ccgagcagtg actggagagg tccctaacaa tccacgagat 900
   atccccatgt ggattggccg gaacgctcct gcaattgaag gggtgtaccc tgtcaccaca 960
   ccagacctga gggctcgcat cattaatgca ctgatcgggg gaaagagcgg cattcatctg 1020
   accgcaccag aggcagtgac atgggcatcc gctgtcgcag ctatcttcac tcgcacccac 1080
   gggtcctttc ctatgcataa tctgtctgca atcctgaccg gaattgccaa cggagagggc 1140
   gtggaaagtg cctacaatct ggggatgatg ctgtcaaacg gagacttcaa tctggtgtat 1200
   ggaatcgtcc gaggactgct gccaggacag gcagccgtgg catacatgca gcagcgcctg 1260
   gatgcagagc caagcgacgc tctgcgggca cagaacttta tccagcacct gcatctggtg 1320
   tacgaaattc tgggcctgaa tcacagaggg cagtctatca ggacaagtct gccaacatca 1380
   actcgacccc gggggcaggg aagaggcagg ggccagggac agggcagtat tccctcaact 1440
   cctaggcgcc cacagtcagg gaggggactg agcaccccta accgaggcag caacaatgcc 1500
   aacaataaca cacagtccaa tgtgcagacc gagacaccac gacggtcttt cggcgggtac 1560
   aacctgagac ctaatacctt taggccacag cgatatggag gaggacaggg acagagaagg 1620
   gattcacagc ctgaccgccg aagccagggg tcaagccaga ataacaggcc atccgctcct 1680
   ctggagagcc ggggcgaaca gagccggggc ccaggaggcg ggggaagagc aggcggcaga 1740
   agaaatcaga acaggaatag cggacaggga aacgagagca gcagccacgc cgtgaatgct 1800
   gtcacacagt ctgccgtgac tgagcagcag aacgaaagtc ccactcctcc acccaccagc 1860
   ggaggcagat cctga 1875
<210> 160
   <211> 613
   <212> PRT
   <213> Simian foamy virus
<400> 160
   Met Ala Gln Gly Asp Pro Ile Ile Asn Gln Gly Asp Leu Asn Val Val
   1 5 10 15
   Glu Leu Thr His Leu Leu Arg Glu Asn Gly Leu Pro Gly Asn Pro Arg
   20 25 30
   Asn Gly Gln Thr Tyr Ala Ile Ala Met Thr Glu Gly Trp Trp Gly Asp
   35 40 45
   His Pro Arg Tyr Arg Ile Ile Arg Val Val Phe Gln Asp Thr Ser Gly
   50 55 60
   Asn Pro Leu Ser Gln Pro Gln Trp Met Asp Glu Asp Arg Gln Met Asn
   65 70 75 80
   Pro Leu Gln Asp Pro Val Val Gly Cys Thr Phe Asn Gln Ala Ser Arg
                   85                  90                  95
   Val Phe Asp Gln Ile Asp Ile Gly Glu Gly Pro Ser Arg Phe Gly Pro
               100                 105                 110
   Leu Ala Asp Gly Met Phe Leu Ile Thr Asp Asn Ala Trp Met Asp Phe
           115                 120                 125
   Val Pro Leu Ser Ala Met Glu Ile Thr Asp Ile Asn Asn Glu Arg Thr
       130                 135                 140
   Leu Arg Asp His Leu Thr Phe Cys Cys Thr Leu Leu Glu Asn Met Met
   145                 150                 155                 160
   Ala Asp Ile Ile Val Thr Gln Arg Glu Asn Arg Asp Leu Asn Thr Thr
                   165                 170                 175
   Ile Asn Asn Leu Arg Ala Thr Ile Pro Val Ala Ser Gln Ala Leu Pro
               180                 185                 190
   His Ser Ser Leu Pro Ala Thr Pro Met Ala Ala Ser Thr Pro Gly Gly
           195                 200                 205
   Pro Ser Gly Leu Pro Pro Ser Thr Phe Asn Ile Gly Ala Val Gly Pro
       210                 215                 220
   Ser Tyr Gln Pro Pro Ala Gln Leu Pro Ser Ala Pro Pro Ile Ala Leu
   225                 230                 235                 240
   Pro Val Ile Pro Ser Ala Pro Leu Ala Pro Ala Val Pro Leu Pro Gln
                   245                 250                 255
   Ser Gln Pro Pro Gln Pro Met Tyr Asn Pro Tyr Met Met Ala Gly Asn
               260                 265                 270
   Leu Gln Gly Asn Val Leu Pro Ile Ser Gln Ile Lys Ala Val Ile Gly
           275                 280                 285
   Glu Thr Pro Thr Asp His Lys Ala Val Pro Leu Trp Val Ala Lys His
       290                 295                 300
   Ala Ala Ala Ile Glu Gly Val Phe Pro Thr Gly Ser Pro Glu Val Arg
   305                 310                 315                 320
   Cys Arg Val Leu Asn Ser Leu Leu Thr Gly His Gly Gly Met Met Leu
                   325                 330                 335
   His Pro Val Asp Cys Val Ser Trp Thr Asn Ala Ala Ser Val Leu Phe
   340 345 350
   Gln Arg Val His Gly Val Val Pro Leu His Gln Leu Pro Lys Thr Leu
   355 360 365
   Glu Glu Val Ala Lys Thr Glu Gly Leu Leu Val Ala Tyr Asn Ile Gly
   370 375 380
   Met Thr Phe Thr Ser Asn Asn Phe Asp Leu Ile Trp Gly Ile Ile Arg
   385 390 395 400
   Pro Leu Val Pro Gly Gln Ala Ala Val Ala Met Leu Gln Gly Tyr Leu
   405 410 415
   Asp Gln Tyr Pro Arg Pro Gln Asp Lys Ile Glu His Phe Pro Arg Phe
   420 425 430
   Leu Arg Arg Thr Phe Glu Val Leu Gly Leu Asn Phe Leu Gly Gln Ser
   435 440 445
   Ile Arg Ser Thr Gln Ala Pro Ser Arg Pro Asn Ser Ala Asn Arg Gly
   450 455 460
   Thr Asn Thr Ala Arg Gly Arg Gly Gly Asn Arg Gly Arg Gly Arg Gly
   465 470 475 480
   Arg Gly Val Pro Pro Gly Pro Pro Pro Gly Arg Gly Thr Ala Thr Pro
   485 490 495
   Gln Ala Ala Gly Thr Ser Arg Ser Ser Ser Gly Ser Phe Gly Glu Gln
   500 505 510
   Pro Arg Tyr Asn Leu Arg Pro Gln Val Asn Arg Pro Ser Arg Tyr Gly
   515 520 525
   Gly Gly Pro Asn Pro Ala Arg Asn Asn Pro Glu Pro Gln Gly Gly Asp
   530 535 540
   Thr Ser Ser Thr Pro Arg Gly Gln Gly Glu Arg Ser Gly Arg Gln Arg
   545 550 555 560
   Asn Asn Asn Ala Arg Gln Asn Gln Gln Pro Pro Arg Arg Pro Asp Arg
   565 570 575
   Thr Val Asn Thr Val Arg Thr Ala Gln Pro Asn Pro Thr Val Ala Ser
   580 585 590
   Gly Ser Gln Asp Asp Asn Ser Thr Pro Pro Ser Thr Ala Arg Gly Ser
   595 600 605
   Glu Glu Asn Arg Ala
   610
<210> 161
   <211> 1842
   <212> DNA
   <213> Artificial Sequence
<220>
   <223> expression optimized polynoucleotide encoding for a simian foamy virus derived capsid protein
<400> 161
   atggcccagg gagacccaat catcaaccag ggggatctga acgtcgtcga actgacacat 60
   ctgctgaggg agaacggact gcccggaaat cctaggaacg gccagacata cgcaatcgcc 120
   atgactgagg gatggtgggg ggaccaccca cgatatcgga tcattagggt ggtcttccag 180
   gatacaagcg gcaaccccct gtcccagcct cagtggatgg acgaggatcg ccagatgaat 240
   ccactgcagg accccgtggt cggatgcacc ttcaaccagg cctcacgggt gtttgaccag 300
   atcgatattg gagaaggccc aagcagattc ggacccctgg ctgatggcat gtttctgatc 360
   accgacaatg cctggatgga tttcgtgcct ctgtccgcta tggagatcac tgacattaac 420
   aatgaaagaa ccctgaggga tcacctgacc ttttgctgta cactgctgga gaacatgatg 480
   gccgacatca ttgtgacaca gcgcgaaaat cgagatctga acaccacaat caacaatctg 540
   agagccacta ttccagtggc ctcccaggct ctgccacata gctccctgcc agctacccct 600
   atggcagctt ctacaccagg aggaccatct ggcctgccac ctagtacctt taatatcgga 660
   gcagtgggac caagttacca gccaccagca cagctgcctt cagcacctcc aatcgccctg 720
   cctgtgattc catctgcacc actggctcct gcagtcccac tgccacagag ccagccacct 780
   cagcctatgt acaacccata tatgatggct ggcaatctgc aggggaacgt gctgccaatc 840
   agccagatta aggcagtcat cggcgagacc cccaccgacc acaaggctgt gccactgtgg 900
   gtcgcaaaac atgcagccgc tattgagggg gtgttcccca ccggaagccc tgaagtgaga 960
   tgcagggtcc tgaattccct gctgacaggc cacggaggca tgatgctgca tcccgtggat 1020
   tgtgtctcat ggaccaacgc agccagcgtg ctgtttcaga gggtccacgg cgtggtccct 1080
   ctgcatcagc tgccaaagac actggaggaa gtggcaaaaa ctgaagggct gctggtcgcc 1140
   tataatatcg gaatgacatt cactagcaac aattttgacc tgatttgggg aatcattcga 1200
   ccactggtgc ccggacaggc tgcagtcgca atgctgcagg gctacctgga ccagtatcct 1260
   cggccacagg ataaaatcga gcatttcccc agatttctgc ggagaacttt cgaagtgctg 1320
   ggcctgaact ttctggggca gtcaattcgg agcacccagg ccccttccag accaaattct 1380
   gctaacaggg gaaccaatac agcacgcggc cgagggggaa accgggggag aggaaggggc 1440
   cgcggggtgc cacccggacc tccaccagga aggggcactg ccaccccaca ggcagctggc 1500
   acatcccgct ctagttcagg gtctttcgga gagcagccca ggtacaatct gcgcccccag 1560
   gtgaaccggc ctagtagata tggcggggga cccaatcctg cacgaaacaa tccagagcca 1620
   cagggagggg acacaagctc cactccccga gggcagggag aacgatctgg ccgacagcga 1680
   aacaataacg ccagacagaa ccagcagcct ccaaggcgcc ctgatcgcac cgtgaataca 1740
   gtccgaactg cccagcctaa cccaaccgtg gcttctggca gccaggacga taatagtact 1800
   cccccttcaa ccgctagggg aagcgaggaa aaccgcgcat ga 1842
<210> 162
   <211> 623
   <212> PRT
   <213> Simian foamy virus
<400> 162
   Met Ala Gln Gly Asn Gln Pro Glu His Ile Asp Ile Gly His Leu Thr
   1               5                   10                  15
   Ile Leu Leu Arg Glu Asn Gly Leu Pro Thr Asn Pro Pro His Gly Thr
               20                  25                  30
   Glu Tyr Ala Val Arg Met Thr Glu Gly Trp Trp Gly Asp Tyr Ala Arg
           35                  40                  45
   Phe Thr Val Ile Arg Val Ala Phe Gln Asp Asn Gln Gly Asn Pro Leu
       50                  55                  60
   Pro Arg Pro Glu Trp Glu Tyr Ile Asn Arg Asp Val Arg Pro Leu Asn
   65                  70                  75                  80
   Asp Ser Ile Ile Gly Cys Thr Phe Arg Gln Ala Gln Gln Ala Phe Asn
                   85                  90                  95
   Asn Ile Asp Leu Ala Arg Thr Pro Ser Arg Tyr Gly Pro Leu Ser Asn
               100                 105                 110
   Gly Leu Phe Leu Ile Thr Asp Pro Glu Trp Leu Thr Phe Asn Pro Leu
           115                 120                 125
   Ser Ala Val Glu Ile Ala Asp Leu Asp Asp Gln Glu Ile Arg Asp Phe
       130                 135                 140
   Leu Ala His Ala Thr Ile Val Leu Asp Asn Val Met Ala Asp Val Val
   145                 150                 155                 160
   Ile Gln Gln Arg Glu Ile Glu Asp Leu Thr Lys Thr Asn Leu Arg Leu
                   165                 170                 175
   Arg Asn Ala Leu Ala Ser Ser Gly Thr Tyr Gln Pro Ile Gly Met Pro
               180                 185                 190
   Ile Ala Gln Ser Ser Pro Ala Gly Pro Ser Ala Ser Val Ala Gly Pro
           195                 200                 205
   His Met Pro Ala Met Pro Ser Val Pro Tyr Ile Ser Ala Ile Pro Ser
       210                 215                 220
   Ala Pro Pro Ala Pro Ile Pro Val Ala Pro Ala Ala Ala Pro Pro Leu
   225                 230                 235                 240
   Gly Pro Leu Pro Ser Ala Pro Ala Pro Pro Val Leu Pro Val Ile Gln
                   245                 250                 255
   Pro Asn Thr Phe Gly Thr Asp Gly Asn Gly Tyr Thr Ala Val Gly Pro
               260                 265                 270
   Val Pro Val Leu Pro Ile Ala Gln Ile Lys Ala Val Ile Gly Glu Ala
           275                 280                 285
   Pro Thr Asp Ala Arg Gln Ile Pro Leu Trp Val Ala Lys His Ala Ala
       290                 295                 300
   Ala Ile Glu Gly Thr Phe Pro Thr Gly Ser Ala Asp Val Arg Cys Arg
   305                 310                 315                 320
   Val Leu Asn Ala Leu Leu Thr Ser His Gly Gly Met Thr Leu Ser Pro
                   325                 330                 335
   Asn Glu Cys Gly Thr Trp Ser Leu Ala Ala Ala Ala Leu Tyr Gln Arg
               340                 345                 350
   Ile Tyr Gly Val Ile Pro Leu His Asp Leu Pro His Thr Met Ala Glu
           355                 360                 365
   Val Ala Arg Arg Glu Gly Ile Leu Val Ala Phe Asn Met Gly Met Thr
       370                 375                 380
   Phe Thr Asn Asn Ser Phe Asp Val Val Trp Gly Ile Ile Arg Pro Leu
   385                 390                 395                 400
   Leu Pro Gly Gln Ala Ser Val Ala Met Leu Gln Gly Tyr Leu Asp Gln
                   405                 410                 415
   Tyr Gln Gly Gln Gln Gln Lys Ala Gln Ala Phe Pro Thr Leu Leu Arg
               420                 425                 430
   Arg Thr Phe Glu Thr Leu Gly Leu Asn Tyr Leu Gly Gln Ser Ile Arg
   435 440 445
   Ser Asn Gln Pro Ala Arg Thr Ser Gly Ser Val Ala Gly Ser Ile Thr
   450 455 460
   Gln Gly Arg Gly Arg Gly Gln Pro Arg Ser Arg Pro Pro Asn Arg Ala
   465 470 475 480
   Ser Asn Arg Ser Asn Asn Pro Pro Arg Pro Pro Pro Pro Arg Ser Gln
   485 490 495
   Glu Gln Gln Pro Ser Pro Ala Gly Arg Gly Ser Ser Ser Ser Ser Ala
   500 505 510
   Pro Pro Gln Arg Ser Gly Tyr Asn Leu Arg Gln Gln Ile Asn Arg Pro
   515 520 525
   Gln Arg Leu Gly Gly Gly Pro Asn Pro Tyr Arg Asn Leu Glu Asn Arg
   530 535 540
   Asn Ile Pro Arg Ser Glu Thr Val Pro Ser Ser Arg Arg Ala Glu Thr
   545 550 555 560
   Ser Glu Thr Asn Arg Pro Asn Gln Ala Arg Gly Gly Gln Gln Gly Ser
   565 570 575
   Ser Asn Arg Asn Gln Thr Arg Ser Ile Asn Thr Val Arg Ala Ser Asp
   580 585 590
   Ser Gln Val Ile Pro Ser Ala Pro Pro Ala Glu Thr Asn Pro Ala Ala
   595 600 605
   Thr Ala Val Thr Pro Asn Gly Pro Asn Gln His Ser Gly Arg Asn
   610 615 620
<210> 163
   <211> 1893
   <212> DNA
   <213> Artificial Sequence
<220>
   <223> expression optimized polynoucleotide encoding for a simian foamy virus derived capsid protein
<400> 163
   atggcccagg gaaaccagcc cgaacatatt gacatcggac atctgactat cctgctgcga      60
   gaaaatggac tgcccactaa ccccccacac ggcactgagt acgccgtgag gatgaccgaa     120
   ggatggtggg gcgactatgc cagattcacc gtgatcaggg tcgcttttca ggataaccag     180
   gggaatcccc tgcctaggcc agagtgggaa tacattaacc gagacgtgcg gcctctgaat     240
   gatagcatca ttggatgcac cttccgccag gcacagcagg cctttaacaa tatcgacctg      300
   gcacgcacac ccagccgata tggacctctg tccaacgggc tgttcctgat tacagatccc      360
   gagtggctga cttttaatcc tctgagcgca gtggagatcg ccgacctgga cgatcaggaa      420
   attcgggatt tcctggccca cgctaccatc gtgctggaca acgtcatggc cgatgtggtc      480
   atccagcaga gagagattga agacctgacc aagacaaacc tgagactgag gaatgcactg      540
   gccagctccg gcacatacca gccaatcggg atgcccattg cacagtctag tcctgccggg      600
   ccaagtgctt cagtggcagg accccatatg cctgcaatgc cttccgtccc ctatatcagt      660
   gccattccct cagccccccc tgctcccatc cctgtggcac ctgccgctgc accaccactg      720
   ggaccactgc catctgctcc agcacctcca gtgctgcctg tcatccagcc aaacacattc      780
   ggcactgatg ggaatggata cactgcagtg ggacctgtgc cagtcctgcc tatcgctcag      840
   attaaggcag tcatcggaga ggcaccaacc gacgctcgac agattcccct gtgggtggcc      900
   aaacacgccg ctgcaatcga aggaaccttt cctacaggca gcgctgatgt gcgatgccgg      960
   gtgctgaacg cactgctgac ctcacatggc gggatgacac tgagcccaaa tgagtgtgga     1020
   acatggagcc tggccgctgc agccctgtac cagcggatct atggcgtgat tccactgcac     1080
   gacctgcccc atactatggc agaggtggcc cggagagaag gaatcctggt cgcctttaac     1140
   atgggcatga ctttcaccaa caattctttt gacgtggtct gggggatcat tagacctctg     1200
   ctgccaggac aggctagtgt ggcaatgctg cagggctacc tggatcagta tcaggggcag     1260
   cagcagaaag cacaggcttt ccccaccctg ctgaggcgaa cttttgagac cctggggctg     1320
   aactatctgg gacagtcaat ccggagcaat cagccagcta gaacaagcgg gtccgtggca     1380
   ggcagcatta ctcagggacg ggggagagga cagccaaggt ctcgcccccc taaccgagca     1440
   tccaatagat ctaacaatcc accccgccct ccaccacctc gatctcagga acagcagccc     1500
   agtcctgcag gaagggggtc aagctcctct agtgctccac cccagaggtc cggctacaac     1560
   ctgcgccagc agatcaatag accacagagg ctgggaggag gaccaaaccc atatcgcaat     1620
   ctggagaaca ggaatattcc tcgcagtgaa accgtgccat caagccgacg ggccgagaca     1680
   tcagaaacta acaggccaaa tcaggctcgc ggaggacagc agggctcctc taaccgaaat     1740
   cagacccgga gcatcaacac cgtgcgggcc tctgatagtc aggtcattcc ttccgcccct     1800
   ccagctgaga caaacccagc tgcaaccgct gtgacaccca acggacctaa tcagcattct     1860
   ggcagaaatt gacaacattc cggtagaaat taa                                   1893
<210> 164
   <211> 572
   <212> PRT
   <213> Simian foamy virus
<400> 164
   Met Ala Arg Gly Asn Leu Asp Ile Ala Arg Leu Thr Val Leu Leu Gln
   1               5                   10                  15
   Glu Asn Ser Leu Ser Gly Asn Pro Pro His Leu Thr His Tyr Val Leu
               20                  25                  30
   Arg Met Thr Glu Gly Trp Trp Gly Pro Phe Pro Arg Tyr Thr Arg Val
           35                  40                  45
   Arg Ile Ile Leu Gln Asp Glu Ser Gly Asn Pro Leu Ser Gln Pro Arg
       50                  55                  60
   Trp Glu Leu Val Asp Arg Met Phe Asn Pro Leu Arg Asp Pro Ile Leu
   65                  70                  75                  80
   Glu Thr Thr Leu Asp Glu Met Asp Arg Val Phe Asp Gly Ile Asn Leu
                   85                  90                  95
   Ser Pro Gly Thr Glu Arg Tyr Gly Pro Leu Cys Asp Gly Asn Phe Leu
               100                 105                 110
   Tyr Thr Asp Asp Ala Trp Asn Asp Phe Asn Pro Met Ser Ala Leu Glu
           115                 120                 125
   Val Ala Asp Val Glu Leu Thr Pro Ala Leu Tyr Arg Glu His Leu Asn
       130                 135                 140
   Phe Leu Ser Arg Val Ala Glu Gly Met Leu Gly Asp Ile Ile Met Leu
   145                 150                 155                 160
   Lys Gln Glu Asn Glu Asp Ala His Glu Thr Ile Thr Arg Leu Arg Glu
                   165                 170                 175
   Arg Ile Ile Asn Gln Pro Thr Ser Ala Pro Ile Ile Thr Ser Thr Pro
               180                 185                 190
   Gly Gly Val Ser Gly Leu Pro Pro Ser Val Tyr Gln Pro Thr Gly Leu
           195                 200                 205
   His Glu Thr Tyr Ile Gln Pro Ser Arg Pro Met Gln Pro Ser Ala Pro
       210                 215                 220
   Pro Leu Pro Leu Met Gly Asp Leu Gly Ser Gly Ile Leu Ser Val Leu
   225                 230                 235                 240
   Pro Ile Ser Gln Ile Arg Thr Val Ile Gly Asn Thr Pro Val Asp Pro
                   245                 250                 255
   Lys Lys Val Pro Leu Trp Ile Ala Lys Ser Ala Ser Ala Ile Glu Gly
               260                 265                 270
   Val Met Pro Thr Asn Thr Pro Asp Ile Arg Cys Arg Leu Val Asn Ala
           275                 280                 285
   Leu Leu Pro Gln His Gly Gly Leu Ile Leu Gln Pro His Glu Cys Asn
       290                 295                 300
   Ser Trp Thr Gln Ile Ala Ser Ala Leu Tyr Thr Arg Val Asn Gly Met
   305                 310                 315                 320
   Ile Pro Leu His Ala Leu Pro Gln Thr Leu Ser Gln Val Thr Lys Glu
                   325                 330                 335
   Glu Gly Ile Leu Val Ala Tyr Gln Ile Gly Met Thr Phe Thr Gly Gln
               340                 345                 350
   Asn Phe Pro Leu Thr Trp Gly Ile Leu Arg Pro Leu Leu Pro Gly Gln
           355                 360                 365
   Ala Val Val Ala Met Met Gln Gly Tyr Leu Asp Gln Tyr Pro Thr Asp
       370                 375                 380
   Asp Leu Lys Ala Val Asn Phe Ala Ser Ile Leu Arg Arg Val Phe Asp
   385                 390                 395                 400
   Ile Leu Gly Leu Asn Tyr Met Gly Gln Asn Ile Arg Asn Gln Ser Ser
                   405                 410                 415
   Pro Ser Leu Thr Val Ser Ser Ala Arg Gly Ala Thr Gly Arg Gly Arg
               420                 425                 430
   Gly Arg Ser Arg Asn Ile Gly Arg Gly Arg Gly Thr Thr Pro Asn Phe
           435                 440                 445
   Gln Gly Arg Gly Gln Pro Ala Thr Thr Pro Thr Val Thr Thr Ser Gly
       450                 455                 460
   Ser Thr Ser Ser Thr Thr Glu Ser Gln Gly Arg Asn Ser Asn Pro Asn
   465                 470                 475                 480
   Tyr Asn Phe Arg Pro Arg Val Asn Gln Pro Pro Arg Tyr Gly Gly Gly
                   485                 490                 495
   Pro Asn Pro Tyr Arg Asn Arg Glu Pro Leu Asn Asn Pro Pro Asn Arg
               500                 505                 510
   Glu Asn Thr Thr Gln Asn Asn Thr Arg Arg Pro Gly Ser Gln Gly Gln
           515                 520                 525
   Asn Arg Asn Arg Asn Gln Asn Arg Thr Val Asn Ala Val Gln Val Val
   530 535 540
   Pro Asn Gln Asp Asn Thr Asn Gln Pro Asn Ile Asn Pro Asn Gln Asn
   545 550 555 560
   Val Ser Thr Pro Ala Ser Ala Ser Gly Gly Asn Ser
   565 570
<210> 165
   <211> 1719
   <212> DNA
   <213> Artificial Sequence
<220>
   <223> expression optimized polynoucleotide encoding for a simian foamy virus derived capsid protein
<400> 165
   atggcaagag ggaatctgga catcgcaagg ctgaccgtgc tgctgcagga aaacagtctg      60
   agcggcaacc caccacatct gacacattac gtgctgcgca tgactgaggg gtggtgggga     120
   ccattccccc gatatacccg ggtgcggatc attctgcagg acgagagcgg aaaccctctg     180
   agccagccac gatgggaact ggtggaccga atgttcaatc ccctgcggga ccccattctg     240
   gagaccacac tggacgaaat ggatagggtg tttgacggca tcaacctgtc tccagggaca     300
   gaacgctacg gacccctgtg cgatggcaat ttcctgtata ctgacgatgc ctggaacgac     360
   tttaatccca tgtctgctct ggaggtggca gatgtcgaac tgacccctgc actgtacaga     420
   gagcacctga actttctgag cagggtggcc gaaggcatgc tgggggacat cattatgctg     480
   aagcaggaga acgaagatgc ccatgagacc attacaagac tgagggaacg catcattaat     540
   cagccaacca gcgctcccat cattacttcc accccaggag gggtgtccgg gctgccacct     600
   tctgtctacc agcccactgg actgcacgag acctatatcc agccctctag gcctatgcag     660
   ccaagtgcac caccactgcc tctgatgggg gacctgggct ctgggattct gagtgtgctg     720
   ccaatcagcc agattcgcac agtcatcggc aacactcccg tggaccccaa gaaggtgccc     780
   ctgtggatcg caaaatccgc ctctgctatt gagggcgtga tgcctacaaa cactccagat     840
   atccgatgcc ggctggtcaa tgctctgctg cctcagcacg gaggcctgat cctgcagcca     900
   catgaatgta actcatggac acagattgca agcgccctgt acactcgggt gaatggaatg     960
   atccctctgc atgctctgcc acagaccctg agccaggtga caaaggagga aggcatcctg    1020
   gtcgcatatc agattgggat gaccttcaca ggacagaact ttcctctgac atggggaatc    1080
   ctgagaccac tgctgccagg acaggcagtg gtcgctatga tgcagggcta cctggaccag    1140
   tatccaaccg acgatctgaa agccgtgaac ttcgcttcca tcctgcggcg ggtgtttgat    1200
   attctgggcc tgaattacat ggggcagaac attcggaatc agagctcccc tagtctgacc    1260
   gtgtctagtg caagaggcgc aacaggaaga ggcagggggc gctcccgaaa catcggacgg    1320
   ggcagaggga ctacccccaa tttccaggga aggggacagc cagcaacaac tcctactgtg    1380
   accacaagtg gctcaacctc aagcactacc gagagccagg ggaggaactc aaatcccaac    1440
   tataatttta ggcctcgcgt gaaccagcct ccacgatacg gaggaggacc taacccatat    1500
   cgaaatcggg agcccctgaa caatccccct aaccgggaaa atacaactca gaacaatacc    1560
   aggcgccctg gaagccaggg ccagaacaga aataggaacc agaatagaac agtgaacgcc    1620
   gtccaggtgg tcccaaatca ggacaacact aatcagccaa acatcaatcc caaccagaat    1680
   gtgtccaccc ccgctagcgc atccggagga aattcttga                           1719
<210> 166
   <211> 544
   <212> PRT
   <213> Bovine foamy virus
<400> 166
   Met Ala Leu Asn Asp Phe Asp Pro Ile Ala Leu Gln Gly Tyr Leu Pro
   1               5                   10                  15
   Ala Pro Arg Val Leu Gln His Asn Asp Ile Ile Ile Cys Arg Ala Thr
               20                  25                  30
   Ser Gly Pro Trp Gly Ile Gly Asp Arg Tyr Asn Leu Ile Arg Ile His
           35                  40                  45
   Leu Gln Asp Pro Ala Gly Gln Pro Leu Pro Ile Pro Gln Trp Glu Pro
       50                  55                  60
   Ile Pro Asn Arg Thr Ala Asn Pro Arg Thr Gln Pro Tyr Pro Val Val
   65                  70                  75                  80
   Ser Ala Pro Met Ala Thr Leu Glu Asn Ile Leu Asn Asn Phe His Ile
                   85                  90                  95
   Pro His Gly Val Ser Arg Tyr Gly Pro Leu Glu Gly Gly Asp Tyr Gln
               100                 105                 110
   Pro Gly Glu Gln Tyr Ser Gln Gly Phe Cys Pro Val Thr Gln Ala Glu
           115                 120                 125
   Ile Ala Leu Leu Asn Gly Gln His Leu Glu Glu Glu Ile Thr Ile Leu
       130                 135                 140
   Arg Glu Ile Thr His Arg Leu Met Gln Gly Val Arg Pro Pro Ala Val
   145                 150                 155                 160
   Pro Gln Gly Pro Ala Pro Pro Pro Pro Pro Ala Gln Pro Pro Ala Pro
   165 170 175
   Leu Pro Ala Pro Pro Ile Gly Pro Pro Pro Pro Ala Ala Pro Ala Pro
   180 185 190
   Ala Pro Gly Pro Met Pro Val Pro Gln His Leu Pro Ile Thr His Ile
   195 200 205
   Arg Ala Val Ile Gly Glu Thr Pro Ala Asn Ile Arg Glu Val Pro Leu
   210 215 220
   Trp Leu Ala Arg Ala Val Pro Ala Leu Gln Gly Val Tyr Pro Val Gln
   225 230 235 240
   Asp Ala Val Met Arg Ser Arg Thr Val Asn Ala Leu Thr Val Arg His
   245 250 255
   Pro Gly Leu Ala Leu Glu Pro Leu Glu Cys Gly Ser Trp Gln Glu Cys
   260 265 270
   Leu Ala Ala Leu Trp Gln Arg Thr Phe Gly Ala Thr Ala Leu His Ala
   275 280 285
   Leu Gly Asp Thr Leu Gly Gln Ile Ala Asn Ser Asp Gly Ile Val Met
   290 295 300
   Ala Ile Glu Leu Gly Leu Leu Phe Ser Asp Asp Asn Trp Asp Leu Val
   305 310 315 320
   Trp Gly Ile Cys Arg Arg Phe Leu Pro Gly Gln Ala Val Cys Val Ala
   325 330 335
   Val Gln Ala Arg Leu Asp Pro Leu Pro Asp Asn Ala Thr Arg Ile Val
   340 345 350
   Met Ile Ser His Ile Ile Arg Asp Val Tyr Ala Ile Leu Gly Leu Asp
   355 360 365
   Pro Leu Gly Arg Pro Met Gln Gln Thr Leu Pro Arg Arg Asn Asn Gln
   370 375 380
   Pro Pro Arg Gln Gln Pro Gln Arg Arg Gln Gln Pro Arg Arg Thr Gly
   385 390 395 400
   Asn Gln Glu Glu Arg Gly Gln Arg Asn Arg Gly Arg Gln Asn Ala Gln
   405 410 415
   Thr Pro Arg Gln Glu Gly Asn Arg Leu Gln Asn Ser Gln Leu Pro Gly
   420 425 430
   Pro Arg Asp Cys Pro Asn Asn Ser Asn Gln Pro Arg Tyr Pro Leu Arg
   435 440 445
   Pro Asn Pro Gln Gln Pro Gln Arg Tyr Gly Gln Glu Gln Asn Arg Gly
   450 455 460
   Asn Asn Pro Asn Pro Tyr Arg Gln Pro Thr Pro Gly Asn Gly Asn Gln
   465 470 475 480
   Asn Arg Asn Phe Ser Arg Gly Pro Ala Pro Val Asn Glu Gln Ser Arg
   485 490 495
   Gly Arg Gly Arg Ser Ser Gln Gly Thr Asn Asn Thr Gly Ser Ser Ala
   500 505 510
   Val His Ser Val Arg Leu Thr Ser Ala Ala Pro Pro Ile Pro Pro Gln
   515 520 525
   Asp Ala Gly Thr Pro Pro Thr Ser Ser Gly Asn Gln Gly Gln Ser Ser
   530 535 540
<210> 167
   <211> 1635
   <212> DNA
   <213> Artificial Sequence
<220>
   <223> expression optimized polynoucleotide encoding for a bovine foamy virus derived capsid protein
<400> 167
   atggctctga acgactttga tccaatcgca ctgcagggct atctgccagc acctcgggtc        60
   ctgcagcaca acgacattat tatctgtagg gcaaccagcg gaccatgggg aatcggcgac       120
   cggtacaacc tgatcagaat tcacctgcag gaccccgcag gacagccact gcccatccct       180
   cagtgggaac caattcccaa caggaccgct aatcctcgca cacagcctta cccagtggtc       240
   tccgccccaa tggctaccct ggagaacatc ctgaacaatt tccacattcc tcatggcgtg       300
   tccagatatg gaccactgga aggaggggac taccagccag gagagcagta ttctcagggc       360
   ttttgccccg tgacacaggc agaaatcgcc ctgctgaatg gccagcacct ggaggaagag       420
   atcacaattc tgcgagagat tactcatcgg ctgatgcagg gggtcaggcc ccctgccgtg       480
   ccacagggac ccgctccacc ccctccaccc gcccagcctc cagctccact gcctgcacca       540
   cctattggac caccacctcc agcagctcca gcaccagctc ctggaccaat gcccgtccct       600
   cagcacctgc ccatcaccca tattcgcgca gtgatcggag aaacacccgc caacattagg       660
   gaggtccctc tgtggctggc acgcgccgtg ccagcactgc agggcgtcta tcccgtgcag       720
   gacgccgtga tgcgcagtcg aactgtcaat gctctgaccg tgcggcaccc aggcctggca       780
   ctggaaccac tggagtgcgg gagctggcag gaatgtctgg cagccctgtg gcagagaaca       840
   ttcggcgcta ctgcactgca tgccctgggg gacaccctgg gacagatcgc taactcagat       900
   ggcatcgtga tggcaattga gctggggctg ctgttcagcg acgataattg ggatctggtg       960
   tggggaattt gccggagatt tctgccagga caggcagtgt gtgtcgctgt gcaggcacgg      1020
   ctggacccac tgcccgataa cgccacacgg atcgtcatga tttctcacat cattagagac      1080
   gtgtacgcta tcctgggcct ggaccccctg ggaaggccta tgcagcagac cctgcctagg      1140
   cgcaacaatc agccacctcg acagcagcct cagcgacgac agcagccaag aaggactggc      1200
   aatcaggaag agagggggca gcggaacaga ggaaggcaga atgcccagac ccccagacag      1260
   gagggaaaca ggctgcagaa ttctcagctg cctggcccac gcgactgtcc aaacaatagt      1320
   aaccagccta gatacccact gaggcccaat cctcagcagc cccagcgcta tgggcaggaa      1380
   cagaaccgag gaaacaatcc aaatccctat cggcagccta ctccagggaa cggaaatcag      1440
   aaccgcaatt ttagccgagg gcccgcccct gtgaacgagc agtcccgcgg ccgagggcgg      1500
   agctcccagg gcaccaacaa tacagggtct agtgccgtcc atagtgtgag actgacatca      1560
   gctgcaccac ccatccctcc acaggatgct ggcactcccc ctacctcaag cggaaatcag      1620
   ggccagtcct cttga                                                       1635

## Description

The present invention relates to means and methods for pseudotyping of foamy viruses and the pharmaceutical application of pseudotyped foamy virus particles. The invention is applicable in biomedical research and in diagnostic and therapeutic applications.

Pseudotyping is used in the art for altering the host tropism or for generating an increased or decreased stability of a virus particle. Therefore, foreign viral envelope proteins (heterologous envelope proteins) are introduced into viral vectors. Viral envelope proteins are typically glycoproteins derived from portions of the membrane of the virus infected host cells or glycoproteins encoded by the virus genome.

Foamy viruses (spuma viruses) represent a special type of retroviruses that have been grouped into a separate retrovirus subfamily, the spumaretrovirinae. Foamy viruses (FV) exhibit specific features in their replication strategy that distinguishes the replication mechanism from other retroviruses. A unique feature in foamy virus replication is the requirement of a specific interaction between foamy virus glycoprotein (FV Env) and foamy virus capsid protein (FV Gag, the terms "Gag protein" and "capsid protein" are used interchangeably) for budding and release of virus-like-particles. This is due to the fact that FV Gag, opposed to other retroviral capsid proteins, lacks a membrane-targeting domain. For membrane-targeting of FV Gag, the interaction between the membrane-associated FV Env and the FV Gag, involving a N-terminal domain of FV Env leader peptide subunit and a coiled domain in the N-terminal region of FV Gag, is essential. For that reason, FV Gag is not able to mediate particle release in the absence of FV Env by itself and pseudotyping of foamy viruses by replacing FV Env by a heterologous envelope protein does not lead to successfully released virus particles.

Thus, for pseudotyping foamy virus particles, an interaction between FV Gag and the membrane has to be induced. In the art this is realized by permanently fusing a heterologous membrane-targeting domain that is applicable to mediate membrane interaction to the FV Gag protein in the form of a fusion protein. This system results in a permanent interaction between FV Gag and the membrane.

Eastman et al. discloses the analysis of the role of specific FV Gag domains for virus budding. It is described that the arginine functionality at position 50 of the FV Gag protein is essential for budding. Introducing a dysfunctional mutation at position 50 a functional membrane interaction resulting in virus budding can be restored by myristoylation of FV Gag. This was realized by Eastman et al. by replacing the 10 N-terminal amino acids of FV Gag by the Src plasma membrane-targeting signal that leads to myristoylation of FV Gag.

Zhadina et al. achieved an envelope-protein independent release of viral particles by providing fusion proteins of FV Gag with membrane-targeting signals, such as proto-oncogene tyrosine-protein kinase Fyn and protein-tyrosine kinase p56lck.

Swiersy (dissertation) discloses the role of FV Env in virus particle morphogenesis. An FV Env independent membrane-targeting is studied using fusion proteins, wherein different heterologous membrane-targeting signals are fused N-terminally to FV Gag. In contrast to Eastman et al. the membrane-targeting signals were fused to the intact N-terminus of FV Gag. This leads to efficient particle release in the absence of FV Env but rather poor infectious titers. It is disclosed that pseudotyping of FV particles using fusion proteins of FV Gag with a membrane-targeting signal is possible. However, infectious titers were significantly lower than that of native foamy virus particles.

It is the object of the invention to provide improved means and methods for pseudotyping foamy virus particles. It is another object of the invention to provide pseudotyped foamy virus particles with increased infectious titers compared to pseudotyped foamy virus particles known in the art.

The object is solved by the use of a first fusion protein and a second fusion protein for the generation of a pseudotyped foamy virus particle according to claim 1.

The first fusion protein comprises a foamy virus capsid (Gag) protein, preferably with intact C-terminus, and a first dimerization domain that is applicable to dimerize with a second dimerization domain from another protein upon binding of a small molecule ligand to both the first and the second dimerization domain (herein also referred to as "FV Gag-HDD").

The second fusion protein comprises a, preferably viral, envelope (Env) protein not derived from foamy virus and a second dimerization domain that is applicable to dimerize with the first dimerization domain of the first fusion protein upon binding of a small molecule ligand to both the first and the second dimerization domain. Fusion proteins used in the invention comprising an envelope protein and a dimerization domain are herein also referred to as "Env-HDD".

Alternatively the second fusion protein comprises a, preferably viral, membrane-targeting domain not derived from foamy virus, and a second dimerization domain that is applicable to dimerize with the first dimerization domain from the first fusion protein upon binding of a small molecule ligand to both the first and the second dimerization domain. Fusion proteins according used in the invention comprising a membrane-targeting domain and a dimerization domain are herein also referred to as "MT-HDD".

With the invention an inducible membrane interaction of FV Gag protein is assured in absence of FV Env. The membrane interaction of the recombinant FV Gag-HDD is induced by adding a small molecule ligand that causes heterodimerization of the dimerizable membrane associated protein Env-HDD and FV Gag-HDD or, alternatively, MT-HDD and FV Gag-HDD. Thereby the invention allows pseudotyping of foamy virus particles. The invention is specifically advantageous as the inventors have found out, that the interaction between FV Env and FV Gag is supposedly not permanent but rather transient. By adding a dimerizable small molecule, virus particle release can be induced at the optimum time point.

Another aspect of the invention is the use of a polynucleotide encoding for a FV Gag-HDD fusion protein that comprises a FV Gag protein, preferably with intact C-terminus, and a dimerization domain for the expression of a pseudotyped foamy virus particle according the invention. Another aspect of the invention is the use of a polynucleotide encoding for an Env-HDD fusion protein comprising a, preferably viral, not foamy virus derived envelope protein and a dimerization domain for the expression of a pseudotyped foamy virus particle according the invention. Another aspect of the invention is the use of a polynucleotide encoding for a MT-HDD fusion protein that comprises a, preferably viral, not foamy virus derived membrane-targeting domain and a dimerization domain for the expression of a pseudotyped foamy virus particle according the invention.

Another aspect of the invention is the use of a vector for the expression of a pseudotyped foamy virus particle according to the invention. The vector used in the invention comprises a polynucleotide encoding for a FV Gag-HDD fusion protein and/or an polynucleotide encoding for a, preferably viral, Env-HDD fusion protein or a, preferably viral, MT-HDD, fusion protein. In the case, that the vector comprises both, a polynucleotide encoding for a FV Gag-HDD fusion protein and another polynucleotide encoding for an Env-HDD fusion protein or a MT-HDD fusion protein, the dimerization domains are not identical and preferably are FK-HDD and FR-HDD, respectively.

Another aspect of the invention is a pseudotyped foamy virus particle according claim 1 comprising a heterologous envelope protein, comprising a first FV Gag-HDD fusion protein, preferably with intact C-terminus of FV Gag, and a second Env-HDD or MT-HDD fusion protein. Upon addition of a small molecule that is able to bind to both, the first and the second dimerization domain, the first and second fusion protein dimerize resulting in membrane targeting of the FV Gag protein.

A further aspect of the invention is the use of a complementation cell line comprising a vector as definded above for the expression of a pseudotyped foamy virus particle according to the invention.

An even further aspect of the invention is a method for the preparation of a pseudotyped foamy virus particle, comprising the steps of:
(i) culturing of a complementation cell line comprising an expression vector encoding for a first FV-Gag HDD fusion protein,
   - an expression vector encoding for a second Env-HDD fusion protein or
   - an expression vector encoding for a second MT-HDD fusion protein and an expression vector encoding for a heterologous envelope protein
   under conditions suitable to permit production of said pseudotyped foamy virus particle,
(ii) addition of an effective amount of a small molecule that is applicable to lead to dimerization between the first and the second fusion protein,
(iii) recovering said pseudotyped foamy virus particle from the cell culture.

The complementation cell line used in the invention comprises, preferably viral, packaging vectors and, optionally, transfer vectors. The packaging vectors include expression vectors as defined under (i) and optionally an expression vector encoding for foamy virus polymerase protein. Transfer vectors comprise a packageable polynucleotide, preferably a therapeutically applicable polynucleotide. Upon virus particle generation, the polynucleotide from the transfer vector is incorporated in the generated pseudotyped foamy virus particle. Transfer vectors are viral or non-viral vectors.

In a method according to the invention a dimerizing small molecule is contacted with the complementation cell line to induce dimerization of FV Gag-HDD with Env-HDD or MT-HDD. The small molecule is added to the cell culture medium preferably at the beginning of the culturing period. Alternatively the dimerizing small molecule is added to the cell culture medium at least 24 hours before harvesting the cells.

Another aspect of the invention is a vertebrate cell, preferably a mammalian cell, infected with a pseudotyped foamy virus particle according to the invention or with a pseudotyped foamy virus particle obtainable by a method according to the invention.

Another aspect of the invention is a pharmaceutical composition comprising an effective amount of a pseudotyped foamy virus particle according to the invention or with a pseudotyped foamy virus particle obtainable by a method according to the invention in combination with a pharmaceutically acceptable carrier.

Another aspect of the invention is the use of a pseudotyped foamy virus particle according to the invention or a pseudotyped foamy virus particle obtainable by a method according to the invention for preparing a medicinal product. A preferred pharmaceutical application is the transfer of genes, that are included in a pseudotyped foamy virus particle, to blood stem cells or neuronal cells. For gene transfer to neuronal cells, glycoprotein G of rabies virus is preferred as envelope protein for pseudotyping.

With respect to the invention the following definitions apply:
A protein is a macromolecule comprising one or more polypeptide domains folded into a characteristic globular or fibrous form and is characterized by its amino acid sequence. The term protein includes naturally occurring proteins (native proteins) or recombinant proteins, such as recombinant fusion proteins. For generation of recombinant fusion proteins (or simply fusion proteins as termed herein) a fusion gene is generated by fusing a gene sequence with another gene sequence after deletion of the stop codon. By translation of the fusion gene a fusion protein is formed. Fusion proteins comprise at least one first protein domain that is fused to another protein domain either at the N-terminal or C-terminal end of the first protein domain. Alternatively the gene of another protein domain can also be located inside the gene encoding for a first fusion protein (internal fusion). However internal fusion is carried out only if the functionality of the first fusion protein is comparable to the individual first protein domain. The at least two domains are preferably linked by a flexible linker peptide, like a glycine serine linker (G₄S)₃. The protein domains comprised in a fusion protein exhibit a comparable functionality than the respective individual domains.

In a fusion protein used in the invention, at least one protein domain (a FV Gag protein, an envelope protein or a membrane targeting domain) is fused with a dimerizable domain. A dimerizable domain is a polypeptide domain, preferably comprising 50 to 300 amino acids. The dimerizable domain of a fusion protein used in the invention is selected such that it is applicable for small molecule dependent dimerization with a second (non-identical) dimerization domain, thereby forming a heterodimer. Therefore, a small molecule binds to the first dimerization domain and with another epitope to a second dimerization domain. By this, two or more fusion proteins each comprising a first or a second dimerization domain heterodimerize upon addition of an effective amount of the dimerizing small molecule.

Small molecules are low molecular weight organic compounds that are not polymers. Small molecules as defined herein bind with high affinity to at least two different protein domains, such as dimerization domains (therefore it is herein also referred to a "dimerizing" small molecule). The upper molecular weight limit of a small molecule is 800 Daltons. An effective amount of a dimerizing small molecule is an amount sufficient to induce heterodimerization of two fusion proteins comprising different dimerization domains that heterodimerize upon addition of the small molecule in a physiological environment.

A polynucleotide as referred to herein means single-stranded or double- stranded nucleic acid polymers of at least 10 nucleotides in length. In certain embodiments, the nucleotides comprised in the polynucleotide are ribonucleotides, deoxyribonucleotides or a modified form of either type of nucleotide. Said modifications include base modifications such as bromuridine, ribose modifications such as arabinoside and 2', 3'-dideoxyribose and internucleotide linkage modifications such as phosphorothioate, phosphorodithioate, phosphoroselenoate, phosphorodiselenoate, phosphoroanilothioate, phoshoraniladate and phosphoroamidate.

The term vector includes a nucleic acid molecule capable of transporting another nucleic acid to which it has been linked. Suitable vectors are a plasmid, which refers to a circular double stranded DNA loop into which additional DNA segments may be ligated or a viral vector, such as replication defective retroviruses, adenoviruses and adeno-associated viruses, preferably retroviral vectors, wherein additional DNA segments may be ligated into the viral genome. Certain vectors are capable of directing the expression of genes to which they are operatively linked. Such vectors are referred to herein as expression vectors which is herein used interchangeably with the term vector.

A pseudotyped virus particle is a virus particle comprising a heterologous envelope protein and not comprising its original envelope protein. A pseudotyped virus particle can be generated by a complementation cell line comprising polynucleotides sufficient for packaging of the pseudotyped virus particle. Therefore the complementation cell line includes a least a viral gag and env gene whereby the env gene is a heterologous gene and optionally a viral pol gene. The genetic information necessary for forming the pseudotyped virus particle is introduced into a, preferably eukaryotic, host cell line to form a complementation cell line by transient or stable transfection. Preferred host cell lines are vertebrate cells, preferably mammalian cells, more preferably HEK293 cells. If cultured *in vitro*, the cells are grown and maintained at an appropriate temperature and gas mixture, typically being 37 °C, 5 % CO₂, in a cell culture medium.

The term "vertebrate cells" as used herein comprises differentiated cells and pluripotent cells, including induced pluripotent cells and stem cells. The term vertebrate cells does not comprise human embryonic stem cells that are obtained by the destruction of a human embryo. A vertebrate cell to be infected with a pseudotyped foamy virus particle according to the invention are either derived from a, preferably human, subject (primary cell) or cells from a vertebrate cell line (preferably a mammalian cell line).

A pharmaceutical composition comprises a, preferably therapeutically effective, amount of a pseudotyped foamy virus particle according to the invention. The pharmaceutical compositions according to the invention include various dosage forms and are preferred for oral or parenteral use, particularly preferably suitable for intravenous administration. Preferably, the parenteral pharmaceutical composition is in a form, which is suitable for injection. Particularly preferred pharmaceutical compositions are solutions, emulsions or suspensions comprising the pseudotyped foamy virus particle in a pharmaceutically acceptable carrier. Pharmaceutically acceptable carriers are preferably sterile liquids, especially water, buffered water, 0.4% saline, 0.3% glycine and the like. The pharmaceutical compositions may be sterilized by conventional, well known techniques. The compositions preferably contain pharmaceutically acceptable auxiliary substances, such as those that are required to assure physiological conditions and/or that increase the stability of the contained pseudotyped foamy virus particles. Preferred auxiliary substances are agents for adjusting the pH and buffering agents, preferably selected from sodium acetate, sodium chloride, potassium chloride, calcium chloride and sodium lactate.

The following embodiments are preferred for the invention:
Preferably the dimerization domain of a fusion protein used in the invention comprises an amino acid sequence with at least 80%, preferably at least 90%, particularly preferred at least 95%, particularly preferred at least 99%, amino acid sequence identity to the amino acid sequence presented in SEQ ID No. 1 (herein also referred to as "FK-HDD") or SEQ ID No. 3 (herein also referred to as "FR-HDD").

A polynucleotide encoding for FK-HDD preferably comprises a nucleic acid sequence with at least 80%, preferably at least 90%, particularly preferred at least 95%, particularly preferred at least 99%, nucleic acid sequence identity to the nucleic acid sequence presented in SEQ ID No. 2. A polynucleotide encoding for FR-HDD preferably comprises a nucleic acid sequence with at least 80%, preferably at least 90%, particularly preferred at least 95%, particularly preferred at least 99%, nucleic acid sequence identity to the nucleic acid sequence presented in SEQ ID No. 4.

The dimerization domains FK-HDD and FR-HDD are able to dimerize upon addition of a small molecule, namely rapamycin or its analogues including rapalog. Therefore, preferred small molecules used for inducing dimerization are rapamycin and its analogues, preferably rapalog. A particularly preferred rapamycin analogue used in the invention is AP21967 that is depicted in the following formula (1):

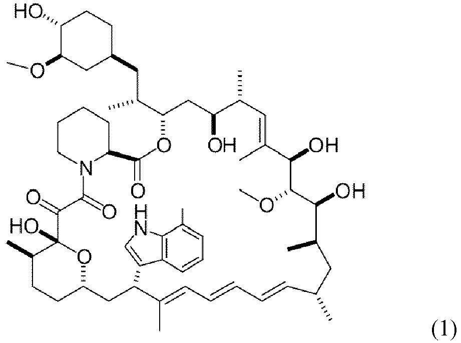

Preferably, the FV Gag protein included in a fusion protein used in the invention is selected from human FV (also called prototype FV, "PFV") Gag proteins, simian FV (SFV) Gag proteins, feline FV (FFV) Gag proteins, bovine FV (BFV) Gag proteins and equine FV (EFV) Gag proteins. Preferred SFV Gag proteins are derived from chimpanzee SFV (SFVcpz), African green monkey SFV (SFVagm), macaque SFV (SFVmac), Gorilla SFV (SFVgor) or Orangutan SFV (SFVora), Spider monkey SFV (SFVspm), Common marmoset SFV (SFVmar), Squirrel monkey SFV (SFVsqu).

Preferably, the FV Gag protein included in a fusion protein used in the invention exhibits at least 80 %, preferably at least 90 %, preferably at least 95 % amino acid sequence identity to one of the amino acid sequences listed in the following table 1. A nucleic acid encoding for the respective preferred FV Gag protein included in a fusion protein used in the invention exhibits at least 80 %, preferably at least 90 %, preferably at least 95 % nucleic acid sequence identity to one of the nucleic acid sequences listed in the following table 1:

**Table 1:**

| **FV Gag protein** | **Amino acid sequence** | **Nucleic acid sequence** |
|---|---|---|
| SFVmac Gag | SEQ ID No. 17 | SEQ ID No. 18 |
| expression optimized SFVmac Gag | SEQ ID No. 152 | SEQ ID No. 153 |
| SFVagm Gag | SEQ ID No. 19 | SEQ ID No. 20 |
| expression optimized SFVagm Gag | SEQ ID No. 154 | SEQ ID No. 155 |
| SFVcpz Gag | SEQ ID No. 21 | SEQ ID No. 22 |
| SFVgor Gag | SEQ ID No. 23 | SEQ ID No. 24 |
| expression optimized SFVgor Gag | SEQ ID No. 156 | SEQ ID No. 157 |
| SFVora Gag | SEQ ID No. 25 | SEQ ID No. 26 |
| expression optimized SFVora Gag | SEQ ID No. 158 | SEQ ID No. 159 |
| SFVmar Gag | SEQ ID No. 27 | SEQ ID No. 28 |
| expression optimized SFVmar Gag | SEQ ID No. 160 | SEQ ID No. 161 |
| SFVspm Gag | SEQ ID No. 29 | SEQ ID No. 30 |
| expression optimized SFVspm Gag | SEQ ID No. 162 | SEQ ID No. 163 |
| SFVsqu Gag | SEQ ID No. 31 | SEQ ID No. 32 |
| expression optimized SFVsqu Gag | SEQ ID No. 164 | SEQ ID No. 165 |
| BFV Gag | SEQ ID No. 33 | SEQ ID No. 34 |
| expression optimized BFV Gag | SEQ ID No. 166 | SEQ ID No. 167 |
| EFV Gag | SEQ ID No. 35 | SEQ ID No. 36 |
| FFV Gag | SEQ ID No. 37 | SEQ ID No. 38 |
| PFV Gag | SEQ ID No. 5 | SEQ ID No. 6 |
| expression optimized PFV Gag | SEQ ID No. 7 | SEQ ID No. 8 |

A particularly preferred FV Gag protein is human FV Gag protein (PFV). Preferably PFV Gag used herein comprises an amino acid sequence with at least 80%, preferably at least 90%, particularly preferred at least 95%, particularly preferred at least 99%, amino acid sequence identity to the amino acid sequence presented in SEQ ID No. 5 (herein also referred to as "native PFV Gag") or SEQ ID No. 7 (herein also referred to as "expression optimized PFV Gag" or "ePFV Gag").

A preferred FV Gag-HDD fusion protein used in the invention comprises an amino acid sequence with at least 80%, preferably at least 90%, particularly preferred at least 95%, particularly preferred at least 99%, amino acid sequence identity to the amino acid sequence presented in one of the following SEQ ID No.:
- SEQ ID. No. 9: This fusion protein comprises expression optimized PFV Gag and FK-HDD fused N-terminally to PFV Gag via a glycine-serine linker peptide (herein "ePFV Gag NFK"). A nucleic acid encoding for the fusion protein according to SEQ ID No. 9 exhibits a nucleic acid sequence according to SEQ ID No. 10.
- SEQ ID No. 11: This fusion protein comprises expression optimized PFV Gag and FR-HDD fused N-terminally to PFV Gag via a glycine-serine linker peptide (herein "ePFV Gag NFR"). A nucleic acid encoding for the fusion protein according to SEQ ID No. 11 exhibits a nucleic acid sequence according to SEQ ID No. 12.
- SEQ ID No. 13: This fusion protein comprises expression optimized PFV Gag and FK-HDD fused C-terminally to PFV Gag via a glycine-serine linker peptide (herein "ePFV Gag CFK"). A nucleic acid encoding for the fusion protein according to SEQ ID No. 13 exhibits a nucleic acid sequence according to SEQ ID No. 14.
- SEQ ID No. 15: This fusion protein comprises expression optimized PFV Gag and FR-HDD fused C-terminally to PFV Gag via a glycine-serine linker peptide (herein "ePFV Gag CFR"). A nucleic acid encoding for the fusion protein according to SEQ ID No. 15 exhibits a nucleic acid sequence according to SEQ ID No. 16.

Particularly preferred nucleic acids used in the invention exhibit at least 80%, preferably at least 90%, particularly preferred at least 95%, particularly preferred at least 99%, nucleic acid sequence identity to one of the nucleic acid sequences presented in SEQ ID No. 10, SEQ ID No. 12, SEQ ID No. 14 or SEQ ID No. 16.

Preferred envelope proteins included in an Env-HDD fusion protein used in the invention are viral envelope proteins. Particularly preferred viral envelope proteins are selected from glycoprotein G of vesicular stomatitis virus (VSV-G); glycoprotein G of rabies virus (rabies G); glycoprotein G of Mokola virus (Mokola G); glycoproteins of murine leukemia virus with ecotropic (MLVeco Env), amphotropic (MLVampho Env), polytropic (MLVpoly Env) or xenotropic host range (MLVxeno Env); glycoprotein of human immunodeficiency viruses type 1 (HIV-1 Env) and type 2 (HIV-2 Env); glycoprotein of simian immunodeficiency virus (SIV Env); glycoprotein of human T-cell leukemia viruses type 1 (HTLV-1 Env), type 2 (HTLV-2 Env) and type 4 (HTLV-4 Env); glycoprotein of endogenous feline virus RD114 (RD114); glycoprotein of gibbon ape leukemia virus (GALV Env); glycoproteins H and F of measles virus; glycoproteins H and F of Nipah virus; hemagglutinin and neuraminidase of influenza A or B; large-, middle- or small S-antigens of hepatitis B virus (HBV LS, HBV MS, HBV SS); E1 and E2 proteins of hepatitis C virus (HCV E1, HCV E2); glycoprotein of lymphocytic choriomeningitis virus (LCMV-G Arm); glycoprotein of Lassa virus (Lassa GP); glycoproteins HN (Sendai HN) and F of Sendai virus (Sendai F), glycoprotein of Ebola virus (Ebola GP); glycoprotein of Marburg virus (Marburg GP).

Aforementioned envelope proteins are also preferred for pseudotyping a foamy virus particle in case a MT-HDD fusion protein is used.

Preferably, the viral envelope protein used in the invention exhibits at least 80 %, preferably at least 90 %, preferably at least 95 % amino acid sequence identity to one of the amino acid sequences listed in the following table 2. A nucleic acid encoding for the respective preferred viral envelope proteins used in the invention exhibits at least 80 %, preferably at least 90 %, preferably at least 95 % nucleic acid sequence identity to one of the nucleic acid sequences listed in the following table 2:

**Table 2:**

| **viral envelope protein** | **Amino acid sequence** | **Nucleic acid sequence** |
|---|---|---|
| VSV-G | SEQ ID No. 39 | SEQ ID No. 40 |
| rabies G | SEQ ID No. 41 | SEQ ID No. 42 |
| Mokola G | SEQ ID No. 43 | SEQ ID No. 44 |
| MLVeco Env | SEQ ID No. 45 | SEQ ID No. 46 |
| MLVampho Env | SEQ ID No. 47 | SEQ ID No. 48 |
| MLVpoly Env | SEQ ID No. 49 | SEQ ID No. 50 |
| MLVxeno Env | SEQ ID No. 51 | SEQ ID No. 52 |
| HIV-1 Env | SEQ ID No. 53 | SEQ ID No. 54 |
| HIV-2 Env | SEQ ID No. 55 | SEQ ID No. 56 |
| SIV Env | SEQ ID No. 57 | SEQ ID No. 58 |
| HTLV-1 Env | SEQ ID No. 59 | SEQ ID No. 60 |
| HTLV-2 Env | SEQ ID No. 61 | SEQ ID No. 62 |
| HTLV-4 Env | SEQ ID No. 63 | SEQ ID No. 64 |
| RD114 | SEQ ID No. 65 | SEQ ID No. 66 |
| GALV Env | SEQ ID No. 67 | SEQ ID No. 68 |
| measles virus F protein | SEQ ID No. 69 | SEQ ID No. 70 |
| measles virus H protein | SEQ ID No. 71 | SEQ ID No. 72 |
| Nipah virus F protein | SEQ ID No. 73 | SEQ ID No. 74 |
| Nipah virus H protein | SEQ ID No. 75 | SEQ ID No. 76 |
| hemagglutinin of influenza A | SEQ ID No. 77 | SEQ ID No. 78 |
| neuraminidase of influenza A | SEQ ID No. 79 | SEQ ID No. 80 |
| HBV LS | SEQ ID No. 81 | SEQ ID No. 82 |
| HBV MS | SEQ ID No. 83 | SEQ ID No. 84 |
| HBV SS | SEQ ID No. 85 | SEQ ID No. 86 |
| HCV E1 | SEQ ID No. 87 | SEQ ID No. 88 |
| HCV E2 | SEQ ID No. 89 | SEQ ID No. 90 |
| LCMV-G Arm | SEQ ID No. 91 | SEQ ID No. 92 |
| Lassa GP | SEQ ID No. 136 | SEQ ID No. 137 |
| Sendai HN | SEQ ID No. 138 | SEQ ID No. 139 |
| Sendai F | SEQ ID No. 140 | SEQ ID No. 141 |
| Ebola GP | SEQ ID No. 142 | SEQ ID No. 143 |
| Marburg GP | SEQ ID No. 144 | SEQ ID No. 145 |

A particularly preferred envelope protein is glycoprotein G of vesicular stomatitis virus (herein also referred to as "VSV-G" as identified above). A preferred fusion protein comprising VSV-G and a dimerization domain that is applicable to dimerize upon addition of rapamycin or its analogues comprises an amino acid sequence with at least 80%, preferably at least 90%, particularly preferred at least 95%, particularly preferred at least 99%, amino acid sequence identity to the amino acid sequence presented in one of the following SEQ ID No.:
- SEQ ID. No. 93: This fusion protein comprises VSV-G and FK-HDD fused N-terminally to VSV-G via a glycine-serine linker peptide (herein "VSV-G NFK"). A nucleic acid encoding for the fusion protein according to SEQ ID No. 93 exhibits a nucleic acid sequence according to SEQ ID No. 94.
- SEQ ID No. 95: This fusion protein comprises VSV-G and FR-HDD fused N-terminally to VSV-G via a glycine-serine linker peptide (herein "VSV-G NFR"). A nucleic acid encoding for the fusion protein according to SEQ ID No. 95 exhibits a nucleic acid sequence according to SEQ ID No. 96.
- SEQ ID No. 97: This fusion protein comprises VSV-G and FK-HDD fused C-terminally to VSV-G via a glycine-serine linker peptide (herein "VSV-G CFK"). A nucleic acid encoding for the fusion protein according to SEQ ID No. 97 exhibits a nucleic acid sequence according to SEQ ID No. 98.
- SEQ ID No. 99: This fusion protein comprises VSV-G and FR-HDD fused C-terminally to VSV-G via a glycine-serine linker peptide (herein "VSV-G CFR"). A nucleic acid encoding for the fusion protein according to SEQ ID No. 99 exhibits a nucleic acid sequence according to SEQ ID No. 100.

In an Env-HDD fusion protein used in the invention with a viral envelope protein, which comprises a signal peptide that is cleaved cotranslationally and is not a potential component of the glycoprotein complex, the dimerization domain is preferably fused to the C-terminal end of the envelope protein. In an alternative Env-HDD fusion protein used in the invention the dimerization domain is fused with the viral envelope protein by internal fusion, thereby being located inside the envelope protein. In this alternative the dimerization domain is preferably localized in the cytoplasmic domain of the envelope protein.

Alternatively preferred envelope proteins are non-viral glycoproteins. Particularly preferred non-viral glycoproteins are signaling receptors, preferably a G-protein coupled receptor (GPCR) (for example Beta-2 adrenergic receptor, SEQ ID No. 146); immunomodulatory membrane proteins, preferably an integrin (for example human intercellular adhesion molecule 1, SEQ ID No. 147) or a T- or B-cell costimulatory protein (for example human CD28, SEQ ID No. 148 and human CD80, SEQ ID No. 149); antigen-presenting molecules, preferably a major histocompatibility antigen (for example human HLA class I histocompatibility antigen, SEQ ID No. 150).

Preferred viral membrane-targeting domains are selected from the Gag matrix subunits of HIV-1 (HIV-1 MA), HIV-2 (HIV-2 MA), HTLV-1 (HTLV-1 MA), HTLV-2 (HTLV-2 MA), HTLV-4 (HTLV-2 MA), SIV (SIV MA), murine leukemia virus (MLV MA), Mason-Pfizer monkey virus (MPMV MA), mouse mammary tumor virus (MMTV MA), avian leukosis virus (ALV MA), Jaagsiekte sheep retrovirus (JSRV MA) and Gibbon ape leukemia virus (GALV MA). An alternatively preferred viral membrane-targeting domain is the Avian sarcoma virus derived myristoylation signal sequence of proto-oncogene tyrosine-protein kinase Src (vSrcM).

Alternatively preferred non-viral membrane-targeting domains are selected from myristoylation signal sequences of proto-oncogene tyrosine-protein kinase Src (nvSrcM), proto-oncogene tyrosine-protein kinase Fyn (nvFynM), protein-tyrosine kinase p56lck (nvLckM).

Preferably, the membrane-targeting domain included in a fusion protein used in the invention exhibits at least 80 %, preferably at least 90 %, preferably at least 95 % amino acid sequence identity to one of the amino acid sequences listed in the following table 3. A nucleic acid encoding for the respective preferred membrane-targeting domain included in a fusion protein used in the invention exhibits at least 80 %, preferably at least 90 %, preferably at least 95 % nucleic acid sequence identity to one of the nucleic acid sequences listed in the following table 3:

**Table 3:**

| **membrane targeting domain** | | **Amino acid sequence** | **Nucleic acid sequence** |
|---|---|---|---|
| HIV-1 MA | viral | SEQ ID No. 101 | SEQ ID No. 102 |
| HIV-2 MA | viral | SEQ ID No. 103 | SEQ ID No. 104 |
| HTLV-1 MA | viral | SEQ ID No. 105 | SEQ ID No. 106 |
| HTLV-2 MA | viral | SEQ ID No. 107 | SEQ ID No. 108 |
| HTLV-4 MA | viral | SEQ ID No. 109 | SEQ ID No. 110 |
| SIV MA | viral | SEQ ID No. 111 | SEQ ID No. 112 |
| MLVMA | viral | SEQ ID No. 113 | SEQ ID No. 114 |
| MPMV MA | viral | SEQ ID No. 115 | SEQ ID No. 116 |
| ALV MA | viral | SEQ ID No. 117 | SEQ ID No. 118 |
| MMTV MA | viral | SEQ ID No. 119 | SEQ ID No. 120 |
| JSRV MA | viral | SEQ ID No. 121 | SEQ ID No. 122 |
| GALV MA | viral | SEQ ID No. 123 | SEQ ID No. 124 |
| vSrcM | viral | SEQ ID No. 151 | |
| nvSrcM | non-viral | SEQ ID No. 125 | |
| nvFynM | non-viral | SEQ ID No. 126 | |
| nvLckM | non-viral | SEQ ID No. 127 | |

A particularly preferred membrane-targeting domain is the Gag matrix subunit of HIV-1 (HIV-1 MA). A preferred fusion protein comprising HIV-1 MA and a dimerization domain that is applicable to dimerize upon addition of rapamycin or its analogues comprises an amino acid sequence with at least 80%, preferably at least 90%, particularly preferred at least 95%, particularly preferred at least 99%, amino acid sequence identity to the amino acid sequence presented in one of the following SEQ ID No.:
- SEQ ID No. 128: This fusion protein comprises HIV-1 MA and FK-HDD fused C-terminally to HIV-1 MA via a glycine-serine linker peptide (herein "HIV-1 MA CFK"). A nucleic acid encoding for the fusion protein according to SEQ ID No. 128 exhibits a nucleic acid sequence according to SEQ ID No. 129.
- SEQ ID No. 130: This fusion protein comprises HIV-1 MA and FR-HDD fused C-terminally to HIV-1 MA via a glycine-serine linker peptide (herein "HIV-1 MA CFR"). A nucleic acid encoding for the fusion protein according to SEQ ID No. 130 exhibits a nucleic acid sequence according to SEQ ID No. 131.

Another particularly preferred membrane-targeting domain is the myristoylation signal sequence of proto-oncogene tyrosine-protein kinase Src (SrcM). A preferred fusion protein comprising SrcM and a dimerization domain that is applicable to dimerize upon addition of rapamycin or its analogues comprises an amino acid sequence with at least 80%, preferably at least 90%, particularly preferred at least 95%, particularly preferred at least 99%, amino acid sequence identity to the amino acid sequence presented in one of the following SEQ ID No.:
- SEQ ID No. 132: This fusion protein comprises SrcM and FK-HDD fused C-terminally to SrcM via a glycine-serine linker peptide (herein "SrcM CFK"). A nucleic acid encoding for the fusion protein according to SEQ ID No. 132 exhibits a nucleic acid sequence according to SEQ ID No. 133.
- SEQ ID No. 134: This fusion protein comprises SrcM and FR-HDD fused C-terminally to SrcM via a glycine-serine linker peptide (herein "SrcM CFR"). A nucleic acid encoding for the fusion protein according to SEQ ID No. 134 exhibits a nucleic acid sequence according to SEQ ID No. 135.

### Description of the drawings

The invention is further illustrated by the following figures and examples without being limited to these.

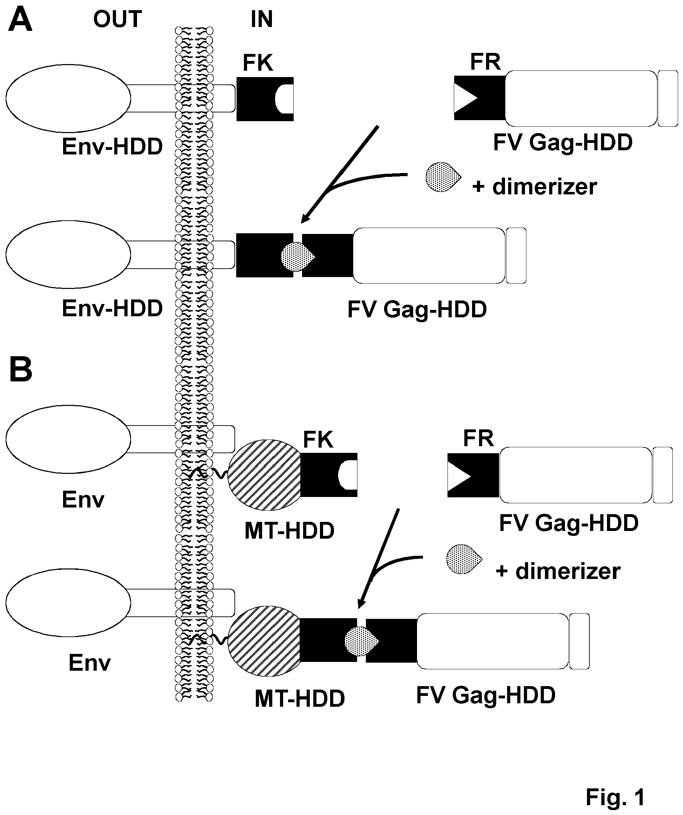

- Fig. 1: Schematic illustration of foamy virus pseudotyping strategies using dimerizable fusion proteins. A) Pseudotyping of foamy virus particles using FV Gag-HDD fusion proteins and Env-HDD fusion proteins; B) Pseudotyping of foamy virus particles using FV Gag-HDD fusion proteins and MT-HDD fusion proteins.

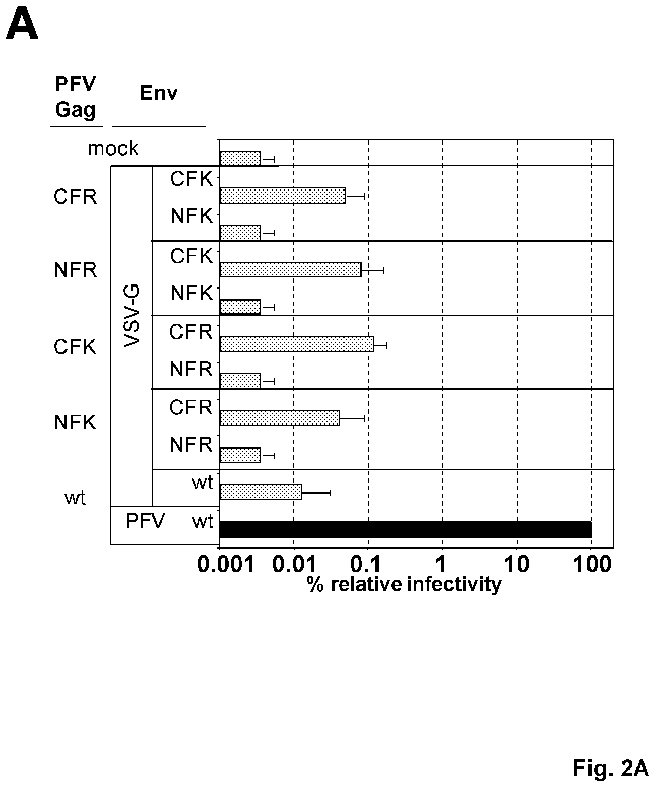

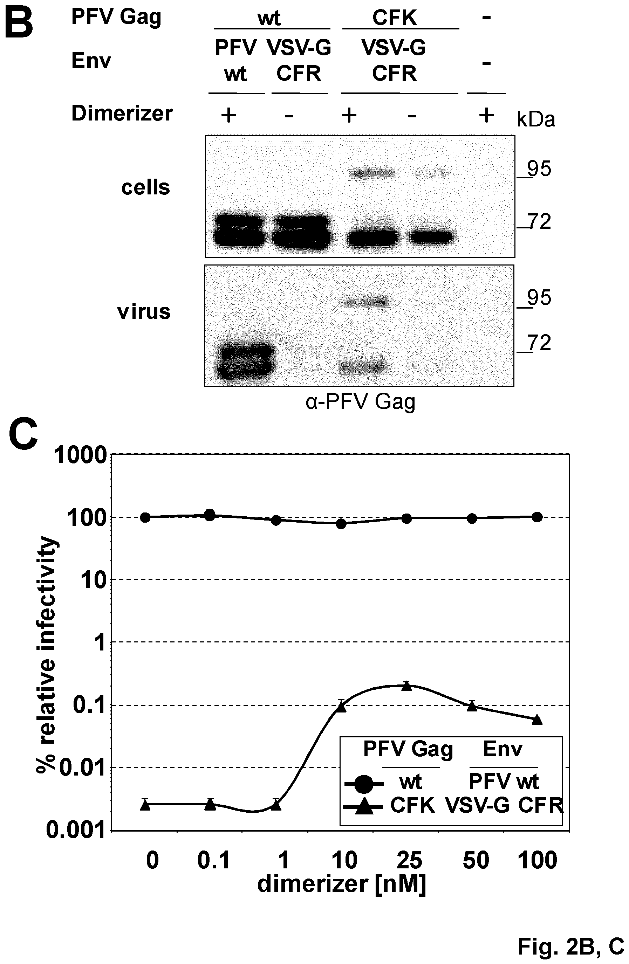

- Fig. 2: Pseudotyping of foamy virus particles using PFV Gag-HDD and Env-HDD with VSV-G as heterologous glycoprotein. A) Relative infectivity of pseudotyped foamy virus particles generated with different PFV Gag and Env packaging constructs in the presence of 10 nM dimerizer; B) Western blot analysis of dimerizer-dependent physical particle release of foamy virus particles pseudotyped with VSV-G using a polyclonal antiserum specific for PFV Gag (α-PFV Gag). (+) 10 nM dimerizer; (-) 0 nM dimerizer; C) Dimerizer dose-dependent production of infectious foamy virus particles pseudotyped with VSV-G.

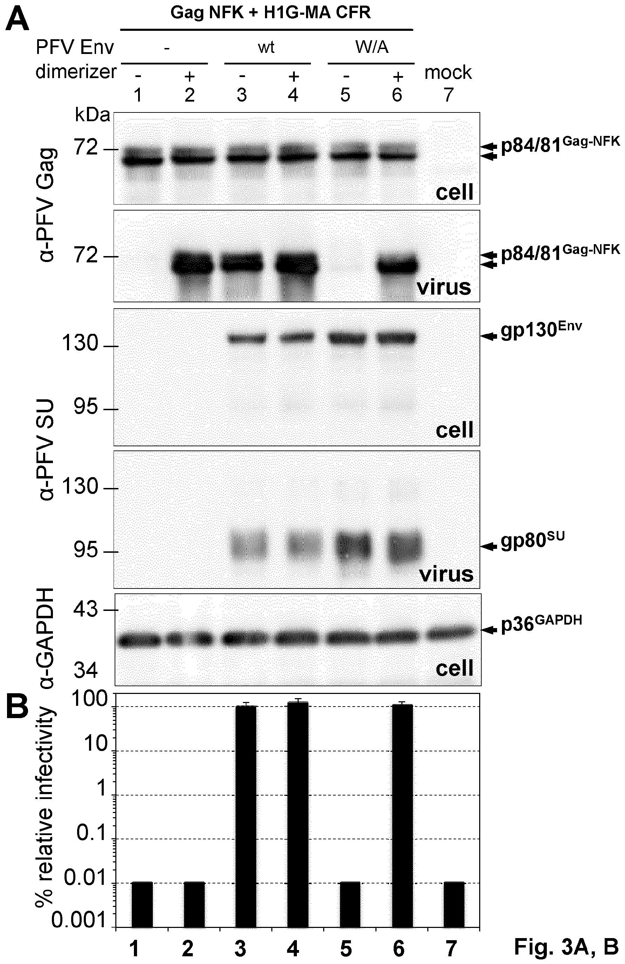

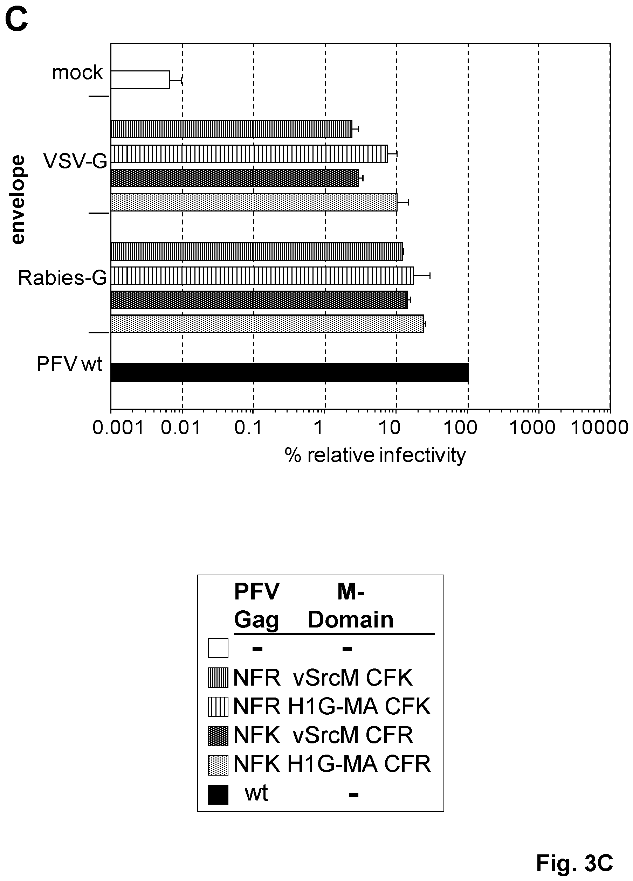

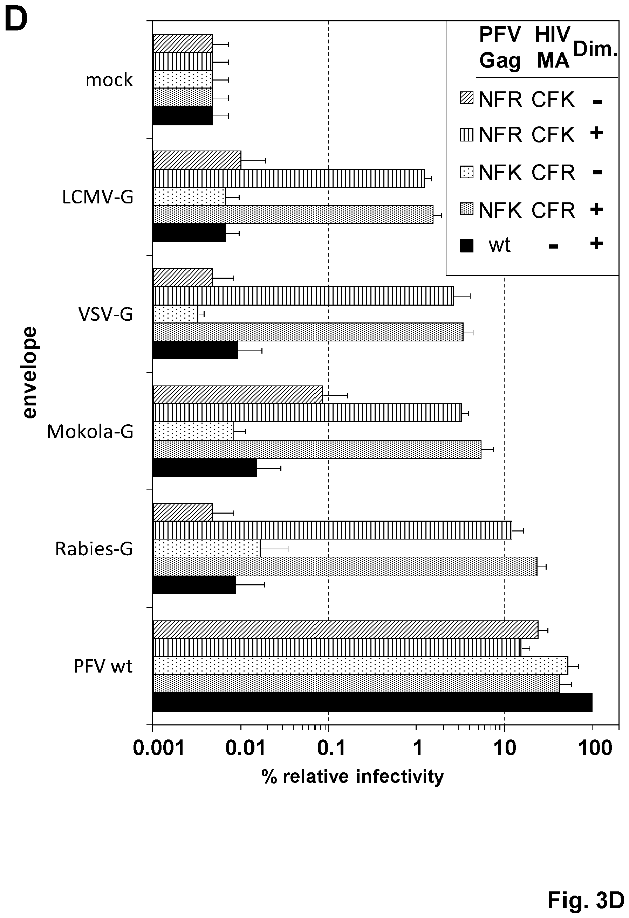

- Fig. 3: Pseudotyping of foamy virus particles using PFV Gag-HDD and different MT-HDD. A) Western blot analysis of HEK 293T cell lysates (cell) and viral particles (virus) of NFK-tagged PFV Gag (Gag NFK) and CFR-tagged HIV-1 Gag MA (H1G-MA CFR) in the absence (-) or presence (+) of 25 nM dimerizer. Detection was performed using anti-PFV Gag (α-PFV Gag), anti-PFV Env SU (α-PFV SU) specific antiserum or anti-GAPDH monoclonal antibody (α-GAPDH). B) Relative infectivity of the supernatants corresponding to A) determined on HT1080 target cells using a flow cytometric EGFP transfer assay. C+D) Relative infectivity of pseudotyped foamy virus particles generated with different PFV Gag-HDD fusion proteins and MT-HDD (HIV-1 Gag MA-HDD, SrcM-HDD) packaging constructs (1:1 ratio) and various Env expression vectors as indicated in the presence of 25 nM dimerizer.

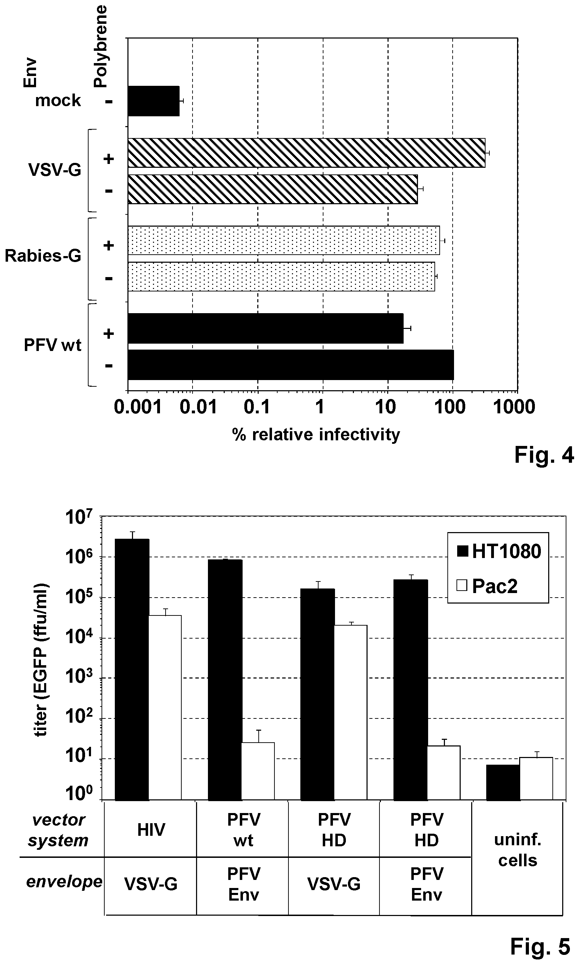

- Fig. 4: Relative infectivity of pseudotyped virus particles with different envelope proteins (VSV-G, Rabies G, wild type PFV Env (PFV wt) for comparison) using dimerizable PFV Gag and dimerizable HIV-1 MA at optimized packaging plasmid amount in the presence (+) or absence (-) of the cationic polymer Polybrene.
- Fig. 5: Susceptibility of zebrafish Pac2 cells towards transduction by different foamy virus vector pseudotypes.

Figure 1 illustrates the induction of a FV Env independent membrane interaction of FV Gag protein using dimerizable fusion proteins. The membrane interaction of FV Gag is required for the physical release of virus particles from the infected host cell. Figure 1A shows membrane interaction using a FV Gag-HDD protein in combination with a dimerizable heterologous envelope protein Env-HDD. Figure 1B illustrated membrane interaction using a FV Gag-HDD protein in combination with a dimerizable heterologous membrane targeting domain MT-HDD for pseudotyping of a foamy virus particle with a heterologous envelope protein (Env). The different heterodimerizable domains are indicated (FK and FR). In both systems, addition of a small molecule ligand (dimerizer) that induces dimerization of the heterodimerizable domains of the two respective fusion proteins leads to heterodimerization of these fusion proteins and thereby to a membrane targeting of FV Gag.

### Example 1: Pseudotyping of foamy virus particles using PFV Gag-HDD fusion proteins and Env-HDD with VSV-G as heterologous glycoprotein

Fusion proteins comprising PFV Gag and a first dimerization domain were prepared. Wild type expression optimized PFV Gag with the amino acid sequence according to SEQ ID No. 7 was used (encoding nucleic acid sequence is shown in SEQ ID No. 8). FK-HDD (SEQ ID No. 1) and FR-HDD (SEQ ID No. 3) as described above were used as dimerization domains. Dimerization was induced by addition of the rapamycin analogue AP21967 according to formula (1). Glycoprotein G of vesicular stomatitis virus (VSV-G) according to SEQ ID No. 39 (encoding nucleic acid sequence according to SEQ ID No. 40) was used as heterologous envelope protein for pseudotyping.

The amino acid sequences of the fusion proteins according to the invention and the encoding nucleic acid sequences are shown in table 4, wherein the terminus "ePFV Gag" refers to the expression optimized PVG Gag protein as described above:

**Table 4:**

| **fusion protein** | **abbreviation** | **Amino acid sequence** | **Nucleic acid sequence** |
|---|---|---|---|
| ePFV Gag with FK-HDD fused N-terminally | Gag NFK | SEQ ID No. 9 | SEQ ID No. 10 |
| ePFV Gag with FR-HDD fused N-terminally | Gag NFR | SEQ ID No. 11 | SEQ ID No. 12 |
| ePFV Gag with FK-HDD fused C-terminally | Gag CFK | SEQ ID No. 13 | SEQ ID No. 14 |
| ePFV Gag with FR-HDD fused C-terminally | Gag CFR | SEQ ID No. 15 | SEQ ID No. 16 |
| VSV-G with FK-HDD fused N-terminally | VSV-G NFK | SEQ ID No. 93 | SEQ ID No. 94 |
| VSV-G with FR-HDD fused N-terminally | VSV-G NFR | SEQ ID No. 95 | SEQ ID No. 96 |
| VSV-G with FK-HDD fused C-terminally | VSV-G CFK | SEQ ID No. 97 | SEQ ID No. 98 |
| VSV-G with FR-HDD fused C-terminally | VSV-G CFR | SEQ ID No. 99 | SEQ ID No. 100 |

HEK 293T cells were cotransfected with different Gag and Env packaging constructs in context of a 4-component replication-deficient PFV vector system comprising Gag, Env and Pol packaging constructs and an EGFP expressing transfer vector as described by Mannigel et al. (Mannigel 2007, specifically Fig. 1). Subsequently infectivity of supernatants was determined on HT1080 target cells using a flow cytometric EGFP transfer assay or protein composition of pelleted particles analyzed.

The relative infectivity of recombinant vector particles generated with different PFV Gag and Env packaging constructs was analyzed in the presence of 10 nM dimerizer (Fig. 2A). VSV-G proteins with N-terminal HDD fusion (VSV-G NFK and VSV-G NFR) were unable to support production of infectious pseudotyped PFV vector particles, most probably due to cotranslational cleavage of the VSV-G signal peptide thereby removing the HDD from the viral glycoprotein (Fig. 2A). In contrast, all VSV-G proteins with C-terminal fusion (VSV-G CFK and VSV-G CFR) yielded infectious pseudotyped PFV particles, with PFV Gag CFK and VSV-G CFR giving rise to the highest titers, although only minor differences were observed with the other combinations (Fig. 2A).

A Western blot analysis of small-molecule-dependent physical particle release of VSV-G containing heterodimerizer domains was carried out using a polyclonal antiserum specific for PFV Gag (α-PFV Gag) (Fig. 2B). The analysis was carried out in presence of 10 nM dimerizer (indicated by "+") and without dimerizer (indicated by "-"). Dimerizer dose-dependent production of infectious VSV-G pseudotyped PFV vector particles is shown in Fig. 2C. A dimerizer-dependent physical release of PFV capsids could be observed for the C-terminal HDD-tagged VSV-G glycoproteins (Fig. 2B). The optimal dimerizer concentration was around 25 nM (Fig. 2C).

### Example 2: Pseudotyping of foamy virus particles using PFV Gag-HDD fusion proteins and MT-HDD

Fusion proteins comprising PFV Gag and a first dimerization domain (Gag NFK and Gag NFR) were prepared as in example 1. FK-HDD and FR-HDD as described above were used as dimerization domains. Dimerization was induced by addition of the rapamycin analogue AP21967 according to formula (1). Either the Gag matrix (MA) subunit of HIV-1 (abbreviated herein "HIV-1 MA" or "H1G MA") or the myristoylation signal sequence of avian sarcoma virus oncogene tyrosine-protein kinase Src (vScrM) was used as membrane targeting domain to form a MT-HDD fusion protein according to the invention.

The amino acid sequences of the MT-HDD fusion proteins used in example 2 and the encoding nucleic acid sequences are shown in table 5:

**Table 5:**

| **fusion protein** | **abbreviation** | **Amino acid sequence** | **Nucleic acid sequence** |
|---|---|---|---|
| HIV-1 MA with FK-HDD fused C-terminally | HIV-1 MA CFK (also termed "H1G MA CFK") | SEQ ID No. 128 | SEQ ID No. 129 |
| HIV-1 MA with FR-HDD fused C-terminally | HIV-1 MA CFR (also termed "H1G MA CFR") | SEQ ID No. 130 | SEQ ID No. 131 |
| vSrcM with FK-HDD fused C-terminally | vSrcM CFK | SEQ ID No. 132 | SEQ ID No. 133 |
| vSrcM with FR-HDD fused C-terminally | vSrcM CFR | SEQ ID No. 134 | SEQ ID No. 135 |

HEK 293T cells were cotransfected with different PFV Gag, HIV-1 MA or vSrcM and different Env packaging constructs in context of a 4-component replication-deficient PFV vector system as indicated in example 1.

Results of the Western blot analysis of HEK 293T cell lysates (cell) and viral particles (virus) purified by ultracentrifugation through 20% sucrose of NFK-tagged PFV Gag (Gag NFK) and CFR-tagged HIV-1 Gag MA (H1G-MA CFR) in the absence (-) or presence (+) of 25 nM dimerizer are shown in Fig. 3A. Proteins were detected using an anti-PFV Gag (α-PFV Gag) or anti-PFV Env SU (α-PFV SU) specific antiserum, or an anti-GAPDH monoclonal antibody (α-GAPDH). Analysis of particle release in the absence of any viral glycoprotein using the combination of Gag NFK and H1G-MA CFR demonstrated that physical particle release was strictly dimerizer-dependent (Fig. 3A, lane 1, 2). This indicates that HDD-tagged PFV Gag and the membrane-targeting domain can interact in a dimerizer-dependent manner enabling a PFV Env-independent release of PFV virus like particles. Upon coexpression of the wildtype PFV Env protein (wt) particle release of infectious particles was dimerizer-independent, demonstrating that the natural PFV Gag - Env interaction is not disturbed by coexpression of an HDD-tagged membrane-targeting domain and its dimerizer-dependent interaction with the PFV capsid protein (Fig. 3A, lane 3, 4). Infectious PFV particles could be reconstituted upon coexpression of the untagged, naturally interaction-deficient PFV Env mutant (W/A; W₁₀A, W₁₃A mutation) with Gag NFK and H1G-MA CFR (Fig. 3A, lane 5, 6). Physical release of infectious particles was strictly dimerizer-dependent demonstrating that PFV Env located in the plasma membrane can be functionally incorporated into PFV particle that bud PFV Env-independently.

Relative infectivity of the pseudotyped virus particles with different envelope proteins (VSV-G, Rabies G, wild type PFV Env (PFV wt) for comparative analyses) using wildtype (wt) or dimerizable PFV Gag (Gag NFK or Gag NFR) and either dimerizable vSrcM (vSrcM CFR or vSrcM CFK) or dimerizable HIV-1 MA (H1G-MA CFR or H1G-MA CFK) was analyzed as described in example 1 (Fig. 3C).

Relative infectivity of the pseudotyped virus particles with different envelope proteins (LCMV-G, VSV-G, Mokola-G, Rabies G and wild type PFV Env for comparative analyses) using wildtype (wt) or dimerizable PFV Gag (Gag NFK or Gag NFR) and dimerizable HIV-1 MA (H1G-MA CFR or H1G-MA CFK) in the presence (+) or absence (-) of dimerizer (Dim.) was analyzed as described in example 1 (Fig. 3D).

All tested envelope proteins gave rise to infectious pseudotyped PFV particles in a dimerizer-dependent manner. The yielded titers were only 5-100 fold below those of standard PFV vectors.

Relative infectivity of the pseudotyped virus particles with different envelope proteins (VSV-G, Rabies G, wild type PFV Env (PFV wt) for comparative analyses) using dimerizable PFV Gag and dimerizable HIV-1 MA (H1G-MA) at optimized packaging plasmid amount in the presence (+) or absence (-) of the cationic polymer Polybrene was analyzed as described in example 1 (Fig. 4). It is demonstrated that for pseudotyped foamy viruses comparable infectious titers as wild type foamy virus are yielded. By adding Polybrene, infectious titers of the VSV-G pseudotyped foamy virus particles can be increased. Whereas Polybrene addition led to a 5-fold decrease of viral infectivity of authentic PFV vector particles, a 10-fold increase was observed for VSV-G pseudotypes and no major influence on Rabies-G pseudotyped particles.

### Example 3: Host range alteration by pseudotyping of foamy virus particles using PVG Gag-HDD fusion proteins and Env-HDD with VSV-G as heterologous glycoprotein

As a proof of principle that pseudotyping of foamy virus particles with the invention can alter the host range of PFV vector particles, pseudotypes with VSV-G as heterologous envelope protein were produced with fusion proteins as described in example 2 using PFV Gag NFK and HIV-1 MA CFR (H1G-MA CFR). VSV-G pseudotypes were examined on Pac2 cells, a fish cell line recently been demonstrated to be resistant to transduction by authentic PFV vector particles. The results are shown in Fig. 5. It is demonstrated that VSV-G pseudotyped PFV vectors are able to transduce Pac2 cells whereas Pac2 cells are refractory to PFV Env-mediated gene transfer (Fig. 5).

### Non patent literature

- Eastman and Linial, Journal of Virology 2001, Vol. 75 No. 15, p. 6857-6864
- Swiersky, Anka "Analyse von orthoretroviral-ähnlich exprimierten PFV Gag-Pol-Fusionsproteinen und N-terminal modifizierten PFV Gag Proteinen", Dissertation, Technische Universität Dresden, 2010
- Mannigel et al., Journal of Virology 2007, Vol. 81 No. 7, p. 3317-3326
- Zhadina et al., PNAS 2007, Vol. 104 No. 50, p. 20031-20036

## Claims

1. Pseudotyped foamy virus particle comprising a heterologous envelope protein not derived from foamy virus or a membrane targeting domain not derived from foamy virus, comprising
- a first fusion protein comprising a foamy virus Gag protein, and a first dimerization domain that is applicable to dimerize with a second dimerization domain from another protein upon binding of a small molecule ligand to both the first and the second dimerization domain; and
- a second fusion protein, wherein the second fusion protein comprises
- the envelope protein not derived from foamy virus and the second dimerization domain that is applicable to dimerize with the first dimerization domain from the first fusion protein upon binding of a small molecule ligand to both the first and the second dimerization domain; or
- the membrane targeting domain not derived from foamy virus, and the second dimerization domain that is applicable to dimerize with the first dimerization domain from the first fusion protein upon binding of a small molecule ligand to both the first and the second dimerization domain.

2. Use of a first fusion protein and a second fusion protein for the generation of a pseudotyped foamy virus particle according to claim 1, wherein the first fusion protein comprises a foamy virus Gag protein and a first dimerization domain and the second fusion protein comprises a second dimerization domain and either a heterologous envelope protein not derived from foamy virus or a membrane targeting domain not derived from foamy virus, wherein the first dimerization domain is applicable to dimerize with the second dimerization domain upon binding of a small molecule ligand to both the first and the second dimerization domain.

3. Pseudotyped foamy virus particle according to claim 1 or use according to claim 2, wherein the foamy virus Gag protein is selected from human foamy virus Gag protein, simian foamy virus Gag proteins, feline foamy virus Gag protein, bovine foamy virus Gag protein and equine foamy virus Gag protein.

4. Pseudotyped foamy virus particle according to claim 1 or 3 or use according to claim 2 or 3, wherein the heterologous envelope protein is selected from glycoprotein G of vesicular stomatitis virus, glycoprotein G of rabies virus, glycoprotein G of Mokola virus, glycoproteins of murine leukemia virus with ecotropic, amphotropic, polytropic or xenotropic host range, glycoprotein of human immunodeficiency viruses type 1 and type 2, glycoprotein of simian immunodeficiency virus, glycoprotein of human T-cell leukemia viruses type 1, type 2 and type 4, glycoprotein of endogenous feline virus RD114, glycoprotein of gibbon ape leukemia virus, glycoproteins H and F of measles virus, glycoproteins H and F of Nipah virus, hemagglutinin and neuraminidase of influenza A or B, large-, middle- or small S-antigens of hepatitis B virus, E1 and E2 proteins of hepatitis C virus and glycoprotein from lymphocytic choriomeningitis virus, glycoprotein of Lassa virus, glycoproteins HN and F of Sendai virus, glycoprotein of Ebola virus and glycoprotein of Marburg virus.

5. Pseudotyped foamy virus particle according to claim 1, 3 or 4 or use of a fusion protein according to claim 2 or 3, wherein the membrane targeting domain is selected from
- the Gag matrix subunits of human immunodeficiency viruses type 1 or type 2, human T-lymphotropic virus type 1, type 2 or type 4, simian immunodeficiency virus, murine leukemia virus, Mason-Pfizer monkey virus, mouse mammary tumor virus, avian leukosis virus, Jaagsiekte sheep retrovirus or Gibbon ape leukemia virus and
- the myristoylation signal sequence of proto-oncogene tyrosine-protein kinase Src of Avian sarcoma virus and
- the myristoylation signal sequence of proto-oncogene tyrosine-protein kinase Src, proto-oncogene tyrosine-protein kinase Fyn or protein-tyrosine kinase p56lck.

6. Use according to one of claims 2 to 5, wherein the dimerization domain of the fusion protein binds to rapamycin and preferably exhibits at least 80% amino acid sequence identity to SEQ ID No. 1 or SEQ ID No. 3.

7. Use of a polynucleotide encoding for a fusion protein as defined in one of the claims 2 to 6 or a Vector comprising a polynucleotide encoding for a fusion protein as defined in one of claims 2 to 6 for the expression of a pseudotyped foamy virus particle according to claim 1, 3, 4 or 5.

8. Use of a complementation cell line comprising a vector as defined in claim 7 for the generation of a pseudotyped foamy virus particle according to claim 1, 3, 4 or 5.

9. Method for the preparation of a pseudotyped foamy virus particle according to claim 1, comprising the steps of:
(i) culturing of a complementation cell line comprising an expression vector encoding for the first fusion protein as defined in one of claims 2 or 3, and
- an expression vector encoding for the second fusion protein comprising a heterologous envelope protein as defined in claim 2 or 4 or
- an expression vector encoding for the second fusion protein comprising a membrane targeting domain as defined in claim 2 or 5 and an expression vector encoding for a heterologous envelope protein
under conditions suitable to permit production of said pseudotyped foamy virus particle,
(ii) addition of an effective amount of a small molecule that is applicable to lead to dimerization between the first and the second fusion protein,
(iii) recovering said pseudotyped foamy virus particle from the cell culture.

10. Vertebrate cell infected with a pseudotyped foamy virus particle of claim 1 or with a pseudotyped foamy virus particle obtainable by a method of claim 9.

11. Pharmaceutical composition comprising an effective amount of a pseudotyped foamy virus particle of claim 1 or a pseudotyped foamy virus particle obtainable by a method of claim 9 in combination with a pharmaceutically acceptable carrier.

12. Use of a pseudotyped foamy virus particle of claim 1 or a pseudotyped foamy virus particle obtainable by a method of claim 9 for preparing a medicinal product.

## Patentansprüche

1. Pseudotypisiertes Foamyvirus-Partikel, umfassend ein heterologes Hüllprotein, das nicht von Foamyvirus stammt, oder eine Membran-Targeting-Domäne, die nicht von Foamyvirus stammt, enthaltend
- ein erstes Fusionsprotein, umfassend ein Foamyvirus-Gag-Protein und eine erste Dimerisierungsdomäne, die geeignet ist, mit einer zweiten Dimerisierungsdomäne von einem anderen Protein bei Bindung eines kleinen Molekülliganden sowohl an die erste als auch an die zweite Dimerisierungsdomäne zu dimerisieren; und
- ein zweites Fusionsprotein, wobei das zweite Fusionsprotein Folgendes umfasst:
- ein Hüllprotein, das nicht von Foamyvirus stammt, und die zweite Dimerisierungsdomäne, die geeignet ist, mit der ersten Dimerisierungsdomäne von dem ersten Fusionsprotein bei Bindung eines kleinen Molekülliganden sowohl an die erste als auch an die zweite Dimerisierungsdomäne zu dimerisieren; oder
- eine Membran-Targeting-Domäne, die nicht von Foamyvirus stammt, und die zweite Dimerisierungsdomäne, die geeignet ist, mit der ersten Dimerisierungsdomäne von dem ersten Fusionsprotein bei Bindung eines kleinen Molekülliganden sowohl an die erste als auch an die zweite Dimerisierungsdomäne zu dimerisieren.

2. Verwendung eines ersten Fusionsproteins und eines zweiten Fusionsproteins für die Herstellung eines pseudotypisierten Foamyvirus-Partikels nach Anspruch 1, wobei das erste Fusionsprotein ein Foamyvirus-Gag-Protein und eine erste Dimerisierungsdomäne umfasst und das zweite Fusionsprotein eine zweite Dimerisierungsdomäne und entweder ein heterologes Hüllprotein, das nicht von Foamyvirus stammt, oder eine Membran-Targeting-Domäne, die nicht von Foamyvirus stammt, umfasst, wobei die erste Dimerisierungsdomäne geeignet ist, mit der zweiten Dimerisierungsdomäne bei Bindung eines kleinen Molekülliganden sowohl an die erste als auch an die zweite Dimerisierungsdomäne zu dimerisieren.

3. Pseudotypisiertes Foamyvirus-Partikel nach Anspruch 1 oder Verwendung nach Anspruch 2, wobei das Foamyvirus-Gag-Protein aus humanem Foamyvirus-Gag-Protein, Simian-Foamyvirus-Gag-Proteinen, felinem Foamyvirus-Gag-Protein, bovinem Foamyvirus-Gag-Protein und equinem Foamyvirus-Gag-Protein ausgewählt ist.

4. Pseudotypisiertes Foamyvirus-Partikel nach Anspruch 1 oder 3 oder Verwendung nach Anspruch 2 oder 3, wobei das heterologe Hüllprotein ausgewählt ist aus Glykoprotein G des Vesicular-Stomatitis-Virus, Glykoprotein G des Tollwutvirus, Glykoprotein G des Mokolavirus, Glykoproteinen des Mausleukämievirus mit ekotropem, amphotropem, polytropem oder xenotropem Wirtsbereich, einem Glykoprotein der Humanen Immundefizienzviren Typ 1 und Typ 2, einem Glykoprotein des Simian-Immundefizienzvirus, einem Glykoprotein der humanen T-Zellleukämieviren Typ 1, Typ 2 und Typ 4, einem Glykoprotein des Endogenen Felinen Virus RD 114, einem Glykoprotein des Gibbonaffen-Leukämievirus, den Glykoproteinen H und F des Masernvirus, den Glykoproteinen H und F des Nipah-Virus, Hämagglutinin und Neuraminidase von Influenza A oder B, den großen, mittleren oder kleinen S-Antigenen des Hepatitis-B-Virus, den E1- und E2-Proteinen des Hepatitis-C-Virus und einem Glykoprotein des Lymphozytischen Choriomeningitisvirus, einem Glykoprotein des Lassavirus, den Glykoproteinen HN und F des Sendaivirus, einem Glykoprotein des Ebolavirus und einem Glykoprotein des Marburgvirus.

5. Pseudotypisiertes Foamyvirus-Partikel nach Anspruch 1, 3 oder 4 oder Verwendung eines Fusionsproteins nach Anspruch 2 oder 3, wobei die Membran-Targeting-Domäne ausgewählt ist aus
- den Gag-Matrix-Untereinheiten der humanen Immundefizienzviren Typ 1 oder Typ 2, des humanen T-Lymphotropen Virus Typ 1, Typ 2 oder Typ 4, des Simian-Immundefizienzvirus, des Mausleukämievirus, des Mason-Pfizer-Affenvirus, des Maus-Mammatumorvirus, das Vogelleukosevirus, des Jaagsiekte-Schaf-Retrovirus oder des Gibbonaffen-Leukämievirus und
- der Myristoylierungsssignalsequenz der Protoonkogen-Tyrosinproteinkinase Src des Vogelsarkomavirus und
- der Myristoylierungsssignalsequenz der Protoonkogen-Tyrosinproteinkinase Src, der Protoonkogen-Tyrosinproteinkinase Fyn oder der Proteintyrosinekinase p561ck.

6. Verwendung nach einem der Ansprüche 2 bis 5, wobei die Dimerisierungsdomäne des Fusionsproteins an Rapamycin bindet und vorzugsweise eine Aminosäuresequenzidentität von mindestens 80 % mit SEQ ID Nr. 1 oder SEQ ID Nr. 3 aufweist.

7. Verwendung eines Polynukleotids, das für ein Fusionsprotein nach der Definition in einem der Ansprüche 2 bis 6 kodiert, oder eines Vektors, umfassend ein Polynukleotid, das für ein Fusionsprotein nach der Definition in einem der Ansprüche 2 bis 6 kodiert, für die Expression eines pseudotypisierten Foamyvirus-Partikels nach Anspruch 1, 3, 4 oder 5.

8. Verwendung einer Komplementarisierungszelllinie, enthaltend einen Vektor nach der Definition in Anspruch 7 für die Erzeugung eines pseudotypisierten Foamyvirus-Partikels nach Anspruch 1, 3, 4 oder 5.

9. Verfahren für die Herstellung eines pseudotypisierten Foamyvirus-Partikels nach Anspruch 1, umfassend die folgenden Schritte:
(i) Kultivieren einer Komplementarisierungszelllinie, enthaltend einen Expressionsvektor, welcher für das erste Fusionsprotein nach der Definition in einem der Ansprüche 2 oder 3 kodiert, und
- einen Expressionsvektor, der für das zweite Fusionsprotein, umfassend ein heterologes Hüllprotein nach der Definition in Anspruch 2 oder 4 kodiert, oder
- einen Expressionsvektor, der für das zweite Fusionsprotein, umfassend eine Membran-Targeting-Domäne nach der Definition in Anspruch 2 oder 5 kodiert, und einen Expressionsvektor, der für ein heterologes Hüllprotein kodiert,
unter Bedingungen, die geeignet sind, um eine Herstellung des pseudotypisierten Foamyvirus-Partikels zu erlauben,
(ii) Zugabe einer wirksamen Menge eines kleinen Moleküls, das geeignet ist, zu einer Dimerisierung zwischen dem ersten und dem zweiten Fusionsprotein zu führen,
(iii) Wiedergewinnung des pseudotypisierten Foamyvirus-Partikels aus der Zellkultur.

10. Wirbeltierzelle, infiziert mit einem pseudotypisierten Foamyvirus-Partikel nach Anspruch 1 oder mit einem pseudotypisierten Foamyvirus-Partikel, das mit einem Verfahren nach Anspruch 9 erhalten werden kann.

11. Pharmazeutische Zusammensetzung, umfassend eine wirksame Menge eines pseudotypisierten Foamyvirus-Partikels nach Anspruch 1 oder eines pseudotypisierten Foamyvirus-Partikels, das mit einem Verfahren nach Anspruch 9 erhalten werden kann, in Kombination mit einem pharmazeutisch akzeptablen Träger.

12. Verwendung eines pseudotypisierten Foamyvirus-Partikels nach Anspruch 1 oder eines pseudotypisierten Foamyvirus-Partikels, das mit einem Verfahren nach Anspruch 9 erhalten werden kann, zur Zubereitung eines medizinischen Produktes.

## Revendications

1. Particule de virus spumeux pseudotypé comprenant une protéine d'enveloppe hétérologue qui n'est pas dérivée d'un virus spumeux ou un domaine de ciblage de membrane qui n'est pas dérivé d'un virus spumeux, comprenant :
- une première protéine de fusion comprenant une protéine Gag de virus spumeux et un premier domaine de dimérisation qui est capable de se dimériser avec un second domaine de dimérisation provenant d'une autre protéine lors de la liaison d'un ligand de petite molécule à la fois au premier et au second domaine de dimérisation ; et
- une seconde protéine de fusion, la seconde protéine de fusion comprenant
- la protéine d'enveloppe qui n'est pas dérivée d'un virus spumeux et le second domaine de dimérisation qui est capable de se dimériser avec le premier domaine de dimérisation provenant de la première protéine de fusion lors de la liaison d'un ligand de petite molécule à la fois au premier et au second domaine de dimérisation ; ou
- le domaine de ciblage de membrane qui n'est pas dérivé d'un virus spumeux, et le second domaine de dimérisation qui est capable de se dimériser avec le premier domaine de dimérisation provenant de la première protéine de fusion lors de la liaison d'un ligand de petite molécule à la fois au premier et au second domaine de dimérisation.

2. Utilisation d'une première protéine de fusion et d'une seconde protéine de fusion pour produire une particule de virus spumeux pseudotypé selon la revendication 1, dans laquelle la première protéine de fusion comprend une protéine Gag de virus spumeux et un premier domaine de dimérisation et la seconde protéine de fusion comprend un second domaine de dimérisation et une protéine d'enveloppe hétérologue qui n'est pas dérivée d'un virus spumeux ou un domaine de ciblage de membrane qui n'est pas dérivé d'un virus spumeux, le premier domaine de dimérisation étant applicable pour se dimériser avec le second domaine de dimérisation lors de la liaison d'un ligand de petite molécule à la fois au premier et au second domaine de dimérisation.

3. Particule de virus spumeux pseudotypé selon la revendication 1 ou utilisation selon la revendication 2, dans laquelle la protéine Gag de virus spumeux est sélectionnée parmi la protéine Gag de virus spumeux humain, les protéines Gag de virus spumeux simien, la protéine Gag de virus spumeux félin, la protéine Gag de virus spumeux bovin et la protéine Gag de virus spumeux équin.

4. Particule de virus spumeux pseudotypé selon la revendication 1 ou 3 ou utilisation selon la revendication 2 ou 3, dans laquelle la protéine d'enveloppe hétérologue est sélectionnée parmi la glycoprotéine G du virus de la stomatite vésiculaire, la glycoprotéine G du virus de la rage, la glycoprotéine G du virus Mokola, les glycoprotéines du virus de la leucémie murine avec une plage d'hôtes écotropiques, amphotropiques, polytropiques ou xénotropiques, la glycoprotéine des virus de l'immunodéficience humaine de type 1 et de type 2, la glycoprotéine du virus de l'immunodéficience simienne, la glycoprotéine des virus de la leucémie à lymphocytes T humaine de type 1, de type 2 et de type 4, la glycoprotéine du virus endogène félin RD114, la glycoprotéine du virus de la leucémie du singe Gibbon, les glycoprotéines H et F du virus de la rougeole, les glycoprotéines H et F du virus Nipah, l'hémagglutinine et la neuraminidase de la grippe A ou B, les antigènes S gros, moyens ou petits du virus de l'hépatite B, les protéines E1 et E2 du virus de l'hépatite C et la glycoprotéine provenant du virus de la chorioméningite lymphocytaire, la glycoprotéine du virus Lassa, les glycoprotéines HN et F du virus Sendai, la glycoprotéine du virus Ebola et la glycoprotéine du virus Marburg.

5. Particule de virus spumeux pseudotypé selon la revendication 1, 3 ou 4 ou utilisation d'une protéine de fusion selon la revendication 2 ou 3, dans laquelle le domaine de ciblage de membrane est choisi parmi :
- les sous-unités de la matrice de Gag des virus de l'immunodéficience humaine de type 1 ou de type 2, du virus T-lymphotropique humain de type 1, de type 2 ou de type 4, du virus de l'immunodéficience simienne, du virus de la leucémie murine, du virus du singe Mason-Pfizer, du virus de la tumeur mammaire de souris, du virus de la leucose aviaire, du rétrovirus de mouton Jaagsiekte ou du virus de la leucémie du singe Gibbon, et
- la séquence signal de la myristoylation de la tyrosine-protéine kinase Src proto-oncogène du virus du sarcome aviaire, et
- la séquence signal de la myristoylation de la tyrosine-protéine kinase Src proto-oncogène, de la tyrosine-protéine kinase Fyn proto-oncogène ou de la tyrosine-protéine kinase p561ck.

6. Utilisation selon l'une quelconque des revendications 2 à 5, dans laquelle le domaine de dimérisation de la protéine de fusion se lie à la rapamycine et de préférence présente une identité de séquence d'acides aminés d'au moins 80 % avec la SEQ ID No. 1 ou la SEQ ID No. 3.

7. Utilisation d'un polynucléotide codant pour une protéine de fusion telle que définie selon l'une quelconque des revendications 2 à 6 ou d'un vecteur comprenant un polynucléotide codant pour une protéine de fusion telle que définie selon l'une quelconque des revendications 2 à 6 pour l'expression d'une particule de virus spumeux pseudotypé selon la revendication 1, 3, 4 ou 5.

8. Utilisation d'une lignée de cellules de complémentation comprenant un vecteur tel que défini selon la revendication 7 pour produire une particule de virus spumeux pseudotypé selon la revendication 1, 3, 4 ou 5.

9. Procédé de préparation d'une particule de virus spumeux pseudotypé selon la revendication 1, comprenant les étapes consistant à :
(i) cultiver une lignée de cellules de complémentation comprenant
- un vecteur d'expression codant pour la première protéine de fusion telle que définie selon la revendication 2 ou 3, et
- un vecteur d'expression codant pour la seconde protéine de fusion comprenant une protéine d'enveloppe hétérologue telle que définie selon la revendication 2 ou 4, ou
- un vecteur d'expression codant pour la seconde protéine de fusion comprenant un domaine de ciblage de membrane tel que défini selon la revendication 2 ou 5 et un vecteur d'expression codant pour une protéine d'enveloppe hétérologue
dans des conditions appropriées qui permettent la production de ladite particule de virus spumeux pseudotypé,
(ii) ajouter une quantité efficace d'une petite molécule qui est capable de entraîner une dimérisation entre la première et la seconde protéine de fusion,
(iii) récupérer ladite particule de virus spumeux pseudotypé de la culture de cellules.

10. Cellule de vertébré infectée avec une particule de virus spumeux pseudotypé selon la revendication 1 ou avec une particule de virus spumeux pseudotypé pouvant être obtenue par un procédé selon la revendication 9.

11. Composition pharmaceutique comprenant une quantité efficace d'une particule de virus spumeux pseudotypé selon la revendication 1 ou d'une particule de virus spumeux pseudotypé pouvant être obtenue par un procédé selon la revendication 9 en combinaison avec un support pharmaceutiquement acceptable.

12. Utilisation d'une particule de virus spumeux pseudotypé selon la revendication 1 ou d'une particule de virus spumeux pseudotypé pouvant être obtenue par un procédé selon la revendication 9 pour la préparation d'un médicament.
